# EUROPEAN PATENT APPLICATION

(11) **EP 4 841 646 A1**
(43) Date of publication of application: **18.11.2026**
(21) Application number: 26178003.5
(22) Date of filing: 11.05.2026
(51) Int. Cl.: G16H 50/20, G16H 50/70, G16H 30/20, G16H 30/40

(54) **IDENTIFYING SIMILAR PAIRS OF MEDICAL IMAGE DATA BASED ON SPATIAL BIOMARKER DATA GENERATED VIA ARTIFICIAL INTELLIGENCE**

(30) Priority: 13.05.2025 US 202563805018 P; 07.05.2026 US 202619670105
(71) Applicant: Altis Labs, Inc., Toronto, ON M5G 1L7 (CA)
(72) Inventor: Baldauf-Lenschen, Felix, Toronto, M5G 1L7 (CA)
(74) Representative: Berggren Oy

(57) **Abstract**

A medical data processing system operates by: generating a first plurality of spatial imaging biomarker data for a first plurality of medical image data based on utilizing artificial intelligence to perform a spatial imaging biomarker generator function upon each of the first plurality of medical image data to generate corresponding first candidate spatial imaging biomarker data of the first plurality of spatial imaging biomarker data; generating a second plurality of spatial imaging biomarker data for a second plurality of medical image data based on utilizing artificial intelligence to perform the spatial imaging biomarker generator function upon each of the second plurality of medical image data to generate corresponding second spatial imaging biomarker data of the second plurality of spatial imaging biomarker data; generating, for each of the first plurality of medical image data, a corresponding plurality of similarity scores corresponding to the second plurality of medical image data based on generating each similarity score of the corresponding plurality of similarity scores via performance of a similarity function upon: the corresponding first spatial imaging biomarker data generated for the each of the first plurality of medical image data, and the corresponding second spatial imaging biomarker data generated for a corresponding one of the second plurality of medical image data; and identifying a proper subset of the second plurality of medical image data based on identifying, for the each of the first plurality of medical image data, one of the second plurality of medical image data having a most favorable similarity score of the corresponding plurality of similarity scores.

## Description

### CROSS-REFERENCE TO RELATED APPLICATIONS

The present U.S. Utility Patent Application claims priority pursuant to 35 U.S.C. § 119(e) to U.S. Provisional Application No. 63/805,018, entitled "IDENTIFYING SIMILAR PAIRS OF MEDICAL IMAGE DATA BASED ON SPATIAL BIOMARKER DATA GENERATED VIA ARTIFICIAL INTELLIGENCE", filed 5/13/2026, which is hereby incorporated herein by reference in its entirety and made part of the present U.S. Utility Patent Application for all purposes.

### STATEMENT REGARDING FEDERALLY SPONSORED RESEARCH OR DEVELOPMENT

Not Applicable.

### INCORPORATION-BY-REFERENCE OF MATERIAL SUBMITTED ON A COMPACT DISC

Not Applicable.

### BACKGROUND OF THE INVENTION

### TECHNICAL FIELD OF THE INVENTION

This invention relates generally to computer systems, computer networking, medial data processing, artificial intelligence, and machine learning.

### BRIEF DESCRIPTION OF THE SEVERAL VIEWS OF THE DRAWING(S)

The patent or application file contains at least one drawing executed in color. Copies of this patent or patent application publication with color drawing(s) will be provided by the Office upon request and payment of the necessary fee.
Figure 1A is a schematic block diagram illustrating communication between a medical data processing system, one or more data source entities, and/or one or more one or more requesting entities in accordance with various embodiments;
Figure 1B is a schematic block diagram illustrating a system that includes a medical data processing system in accordance with various embodiments;
Figure 1C is a schematic block diagram illustrating communication between a scientific data processing system, one or more data source entities, and/or one or more one or more requesting entities in accordance with various embodiments;
Figure 2A is a schematic block diagram of a medical data processing system that includes a memory module, processing module, and/or network interface in accordance with various embodiments;
Figure 2B is a schematic block diagram of a medical data processing system that includes a data storage system, a function library, and/or a set of subsystems in accordance with various embodiments;
Figure 2C is a schematic block diagram of a medical data processing system that implements a medical outcome prognostication model training system, a medical outcome prognostication system, and/or a prognosis-based trial arm assignment system in accordance with various embodiments;
Figure 2D is a schematic block diagram of a subsystem in accordance with various embodiments;
Figure 2E is a schematic block diagram of a computing device in accordance with various embodiments;
Figure 2F is a schematic block diagram of a scientific data processing system that includes a memory module, processing module, and/or network interface in accordance with various embodiments;
Figure 2G is a schematic block diagram of a scientific data processing system that includes a data storage system, a function library, and/or a set of subsystems in accordance with various embodiments;
Figure 2H is a schematic block diagram of a scientific data processing system that implements an outcome prediction model training system, an outcome prediction system, and/or a prediction-based trial arm assignment system in accordance with various embodiments;
Figure 2I is a schematic block diagram of a data source that implements a medical data processing system in accordance with various embodiments in accordance with various embodiments;
Figure 2J is a schematic block diagram of a requesting entity that implements a medical data processing system in accordance with various embodiments in accordance with various embodiments;
Figure 2K is a schematic block diagram of at least one medical product manufacturing and/or testing entity that communicates with a medical data processing system in accordance with various embodiments;
Figure 2L is a schematic block diagram of at least one medical product manufacturing and/or testing entity that implements a medical data processing system in accordance with various embodiments;
Figure 2M is a schematic block diagram of at least one scientific study conducting entity that communicates with a scientific data processing system in accordance with various embodiments;
Figure 2N is a schematic block diagram of at least one scientific study conducting entity that implements a scientific data processing system in accordance with various embodiments;
Figure 2O is a schematic block diagram of at least one trial participant assignment entity that communicates with a medical data processing system and/or a scientific data processing system in accordance with various embodiments;
Figure 2P is a schematic block diagram of at least one trial participant assignment entity that that implements a medical data processing system and/or a scientific data processing system in accordance with various embodiments;
Figure 2Q is a schematic block diagram of at least one Artificial Intelligence (AI) Platform that communicates with a medical data processing system and/or a scientific data processing system in accordance with various embodiments;
Figure 2R is a schematic block diagram of at least one AI platform that implements a medical data processing system and/or a scientific data processing system in accordance with various embodiments;
Figure 3A illustrates an embodiment of medical data and corresponding medical data metadata in accordance with various embodiments;
Figure 3B illustrates an embodiment of patient data in accordance with various embodiments;
Figure 3C illustrates an embodiment of clinical trial data in accordance with various embodiments;
Figure 3D illustrates an embodiment of a function entry in accordance with various embodiments;
Figure 3E illustrates an embodiment of a function library in accordance with various embodiments;
Figure 3F is a schematic block diagram of a medical data processing system that implements a data collection module in accordance with various embodiments;
Figure 3G is a schematic block diagram of a medical data processing system that implements a data dissemination module in accordance with various embodiments;
Figure 3H is a schematic block diagram of a medical data processing system that implements a medical modeling platform 505 in accordance with various embodiments;
Figure 4A is a schematic block diagram of a medical data processing system that generates trial arm assignment data in accordance with various embodiments;
Figure 4B is a schematic block diagram of a medical data processing system that communicates with a medical product manufacturing and/or testing processing system in accordance with various embodiments;
Figure 4C illustrates an embodiment of a process of producing a medical product in accordance with various embodiments;
Figure 4D is a schematic block diagram of a medical outcome prognostication model training system in accordance with various embodiments;
Figure 4E is a schematic block diagram of a medical outcome prognostication system in accordance with various embodiments;
Figures 4F - 4H illustrate embodiments of an input feature set in accordance with various embodiments;
Figure 4I is a schematic block diagram of a prognosis-based trial arm assignment system in accordance with various embodiments;
Figure 4J is a schematic block diagram of a medical data processing system communicating with at least one trial participant assignment entity in accordance with various embodiments;
Figure 5A is a schematic block diagram of a scientific data processing system that generates trial arm assignment data in accordance with various embodiments;
Figure 5B is a schematic block diagram of a scientific data processing system that communicates with a scientific study conducting processing system in accordance with various embodiments;
Figure 5C is a schematic block diagram of an outcome prediction model training system in accordance with various embodiments;
Figure 5D is a schematic block diagram of an outcome prediction system in accordance with various embodiments;
Figures 5E - 5G illustrate embodiments of an input feature set in accordance with various embodiments;
Figure 5H is a schematic block diagram of a prediction-based trial arm assignment system in accordance with various embodiments;
Figure 5I is a schematic block diagram of a scientific data processing system communicating with at least one trial participant assignment entity in accordance with various embodiments;
Figure 6A is a schematic block diagram of a trial arm assignment module that assigns participant sets to a plurality of trial arms based on medical outcome prognosis scores in accordance with various embodiments;
Figure 6B is a schematic block diagram of a trial arm assignment module that assigns participant sets to a plurality of trial arms based on outcome prediction scores in accordance with various embodiments;
Figure 6C is a schematic block diagram of a trial arm assignment module that assigns participant sets to a plurality of trial arms based on target participant set parameters;
Figure 6D is a schematic block diagram of a trial arm assignment module that generates a first participant set for a control arm and a second participant set for an experimental arm in accordance with various embodiments;
Figure 6E is a schematic block diagram of a trial arm assignment module that generates participant sets for one or more control arms and/or that generates participant sets for one or more experimental arms in accordance with various embodiments;
Figure 6F is a schematic block diagram of a trial arm assignment module that implements a stratification process and a plurality of per-group randomization processes in accordance with various embodiments;
Figure 6G is a schematic block diagram of a trial arm assignment module that implements a stratification process that applies a stratification function based on a plurality of score ranges in accordance with various embodiments;
Figure 6H is a schematic block diagram of a trial arm assignment module that implements a stratification process that applies a stratification function based on a plurality of categorical scores in accordance with various embodiments;
Figure 6I is a schematic block diagram of a trial arm assignment module that implements a per-group randomization process that utilizes a random trial assignment ordering in accordance with various embodiments;
Figure 6J is a schematic block diagram of a trial arm assignment module that implements a stratification process that implements outcome prediction-based stratification and additional factor-based stratification in accordance with various embodiments;
Figure 6K is a is a schematic block diagram of a trial arm assignment module that collectively processes scores that assigns participant sets to a plurality of trial arms based on target participant set parameters;
Figure 7A is a schematic block diagram of a medical outcome prognostication system 507 that generates a medical outcome prognosis score from medical data in accordance with various embodiments;
Figure 7B is a schematic block diagram of an outcome prediction system 507 that implements an image-based modeling prediction pipeline in accordance with various embodiments;
Figure 8A is a schematic block diagram of a medical outcome prognostication system 507 that generates a medical outcome prognosis score from a medical image in accordance with various embodiments;
Figures 8B - 8G are block diagrams illustrating embodiments of processes functioning in accordance with various embodiments;
Figure 8H is a graphical comparison of mortality risk prediction accuracy at 1 year, 2 years, and 5 years for a particular example of an image-based modeling prediction pipeline in accordance with various embodiments;
Figure 8I illustrates Kaplan-Meier curves and corresponding data for 5-year IPRO mortality risk deciles (includes all TNM stages) for a particular example of an image-based modeling prediction pipeline in accordance with various embodiments;
Figure 8J illustrates stage-specific Kaplan-Meier curves for 5-year IPRO mortality risk quintiles for a particular example of an image-based modeling prediction pipeline in accordance with various embodiments;
Figure 8K illustrates activation or attention maps for patients who received high IPRO mortality risk scores in stage I (top) and stage II (middle and bottom) for a particular example of an image-based modeling prediction pipeline in accordance with various embodiments;
Figure 8L illustrates exclusion criteria for experimental datasets for a particular example of an image-based modeling prediction pipeline in accordance with various embodiments;
Figure 8M illustrates Kaplan-Meier curves and corresponding data for 1-year IPRO mortality risk deciles (includes all TNM stages) for a particular example of an image-based modeling prediction pipeline in accordance with various embodiments;
Figure 8N illustrates Kaplan-Meier curves and corresponding data for 2-year IPRO mortality risk deciles (includes all TNM stages) for a particular example of an image-based modeling prediction pipeline in accordance with various embodiments;
Figure 8O illustrates stage-specific Kaplan-Meier curves for 1-year IPRO mortality risk quintiles for a particular example of an image-based modeling prediction pipeline in accordance with various embodiments;
Figure 8P illustrates stage-specific Kaplan-Meier curves for 2-year IPRO mortality risk quintiles for a particular example of an image-based modeling prediction pipeline;
Figures 9A - 9I illustrate example graphical user interfaces that can be generated by the modeling platform in accordance with various embodiments;
Figures 9J - 9L illustrate example case studies comparing patients and their risk predictions generated in accordance with various embodiments;
Figure 9M illustrates an example activation or attention map for different patients generated in accordance with various embodiments;
Figures 9N - 9R illustrate example graphical user interfaces that can be generated by the modeling platform in accordance with various embodiments;
Figures 10A - 10G are flow diagrams illustrating methods for execution in accordance with various embodiments;
Figure 11A is a schematic block diagram illustrating an adverse reaction model training system, an adverse reaction prediction system, and a treatment safety assessment system in accordance with various embodiments;
Figure 11B is a schematic block diagram of an adverse reaction prediction system that processes medical image data to generate adverse reaction prediction data in accordance with various embodiments;
Figure 11C is a schematic block diagram of a medical data processing system that implements an adverse reaction model training system, an adverse reaction prediction system, and/or a treatment safety assessment system in accordance with various embodiments;
Figure 11D is a schematic block diagram of an adverse reaction model training system implemented via a medical outcome prognostication model training system and an adverse reaction prediction system implemented via a medical outcome prognostication system in accordance with various embodiments;
Figure 11E is a schematic block diagram illustrating an embodiment of a treatment safety assessment system operable to communicate treatment safety data to a requesting entity in accordance with various embodiments;
Figure 11F illustrates an embodiment of medical data metadata in accordance with various embodiments;
Figure 11G illustrates an embodiment of patient data in accordance with various embodiments;
Figure 11H illustrates an embodiment of a function library in accordance with various embodiments;
Figure 12A is a schematic block diagram illustrating a medical condition detection model training system, a medical condition detection system, and a treatment candidacy identification system in accordance with various embodiments;
Figure 12B is a schematic block diagram of a medical condition detection system that processes medical image data to generate model-detected findings data in accordance with various embodiments;
Figure 12C is a schematic block diagram of a medical data processing system that implements a medical condition detection model training system, a medical condition detection system, and/or a treatment candidacy identification system in accordance with various embodiments;
Figure 13A is a schematic block diagram illustrating an adverse reaction prediction system, a treatment safety assessment system, and an adverse reaction-based trial participant exclusion system in accordance with various embodiments;
Figure 13B is a schematic block diagram of a medical data processing system that implements an adverse reaction-based trial participant exclusion system in accordance with various embodiments;
Figure 13C is a schematic block diagram illustrating an adverse reaction prediction system, a treatment safety assessment system, and an adverse reaction-based trial participant exclusion system in accordance with various embodiments;
Figure 13D illustrates an embodiment of a process of producing a medical product in accordance with various embodiments;
Figures 14A - 14E are flow diagrams illustrating methods for execution in accordance with various embodiments; and
Figure 15A is a flow diagram illustrating a method for execution in accordance with various embodiments.

### DESCRIPTION OF THE INVENTION

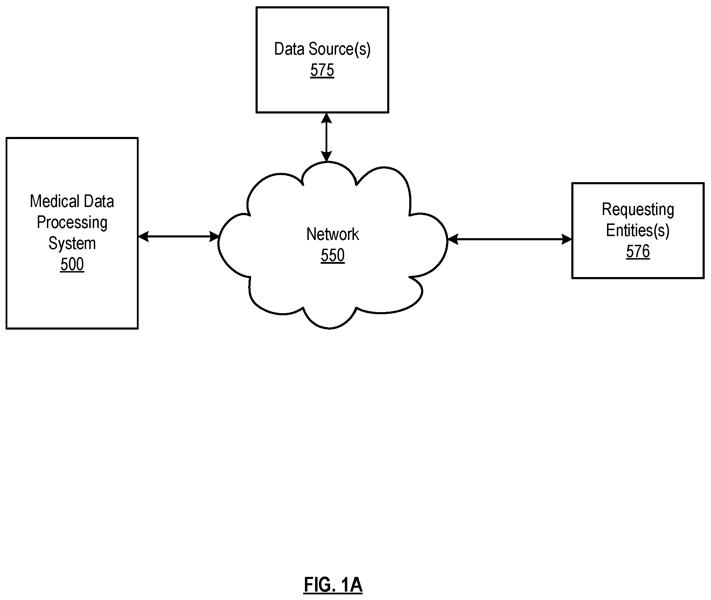

Figure 1A is a schematic block diagram of an embodiment of a medical data processing system 500 that communicates with one or more data source entities 575 and/or with one or more requesting entities 576 via a network 550. The network 550 can be implemented via: one or more wireless and/or wired communication systems; one or more non-public intranet systems and/or public internet systems; one or more satellite communication systems; one or more cellular communication systems; one or more fiber optic communication systems; one or more local area networks (LAN); one or more wide area networks (WAN); the Internet; and/or one or more other communication networks.

One or more data sources 575 can be implemented via medical devices, computing devices, servers, equipment, graphical user interfaces, user input devices, and/or other data sources operable to collect, generate, measure, pre-process, store, and/or send data to the medical data processing system 500 for use in the medical data processing system 500 performing some or all functionality described herein. One or more requesting entities 576 can be implemented via computing devices, servers, equipment, graphical user interfaces, display devices, and/or other data sources operable to operable to receive, store, display, communicate, convey, and/or further process data that was generated and/or sent by the medical data processing system 500 (e.g. for use by corresponding person and/or other entity that requested this data be generated and/or that uses the resulting data for a corresponding purpose, such as administering healthcare or conducting a clinical trial or other scientific experiment).

For example, the medical data processing system 500 receives some or all medical data described herein (e.g. medical images, other device-captured medical data, and/or human-generated medical data) from one or more different data sources 575. For example, at least one function (e.g. an inference function characterized by a corresponding model trained via artificial intelligence and/or machine learning techniques) is trained by medical data processing system 500 based on using a set of medical data received from one or more data sources 575 as training data. Alternatively or in addition, at least one function (e.g. an inference function characterized by a corresponding model trained via artificial intelligence and/or machine learning techniques) is executed upon medical data received from one or more data sources 575 to generate corresponding inference data (e.g. predictions corresponding to prognosis, diagnosis, detection of abnormalities, automatically generated measurements, automatically detected test results, etc.), for example, which can be sent or otherwise communicated to one or more different requesting entities 576 for use.

In some embodiments, medical devices, computing devices, servers, and/or other computing resources/data sources/requesting entities implementing the data sources 575 and/or requesting entities 576 are used by, owned by, located within, associated with, and/or correspond to: at least one hospital, medical clinic, and/or at least one other healthcare treatment facility; at least one pharmaceutical company, healthcare company, university, research organization, company and/or other entity that conducts clinical trials and/or scientific experiments and/or that designs, produces, administers and/or sells foods/drugs/other products and/or treatments/other processes that have been, will be, and/or are currently undergoing analysis via a corresponding clinical trial and/or scientific experiment; at least one physician, radiologist, medical technician, clinical trial administrator and/or worker; and/or other person associated with providing healthcare and/or conducting a clinical trial and/or other scientific experiment; at least one patient or individual (and/or other person/entity associated with the individual such as a parent and/or guardian of a patient that is a minor, and owner of the individual if the individual is a pet/animal/other organism/object, etc.), where this individual has been, will be, and/or is currently a participant of a clinical trial and/or other scientific experiment; and/or where this individual has been, will be, and/or is currently undergoing medical testing, medical treatment and/or other care, and/or where this individual has had, will have, and/or is currently having corresponding medical data collected for processing by medical data processing system 500.

Some or all data sources 575 can be distinct from some or all requesting entities 576 (e.g. a particular hospital collects medical data for patients, which is processed by the medical data processing system 500 to generate output data sent to a research company conducting a clinical trial). Alternatively or in addition, some or all data sources 575 can also be requesting entities 576 (e.g. a particular research entity collects medical data in conjunction with conducting a clinical trial, sends the medical data to the medical data processing system 500 for processing, and receives corresponding output data generated by the medical data processing system 500 in response for use in conducting the clinical trial).

The medical data processing system 500 can implement a modeling platform that provides image-based modeling and/or other modeling operable to assist in clinical trials, scientific experiments, medical tests, healthcare treatment, other health-related events, and/or other scientific testing, treatment, products, and/or processes. Some of the embodiments of medical data processing system 500 described herein are directed towards analyzing a clinical trial(s) and/or other scientific experiment(s) (e.g., not yet started, on-going, and/or completed), however, other embodiments are directed to analyzing patient treatment which may be occurring within a clinical trial or may be occurring outside of or otherwise not associated with any clinical trial (e.g., analysis of on-going treatment of a patient where the treatment was already approved).

In one or more embodiments, the medical data processing system 500 can be implemented to perform medical data-based modeling. This medical data-based modeling can include image-based modeling, for example, based on training a corresponding image processing function (e.g. based on training a computer vision model utilizing artificial intelligence techniques and/or machine learning techniques) and/or based on executing this trained image processing function. In some embodiments, such image-based modeling is applied only to images (which can include data representative of the images) for determining predicted variable(s) and/or other function output of a corresponding image processing function. In some embodiments, the image-based modeling is applied to images in conjunction with other data (e.g. medical/user data) that is ingested by or otherwise analyzed by the model to facilitate the determining of the predicted variable(s). The predicted variable(s) alone or in conjunction with other information (including imputed variables that are determined from analysis of the images) can be used to generated event estimation information including time-to-event curves, survival curves, Kaplan Meier curves, and other outcome models. In some embodiments, the predicted variables can include mortality risk scores. In one or more embodiments, the modeling platform can extract and utilize other data from the images (and/or can obtain the other data from other sources independent of the model's analysis of the images), which may or may not be a clinical variable (e.g., tumor size, cleanliness of margins, etc.), and which is optionally not be a variable per se, but can be utilized for or otherwise facilitate some of the determinations (e.g., survival predictions). In one or more embodiments, the medical data processing system 500 can be implemented to apply image-based models to particular imaging modalities (e.g., computed tomography (CT) scans), however, other embodiments can apply the image-based models to other types of images or combinations of types (e.g., X-ray, Magnetic Resonance Imaging (MRI), etc.). In one or more embodiments, the medical data processing system 500 can be implemented to apply multi-modal image-based models to multiple imaging modalities (e.g. two or more different types of imaging scans, or two or more different types of other input data).

In one or more embodiments, the medical data processing system 500 can be implemented in conjunction with the conducting of clinical trials (which can include various types of medical studies or other scientific studies such as ones that utilize a control group and an investigational group). In some embodiments, a cloud platform is implemented so that automated patient eligibility determinations, screening and randomization can be derived by the image-based model from baseline images (e.g., pre-treatment images such as CT scans). In this cloud platform, ongoing treatment efficacy and prediction can be derived by the image-based model from follow-up images (e.g., CT scans during (i.e., on-treatment or in-treatment images) and after treatment), which can be reviewed by various entities such as the clinical operations manager. In this cloud platform, data submissions for the clinical trial(s) can be submitted to the FDA and/or other governmental agency/entity, for example, according to any requirements to obtain approval for the treatment of the clinical trial(s). The particular interaction with governmental regulatory bodies can be differ and can be accommodated by the exemplary systems and methodologies described herein including submissions of data from multiple clinical trials associated with a treatment which can then be evaluated by the agency (e.g., FDA) for approval. In one or more embodiments, the data generated or otherwise determined from the systems and methodologies described herein can be accessed (e.g., via the cloud platform) and/or utilized for various purposes including internal business decisions, regulatory authorities, or other purposes. In one or more embodiments, data can be generated or otherwise determined via the systems and methodologies described herein for various clinical endpoints which can include survival or survivability assessments, but which can also include other types of clinical endpoints.

In one or more embodiments, the medical data processing system 500 can be implemented to allow for predicting success of a trial during the trial at different time periods, such as based on particular clinical endpoints (e.g. based on predictions such as survival or survivability data or mortality time that are generated from the image-based model applied to baseline/pre-treatment images and/or follow-up images). In some embodiments, medical data processing system 500 (e.g. based on predictions such as survival data or mortality time that are generated from the image-based model applied to baseline/pre-treatment images and/or follow-up images) can be implemented to measure current treatment effect and/or predicting treatment effect during an on-going clinical trial. Some or all of this information can be sent to a corresponding requesting entity 575 and/or can otherwise be accessed by an entity corresponding to a clinical trial manager, pharmaceutical company, and/or or other entity involved in a clinical trial, which can improve the technology of operating or managing clinical trial(s).

In one or more embodiments, the medical data processing system 500 can be implemented to enable generating of event estimation curves according to predictive analysis of various images (e.g., pre-treatment, on-treatment and/or post treatment) which can be associated with various data or be of various types, including clinical endpoint estimation, time-to-event estimation, survival estimation, random forest, Kaplan Meier curves, and so forth. One or more of the embodiments described herein can generate the event estimation curves or data representations in a format (or of a selected type) that can be best suited for providing an analysis of the data and/or an analysis of the clinical trial.

In one or more embodiments, the medical data processing system 500 (e.g., based on predictions such as survival data or mortality time that are generated from the image-based model applied to baseline/pre-treatment images and/or follow-up images) can be used with, or in place of, radiologists manually interpreting or annotating regions of interest. In one or more embodiments, the medical data processing system 500 can improve efficiency, can avoid use of limited resources such as radiological expertise, is not subject to inter-reader variability, and/or avoids the implication that only annotated regions of interest are correlated with outcomes. Further efficiency can be added by the medical data processing system 500, for example, through a corresponding cloud-based platform, for example, to mitigate the need for a hospital to download the image onto a DVD and mail it to the organization managing the clinical trial, which is a time consuming and inefficient process typical in traditional conducting of clinical trials.

In one or more embodiments, the trained image-based model(s) can be generalizable to a broader population based on the size of the training dataset (e.g., 5% of all lung cancer patients across a country such as Canada although other sizes of datasets from various places can be utilized), which will include patients having various sorts of conditions, diseases and other comorbidities.

In one or more embodiments, the image-based modeling can provide time to event predictions. For example, these predictions can be according to treatment (e.g., surgery vs chemotherapy vs different chemotherapy vs. radiation). As another example, these predictions can be done longitudinally (i.e., predicting at different time points to show improvement or deterioration). This can include imaging before, during and/or after treatments for each patient, looking at visual changes in images over times for prediction, and/or predicting whether a tumor will return. As another example, these predictions can be by comorbidity, such as taking into account competing risks (e.g., heart disease).

In one or more embodiments, the medical data processing system 500 can provide explainability. For example, information can be generated as to why the model made a particular prediction. As another example, the model can generate a predicted image representative of the predicted tumor size and/or predicted shape corresponding to various points in the future. In one or more embodiments, the image-based modeling allows for inputting image(s) of a body part (e.g., lung) and the model can generate outcome prediction and a new image showing what the tumor/organ/image is predicted to look like in 3 months, 6 months, 1 year, and so forth to show how the tumor is expected to grow or shrink supporting the model's outcome prediction. In one or more embodiments, the image-based modeling can provide information corresponding to predictions being made that are categorized by various criteria such as by organ, by clinical variable, and so forth.

In one or more embodiments, the image-based modeling can provide predictions for treatment planning. These predictions can be done in conjunction with patients that may or may not be enrolled in a clinical trial. For example, the model can predict from an image (e.g., pre-treatment CT scan) outcomes for specific treatments. The clinician would then choose treatment that offers optimal outcome. As another example, the model can predict from an image (e.g., pre-treatment CT scan) optimal radiation dose by anatomical region to also reduce toxicity risk (i.e., radiation-induced pneumonitis). In another example, image guided treatment can be facilitated such as via an overlay on the image which is fed to the model and the model quantifies the input. As another example, the model can predict from an image (e.g., pre-treatment CT scan) treatment toxicity by treatment type or plan so that the physician can select or plan optimal treatment. As another example, the model can predict from an image (e.g., pre-treatment CT scan) functional test results (e.g., cardiopulmonary function) to quantify fitness for specific treatments (e.g., surgery). For example, the model can predict lung capacity which is used for qualifying patients for surgery. In this example, the prediction from the pre-treatment image can be used to determine at what point in the future the patient may no longer be eligible for surgery. As another example, the model can predict from an image (e.g., pre-treatment CT scan) a quantification of quality of life for various treatment options. In this example, the prediction from the pre-treatment image can be used to assess quality of life at particular time periods in the future, which may be used in place of or in conjunction with test walks, surveys, or other quantification techniques.

In one or more embodiments, the modeling platform can obtain information from personal data sources (e.g., smartwatch, pedometer, HR monitor, and so forth) of the patient which can be utilized as part of the prediction analysis and/or can be provided as additional medical data along with the predicted variables to assist in treatment planning.

In one or more embodiments, the image-based modeling and modeling platform can be utilized to facilitate and improve clinical trials, such as through use of a digital twin that is generated from an image (e.g., a pre-treatment CT scan of a candidate that will be in the investigational arm) where the digital twin can be utilized in a control arm of the clinical trial. The digital twin can be imputed with various information based on predictions from the image-based model applied to the baseline/pre-treatment image, similar to the information that an actual candidate in the control trial arm would exhibit or be associated with (e.g., survival data). In one or more embodiments, the use of a digital twin can speed up clinical trials and make them more efficient by reducing the number of actual candidates required to be utilized in the control arm, such as populating the control arm with a digital twin(s) derived from a candidate(s) that is in the investigational arm. In one or more embodiments, the digital twin can speed up clinical trials and make them more efficient by improving randomization between the investigational arm and the control arm such that the control arm can be balanced by digital twin(s) derived from a candidate(s) that is in the investigational arm. In one or more embodiments, digital twins can be utilized that are simulated control outcomes for individual patients/candidates. For example, during a clinical trial or before treatment, a digital twin can be created from the data collected from a patient/candidate, which can be solely image-based data or can be other information utilized in conjunction with the image-based data. In this example, this baseline data can be fed into a generative AI-model (e.g., a three-dimensional convolutional neural network (3DCNN) or other image-based model) that has been pre-trained, such as on a database of longitudinal patient data (e.g., image data of the patient) from historical trials, observational studies, and/or treatments. The AI-model can predict the likely outcome for that patient/candidate if the patient/candidate was to receive the control while the actual patient/candidate goes on to receive the treatments (which can be active or control) and the outcome under that treatment is observed. In one or more embodiments, generative AI-models can be trained on historical data which can then be used to create digital twins that predict what would likely happen to a particular patient/candidate over the course of a trial if the patient/candidate was treated with the current standard of care (which may be in addition to a placebo).

As an example, the modeling platform can provide automated eligibility screening and/or matching to clinical trials based on a pre-treatment image (alone or in conjunction with other medical/user data for the candidate). As another example, the modeling platform can provide automated trial randomization (investigational arm vs control arm) to clinical trial(s) based on analysis of a pre-treatment image (alone or in conjunction with other medical/user data for the participant). As another example, the modeling platform can provide imaging-based prognostic enrichment for participants in the clinical trial. As another example, the modeling platform can provide imaging-based companion diagnostic to qualify patients for treatment. For example, past clinical trial data can be used to identify ideal patient type for clinical trial success. As another example, inclusion/exclusion criteria based on historical trials can be utilized. As is described herein, the functions of the systems and methodologies described herein including the application of the image-based modeling can have many practical uses which not only improve clinical trials but also allow for a better understanding of the outcome of a clinical trial such as predicting commercial value of a new drug, such as based on changes in predicted patient outcomes. In one or more embodiments, the image-based modeling and modeling platform can automate or otherwise provide information for commercial value and/or pricing of treatment/medications, such as based on cost of current treatments and in consideration of demonstrated benefit during clinical trial. In one or more embodiments, the image-based modeling and modeling platform can predict the cost of a clinical trial, such as based on predicted variables including time of treatment, time at which treatment difference (i.e., treatment effect) will be detectable, and so forth. As is described herein, the functions of the systems and methodologies described herein including the application of the image-based modeling can have other practical uses in the context of patient treatment which not only provides predictions as to treatment results but also allow for a better understanding of the outcome of the treatment and whether changes to the treatment plan could or should be made.

In one or more embodiments, the modeling platform provides tools to assist various entities including pharmaceutical companies, clinical trial managers, healthcare providers and/or patients. As an example, the modeling platform can automate collection of terms via common language, abbreviations, spelling errors, etc. As another example, the modeling platform can automate protected health information (PHI) aggregation creating uniform formats. As another example, the modeling platform can make it easier to interpret data in a more uniform way out of multiple datasets. In one or more embodiments, the modeling platform can automate evaluation of clinical trial design such as improved endpoints, broader patient population, and so forth. In one or more embodiments, the image-based modeling can automate identification of organs (or other body parts) from image and/or automate annotations to the data including points of interest. In one or more embodiments, the modeling platform can create a searchable tool based on the identified organs or other body parts. In one or more embodiments, the modeling platform can create or otherwise provide automatic QA tools to ensure imaging protocols are properly followed. In one or more embodiments, the modeling platform allows for a reverse image search, such as finding similar images (e.g., similar tumor size and/or shape, similar organ size and/or shape, and so forth) based on a submitted image.

In one or more embodiments, the modeling platform facilitates and/or guides preventative care, which may or may not be for a patient participating in a clinical trial. As an example, the modeling platform through use of the image-based modeling can ingest a whole-body scan (or scans of target areas/organs of the body) to identify long term health risks. In this example, various models can be trained and utilized for the analysis such as models particular to a single organ or body part, models particular to groups of organs or body parts, or whole-body scan models. As another example, the modeling platform can rank health care risk by organ(s) and/or by comorbidity risk(s). As another example, the modeling platform can interface with portable devices to auto-screen without the need for manual interpretation, such as for use in a breast cancer screening.

In one or more embodiments, image-based modeling and the modeling platform can be combined with or otherwise used in conjunction to pathology, genomic sequencing, proteomics, transcriptomics. For example, digitized pathology images can be processed and included in the modeling platform in conjunction with the patient's images (e.g., CT imaging). In another example, results of genomic sequencing can be provided as an input into the modeling platform.

In one or more embodiments, image-based modeling and the modeling platform can be used by consumers for predicting optimal financial portfolio construction, predicting optimal diet, predicting optimal workout, physical therapy exercises. In one or more embodiments, image-based modeling and the modeling platform can be used by consumers for ranking long-term care facilities based on residents' health deterioration compared to the expected outcome.

In one or more embodiments, image-based modeling and the modeling platform can be used in veterinary medicine to create organ-based risk assessment for pets along with an expected response to treatment; decrease pet insurance based on the animal's risk score and/or recommend pet food based on animal's risk score. Other embodiments are described in the subject disclosure.

One or more aspects of the subject disclosure include a method performed by one or more processors or processing systems. For example, the method can include obtaining, by a processing system, a baseline/pre-treatment image for each candidate of a group of candidates for a clinical trial resulting in a group of baseline/pre-treatment images, where the baseline/pre-treatment image captures at least an organ that is to be subject to treatment for a disease in the clinical trial, and where the group of baseline/pre-treatment images are captured prior to the treatment. The method can include analyzing, by the processing system, the group of baseline/pre-treatment images according to an imaging model that includes a machine learning model (e.g., a neural network such as a convolutional neural network (CNN), 3DCNN, recurrent neural network (RNN), long short term memory (LSTM), and other modeling networks including current or future models). The method can include predicting, by the processing system according to the analyzing of the group of baseline/pre-treatment images, one or more clinical variables for the group of baseline/pre-treatment images resulting in predicted variables. The method can include determining, by the processing system, a first subset of candidates of the group of candidates that are eligible for the clinical trial based on the predicted variables and based on study criteria of the clinical trial, where the study criteria include inclusion criteria and/or exclusion criteria. The method can include determining, by the processing system, a second subset of candidates of the group of candidates that are ineligible for the clinical trial based on the predicted variables and based on the study criteria of the clinical trial. In other embodiments, the method can include obtaining consent for participation in the clinical trial according to the various laws, rules and/or regulations that are applicable to that jurisdiction which in some instances can include generating notices and obtaining consent to participate in the clinical trial(s).

One or more aspects of the subject disclosure include a device having a processing system including a processor; and having a memory that stores executable instructions that, when executed by the processing system, facilitate performance of operations. The operations can include obtaining a group of baseline/pre-treatment images for a group of candidates for a clinical trial, where the group of baseline/pre-treatment images capture at least an organ that is to be subject to treatment for a disease in the clinical trial, and where the group of baseline/pre-treatment images are captured prior to the treatment. The operations can include analyzing the group of baseline/pre-treatment images according to an imaging model that includes a machine learning model. The operations can include predicting, according to the analyzing of the group of baseline/pre-treatment images, one or more clinical variables for the group of baseline/pre-treatment images resulting in predicted variables. The operations can include generating, based on the predicted variables, digital twins for the group of candidates. The operations can include generating a graphical user interface and providing equipment of an entity managing the clinical trial with access to the graphical user interface. The operations can include obtaining images for the group of candidates participating in the clinical trial resulting in a group of on-treatment images, where the group of on-treatment images are associated with a time period of the treatment. The operations can include analyzing the group of on-treatment images according to the imaging model. The operations can include predicting, based on the analyzing of the group of on-treatment images, the one or more clinical variables for the group of on-treatment images resulting in predicted on-treatment variables. The operations can include generating event estimation curves (e.g., survival curves such as Kaplan Meier (KM) curves) based on the predicted on-treatment variables for an investigational trial arm and a control trial arm of the clinical trial, where the investigational arm includes the group of candidates and the control arm includes the digital twins. The operations can include presenting the event estimation curves in the graphical user interface.

One or more aspects of the subject disclosure include a non-transitory machine-readable medium, including executable instructions that, when executed by a processing system(s) including a processor(s), facilitate performance of operations. The operations can include obtaining a group of baseline/pre-treatment images for a group of candidates for a clinical trial, the group of baseline/pre-treatment images capturing at least an organ that is to be subject to treatment for a disease in the clinical trial, where the group of baseline/pre-treatment images are captured prior to the treatment. The operations can include analyzing the group of baseline/pre-treatment images according to an imaging model that includes a machine learning model. The operations can include predicting, according to the analyzing of the group of baseline/pre-treatment images, one or more clinical variables for the group of baseline/pre-treatment images resulting in predicted variables. The operations can include randomizing, based at least on the predicted variables, each candidate of the group of candidates to one of an investigational trial arm or a trial control arm of the clinical trial. The operations can include generating a graphical user interface and providing equipment of an entity managing the clinical trial with access to the graphical user interface. The operations can include obtaining images for the group of candidates participating in the clinical trial resulting in a group of on-treatment images, where the group of on-treatment images are associated with a time period of the treatment. The operations can include analyzing the group of on-treatment images according to the imaging model. The operations can include predicting, based on the analyzing of the group of on-treatment images, the one or more clinical variables for the group of on-treatment images resulting in predicted on-treatment variables. The operations can include generating event estimation curves (e.g., KM curves) based on the predicted on-treatment variables for the investigational trial arm and the control trial arm of the clinical trial. The operations can include presenting the event estimation curves in the graphical user interface.

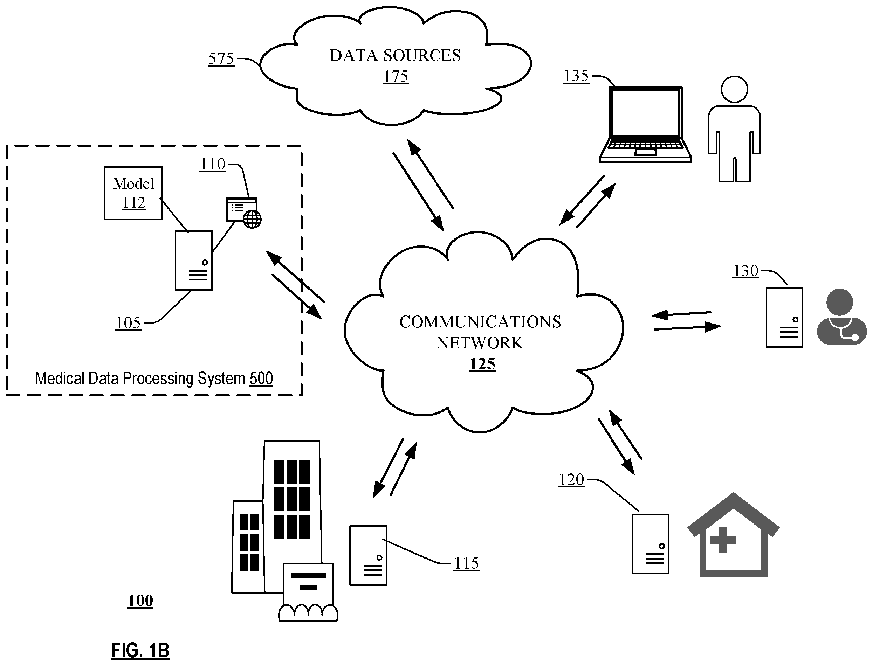

Figure 1B presents a block diagram illustrating an example, non-limiting embodiment of a system 100 in accordance with various aspects described herein. Some or all features and/or functionality of system 100 of Figure 1B can implement any embodiment of medical data processing system 500 described herein. Some or all features and/or functionality of system 100 of Figure 1B can alternatively or additionally implement any embodiment of the data source 575 and/or requesting entity 576 described herein.

In some embodiments, system 100 can facilitate in whole or in part providing medical data-based modeling to assist in clinical trials, healthcare treatment or other health-related events. As an example, the medical data -based modeling can be performed based solely on analysis of device-captured medical data(s) (e.g. medical images) according to a trained model (e.g. trained image model such as a trained computer vision model) or can be performed in conjunction with consideration, incorporation and/or analysis of other information, such as medical/user data for the individual (e.g., one or more of age, sex, weight, Eastern Cooperative Oncology Group (ECOG) status, smoking status, competing mortality risk, cardiac and pulmonary toxicity, TNM (Tumor, Nodes and Metastases) stage, pulmonary function, or other characteristics associated with the individual) or other clinical factors depending on the disease. In one or more embodiments, the other information that can be utilized as part of the medical data-based modeling via one or more imputed variable(s) (such as one or more described above) can be derived, generated or otherwise determined based solely on an analysis of the image (e.g., baseline/pre-treatment image) or can be derived, generated or otherwise determined based on other information (e.g., user input information, corresponding data collected for the potential candidates, etc.) and which can be in conjunction with the analysis of the image. In one or more embodiments, the medical images can be 2D and/or 3D images, such as CT scans and the image-based modeling can be according to 2D and/or 3D modeling. In one or more embodiments, a given medical image can indicate a plurality of image slices (e.g. each corresponding to a two dimensional image. In one or more embodiments given medical image can indicate additional of types imaging information (e.g. implemented as multiple layers and/or masks), for example, generated via application of an additional imaging modality (e.g. information derived from a PET scan that is implemented in addition to the three-dimensional information of a CT scan, where both sets of information were generated via conducting of a corresponding PET-CT scan). In one or more embodiments, system 100 can apply the image-based modeling to various organs (e.g., lungs, brain, liver, pancreas, colon, and so forth) alone or in combination, or to various regions of the body, including regions that have a tumor. In one or more embodiments, system 100 can apply the image-based modeling to volumes surrounding and including various organs, such as the thorax which includes the lungs. In one or more embodiments, system 100 can apply the image-based modeling to humans or animals. In one or more embodiments, system 100 can apply the image-based modeling for generating predicted variables for patients who are or are not part of a clinical trial.

In one or more embodiments, system 100 includes one or more servers and/or computing devices 105 (only one of which is shown) which can manage or otherwise provide image-based modeling to equipment of various entities to assist in clinical trials, healthcare treatment and/or other health-related events. For example, the one or more servers and/or computing devices 105 are implemented as medical data processing system 500.

As an example, the server 105 can communicate over a communications network 125 with equipment of a pharmaceutical entity(ies) or other entity(ies) managing a clinical trial(s), such as a computing device or server 115 (only one of which is shown). The server 105 can communicate over the communications network 125 with equipment of a hospital(s) or other healthcare treatment facility(ies) which may have a patient(s) that is, was or will be taking part in a clinical trial(s), such as a computing device or server 120 (only one of which is shown). The server 105 can communicate over the communications network 125 with equipment of a healthcare provider(s) such as a physician that may have a patient(s) who is, was, or will be taking part in the clinical trial(s), such as a computing device or server 130 (only one of which is shown). The server 105 can communicate over the communications network 125 with equipment of a patient(s) who is, was, or will be taking part in the clinical trial(s), such as a computing device or server 135 (only one of which is shown). Any number of devices or servers 105, 115, 120, 130, 135 can be utilized at any number of locations for facilitating image-based modeling that assists in clinical trials, healthcare treatment and/or other health-related events.

One or more devices or servers 115, 120, 130, 135 can implement one or more data sources 575 of Figure 1A, for example, based on corresponding pharmaceutical entities, entities, managing a clinical trial hospitals, healthcare treatment facilities, physicians, healthcare providers, patients, etc. providing medical data (e.g. medical images), patient data, and/or clinical trial data to the medical data processing system 500 for processing or storage (e.g. to train models, to generate predictions via use of trained models, etc.).

One or more devices or servers 115, 120, 130, 135 can implement one or more requesting entities 576 of Figure 1A, for example, based on corresponding pharmaceutical entities, entities, managing a clinical trial hospitals, healthcare treatment facilities, physicians, healthcare providers, patients, etc. requesting that medical data (e.g. medical images), patient data, and/or clinical trial data be processed by medical data processing system 500 (e.g. to train models, to generate predictions via use of trained models, etc.), where corresponding function output (e.g. predictions) are sent to and/or communicated to these devices or servers 115, 120, 130, 135.

In one or more embodiments, server 105 can provide a modeling platform 110 accessible (in whole or in part) to devices or servers 115, 120, 130, 135. In one or more embodiments, the modeling platform 110 can provide one, some or all of the functions described herein, including image-based modeling which facilitates clinical trials, healthcare treatment and/or other health-related events. It should be understood by one of ordinary skill in the art that the modeling platform 110 can operate in various architectures including centralized or distributed environments, browser-based, installed software, and so forth. As an example, server 115 of the pharmaceutical entity or the other entity managing a clinical trial and server 120 of the hospital(s) or the other healthcare treatment facility may utilize installed software, while server 130 of the healthcare provider(s) and device 135 of the patient(s) utilize a browser-based access to the modeling platform 110.

In one or more embodiments, modeling platform 110 applies a trained image-based model to baseline (e.g., prior to treatment), on-treatment and/or post-treatment images (e.g., CT scans) to predict one or more clinical variables, such as mortality risk score, age, sex, weight, ECOG status, smoking status, competing mortality risk, cardiac and pulmonary toxicity, TNM stage, pulmonary function, or a combination thereof. In one or more embodiments, modeling platform 110 can selectively obtain, train and/or apply one of multiple trained image-based models, only one of which is shown (model 112), to one or more clinical trials, treatments, and so forth. In one or more embodiments, the modeling platform 110 can selectively apply the trained image-based model to each of the images (e.g., baseline/pre-treatment, on-treatment and post-treatment images), for instance as they are obtained or acquired, to predict the one or more clinical variables and to show changes in the predictions over time (i.e., different time periods of each of the images). In one or more embodiments, the baseline images (e.g., pre-treatment images) can be captured before and/or after a candidate(s) is accepted to the clinical trial, such as analyzing a first baseline/pre-treatment image as part of evaluating whether the candidate should participate in the clinical trial and analyzing a second baseline/pre-treatment image (captured later after being accepted to the clinical trial but before treatment commences such as according to a time limit for capturing imaging) as part of generating predicted variables and/or generating event estimation curves such as survival curves.

As an example, an image-based model 112 (e.g., a deep learning model such as a 3DCNN) can be trained based on images associated with a particular organ and/or a particular disease (e.g., which may be pre-treatment images where the treatment was the standard of care at the time), as well as survival data for the individuals associated with the images. The image-based model 112 can be, or can be derived from, various types of machine-learning systems and algorithms. The dataset (e.g., pre-treatment CT scans of individuals that underwent standard of care treatment and/or for whom survival or other data is available) for training the image-based model 112 can be from one or more of various data sources 175 which can be private and/or public data in various formats and which may or may not be anonymized data). In one or more embodiments, the training of the model can be performed based on historical relevant data (e.g., images where outcomes of treatment are known) from individuals that are different from the clinical trial candidates (e.g., where outcomes of treatment have not yet occurred and are unknown). In one embodiment, 80% of the historical relevant data can be utilized to train the model while 20% of the historical relevant data is utilized to validate the model. Other percentages for training and validation distribution can also be utilized. The model training can be done utilizing only images (e.g., from a private and/or public source) and survival data, or can be done in conjunction with other medical/user data (e.g., one or more of age, sex, weight, ECOG status, smoking status, comorbidities, cardiac and pulmonary toxicity, TNM stage, pulmonary function, and so forth) for each of the individuals. Various modeling techniques can be applied for validation and/or improvement of the model, such as generating class activation maps as a visual explanation to indicate upon which anatomical regions the image-based model placed attention to generate its clinical variables (e.g., a mortality risk prediction). In one embodiment, the model 112 is not expressly or directly trained to focus on tumors.

In one embodiment, the modeling platform 110 can obtain a baseline/pre-treatment image(s) (e.g., CT scan) for each candidate of a group of candidates for a clinical trial resulting in a group of baseline/pre-treatment images. The baseline/pre-treatment images can capture an organ (which may also include capturing a surrounding area around the organ) that is to be subject to future treatment for a disease in the clinical trial. The group of baseline/pre-treatment images are captured prior to the treatment and can be provided to the modeling platform 110 from various equipment such as servers 120, 130. The modeling platform 110 can analyze the group of baseline/pre-treatment images according to the image-based model 112 which in this example is a three dimensional convolutional neural network (3DCNN) trained model. According to the analysis of the group of baseline/pre-treatment images (which in one embodiment can be limited to only the images and not other medical/user data), the modeling platform 110 can predict one or more clinical variables (i.e., predicted variables) for the group of baseline/pre-treatment images. As an example, the predicted variables can include (or in one embodiment be limited to) a mortality risk score or other survival valuation for each candidate corresponding to each of the baseline/pre-treatment images. The baseline/pre-treatment images can also be obtained and analyzed for candidates who are to be part of the control trial arm (e.g., receive the standard of care treatment) to generate predicted variables for the control trial arm.

In one embodiment, the modeling platform 110 can assess eligibility for the clinical trial based on the predicted variables. In one embodiment, the modeling platform 110 can determine or otherwise identify a first subset of the candidates that are eligible for the clinical trial based on the predicted variables and based on study criteria of the clinical trial, such as inclusion criteria and exclusion criteria defined by the manager of the clinical trial. In one embodiment, the modeling platform 110 can determine a second subset of the candidates that are ineligible for the clinical trial based on the predicted variables and based on the study criteria of the clinical trial. For instance, the clinical trial manager can access the modeling platform 110 via the server 115 to view a graphical user interface (e.g., a Trial View) in order see the eligibility determinations that have been made as well as other information indicating the status of the clinical trial, such as subjects screened, screen failures, subject enrolled, which may be broken down by various criteria such as site names, investigators, and so forth.

Various techniques can be utilized to determine which of the candidates will be participating in the clinical trial from those that have been selected as eligible by the modeling platform, where those techniques may or may not be implemented by the modeling platform 110. As an example, although other techniques can be implemented, the modeling platform 110 can generate notices for the first subset of candidates regarding eligibility, such as communications that can be sent to the second subset of candidates via their devices 135 (or otherwise sent to them) and/or communications that can be sent to healthcare providers of the second subset of candidates via their devices 130 (or otherwise sent to them). In one embodiment, the modeling platform 110 can obtain consent for the second subset of candidates to participate in the clinical trial according to the particular requirements of the jurisdiction.

In one embodiment, modeling platform 110 generates survival estimation curves such as Kaplan Meier curves based on the predicted variables for an investigational trial arm and a control trial arm of the clinical trial. In one embodiment, the modeling platform 110 can determine or detect an improper or erroneous randomization of the clinical trial (e.g., the control arm predictions such as survival are better than the investigational arm predictions). In this example, the investigational arm data can be calibrated or adjusted such as based on a difference in the KM curves between the investigational trial arm and the control trial arm (e.g., at baseline). Continuing with this example, the calibrating can occur after the treatment begins or after the treatment has finished.

In one embodiment, as follow-up images are captured or obtained for the candidates after treatment commences, the model 112 can be applied to the follow-up images to generate on-treatment predicted variables and the KM curves can be updated according to the updated data. In one embodiment, the generating of the on-treatment predicted variables and updating of the data can be performed for both the investigational arm and the control arm. In one embodiment, the process of capturing follow-up images, generating on-treatment predicted variables according to the model 112 being applied to the follow-up images, and updating of the data for the KM curves can be repeated, such as throughout the length of treatment.

In one embodiment, a graphical user interface of the modeling platform 110 can provide an option for selecting different time periods of the treatment and presenting particular KM curves for the investigational arm and/or the control arm corresponding to the selection.

In one or more embodiments, the modeling platform 110 provides information that allows a clinical manager or other entity to determine whether to make an adjustment to the clinical trial according to the predicted variables (e.g., baseline/pre-treatment and/or on-treatment) which can include, but is not limited to, one of: continuing the clinical trial, terminating the clinical trial or accelerating the clinical trial.

In one embodiment, a graphical user interface of the modeling platform 110 can be accessed by one or more of the devices 120, 130, 135 to view a patient portion of the graphical user interface that is related to a particular candidate without providing access to a remainder of the graphical user interface (e.g., data of other candidates). In one embodiment, the patient portion of the graphical user interface can include a predicted image(s) of the organ or body part at a future time(s) that is generated based on the image-modeling of the baseline/pre-treatment and/or on-treatment images, and/or based on the predicted variables and/or the predicted on-treatment variables. As an example, the predicted image(s) of the organ or body part at the future time(s) can be generated based on predicted tumor size, predicted tumor shape, predicted growth pattern, and/or predicted tumor location (which can be generated based on the image-modeling of the baseline/pre-treatment and/or on-treatment images). In one embodiment, the patient portion including the predicted image(s) of the organ or body part at the future time(s) for all of the candidates can be viewed in a Trial View by the pharmaceutical company and/or clinical manager. In one or more embodiments, the patient portion of the graphical user interface of the modeling platform 110 can be used to facilitate treatment and treatment decisions for the particular patient as described herein. In one embodiment, the graphical user interface of the modeling platform 110 allows a viewer to toggle on or off the image predictions for any follow up images such that if toggled on then the KM curve will include those images in the predictions.

Modeling platform 110 allows for imaging data acquisition from various sources, including trial sites, private and/or public data repositories, and so forth, which can accelerate clinical trial operations, and can increase their transparency. Modeling platform 110 can generate clinically meaningful predictions from each imaging study, which can be utilized alone or can complement traditional imaging interpretation frameworks. Modeling platform 110 can assist clinical trial sponsors in optimizing or improving internal decision making and allow for treatments to be brought to market sooner at a lower cost.

Modeling platform 110 can facilitate and enhance data management associated with a clinical trial. In one or more embodiments, modeling platform 110 provides automated imaging de-identification and quality control to be implemented for acquired baseline/pre-treatment, on-treatment and/or post-treatment images. In one or more embodiments, modeling platform 110 provides centralized cloud and/or on-premises storage of data. In one or more embodiments, modeling platform 110 provides a secure and access-controlled environment, such as based on entity-based permissions (e.g., clinical manager having full access while patients and physicians have limited access pertaining to their own treatment).

Modeling platform 110 can facilitate and enhance collaboration associated with a clinical trial. In one or more embodiments, modeling platform 110 can communicate image, patient, and/or cohort specific findings to a particular team (or other authorized groups of recipients). In one or more embodiments, modeling platform 110 can conduct research anytime, anywhere over the Internet or web. In one or more embodiments, modeling platform 110 can upload, download and/or transfer data associated with the clinical trial or entities, including patients.

Modeling platform 110 can facilitate and enhance analysis associated with the clinical trial and/or treatment of patients. In one or more embodiments, modeling platform 110 can streamline customizable imaging workflows using a Platform Viewer. In one or more embodiments, modeling platform 110 can increase reproducibility of imaging interpretation. In one or more embodiments, modeling platform 110 can generate (e.g., with or without user input or user assistance) annotations for ML research and biomarker discovery. In other embodiments, the modeling platform 110 can allow for editing annotations after their generation.

Modeling platform 110 can facilitate and enhance obtaining or otherwise determining insights associated with the clinical trial and/or treatment of patients. In one or more embodiments, modeling platform 110 can enhance trial design, patient stratification, and/or covariate analyses. In one or more embodiments, modeling platform 110 can facilitate patient enrichment strategies, such as adjustments or supplements to treatment. In one or more embodiments, modeling platform 110 can improve biomarker surrogacy.

Communications network 125 can provide various services including broadband access, wireless access, voice access and/or media access utilizing a plurality of network elements which can also facilitate the distribution of data (e.g., images, medical/user data, and so forth) from data sources 175, which may be any number of data sources that can be private and/or public sources. The communications network 125 can include a circuit switched or packet switched network, a voice over Internet protocol (VoIP) network, Internet protocol (IP) network, a cable network, a passive or active optical network, a 4G, 5G, or higher generation wireless access network, WIMAX network, UltraWideband network, personal area network or other wireless access network, a broadcast satellite network and/or other communications network. The computing devices or servers 105, 115, 120, 130, 135 can be various devices including personal computers, laptop computers, netbook computers, tablets, mobile phones, e-readers, phablets, or other computing devices and can communicate via various devices such as digital subscriber line (DSL) modems, data over coax service interface specification (DOCSIS) modems or other cable modems, a wireless modem such as a 4G, 5G, or higher generation modem, an optical modem and/or other access devices. Communications network 125 can include wired, optical and/or wireless links and the network elements can include service switching points, signal transfer points, service control points, network gateways, media distribution hubs, servers, firewalls, routers, edge devices, switches and other network nodes for routing and controlling communications traffic over wired, optical and wireless links as part of the Internet and other public networks as well as one or more private networks, for managing subscriber access, for billing and network management and for supporting other network functions.

In one or more embodiments, system 100 can provide an end-to-end imaging research stack to accelerate clinical trials, which can include patient eligibility screening, randomization of participating candidates, efficacy predictions and/or FDA submissions. In one or more embodiments, the modeling platform 110 can analyze other related organs as part of the image-based modeling (which is trained accordingly) and prediction process, such as the liver where the disease is lung cancer. In another embodiment, multiple organs (as a single image or multiple images) can be fed into the appropriately trained model to generate the predicted variables. In one or more embodiments, the modeling platform 110 can be applied to (and the image-based models trained for) various diseases such as cardiovascular disease. In one or more embodiments, model 112 can be trained as a new version of the algorithm on individual treatment types then utilized to predict a patient's response to multiple treatment types. For example, this could be used to inform a doctor's decision on how to treat a patient.

In one or more embodiments, model 112 can be trained utilizing pre-, in- and/or post-treatment images (e.g., where the treatment was the standard of care or another treatment). In one embodiment, the training images can include images from disease-free individuals. In one or more embodiments, treatment information such as lab reports, type of treatment, and so forth may or may not be incorporated into the longitudinal model to adjust for changes visible or detectable in the follow-up images.

In one or more embodiments, model 112 can be adjusted, revised or otherwise fine-tuned to take into account additional newer data points. In this example, this allows the model to retain what it has already learned and only adjust the weights by a specified factor. In one or more embodiments, model 112 can be versioned for any iteration. For example, a study or clinical trial can reference the version of the model used. In one or more embodiments, model 112 can be trained on a first clinical trial and then used to predict outcomes of another clinical trial cohort's response to the treatment. This would provide a comparison of two clinical trials. This technique can be repeated over multiple treatments for comparison of multiple clinical trials. In one or more embodiments, model 112 can stratify patients in a clinical trial or otherwise associated with a particular treatment based on the image (e.g., baseline/pre-treatment CT scan) alone.

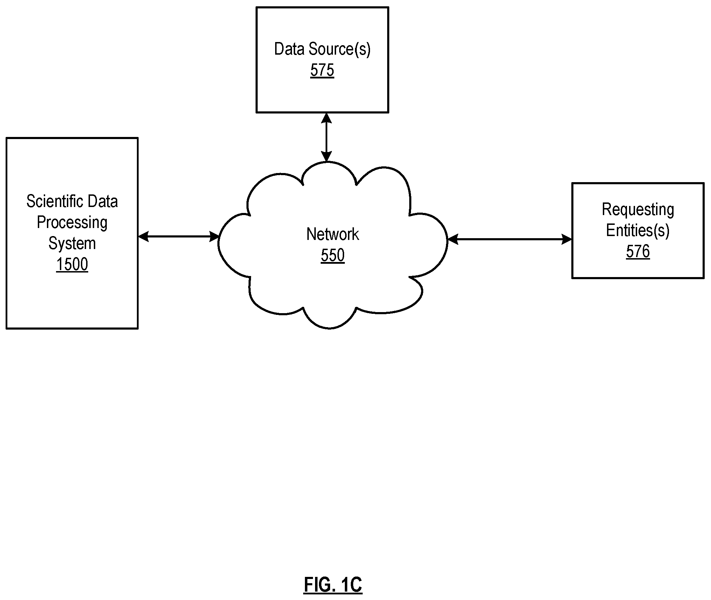

Figure 1C illustrates an embodiment of a scientific data processing system 1500 that communicates with one or more data source entities 575 and/or with one or more requesting entities 576 via a network 550. The one or more data source entities 575, one or more requesting entities 576, and/or network 550 of Figure 1C can be implement in a same or similar fashion as discussed in conjunction with Figure 1A and/or any other embodiment of data source entities 575, requesting entities 576, and/or network 550 described herein.

The scientific data processing system 1500 can be implemented in a same or similar fashion as medical data processing system 500, and/or can be implemented to perform some or all of the features and/or functionality of the medical data processing system 500. The scientific data processing system 1500 can be further implemented to apply some or all features and/or functionality of medical data processing system 500 described herein to non-medical data and/or non-healthcare data. For example, non-medical outcome predictions can be generated for non-medical data, where these predictions correspond to outcomes, for example of a scientific experiment and/or any other outcomes that are not necessarily medical. The scientific data processing system 1500 can thus apply some or all features and/or functionality of the medical data processing system 500 as related to clinical trials to any scientific experiments/studies (e.g. that similarly have a control arm and an investigational arm, where the corresponding experiment being applied is not necessarily a medical treatment and/or healthcare treatment). For example, the medical data processing system 500 can be considered a particular type of scientific data processing system 1500 that is implemented for the medical settings (e.g. scientific experiments that are clinical trials; scientific data that is medical data; trial participants that are patients receiving medical treatment; etc.). where any features of medical data processing system 500 can be similarly applied to other scientific settings/corresponding data, even if non-medical/non-healthcare related.

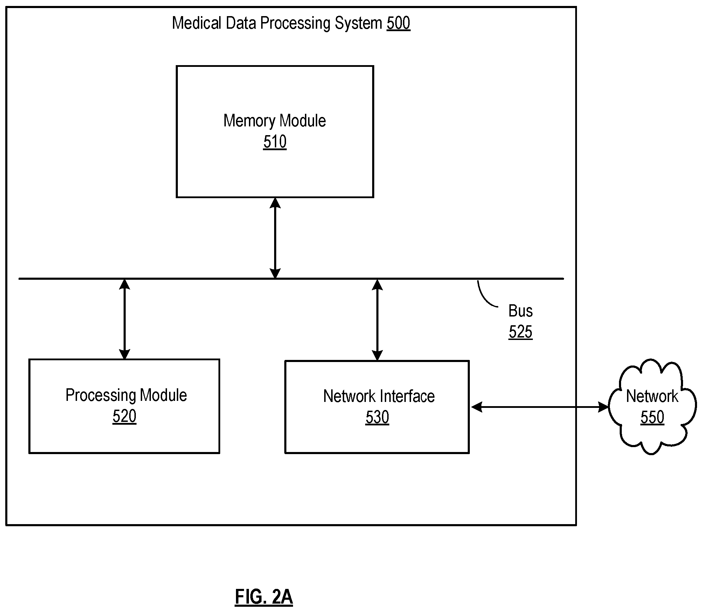

Figure 2A illustrates an embodiment of the medical data processing system 500. Some or all features and/or functionality of medical data processing system 500 of Figure 2A can implement any embodiment of medical data processing system described herein.

As illustrated in Figure 2A, the medical data processing system 500 can be implemented via one or more processing modules 520, one or more memory modules 510, and/or one or more network interfaces 530, communicating via a bus 525. The one or more network interfaces 530 can be operable to send and/or receive data via the network 550 and/or via any other communication system. Bus 525 can facilitate communication of data between the one or more processing modules 520, one or more memory module 510, and/or one or more network interfaces 530 via one or more wired and/or wireless communication resources.

The memory module 510 can include memory that stores operational instructions that, when executed by the one or more processing modules 520, cause the medical data processing system 500 to execute some or all of the functionality described herein.

As another example, the operational instructions, when executed by the one or more processing modules 520, can cause medical data processing system 500 to utilize network interface 530 to receive data from one or more data sources 575 via network 550. For example, this data can include medical data stored by, received by, generated by, and/or sent by one or more data sources 575.

As another example, the operational instructions, when executed by the one or more processing modules 520, can cause medical data processing system 500 to utilize network interface 530 to send a request to one or more data sources 575 via network 550. For example, this request can include a request for medical data stored by, received by, generated by, and/or sent by one or more data sources 575, where medical data 600 is received in response, such as only, and/or all, medical data meeting requirements specified by the request.

As another example, the operational instructions, when executed by the one or more processing modules 520, can cause the one or more processing modules 520 to generate data. For example, this data is generated based on: receiving and processing other data, such medical data stored by, received by, generated by, and/or sent by one or more data sources 575; retrieving and processing other data, retrieved from one or more memory modules 510; performing one or more functions on other data, for example, based on corresponding function entries of a function library; and/or one or more other mechanisms.

As another example, the operational instructions, when executed by the one or more processing modules 520, can further cause the one or more processing modules 520 to store data in one or more memory modules 510. For example, such data can be stored as medical data, function output data, medical outcome score data, patient data, clinical trial data, and/or a function entry, for example, indicating data for a newly trained model. This data can be obtained prior to storage in the one or more memory modules 510 based on being: generated by the one or more processing modules 510; stored in and retrieved from one or more memory modules 250; configured via user input by an administrator; received via network 150; and/or otherwise being determined.

As another example, the operational instructions, when executed by the one or more processing modules 520, can cause the medical data processing system 500 to utilize network interface 530 to send data to one or more requesting entities 576 via network 150. This data can include information, instructions, and/or prompts for display via an interactive user interface of the requesting entities 576. This data can alternatively or additionally include application data for storage and/or execution by requesting entities 576. This data can alternatively or additionally include function output data, such as medical outcome score data, for storage, display, further processing, further communication, or other use by requesting entities 576. This data can be obtained prior to transmission to the one or more requesting entities 576 based on being: generated by the one or more processing modules 520; stored in and retrieved from one or more memory modules 510; configured via user input by an administrator; received via network 550; and/or otherwise being determined. This data can be transmitted to the one or more requesting entities 576 based on processing a corresponding request received from the one or more requesting entities 576 indicating a request to generate, access, retrieve, and/or otherwise send the corresponding data. This data can be transmitted to the one or more requesting entities 576 based on otherwise determining to generate, access, retrieve, and/or otherwise send the corresponding data to the one or more requesting entities 576.

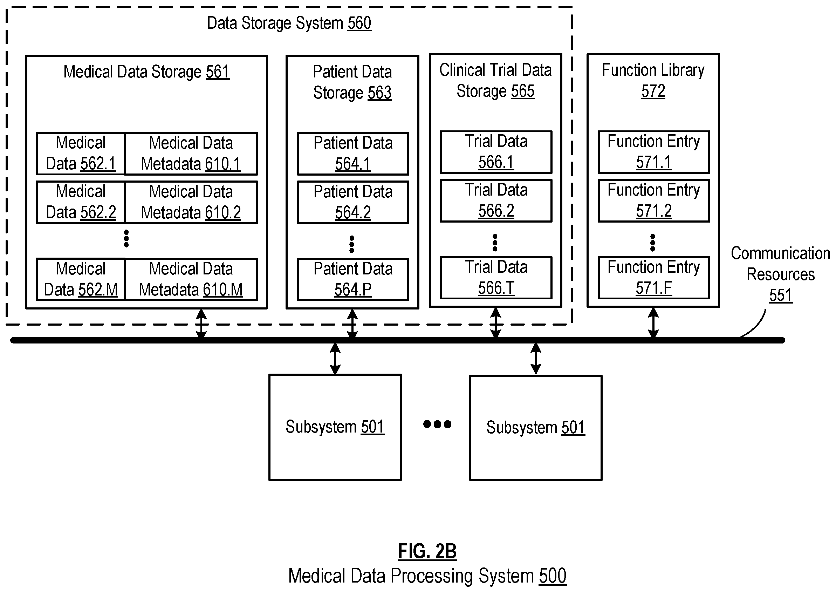

As illustrated in Figure 2B, the medical data processing system 500 can include and/or can communicate with: a data storage system 560; a function library 572; and/or one or more subsystems 501, all communicating via communication resources 551. The data storage system 560 can be implemented to include one or more medical data storages 561; one or more patient data storages 563; and/or one or more clinical trial data storages 565, for example, via implementing memory resources of memory module 510. Communication resources 551 can facilitate communication of data between the one or more medical data storages 561; the one or more patient data storages 563; the one or more clinical trial data storages 565l; the one or more function libraries 572; and/or the one or more subsystems 501 via one or more wired and/or wireless communication resources (e.g. via some or all communication resources implemented by bus 525). Communication resources 551 can correspond strictly to internal communication resources of medical data processing system 500 and/or can be implemented via some or all communication resources of network 550.

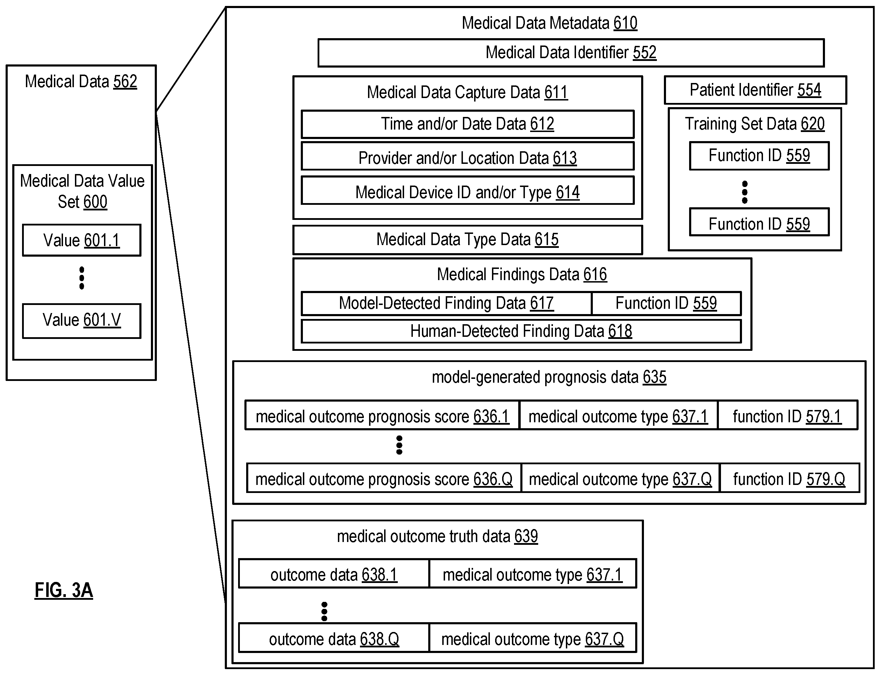

The one or more medical data storage 561 can store a plurality of medical data 562.1 - 562.M and/or a corresponding plurality of medical data metadata 610.1 - 610.M. Each medical data 562 can be any medical data (e.g. a medical image, output of a medical device, medical test data, etc.) captured for a given patient, where corresponding metadata 610 includes additional information describing/regarding/generated based on this medical data 562. Example embodiments of information included in and/or conveyed by medical data 562 and/or corresponding medical data metadata 610 are discussed in conjunction with Figure 3A.

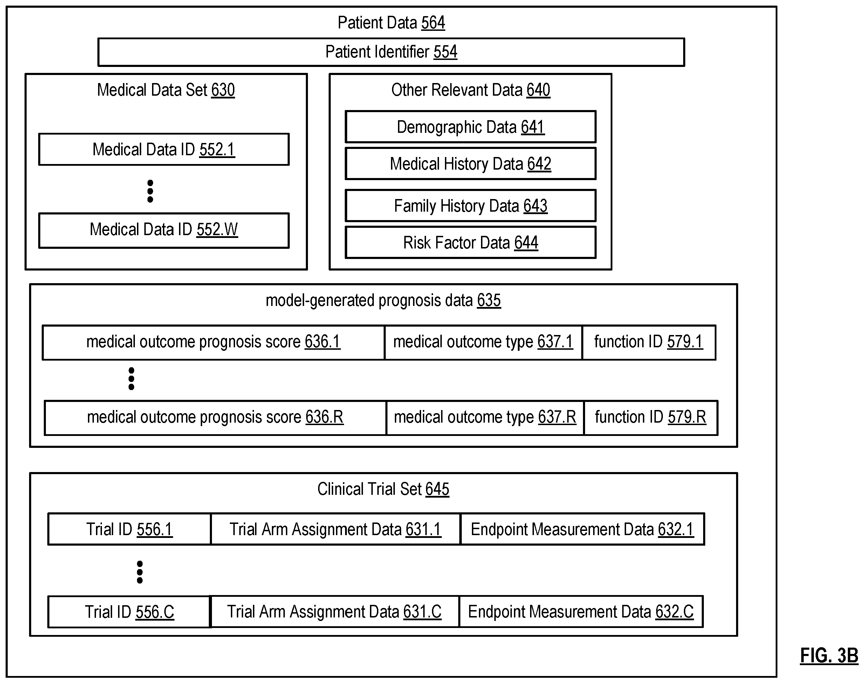

The one or more patient data storage 563 can store a plurality of patient data 564.1 - 564.P. Each patient data 564 can include and/or indicate data regarding a given patient. As used herein, a patient can correspond to any individual (e.g. a person, an animal and/or any subject of a particular procedure, trial, experiment, etc.). As used herein, a patient can correspond to any individual for example, for whom one or more types of and/or instances of medical data 562 has been and/or will be captured, and/or any individual that is a prospective participant/is a confirmed participant of an upcoming clinical trial/experiment and/or that is a current/past participant of a current/past clinical trial. Example embodiments of information included in and/or conveyed by patient data 564 are discussed in conjunction with Figure 3B.

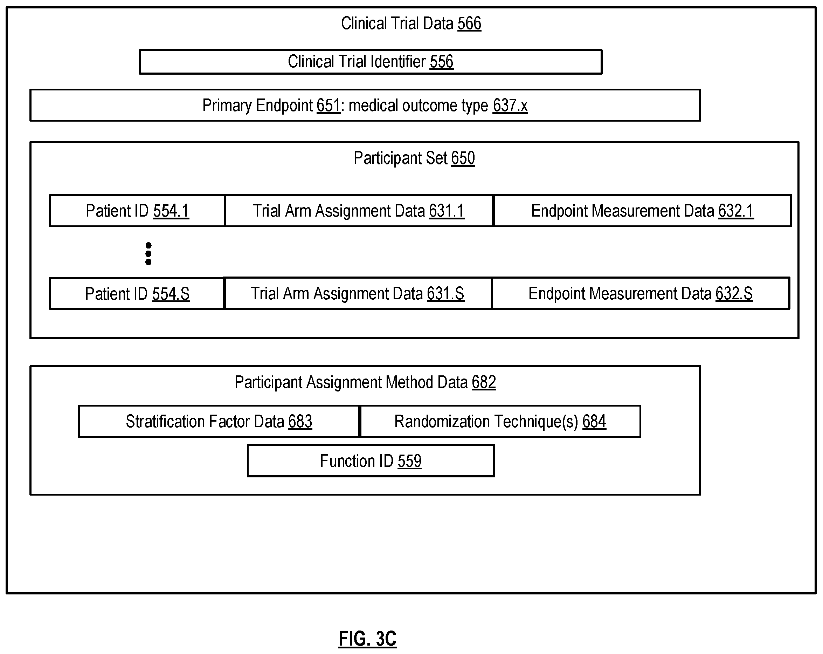

The one or more clinical trial data storage 565 can store a plurality of clinical trial data 566.1 - 566.T. Each clinical trial data 566 can include and/or indicate data regarding a given clinical trial (e.g. for a medical treatment, drug, healthcare product/treatment, food, etc.) and/or optionally any other trial/study/experiment conducted in any scientific field, such as a non-medical/non-healthcare field upon any group of individuals (e.g. a group people, animals, organisms and/or objects observed in at least one control group and at least one investigational group). Example embodiments of information included in and/or conveyed by clinical trial data 566 are discussed in conjunction with Figure 3C.

Some or all medical data 562.1 - 562.M of medical data storage 561, some or all patient data 564.1 - 564.P of patient data storage 563, and/or some or all clinical trial data 566.1 - 566.T of clinical trial data storage 565 can be received by the medical data processing system from a corresponding data source 575 that generated, collected, modified, stores, received, and/or sent the corresponding medical data 562, patient data 564, and/or clinical trial data 566. Alternatively or in addition, some or all medical data 562.1 - 562.M of medical data storage 561, some or all patient data 564.1 - 564.P of patient data storage 563, and/or some or all clinical trial data 566.1 - 566.T of clinical trial data storage 565 can be generated by the medical data processing system itself.

The one or more medical data storages 561, patient data storages 563, and/or clinical trial data storages 565 can be implemented as one or more relational and/or non-relational databases, one or more file storage systems, one or more object storage systems, and/or can be implemented via any one or more memory devices accessible by the medical data processing system 500 that is operable to store and/or access the plurality of medical data 562.1 - 562.M, patient data 564.1 - 564.P, and/or clinical trial data 566.1 - 566.T. The corresponding medical data 562.1 - 562.M, patient data 564.1 - 564.P, and/or clinical trial data 566.1 - 566.T can otherwise be accessed and/or determined by the medical data processing system 500 to enable the medical data processing system 500 to process and/or generate some or all of the corresponding data accordingly in performing some or all functionality described herein.

Alternatively or in addition, some or all medical data 562.1 - 562.M, some or all patient data 564.1 - 564.P, some or all clinical trial data 566.1 - 566.P, and/or some or all function entries 571 are stored externally (e.g. by a particular third party company and/or other entity, such as a particular hospital, a particular medical entity, a particular individual, a particular drug company, a particular medical research company conducting clinical trials, a particular company that collects and/or stores health information or other information for various individuals, etc.), for example, via a corresponding data source 575, requesting entity 576, and/or other computing device, where medical data 562 is processed based on being accessed via communications with a corresponding one or more external entities.

In some embodiments, medical data 562.1 - 562.M, patient data 564.1 - 564.P, clinical trial data 566.1 - 566.P are stored by and/or otherwise used by medical data processing system 500 in a secure fashion, for example, in accordance with protecting patient privacy and/or in accordance with government regulation and/or privacy laws. For example, medical data 562.1 - 562.M, patient data 564.1 - 564.P, clinical trial data 566.1 - 566.P is stored by, accessed by, and/or used by medical data processing system 500 in compliance with: the Health Insurance Portability and Accountability Act (HIPPA); the Standards for Privacy of Individually Identifiable Health Information and/or other requirements issued by the Department of Health and Human Services (HHS); Protection of Human Subjects Regulations and/or other requirements issued by the Food and Drug Administration (FDA); and/or other governmental-based/agency-based/company-based requirements regarding privacy and/or ethical conduct in handing patient information, in administering drugs and/or treatment, and/or in conducting clinical trials. As another example, some or all medical data 562.1 - 562.M, patient data 564.1 - 564.P, clinical trial data 566.1 - 566.P, such as protected health information (PHI) for one or more corresponding individuals, is de-identified and/or identifying markers/information that can map underlying data to a corresponding patient is removed, for example, by the medical data processing system 500 via execution of a corresponding function and/or via another entity, for example, prior to receipt of the medical data by the medical data processing system 500.

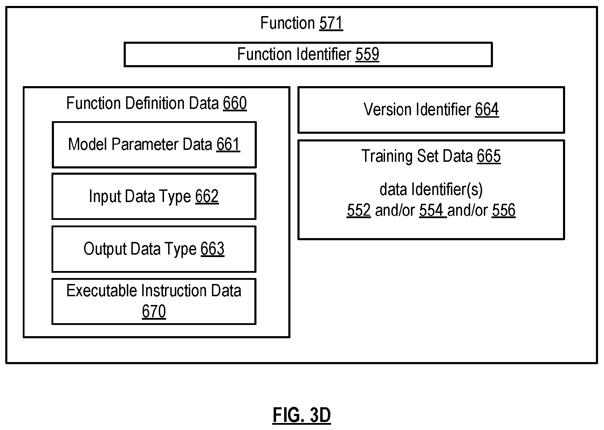

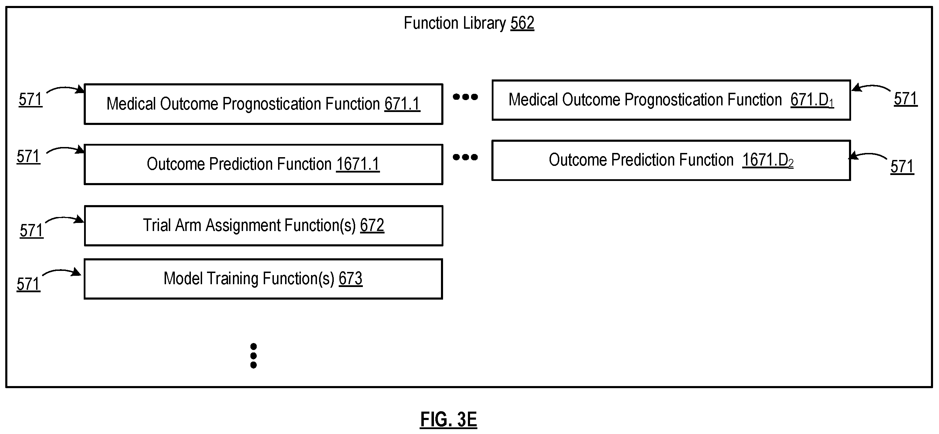

The function library 572 can include a plurality of function entries 571. The function library 572 can be implemented via any one or more memory devices accessible by the medical data processing system 500 that is operable to store and/or access the plurality of function entries 571. Example embodiments of function entries included in function library 572 and/or that are otherwise executed by medical data processing system 500 are discussed in conjunction with Figures 3D and 3E.

Some or all function entries 571 of the function library 572 can be: predetermined; configured via user input by an administrator of the medical data processing system 500; stored in and retrieved from memory accessible by the medical data processing system 500; received by the medical data processing system 500; automatically generated, trained, and/or updated by the medical data processing system 500; and/or otherwise determined by medical data processing system 500.

Each function entry can include information and/or instructions utilized to perform a corresponding function. Some or all functions described herein can be stored in function library 572 and/or can be executed in accordance with the information and/or instructions of a corresponding function entry 571.

A function entry 571 can correspond to a function that can be executed by the medical data processing system 500 to perform functionality of the medical data processing system 500. For example, the medical data processing system 500 performs a given function based on accessing and/or executing information and/or instructions stored in and/or indicated by the corresponding a function entry 571. Alternatively or in addition, the medical data processing system 500 executes operational instruction stored in memory module 510 that cause the medical data processing system 500 to execute a given function that corresponds to a function entry 571.

Alternatively or in addition, a function entry 571 can correspond to a function that can be executed by another external computing device, such as computing resources implemented via a data source 575 and/or computing resources implemented via a requesting entity 576. For example, a computing device implemented via data source 575 and/or requesting entity 576 performs a given function based on receiving information and/or instructions stored in and/or indicated by the corresponding function entry 571, and/or based on storing and/or executing the received function entry 571. Alternatively or in addition, the computing device implemented via data source 575 and/or requesting entity 576 can receive application data from the medical data processing system 500 via the network 550 that includes information and/or instructions corresponding to one or more function entries 571, the corresponding computing device can client can store this application data via its memory resources, and/or the corresponding computing device can perform one or more corresponding functions based on accessing and/or executing this application data.

The plurality of subsystems 501 can be utilized to implement different ones of the various functionality of the medical data processing system 500 described herein. Each of the plurality of subsystems 501 can perform some or all of its functionality based on accessing and/or communicating with: other subsystems 501; the medical data storage 561; the patient data storage 563; the clinical trial data storage 565; and/or the function library 572. In some embodiments, different subsystems 501 each access and/or maintain their own medical data storage 561; patient data storage 563; clinical trial data storage 565; and/or the function library 572.

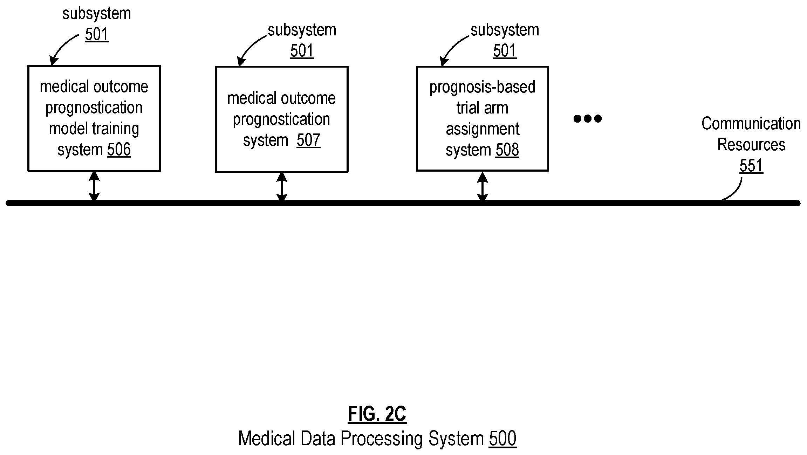

Figure 2C illustrates an example plurality of subsystems 501 implemented by medical data processing system 500. Some embodiments of the medical data processing system 500. can implement all of the set of subsystems 501 of Figure 2C. Some embodiments of the medical data processing system 500 implement only a proper subset of the set of subsystems 101 of Figure 2C. Some embodiments of the medical data processing system 500 implement additional subsystems 501 not illustrated in Figure 2C.

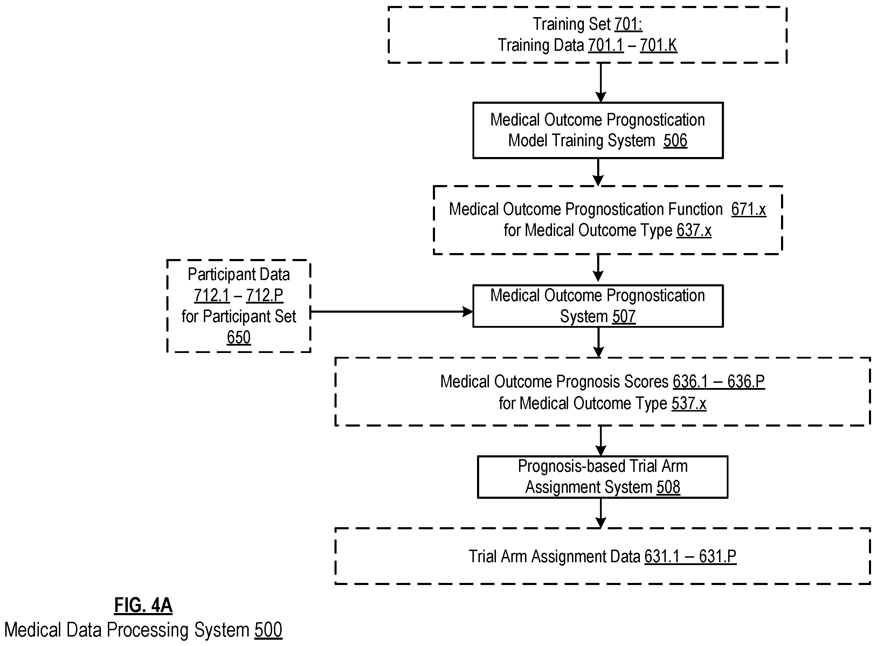

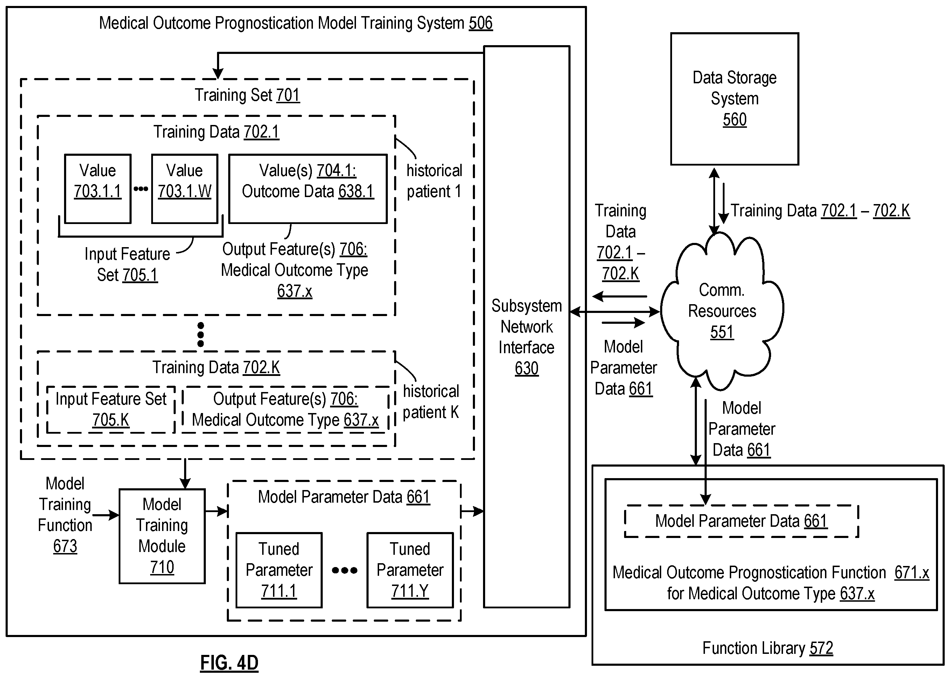

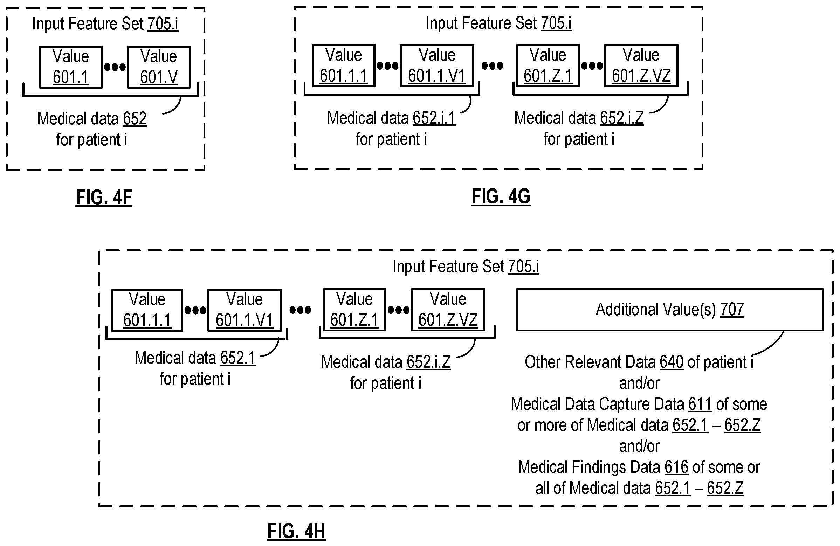

The plurality of subsystems implemented by the medical data processing system 500 can include a medical outcome prognostication model training system 506. Embodiments of medical outcome prognostication model training system 506 are discussed in further detail herein, for example, in conjunction with Figures 4A, 4D, and 4F- 4G.

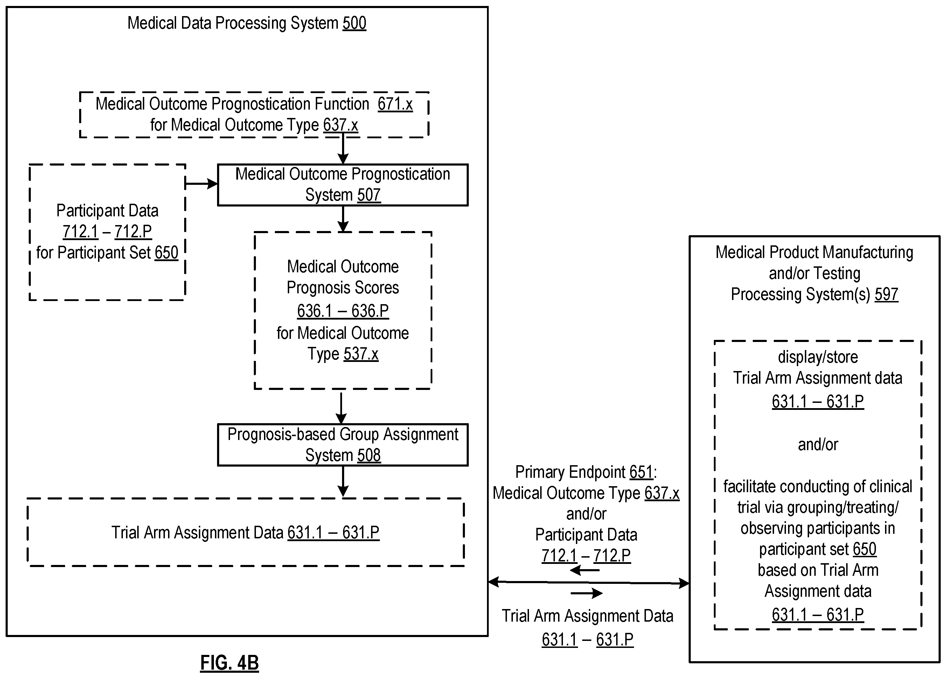

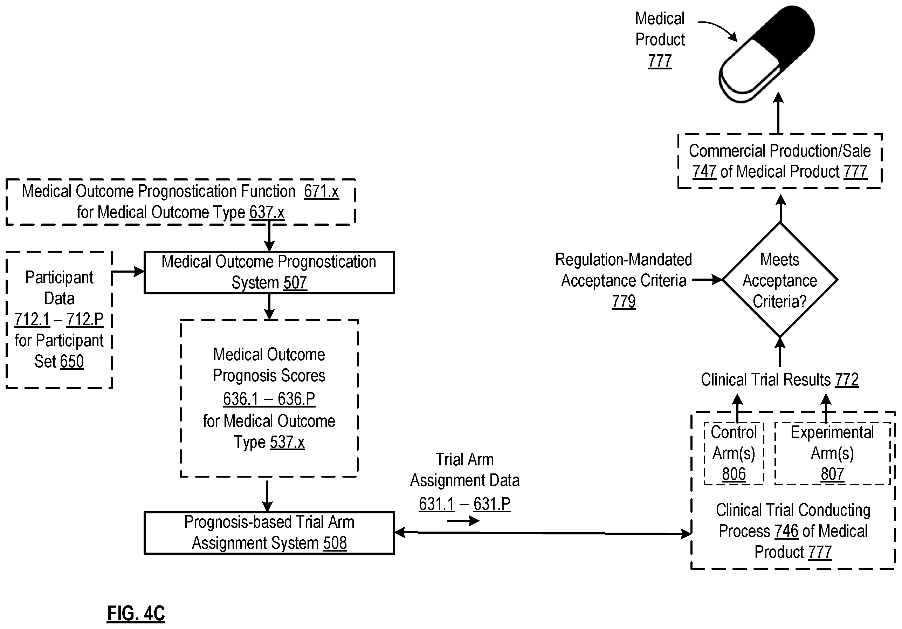

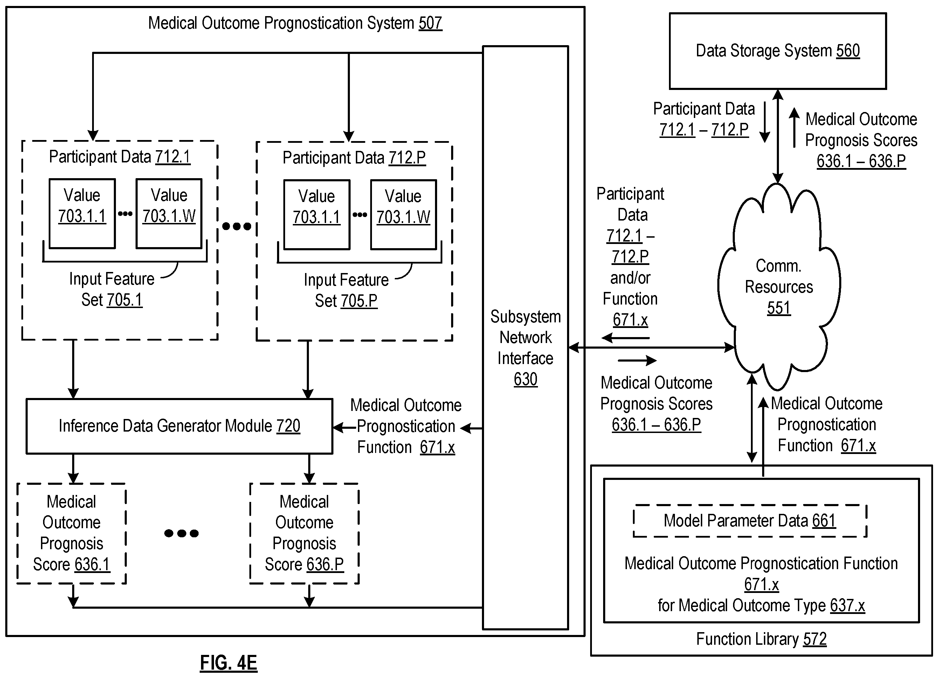

The plurality of subsystems implemented by the medical data processing system 500 can alternatively or additionally include a medical outcome prognostication system 507. Embodiments of medical outcome prognostication system 507 are discussed in further detail in conjunction with Figures 4A, 4B, 4C, and 4E - 4H.

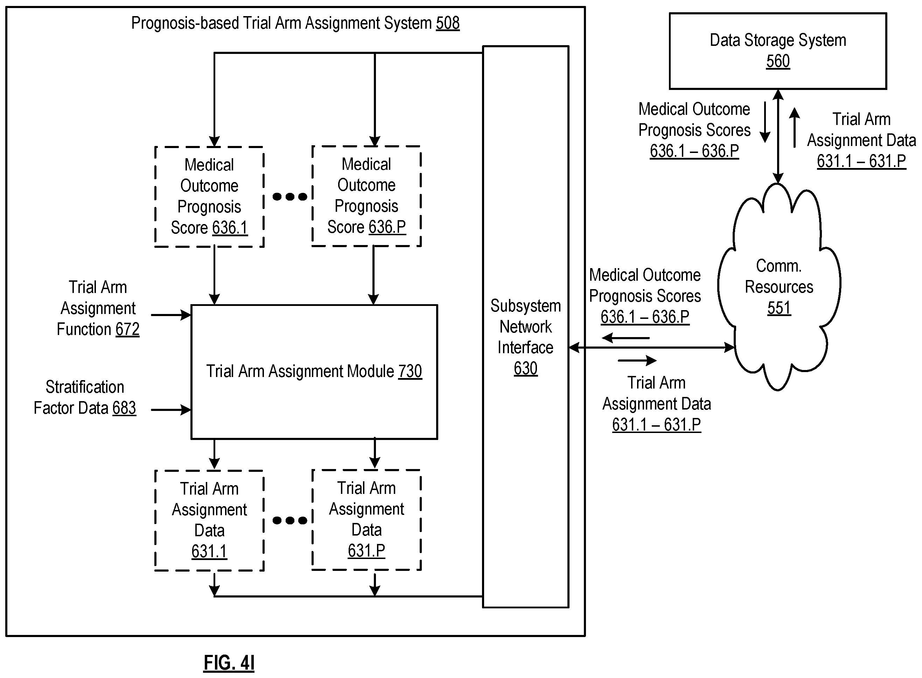

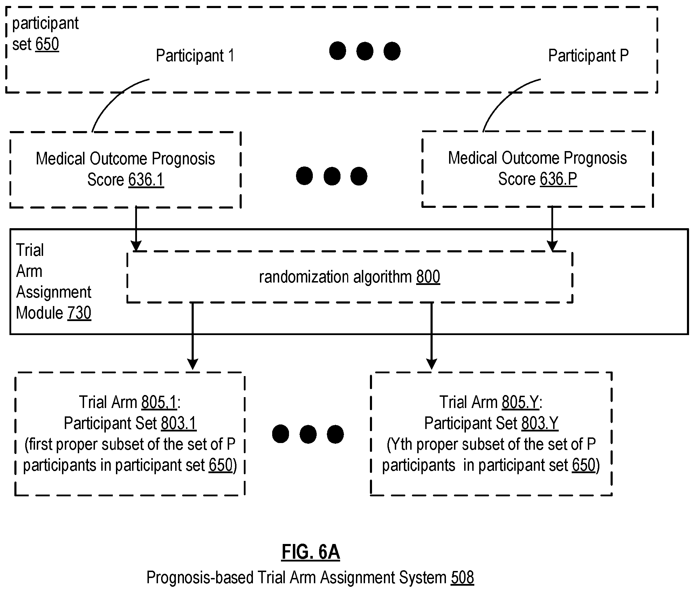

The plurality of subsystems implemented by the medical data processing system 500 can alternatively or additionally include a prognosis-based trial arm assignment system 508. Embodiments of prognosis-based trial arm assignment system 508 are discussed in further detail in conjunction with Figures 4A, 4B, 4C, 4I, and 6A - 6K.

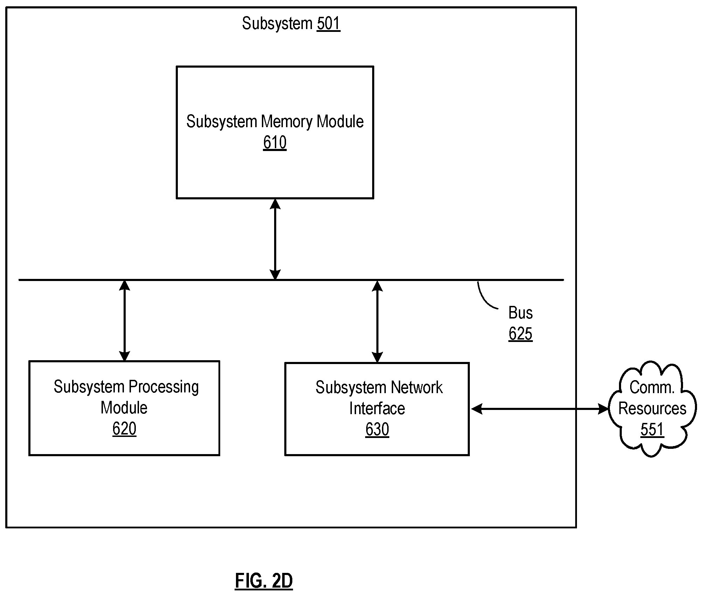

As illustrated in Figure 2D, a given subsystem 501 can be implemented via: one or more subsystem processing modules 620; one or more subsystem memory modules 610; and/or one or more subsystem network interfaces 630, communicating via bus 625. The subsystem memory module 610 can include memory that stores operational instructions that, when executed by the one or more subsystem processing modules 620, cause the corresponding subsystem 501 to execute some or all of the functionality described herein. The one or more network interfaces 630 can be operable to send and/or receive data via the network 550 and/or via any other communication system. Bus 625 can facilitate communication of data between the: one or more subsystem processing modules 460; one or more subsystem memory modules 610; and/or one or more subsystem network interfaces 630 via one or more wired and/or wireless communication resources. Bus 625 of a given subsystem 501 can be implemented by, or can be distinct from, bus 525.

The one or more subsystem processing modules 620 of a given subsystem 501 can be implemented by, or can be distinct from, the one or more processing modules 520 of the medical data processing system 500. The one or more subsystem memory modules 610 of a given subsystem 501 can be implemented by, or can be distinct from, the one or more memory modules 510 of the medical data processing system 500. The one or more subsystem network interfaces 630 of a given subsystem 501 can be implemented by, or can be distinct from, the one or more network interfaces 530 of the medical data processing system 500.

Different subsystems 501 can be implemented via shared resources and/or via distinct resources. For example, a first subsystem 501 is implemented via a first subsystem processing module 620; a first subsystem memory module 610; a first subsystem network interface 430; and and/or a first bus 625, while a second subsystem 501 is implemented via a second subsystem processing module 620; a second subsystem memory module 610; a second subsystem network interface 630; and/or a second bus 625. The first subsystem processing module 620 can have shared processing resources with, or can be entirely distinct from, the second subsystem processing module 620. The first subsystem memory module 620 can have shared memory resources with, or can be entirely distinct from, the second subsystem processing module 620. The first subsystem network interface 630 can have shared network interface resources with, or can be entirely distinct from, the second subsystem network interface 630. The first bus 625 can have shared communication resources with, or can be entirely distinct from, the second bus 625.

As a particular example, multiple subsystems 501 can optionally be implemented via shared resources based on their functionality being implemented in tandem. Alternatively or in addition, one or more different subsystems 501 can optionally be implemented via separate resources, such as separate devices and/or server systems, based on their functionality being implemented separately.

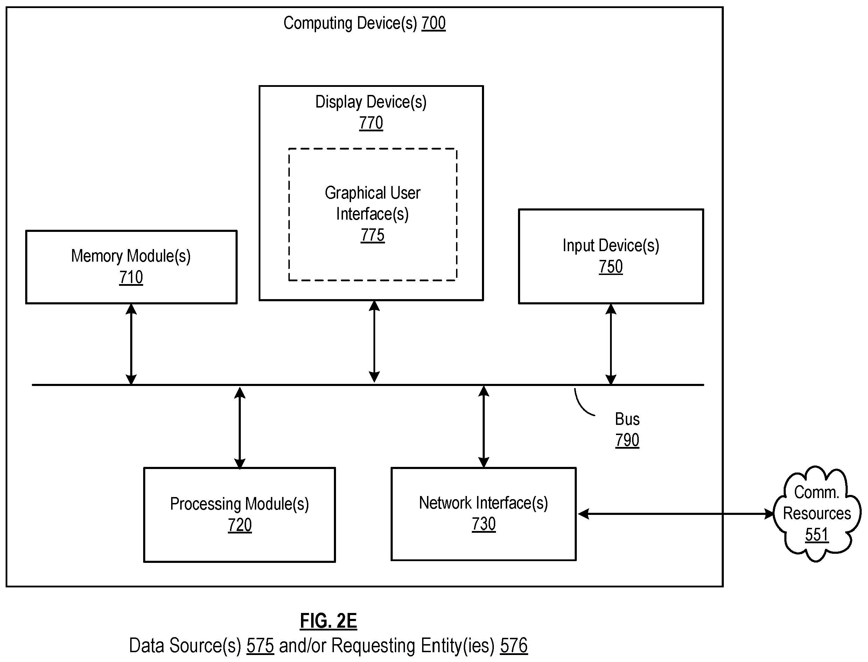

As illustrated in Figure 2E, one or more computing devices 700 implementing requesting entities 576 and/or data sources 575 can be implemented via: one or more processing modules 720; one or more memory modules 710; one or more network interfaces 730; one or more display devices 770; and/or one or more input devices 750, communicating via bus 790. The one or more network interfaces 730 can be operable to send and/or receive data via the network 550 and/or via any other communication system. Bus 790 can facilitate communication of data between the: one or more processing modules 720; one or more memory modules 710; one or more network interfaces 730; one or more display devices 770; and/or one or more input devices 750. One or more computing devices 700 can be implemented as: a computer, a laptop computer, a desktop computer, a mobile device, a cellular phone, a tablet, a smart device, a wearable device, a server system, (e.g. associated with a hospital, pharmaceutical company, research institution, healthcare system, etc.), a medical device and/or medical equipment, and/or any other one or more computing devices.

The display device 770 can be operable to display one or more views of a graphical user interface 375. Graphical user interface 775 can be implemented as an interactive user interface, can present prompts and/or information, can facilitate user selection and/or user entering of text and/or uploading of files corresponding to medical information; can facilitate displaying of function output or other data generated by medical data processing system 500; etc. For example, display device 770 can be implemented via at least one touchscreen, at least one monitor, at least one screen, and/or at least one other display device. In some embodiments, computing device 700 is implemented to convey some or all information and/or prompts as audio data, for example, via at least one speaker of the client device 700.

The input device 750 can be operable to collect user input, for example, in response to one or more prompts presented via graphical user interface 775. For example, input device 750 can be implemented via at least one keyboard, at least one touchscreen, at least one mouse, at least one knob or button, at least one microphone, at least one camera, at least one interface to one or more memory drives storing document files, and/or at least one other input device that collects data utilized as user input, such as any form of medical data described herein.

The memory module 710 can include memory that stores operational instructions that, when executed by the one or more client processing modules 720, cause the corresponding computing device 700 to execute some or all of the functionality described herein. For example, the operational instructions, when executed by the one or more client processing modules 720, can cause the one or more processing modules 720 to utilize network interface 730 to receive data from the medical data processing system 500 via network 550. As another example, the operational instructions, when executed by the one or more processing modules 720, can cause the corresponding display device 770 to display information and/or prompts via graphical user interface 775 in one or more views, for example, based on instructions, information and/or prompts included in the data received from the medical data processing system 500 (e.g. output of a function executed by medical data processing system 500; prompts with which a user should interact; etc.). As another example, the operational instructions, when executed by the one or more processing modules 720, can cause the one or more processing modules 720 to generate data based on user input to input device 750, for example, as medical data generated based on entered user text/selections, and/or uploaded files in response to a corresponding prompt displayed via interactive user interface 775. As another example, the operational instructions, when executed by the one or more processing modules 720, can cause the one or more processing modules 720 to utilize network interface 730 to send data generated by input device 750, for example, to the medical data processing system 500 via network 550.

The memory module 710 can further include memory that stores medical data 562, patient data 564, and/or clinical trial data 566. For example, this medical data 562, patient data 564, and/or clinical trial data 566 is uploaded and sent to medical data processing system 500 for processing in accordance with features and/or functionality of medical data processing system 500 described herein, for example, when computing device is implementing some or all of data sources 575. Alternatively or in addition, this medical data 562, patient data 564, and/or clinical trial data 566 was received from medical data processing system 500 based on being generated/modified by medical data processing system 500 in accordance with features and/or functionality of medical data processing system 500, for example, when computing device is implementing some or all of requesting entities 576.

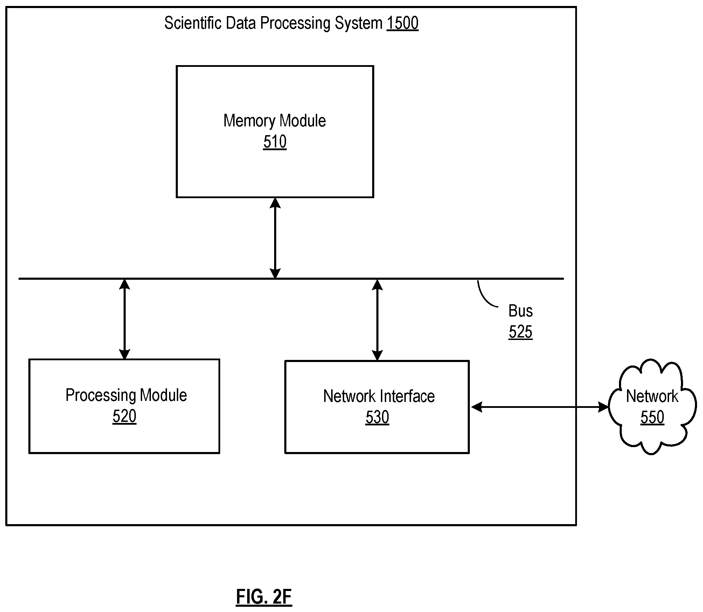

Figures 2F - 2H present embodiments of a scientific data processing system 1500. Some or all features and/or functionality of scientific data processing system 1500 of Figures 2F - 2H can implement any embodiment of scientific data processing system 1500 and/or medical data processing system 300 described herein.

As illustrated in Figure 2F, the scientific data processing system 1500 can be implemented via one or more processing modules 520, one or more memory modules 510, and/or one or more network interfaces 530, communicating via a bus 525. The one or more network interfaces 530 can be operable to send and/or receive data via the network 550 and/or via any other communication system. Bus 525 can facilitate communication of data between the one or more processing modules 520, one or more memory module 510, and/or one or more network interfaces 530 via one or more wired and/or wireless communication resources. Some or all features and/or functionality of processing modules 520. memory modules 510, and/or network interfaces 530 of Figure 2F can implement scientific data processing system 1500 in a same of similar fashion as processing modules 520 memory modules 510, and/or network interfaces 530 of Figure 2A implementing medical data processing system 500. For example, the processing modules 520. memory modules 510, and/or network interfaces 530 of Figure 2F can implement any functionality of scientific data processing system 1500 described herein, such as any functionality of medical data processing system 500 described herein applied to any scientific data/setting, even for non-medical and/or non-healthcare scientific applications.

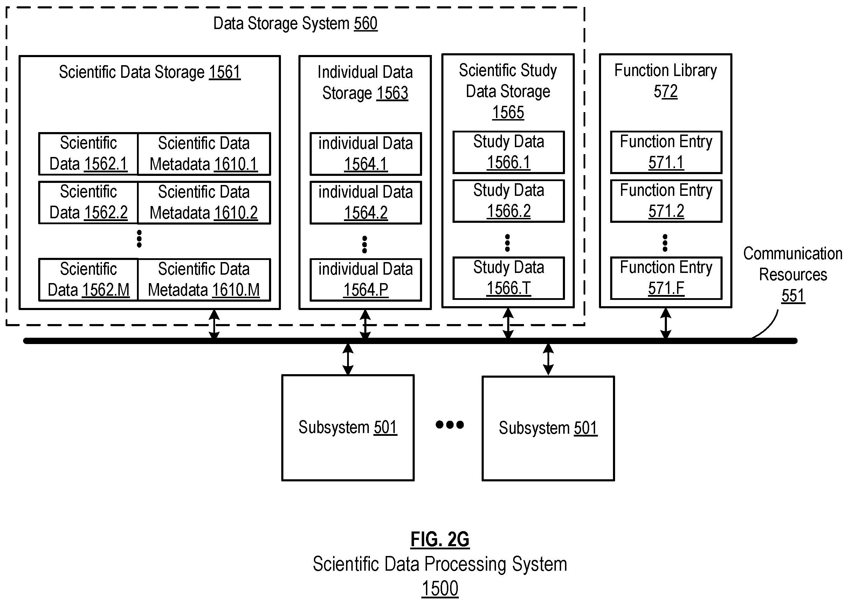

As illustrated in Figure 2G, the scientific data processing system 1500 can include and/or can communicate with: a data storage system 560; a function library 572; and/or one or more subsystems 501, all communicating via communication resources 551. The data storage system 560 of Figure 2G can be implemented in a same or similar or similar fashion as the data storage system of Figure 2B. The data storage system 560 can be implemented to include one or more scientific data storages 1561; one or more individual data storages 1563; and/or one or more scientific study data storages 1565, for example, via implementing memory resources of memory module 510. Communication resources 551 can facilitate communication of data between the one or more scientific data storages 1561; the one or more individual data storages 1563; the one or more scientific study data storages 1565; the one or more function libraries 572; and/or the one or more subsystems 501 via one or more wired and/or wireless communication resources (e.g. via some or all communication resources implemented by bus 525). Communication resources 551 can correspond strictly to internal communication resources of scientific data processing system 1500 and/or can be implemented via some or all communication resources of network 550.

The one or more scientific data storage 1561 can store a plurality of scientific data 1562.1 -1562.M and/or a corresponding plurality of scientific data metadata 1610.1 - 1610.M. Each scientific data 1562 can be any scientific data (e.g. scientific measurements, output of a scientific device, image data, etc.) captured for a given individual, where corresponding metadata 1610 includes additional information describing/regarding/generated based on this scientific data 1562. For example, the medical data 562 can be a type of scientific data 1652 corresponding to data captured in a medical/healthcare-based setting. The scientific data 1652 can be implemented in a same or similar fashion as medical data 562 (e.g. can include a set of one or more values 601, for example, corresponding to one or more measurements collected via scientific equipment), where the scientific data 1652 is not necessarily medical in nature, and can optionally correspond to measurements/other data corresponding to any other scientific field (e.g. collected for individuals being studied/observed/treated in any scientific setting).

The one or more individual data storage 1563 can store a plurality of individual data 1564.1 - 1564.P. Each individual data 1564 can include and/or indicate data regarding a given individual (e.g. a person, an animal and/or any subject of a particular scientific procedure, scientific trial, scientific experiment, scientific study, etc.). As used herein, an individual can correspond to any individual for example, for whom one or more types of and/or instances of individual data 1564 has been and/or will be captured, and/or any individual that is a prospective participant/is a confirmed participant of an upcoming scientific study/experiment and/or that is a current/past participant of a current/past scientific experiment. The individual data 1564 can be implemented in a same or similar fashion as patient data 564, where the individual data 1564 is not necessarily describing a patient of a medical/healthcare treatment/study, and can optionally correspond to any other individual undergoing scientific procedures/testing in any scientific field (e.g. participating in any scientific study in any field).

The one or more scientific study data storage 1565 can store a plurality of clinical trial data 1566.1 - 1566.T. Each scientific study data 1566 can include and/or indicate data regarding a given scientific study (e.g. testing a scientific hypothesis, testing effectiveness/outcome of a corresponding procedure, etc.) upon any group of individuals (e.g. a group people, animals, organisms and/or objects observed in at least one control group and at least one investigational group). The scientific study data 1566 can be implemented in a same or similar fashion as clinical trial data 566, where the scientific study data 1566 is not necessarily describing a clinical trial and/or experiment relating to testing of medical/healthcare-related product/procedure, and can optionally correspond to any other scientific experimentation in any scientific field.

Some or all scientific data 1562.1 - 1562.M of scientific data storage 1561, some or all individual data 1564.1 - 1564.P of individual data storage 1563, and/or some or all scientific study data 1566.1 - 1566.T of scientific study data storage 1565 can be received by the scientific data processing system from a corresponding data source 575 that generated, collected, modified, stores, received, and/or sent the corresponding scientific data 1562, individual data 1562, and/or scientific study data 1566 (e.g. a corresponding scientific entity studying science and/or conducting scientific experiments such as a university, research institution, company manufacturing products/procedures undergoing scientific testing, devices/equipment collecting corresponding measurements of individuals as scientific data, etc.). Alternatively or in addition, some or all scientific data 1562.1 - 1562.M of scientific data storage 1561, some or all individual data 1564.1 - 1564.P of individual data storage 1563, and/or some or all scientific study data 1566.1 - 1566.T of scientific study data storage 1565 can be generated by the scientific data processing system itself.

The one or more scientific data storages 1561, individual data storages 1563, and/or scientific experiment data storages 1565 can be implemented as one or more relational and/or non-relational databases, one or more file storage systems, one or more object storage systems, and/or can be implemented via any one or more memory devices accessible by the scientific data processing system 1500 that is operable to store and/or access the plurality of scientific data 1562.1 - 1562.M, individual data 1564.1 - 1564.P, and/or scientific study data 1566.1 - 1566.T. The corresponding scientific data 1562.1 - 1562.M, individual data 1564.1 - 1564.P, and/or scientific study data 1566.1 - 1566.T can otherwise be accessed and/or determined by the scientific data processing system 1500 to enable the scientific data processing system 1500 to process and/or generate some or all of the corresponding data accordingly in performing some or all functionality described herein.

Alternatively or in addition, some or all scientific data 1562.1 - 1562.M, some or all individual data 1564.1 - 5164.P, some or all scientific study data 1566.1 - 1566.P, and/or some or all function entries 571 are stored externally (e.g. by a particular third party company and/or other entity, such as a particular university, a particular research institution, a particular company, a particular individual,, a particular company that collects and/or stores scientific information or other information for various individuals, etc.), for example, via a corresponding data source 575, requesting entity 576, and/or other computing device, where scientific data 1562 is processed based on being accessed via communications with a corresponding one or more external entities.

The function library 572 of Figure 2G can be implemented in a same or similar fashion as the function library 572 of Figure 2B, and can similarly include a plurality of function entries 571. The function library 572 can be implemented via any one or more memory devices accessible by the scientific data processing system 1500 that is operable to store and/or access the plurality of function entries 571. Some or all features and/or functionality of function library 572 of Figure 2G can be implemented via the function library 572 of Figure 2B and/or any other embodiment of function library 572 described herein. Some or all function entries 571 of function library 572 of scientific data processing system 1500 can be the same as or similar to function entries 571 of function library 572 of medical data processing system 500. For example, function entries 571 of function library 572 of medical data processing system 500 (and/or any functions performed by medical data processing system 500) can be trained/executed in a similar fashion, where the corresponding functionality is extended/adapted to processing any scientific data, individual data, and/or scientific study data of any scientific field rather than data specific to the medical field/healthcare industry.

The plurality of subsystems 501 of Figure 2G can be implemented in a same or similar fashion as the plurality of subsystems 501 of Figure 2B, and/or can otherwise be utilized to implement different ones of the various functionality of the scientific data processing system 1500 described herein. Each of the plurality of subsystems 501 can perform some or all of its functionality based on accessing and/or communicating with: other subsystems 501; the scientific data storage 1561; the individual data storage 1563; the scientific study data storage 1565; and/or the function library 572. In some embodiments, different subsystems 501 each access and/or maintain their own scientific data storage 1561; individual data storage 1563; scientific study data storage 1565; and/or the function library 572.

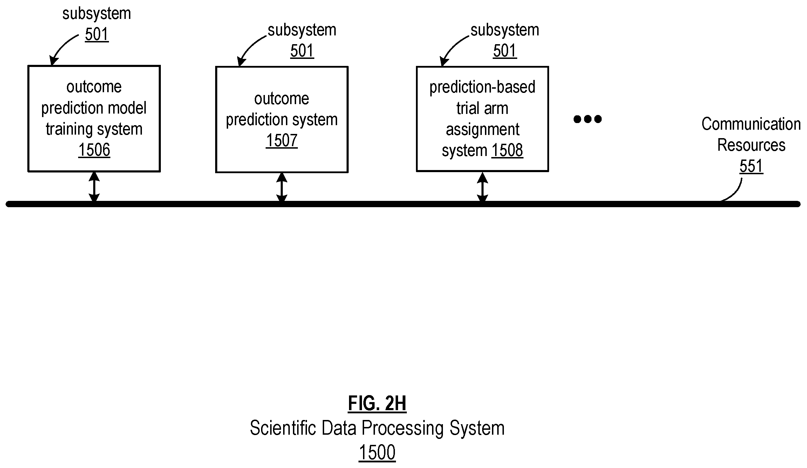

Figure 2H illustrates an example plurality of subsystems 501 implemented by scientific data processing system 1500. Some embodiments of the scientific data processing system 1500 can implement all of the set of subsystems 501 of Figure 2H. Some embodiments of the scientific data processing system 1500 implement only a proper subset of the set of subsystems 101 of Figure 2H. Some embodiments of the scientific data processing system 500 implement additional subsystems 501 not illustrated in Figure 2H.

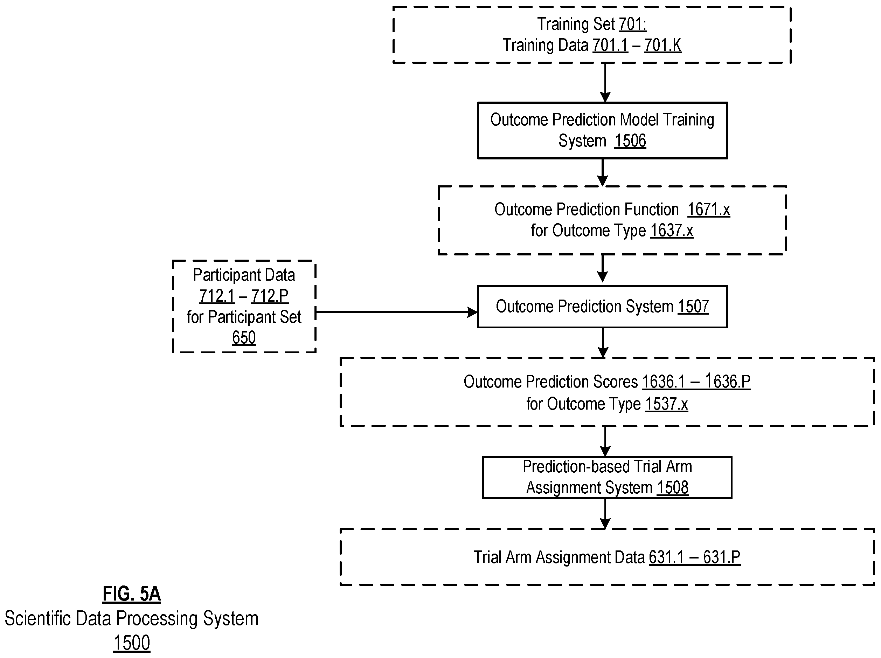

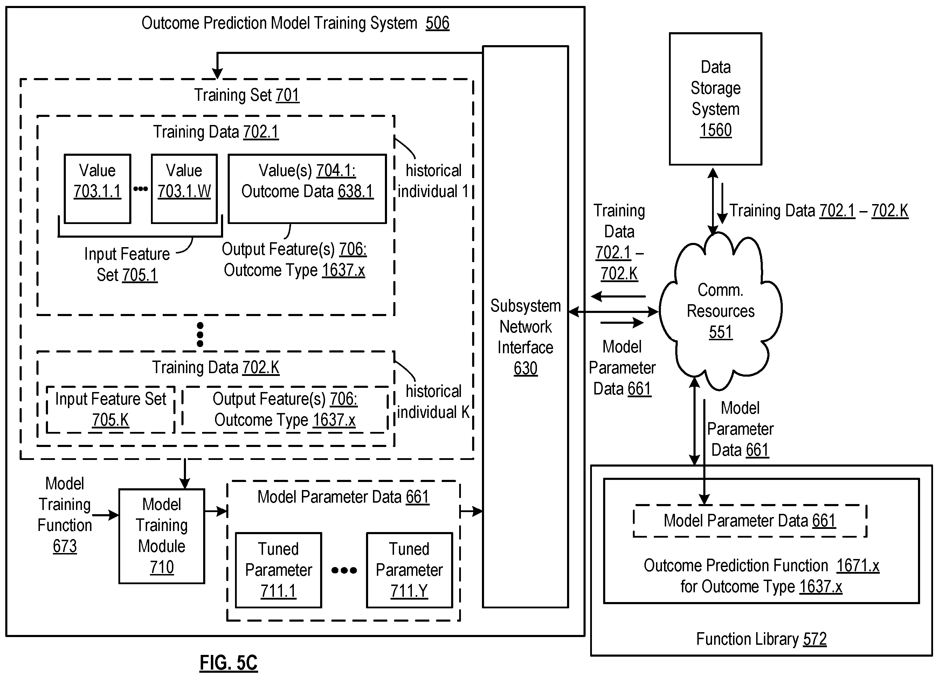

The plurality of subsystems implemented by the scientific data processing system 500 can include an outcome prediction model training system 1506. Embodiments of outcome prediction model training system 1506 are discussed in further detail in conjunction with Figures 5A and 5C - 5F. The outcome prediction model training system 1506 of scientific data processing system 1500 can be implemented in a same or similar fashion as the medical outcome prognostication model training system 506 of medical data processing system 500. For example, the medical outcome prognostication model training system 506 is a type of outcome prediction model training system 1506 that trains models to predict medical outcomes, while other outcome prediction model training systems 1506 can be implemented to train models to predict any other type of outcome (e.g. any primary endpoint of any scientific study).

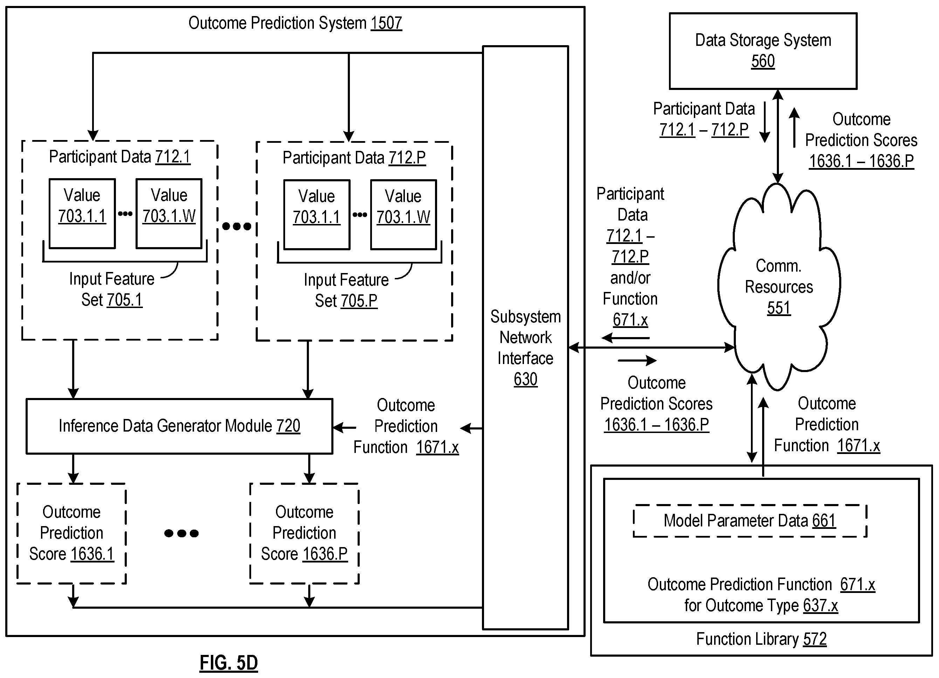

The plurality of subsystems implemented by the scientific data processing system 500 can alternatively or additionally include an outcome prediction system 1507. Embodiments of outcome prediction system 1507 are discussed in further detail in conjunction with Figures 5A and 5D - 5G. The outcome prediction system 1507 of scientific data processing system 1500 can be implemented in a same or similar fashion as the medical outcome prognostication system 507 of medical data processing system 500. For example, the medical outcome prognostication system 507 is a type of outcome prediction system 1507 that predicts medical outcomes for patients based on processing corresponding medical data, while other outcome prediction model training systems 1507 can be implemented to predict any other type of outcome for individuals (e.g. any primary endpoint of any scientific study) based on processing corresponding scientific data.

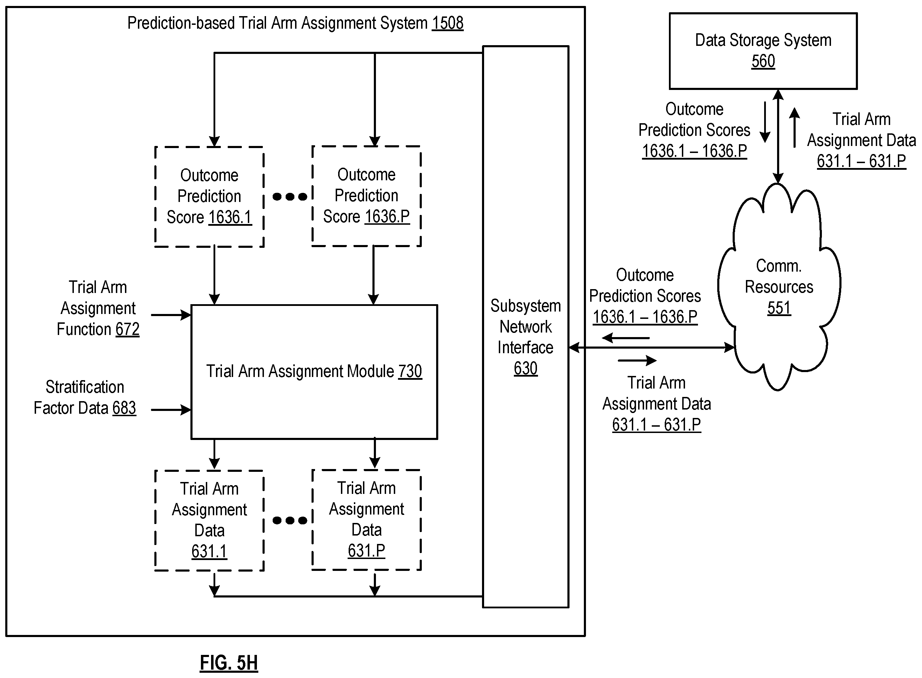

The plurality of subsystems implemented by the scientific data processing system 1500 can alternatively or additionally include a prediction-based trial arm assignment system 1508. Embodiments of prediction-based trial arm assignment system 1508 are discussed in further detail in conjunction with Figures 5A, 5D - 5F and 5H. The prediction-based trial arm assignment system 1508 of scientific data processing system 1500 can be implemented in a same or similar fashion as the prognosis-based medical outcome prognostication system 508 of medical data processing system 500. For example, the prognosis-based medical outcome prognostication system 508 is a type of prediction-based trial arm assignment system 1508 that groups clinical trial participants based on corresponding medical outcome prognosis scores, while other prediction-based trial arm assignment system 1508 can be implemented to groups individuals of any scientific study based on corresponding outcome prediction scores (e.g. predicting the primary endpoint of the scientific study).

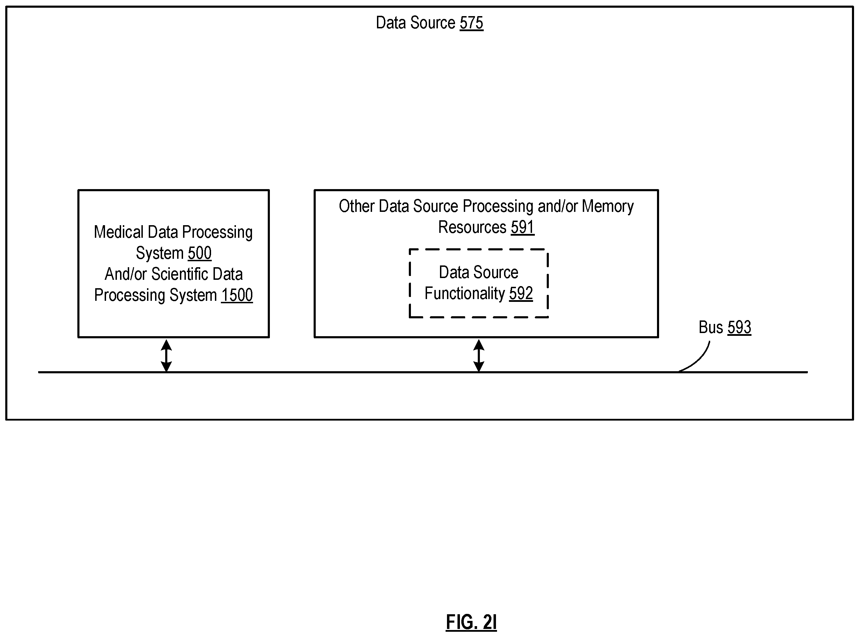

Figure 2I illustrates an embodiment of a data source 575 that implements medical data processing system 500 and/or scientific data processing system 1500. For example data source 575 (e.g. one or more corresponding computing devices 700) implements medical data processing system 500 and/or scientific data processing system 1500 and/or otherwise performs some or all features and/or functionality of medical data processing system 500 and/or scientific data processing system 500 (e.g. in conjunction with executing corresponding application data that was received/downloaded/installed, or otherwise being implemented to performing corresponding features and/or functionality upon the collected data) alternatively or in addition to performing other data source functionality 592 (e.g. collecting/storing corresponding data for processing) via other data source processing and/or memory resources 591, which can be shared with and/or separate from processing and/or memory resources implementing the medical data processing system 500 and/or scientific data processing system 1500, and/or can communicate with processing and/or memory resources implementing the medical data processing system 500 and/or scientific data processing system 1500 via a corresponding bus 593.

In such embodiments, a data source optionally performs some or all features and/or functionality of medical data processing system 500 and/or scientific data processing system 1500 upon its own data (e.g. its own collected medical data or other data), for example, instead of sending this data to a separate medical data processing system 500 and/or scientific data processing system 1500 as illustrated in Figures 1A and 1C. Some or all embodiments of medical data processing system 500 and/or scientific data processing system 1500 can be implemented via a corresponding data source 575, for example, instead of or in addition to communicating with one or more data sources 575.

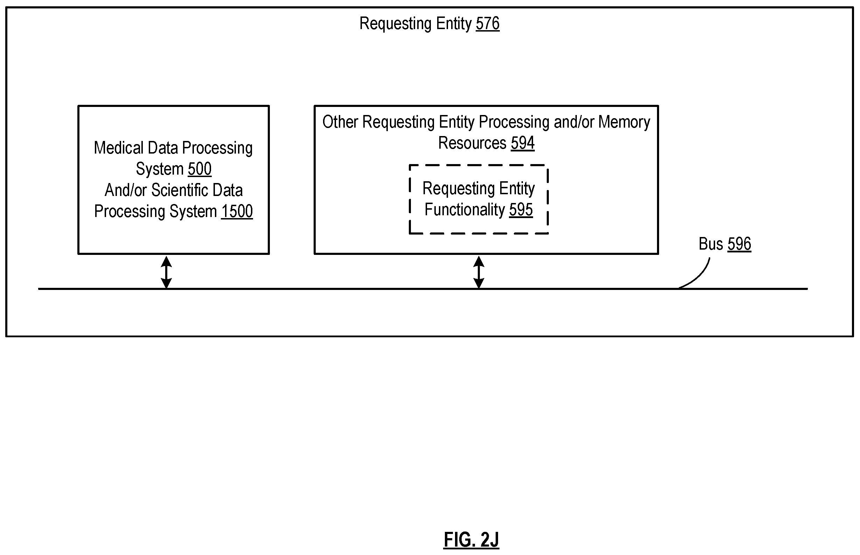

Figure 2J illustrates an embodiment of a requesting entity 576 that implements medical data processing system 500 and/or scientific data processing system 1500. For example requesting entity 576 (e.g. one or more corresponding computing devices 700) implements medical data processing system 500 and/or scientific data processing system 1500 and/or otherwise performs some or all features and/or functionality of medical data processing system 500 and/or scientific data processing system 500 (e.g. in conjunction with executing corresponding application data that was received/downloaded/installed, or otherwise being implemented to performing corresponding features and/or functionality as requested by the requesting entity and/or for use by the requesting entity) alternatively or in addition to performing other requesting functionality 595 (e.g. conducting clinical trials/scientific studies, etc.) via other requesting entity processing and/or memory resources 594, which can be shared with and/or separate from processing and/or memory resources implementing the medical data processing system 500 and/or scientific data processing system 1500, and/or can communicate with processing and/or memory resources implementing the medical data processing system 500 and/or scientific data processing system 1500 via a corresponding bus 596

In such embodiments, a requesting entity 576 optionally performs some or all features and/or functionality of medical data processing system 500 and/or scientific data processing system 1500 itself (e.g. generates its own requested/desired), for example, instead of receiving this data from a separate medical data processing system 500 and/or scientific data processing system 1500 as illustrated in Figures 1A and 1C. Some or all embodiments of medical data processing system 500 and/or scientific data processing system 1500 can be implemented via a corresponding requesting entity 576, for example, instead of or in addition to communicating with one or more requesting entities 576.

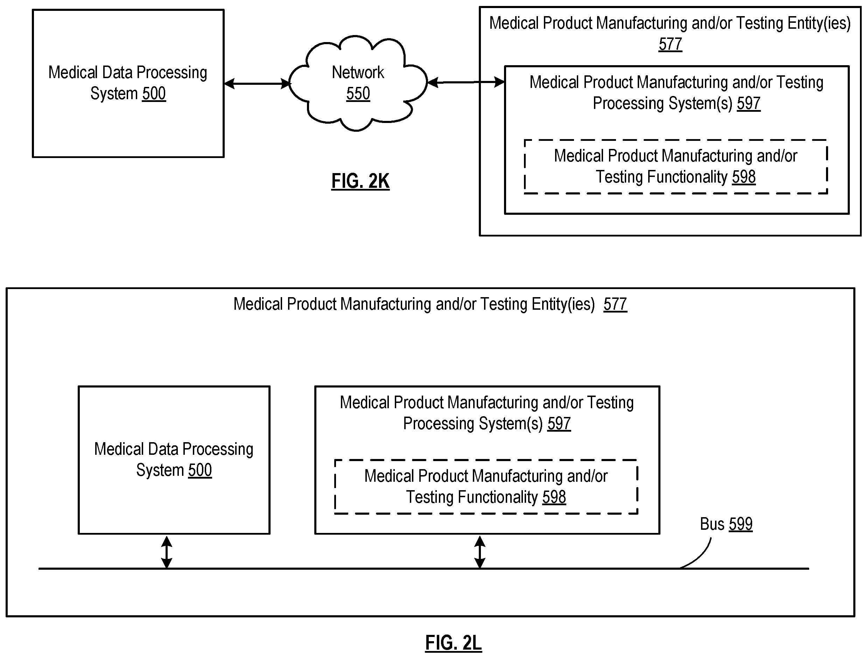

Figures 2K and 2L illustrate embodiments of one or more medical product manufacturing and/or testing entities 577 that communicate with and/or implement medical data processing system 500, for example, in conjunction with performing corresponding medical product manufacturing and/or testing system functionality 598 (e.g. in conjunction with facilitating conducting of clinical trials upon corresponding patients to test a medical product, such as a corresponding pharmaceutical to enable the medical product to ultimately be manufactured for commercial use) via processing and/or memory resources of one or more corresponding medical product manufacturing and/or testing processing systems 597. The one or more medical product manufacturing and/or testing entities 577 can be implemented as a particular type of data source 575 and/or a particular type of requesting entity 576, and/or can otherwise be implemented via one or more computing devices 700. For example, a medical product manufacturing and/or testing entity 577 corresponds to a pharmaceutical company, company producing medical products such as pharmaceutical compounds (e.g. medications, drugs, etc.), medical devices, products developed to treat medical conditions, products developed to improve healthcare, company/institution that tests such products via conducting/assisting in conducting of corresponding clinical trials, etc.

As illustrated in the embodiment of Figure 2K, the one or more medical product manufacturing and/or testing entities 577 can be separate from medical data processing system 500, and/or can otherwise communicate with medical data processing system 500 via network 550 (e.g. receive corresponding trial arm assignment data assigning patients to clinical trial arms in conjunction with testing the corresponding medical product, etc.).

As illustrated in the embodiment of Figure 2L, the one or more medical product manufacturing and/or testing entities 577 can alternatively or additionally implement medical data processing system 500 (e.g. can execute corresponding application data corresponding to medical data processing system 500 and/or otherwise perform some or all features and/or functionality of medical data processing system 500 themselves), where the medical data processing system 500 communicates with/is implemented in conjunction with one or more medical data manufacturing and/or testing processing systems 597, for example, via bus 599.

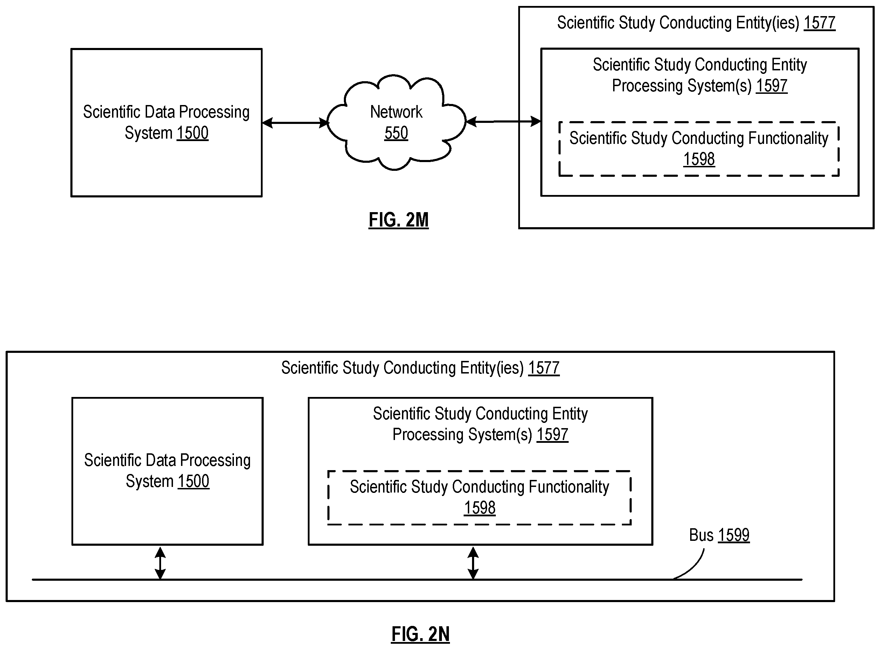

Figures 2M and 2N illustrate embodiments of one or more scientific study conducting entities 1577 manufacturing and/or testing entities 577 that communicate with and/or implement scientific data processing system 1500, for example, in conjunction with performing corresponding scientific study conducting functionality 1598 (e.g. in conjunction with facilitating conducting of scientific studies and/or testing a scientific hypothesis, process, and/or product upon corresponding individuals) via processing and/or memory resources of one or more corresponding scientific study conducting entity processing systems 1597. The one or more scientific study conducting entities 1577 can be implemented as a particular type of data source 575 and/or a particular type of requesting entity 1576, and/or can otherwise be implemented via one or more computing devices 700. For example, a scientific study conducting entity can correspond to any entity that conducts scientific studies (e.g. clinical trials, or optionally non-healthcare/non-medical experiments), such as a university; research institution; hospital system; company testing its own products and/or services via a corresponding scientific study; a third party company testing another company's products and/or services via a corresponding scientific study; a company that administers polling and/or surveys to collect and/or analyze corresponding polling data and/or survey data, for example in accordance with a scientific process; an entity that produces and/or products and/or services in accordance with corresponding governmental regulations and/or other regulatory requirements, for example, as required for commercial production/sale of such products or services; and/or other entity that conducts scientific trials/experiments/studies (e.g. testing a corresponding product, service, and/or any scientific hypothesis, for example, testing and/or relating to: effectiveness of a product or service; safety of a product or service; effectiveness of corresponding commercial impact and/or marketing strategy, for example, in selling a product or service; psychological studies, sociology studies, social-science studies, and/or other studies studying human behavior; user experience studies relating to a corresponding product or service, such as testing of use-ability of a corresponding graphical user interface; predicting and/or studying elections and/or political data via polling data; earth science, environmental science, climate science, astronomical science, and/or physical science related-testing/experiments utilized to better understand/predict natural phenomena; engineering-related testing of a corresponding product/infrastructure design/etc.; simulation-based testing; any testing of a hypothesis via a at least one control group and/or at least one experimental group; any conducting of experiments via application of the scientific method; and/or any other scientific testing/scientific study.

As illustrated in the embodiment of Figure 2M, the one or more scientific study conducting entities 1577 can be separate from scientific data processing system 1500, and/or can otherwise communicate with scientific data processing system 1500 via network 550 (e.g. receive corresponding trial arm assignment data assigning patients to scientific study trial arms in conjunction with conducting the corresponding scientific study, etc.).

As illustrated in the embodiment of Figure 2L, the one or more scientific study conducting entities 1577 can alternatively or additionally implement scientific data processing system 1500 (e.g. can execute corresponding application data corresponding to scientific data processing system 1500 and/or otherwise perform some or all features and/or functionality of scientific data processing system 1500 themselves), where the scientific data processing system 1500 communicates with/is implemented in conjunction with one or more scientific study conducting entity processing systems 1597, for example, via bus 1599.

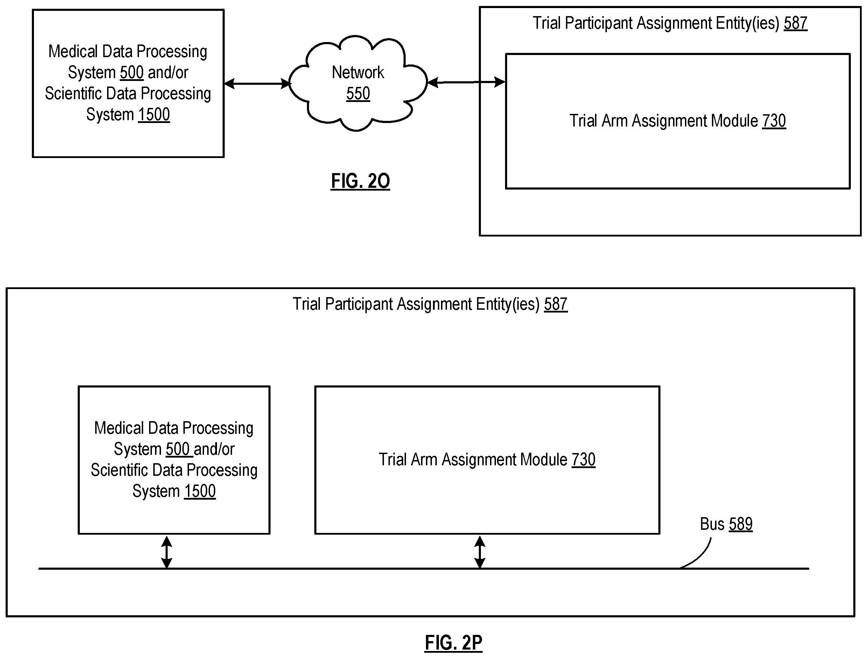

Figures 2O and 2P illustrate embodiments of one or more trial participant assignment entities 587 that communicate with and/or implement scientific data processing system 1500 and/or medical data processing system 500, for example, in conjunction with randomizing trial participants for a corresponding clinical trial and/or scientific study via trial participant assignment entities 587, and/or optionally via human interventions. The one or more trial participant assignment entities 587 can be implemented as a particular type of data source 575 and/or a particular type of requesting entity 1576, and/or can otherwise be implemented via one or more computing devices 700. For example, a trial participant assignment entity can correspond to any entity that identifies and/or randomizes prospective participants of a clinical trial and/or scientific via a human-conducted or automated process (or combination of both), e one or more trial participant assignment entities 587 can perform the corresponding randomization procedure to randomize participants by implementing a trial arm assignment module 730, which can implement some or all features and/or functionality of the trial arm assignment module 730 described herein via corresponding automated processes and/or human intervention that perform functionality of the trial participant assignment entity 587.

As illustrated in the embodiment of Figure 2O, the one or more trial participant assignment entities 587 can be separate from scientific data processing system 1500 and/or medical data processing system 500, and/or can otherwise communicate with scientific data processing system 1500 and/or medical data processing system 500 via network 550 (e.g. the trial participant assignment entity generates trial arm assignment data assigning participants to trial arms in accordance with conducting a randomized process based on outcome prediction scores/medical outcome prognosis scores received from the scientific data processing system 1500 and/or medical data processing system 500, etc.)

As illustrated in the embodiment of Figure 2P, the one or more trial participant assignment entities 587 can alternatively or additionally implement scientific data processing system 1500 and/or medical data processing system 500 (e.g. can execute corresponding application data corresponding to scientific data processing system 1500 and/or medical data processing system 500; and/or otherwise perform some or all features and/or functionality of scientific data processing system 1500 and/or medical data processing system 500 themselves), where the scientific data processing system 1500 and/or medical data processing system 500 communicates with/is implemented in conjunction with one or more trial arm assignment modules 730, for example, via bus 589.

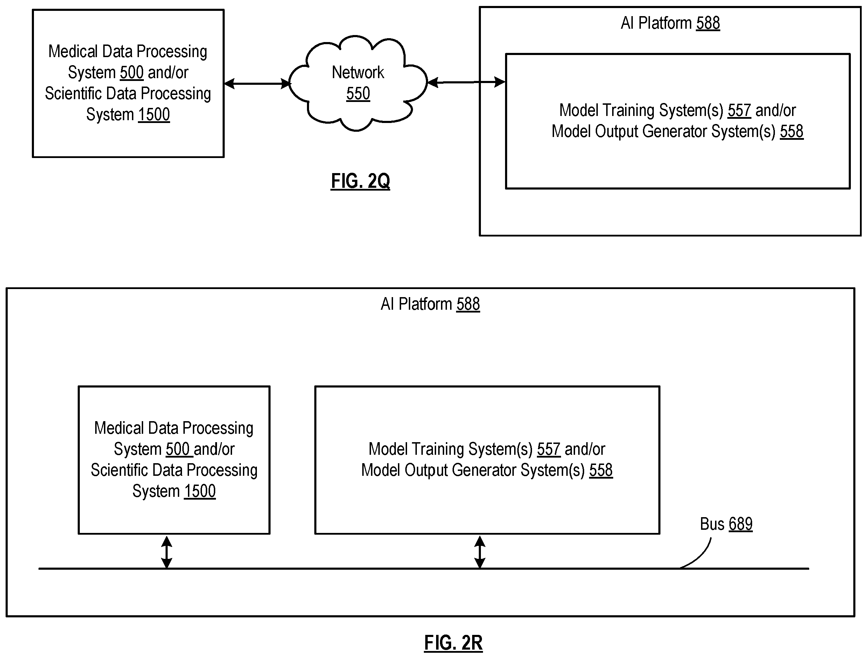

Figures 2Q and 2R illustrate embodiments of one or more AI platforms 588 that communicate with and/or implement scientific data processing system 1500 and/or medical data processing system 500, for example, in conjunction with training and/or applying corresponding AI models implementing some or all features and/or functionality of any machine learning and/or artificial intelligence functionality described herein. The one or more AI platforms 588 can be implemented as a particular type of data source 575 and/or a particular type of requesting entity 1576, and/or can otherwise be implemented via one or more computing devices 700. For example, AI platform 588 can correspond to any entity that trains one or more AI models via one or more corresponding model training systems 557, and/or can correspond to any entity that executes/applies one or more AI models to generate and/or communicate AI model output via one or more model output generator systems 558 (e.g. in response to supplied input). The model training system 557 and/or model output generator system 558 can be implemented via computing resources (e.g. a server system and/or computing devices across one or more physical/geographic location). The corresponding AI platform can correspond to a generative AI platform operable to generate model output via generative AI. The corresponding AI platform can implement any type of artificial intelligence to train models and/or produce corresponding output. The corresponding AI platform can be a third party platform that is optionally separate from the scientific data processing system 1500 and/or the medical data processing system 500.

As illustrated in the embodiment of Figure 2Q, the one or more AI platforms 588 can be separate from scientific data processing system 1500 and/or medical data processing system 500, and/or can otherwise communicate with scientific data processing system 1500 and/or medical data processing system 500 via network 550 (e.g. the AI platform 588 trains some or all models applied by medical data processing system 500 and/or scientific data processing system 1500; the AI platform 588 trains an initial models applied by medical data processing system 500 and/or scientific data processing system 1500 and the medical data processing system 500 and/or scientific data processing system 1500 automatically improves/retrains this initial model; the medical data processing system 500 and/or scientific data processing system 1500 automatically generates an initial model and the AI platform 588 automatically improves/retrains this initial model; the medical data processing system 500 and/or scientific data processing system 1500 receives model data from the AI platform 588 and executes the model accordingly; the medical data processing system 500 and/or scientific data processing system 1500 sends model input to the AI platform 588 and receives model output generated by the AI platform 588 accordingly based on the AI platform 588 processing the model input via one or more AI models; etc.).

As illustrated in the embodiment of Figure 2R, the one or more AI platforms 588 can alternatively or additionally implement scientific data processing system 1500 and/or medical data processing system 500 (e.g. can execute corresponding application data corresponding to scientific data processing system 1500 and/or medical data processing system 500; and/or otherwise perform some or all features and/or functionality of scientific data processing system 1500 and/or medical data processing system 500 themselves), where the scientific data processing system 1500 and/or medical data processing system 500 communicates with/is implemented in conjunction with one or more model training systems 557 and/or model output generator systems 558, for example, via bus 689.

In some embodiments, some or all features and/or functionality of medical outcome prognostication model training system 506 and/or outcome prediction model training system 1506 described herein is alternatively or additionally implemented via model training systems 557 of an AI platform 588, for example, instead of or in addition to being implemented by the scientific data processing system 1500 and/or the medical data processing system 500. For example, the AI platform 588 trains some or all versions of medical outcome prognostication functions 671 and/or outcome prediction functions 1671 described herein, alternatively or in addition to some or all versions of medical outcome prognostication functions 671 and/or outcome prediction functions 1671 being trained by the scientific data processing system 1500 and/or medical data processing system 500 (e.g. in response to receiving and processing corresponding training data from the scientific data processing system 1500 and/or medical data processing system 500).

In some embodiments, some or all features and/or functionality of Medical Outcome Prognostication System 507 and/or outcome prediction system 1507 described herein is alternatively or additionally implemented via model training systems 557 of an AI platform 588, for example, instead of or in addition to being implemented by the scientific data processing system 1500 and/or the medical data processing system 500. For example, the AI platform 588 performs some or all features and/or functionality of medical outcome prognostication functions 671 and/or outcome prediction functions 1671 described herein, alternatively or in addition to medical outcome prognostication functions 671 and/or outcome prediction functions 1671 being performed by the scientific data processing system 1500 and/or medical data processing system 500. In such embodiments, the AI platform 588 optionally generates some or all Outcome Prediction Scores 1636 and/or medical outcome prognosis scores 636 described herein, alternatively or in addition to all outcome prediction scores 1636 and/or medical outcome prognosis scores 636 being generated by the scientific data processing system 1500 and/or medical data processing system 500 (e.g. in response to receiving and processing corresponding participant data from the scientific data processing system 1500 and/or medical data processing system 500).

Figure 3A presents an embodiment of information that is included in, conveyed by, mapped to, generated for, and/or otherwise associated with given medical data 562. Some or all features of medical data 562 of Figure 3A can implement any embodiment of medical data 562 described herein.

A given medical data 562 can include, can be mapped to, be based on, and/or can otherwise indicate a set of one or more values 601.1 - 601.V of a medical data value set 600. The set of values of medical data value set 600 can be dictated by a type of the corresponding medical data, and the corresponding values can correspond to raw sensor values, measurement, and/or other corresponding data of the medical data. For example, the medical data value set 600 is a plurality of pixel values and/or density values of a medical image, for example, where the medical data value set 600 includes a plurality of sets of pixel values and/or density values corresponding to a plurality of image slices, where each image slice has its own set of values. As another example, the medical data value set includes a plurality of signal values of a corresponding signal and/or includes a plurality of measurements/sensor data captured over time in accordance with the medical data being temporal based medical data. As another example, the medical data value set includes test results of a medical text. As another example, the medical data values set includes a plurality of text data corresponding to a medical report. As another example, the medical data values set includes a plurality of user supplied responses to a questionnaire/corresponding set of prompts (e.g. presented via GUI 775).

In some embodiments, some or all of the set of one or more values 601.1 - 601.V of a medical data value set 600 of given medical data 562 can include and/or indicate raw data and/or processed data, for example, indicating medical information and/or heath information, and/or that was collected based on a medical process and/or scientific experiment. For example, the medical data 562 can include and/or indicate data captured for an individual in conjunction with testing, treatment, and/or other data collection and/or processing in the field of medicine, clinical trials, scientific experiments, etc.

In some embodiments, some or all given medical data 562 described herein is implemented as device-captured medical data that was captured/collected/measured/stored/analyzed in whole and/or in part by use of a medical device. For example, the medical device is operable to capture/collect/measure/store/analyze medical data, health data, and/or other data for an individual that can be useful in fields of medicine, clinical trials, scientific experiments, etc. For example, some or all of the of the set of one or more values 601.1 - 601.V of a medical data value set 600 of given medical data 562, and/or other information of medical data 562, is generated via (and/or based on use of) a corresponding medical device, such as: a medical imaging machine, medical test equipment, medical robots, and/or any type of medical device described herein.

Some or all medical devices described herein can optionally be implemented as data sources 575. Alternatively or in addition, medical data 562 collected via a given medical device can otherwise be sent to/received by/stored by/determined by/accessed by a data source 575 and/or by medical data processing system 500.

In some embodiments, some or all some or all of the set of one or more values 601.1 - 601.V of a medical data value set 600 of given medical data 562 is captured for a given individual and/or captured/collected/measured/stored in whole and/or in part via human observation and/or human logging, for example, where some or all medical data 562 indicates information collected by and/or logged by a human based on the human observation. Such medical data can include: demographic data; risk factor data; patient history data; medical report data; speech and/or test data denoting human annotations and/or observations. For example, some or all of the set of one or more values 601.1 - 601.V of a medical data value set 600 of given medical data 562 can indicate basic patient information such as name or anonymized identifier protecting the confidentiality of the patient; age; birthdate; sex; medical history; family medical history; smoking and/or drug habits, pack years corresponding to tobacco use; environmental exposures; current medications; patient symptoms; etc.

In some embodiments, some or all given medical data 562 corresponds to user input data to a graphical user interface (GUI) and/or other user input system implemented by a client device, for example, implemented via a desktop computer, laptop, tablet, mobile device, smart phone, keyboard, mouse, microphone, touchscreen, camera, and/or other devices. For example, some or all of the set of one or more values 601.1 - 601.V of a medical data value set 600 of given medical data 562 is generated based on user interaction with the GUI (e.g. responding to questions presented via a questionnaire; entering labels/text/selections/annotations corresponding to the medical data to describe, annotate the medical data and/or supply metadata for the medical data; logging observed test measurements/results; etc.). In such embodiments, computing devices utilized to display the GUI, collect the corresponding user input data, and/or send the user input data to the medical data processing system 500, can be considered data sources 575 and/or medical devices collecting the corresponding medical data.

In some embodiments, some or all human-supplied medical data of an individual is collected/supplied via the individual themselves. For example, a patient described their family medical history and/or supplies responses regarding current symptoms. As another example, a patient interacts with a GUI (e.g. on their smart phone or personal device, or at a terminal at a medical location) to supply the medical data. Alternatively or in addition, some or all human-supplied medical data of an individual is collected/supplied via a medical professional. For example, a doctor/nurse records answers supplied by a patient; a doctor/nurse records measurements taken of a patient (e.g. via a medical device); a radiologist provides human annotations and/or other description of abnormalities identified in a medical scan or otherwise characterizes findings in a medical scan; etc. Alternatively or in addition, some or all human-supplied medical data of an individual is collected/supplied via an administrator/worker associated with a clinical trial/experiment. For example, the administrator/worker records answers supplied by a patient in conjunction with administering the corresponding clinical trial/experiment; the administrator/worker records observations/measurements taken of a patient (e.g. via a medical device); the administrator/worker identifies whether a primary endpoint and/or secondary endpoint has been reached for the patient in the corresponding clinical trial/experiment; etc.

In some embodiments, some or all of the set of one or more values 601.1 - 601.V of a medical data value set 600 of given medical data 562 can indicate medical data collected via one or more medical tests, such as at least one imaging test, at least one laboratory test, at least one endoscopy test, at least one biopsy test, at least one PCR (polymerase chain reaction) tests, at least one virology test, at least one biopsy test, at least one necropsy tests, and/or at least one other test. For example, some or all of the set of one or more values 601.1 - 601.V of a medical data value set 600 of given medical data 562 includes and/or indicates raw data collected via the test. As another example, some or all of the set of one or more values 601.1 - 601.V of a medical data value set 600 of given medical data 562 includes and/or indicates at least one test result of the test (e.g. one or more measurements characterizing one or more attributes of the raw data; detection of one or more abnormalities and/or conditions observable and/or indicated via test results of the test). The medical test can be conducted based on utilizing a corresponding medical device, for example, to collect corresponding raw data and/or samples; to measure the raw data and/or samples; to analyze the raw data and/or samples; and/or to detect a condition and/or provide a diagnosis/prognosis based on the analysis of the raw data and/or samples. In some embodiments, some or all of the medical test can be conducted via human intervention (e.g. samples/raw measurements are collected by a human; raw measurements/samples are analyzed in whole or in part by human observation; and/or a medical device output is analyzed in whole or in part by human observation).

In some embodiments, a given medical data 562 can be of a medical image type, and/or a given medical data 562 can be generated based on having been captured by a medical device implemented as a medical imaging device. For example, the medical image type can correspond to a medical image captured via a given one or more modalities, capturing one or more given anatomical regions, and/or being captured via one or more planes relative to the corresponding individual. For example, the medical data 562 can include and/or indicate raw and/or processed data of at least one CT scan, x-ray, MRI, PET scan, Ultrasound, EEG, mammogram, photograph, video, combination of different types of scans taken in tandem (e.g. PET-CT scan, PET-MRI scan, etc.) and/or or other type of radiological scan, medical scan, and/or digital image taken of an anatomical region of an individual (e.g. human body, animal, organism, or object). For example, some or all of the medical data 562 can be formatted in accordance with a Digital Imaging and Communications in Medicine (DICOM) format or other standardized image format, and some or more of the fields of the medical scan entry can be included in a DICOM header or other standardized header of the medical scan.

In some embodiments, corresponding measurements/conditions/abnormalities can be automatically detected/characterized in such image data via at least one function (e.g. performed by the medical image processing system 500) that is operable to process this image data/corresponding medical reports and/or other metadata of these medical images (e.g. via a trained computer vision model and/or trained natural language processing model) to automatically detect/predict corresponding measurements/conditions/abnormalities accordingly. This automatically detected information can be implemented as some or all of the medical data.

In some embodiments, a given medical data 562 can indicate temporal-based measurement data. For example, the temporal-based measurement corresponds to signal data, such as electrical signals or data points sampled over time, for example, by a medical device in proximity to an individual. For example, given medical data 562 is collected via a wearable device such as fitness tracking device, smart watch, smart ring, cellular device and/or other mobile device carried by a user; a pacemaker and/or other implanted device operable to measure/collect temporal data over time; and/or other device. Alternatively or in addition, given medical data 562 can be indicate temporal-based measurement data based on a plurality of discrete tests being performed over time and/or corresponding raw data/measurements/results being generated/logged over time, for example, via at least one medical device and/or via human observation.

In some embodiments, corresponding measurements/conditions/abnormalities can be automatically detected/characterized in such signal data and/or other temporal-based measurement data via at least one function (e.g. performed by the medical image processing system 500) that is operable to process these signals/series of discrete measurements and/or other metadata of this temporal-based measurement data (e.g. via a trained computer vision model and/or trained natural language processing model) to automatically detect/predict corresponding measurements/medical conditions/abnormalities accordingly. This automatically detected information can be implemented as a some or all of the medical data.

In some embodiments, given medical data 562 can indicate measurements and/or detection of one or more properties, such as biochemical properties and/or compounds, and/or other data. For example, the medical data 562 is generated based on at least one medical device collecting, measuring, and/or processing a part of the body, such as organic samples and/or any fluids, tissue, body parts, and/or other substances observed on and/or collected from a body of an individual. For example, the given medical data 562 can correspond to and/or indicate measured test results generated by and/or generated via human and/or machine-based analysis/observation of a medical device such as: a test strip; swab; histological slide; medical equipment that collects and/or stores organic samples; medical equipment that generates test results from organic samples, for example, that was stored and/or stored by other medical equipment; medical equipment that enhances human observation of an organic sample and/or part of the human body (e.g. a microscope), and/or other medical device operable to collect, analyze, store, and/or generate measurements of organic matter. Alternatively or in addition, the medical data 562 can correspond to measured levels indicating detection, size, amount, and/or characterization of one or more particular attributes such as: drugs, glucose, hormones, diseases, viruses, bacteria, particular types of cells, pH levels, blood count, chemical compounds, DNA sequencing, abnormalities, and/or other measurable qualities in test samples of fluids, tissue, and/or other organic matter/substances collected from a body of an individual. Alternatively or in addition, the medical data 562 can indicate one or more measurements generated via analysis of an organic sample, such as a sample of: cerebrospinal fluid (CSF), serous fluid (pleural, peritoneal and/or pericardial), synovial fluid, amniotic fluid, drain fluid, semen, urine, dialysate, saliva, sputum/phlegm, feces, vomit, hair, bones, muscle, skin, cells, tissue, and/or any other body fluid sample/tissue sample/organic sample. Alternatively or in addition, the medical data 562 can indicate one or more measurements generated via analysis of cells and/or tissue. Alternatively or in addition, the medical data 562 can indicate one or more measurements corresponding to proteomic data, genomic data, metabolomic data, metagenomic data, phenomics data, and/or transcriptomic data (e.g. RNA transcriptions), for example, in conjunction with proteomic, genomic, metabolomic, metagenomic, phenomics, and/or transcriptomic fields of study.

In some embodiments, given medical data 562 can indicate any measurement data/sensor data and/or other data, for example, collected via a medical device operable to measure, collect, and/or can be indicative of corresponding medical information and/or health information for an individual, such as: heart rate data; blood pressure data; weight data; blood oxygen measurement data; internal temperature data; respiratory rate data; pedometer data; sleep cycle data; blood glucose data; protein measurements; DNA sequence data; EKG data; ovulation data; mensural cycle data; pregnancy data; measured levels indicative of one or more particular drugs, hormones, diseases, viruses, bacteria, particular types of cells, pH levels, blood count, chemical compounds, DNA data, RNA data, proteomic data, genomic data, metabolomic data, metagenomic data, phenomics data, transcriptomic data, abnormalities, and/or measurable attributes.

In some embodiments, given medical data 562 can indicate any measurement data/sensor data and/or other data, for example, collected via a medical device, denoting data relating to types of measurements indicative of a corresponding medical condition, for example, having treatment tested in a corresponding clinical trial. For example, the medical data is indicative of symptoms of; metrics indicative of; and/or other data related to infectious and/or non-infectious diseases, illnesses, viruses, injuries, disorders, and/or other medical conditions such as: one or more types of cancer, such as metrics relating to one or more tumorous growths; cardiovascular conditions such as heart disease, stroke, etc.; diabetes; arthritis; epilepsy; Alzheimer Disease; one or more autoimmune diseases; one or more genetic disorders; one or more mental illnesses; one or more bacterial infections; dermatological conditions affecting skin, hair, and/or nails such as acne, eczema, etc.; viral and/or bacterial infection; musculoskeletal conditions; neurological disorders; and/or any other type of medical condition.

In some embodiments, some or all of the given medical data 562 can include one or more medical codes characterizing a corresponding measurement/medical condition. Such medical codes can be in accordance with a medical standard, for example, where a discrete set of possible codes (e.g. words/alphanumeric patterns) are implemented as a corresponding set of medical codes each indicative of a corresponding medical condition/state. Such medical codes can be implemented as SNOMED codes, Current Procedure Technology (CPT) codes, ICD-9 codes, ICD-10 codes, and/or other standardized medical codes used to label or otherwise describe medical conditions. Some or all of the medical codes can optionally be implemented as DICOM data/other metadata of a corresponding medical image/medical scan (e.g. describing human-detected findings and/or findings automatically generated as output of one or more functions that utilizes a computer vision model, for example, performed by medical data processing system 500).

In some embodiments, given medical data 562 can indicate abnormality data characterizing at least one abnormality (e.g. one or more lesions/nodules/tumors other abnormalities). For example, the medical data 562 can indicate a count of detected abnormalities; location of one or more abnormalities, classification data for an abnormality such as abnormality area, diameter, shape, size, volume, pre-post contract, doubling time, calcification, components, smoothness, texture, diagnosis data, one or more medical codes, a malignancy rating such as a Lung-RADS score, or other classifying data as described herein can be determined based on the detected abnormality. The abnormality data can optionally indicate change in such abnormality data over time (e.g. change in size/shape, such as whether growth or shrinkage has occurred; whether count has increased or decreased etc.) For example, such changes are reflected as temporal-based measurement data captured over time (e.g. in multiple medical images or multiple other device-captured medical data captured over time). In some embodiments, abnormality data implemented as medical data 562 can be automatically detected/characterized in device-captured medical data (e.g. medical images) via at least one function (e.g. performed by the medical image processing system 500) that is operable to process medical images data (optionally in conjunction with corresponding human annotations, corresponding metadata, and/or corresponding medical reports) to automatically detect and/or characterize abnormalities observable in the medical image data. The abnormality data can optionally indicate binary value for one or more classification categories, indicating whether or not such a category is present for a given abnormality/given individual. For example, this binary value can be determined by comparing at least one continuous or discrete value (e.g. of a measurement/of output of a function performed by the medical data processing system 500) to a threshold, for example, where the at least one continuous or discrete value indicates a calculated likelihood that a corresponding abnormality classifier category is present, or indicating another at least one measured value (e.g. raw measurement). In some embodiments, abnormality classifier categories can be assigned one or more non-binary values, such as one or more of these continuous or discrete values indicating a likelihood that the corresponding classifier category is presents/indicating another at least one measured value. The abnormality data can optionally include malignancy data for at least one malignancy category, for example, where the abnormality data includes a malignancy rating such as a Lung-RADS score, a Fleischner score, and/or one or more calculated values that indicate malignancy level, malignancy severity, and/or probability of malignancy. Alternatively or in addition, the malignancy category can be assigned a value of "yes", "no", or "maybe". The abnormality data can optionally indicate abnormality pattern data, for example, denoting whether cardiomegaly, consolidation, effusion, emphysema, fracture, and/or other patterns are present/detected. The abnormality data can optionally indicate Response Evaluation Criteria in Solid Tumors (RECIST) eligibility data, for example, for one or more RECIST evaluation categories. For example, abnormality data can have corresponding abnormality classification data indicating a binary value (e.g. "yes" or "no") and/or can indicate whether the abnormality is a "target lesion" and/or a "non-target lesion." As another example, the abnormality data category can be determined based on longitudinal data (e.g. temporal-based data, such as multiple medical scan collected over time) and can have corresponding abnormality classification data that includes one of the set of possible values "Complete Response", "Partial Response", "Stable Disease", or "Progressive Disease."

In some embodiments, given medical data 562 can indicate one or more biomarkers, such as biomarker data and/or other measurable indicators of a corresponding medical/biological state or condition, for example, measured via a corresponding medical test and/or via a corresponding medical device. The biomarkers can correspond to one or more molecular biomarkers, one or more physiologic biomarkers, one or more histologic biomarkers, one or more radiographic biomarkers; one or more cellular biomarkers; one or more imaging biomarkers; and/or other biomarkers. The one or more biomarkers can be detected automatically via a machine learning function and/or can be observed via a medical device/medical process. The one or more biomarkers can be identified based on at least one medical test/procedure administered to a corresponding patient and can be predictive/indicative of a corresponding condition, for example, being treated in a corresponding clinical study.

In some embodiments, the medical data can correspond to image data, such as a photograph/video/digital image data capturing the medical device itself, and/or capturing corresponding results indicated by/outputted by the medical device and/or otherwise logged by a person observing the output of the medical device, for example, indicating results of a corresponding analysis of organic matter, indicating sensor data output, and/or indicating collected data of/results of any corresponding test. For example, the image data captures a test strip, swab, organic sample, histological slide, medical report such as text written by a medical professional and/or otherwise describing results of a test, test result data, ECG/EKG data, medical image data, measurement data, medical report data, human-supplied text, human-supplied answers to a hard copy of a questionnaire (e.g. filled out on paper), and/or other information. In such embodiments, corresponding measurements/results can be visually indicated in the image data and/or are automatically detected in the image data via at least one function (e.g. performed by the medical image processing system 500) that is operable to process image data/extracted text data of such devices/results (e.g. via a trained computer vision model and/or trained natural language processing model) to automatically extract/detect/predict corresponding measurements/conditions/results accordingly. This automatically extracted/detected information can be implemented as a some or all of the medical data.

As illustrated in Figure 3A, medical data 562 can further have corresponding medical data metadata 610, which can be: mapped to, conveyed by, stored in conjunction with, included in, and/or can be otherwise associated with medical data 562 to indicate additional information relevant to the given medical data 562.

For example, some or all medical data metadata 610 included in/conveyed by/mapped to/generated for medical data 562 is: received by medical data processing system 500 from a corresponding data source based on being stored by, generated by, received by, accessed by, and/or determined by the data source; is stored as a corresponding entry in medical data storage 561 in conjunction with/mapped to the medical data 562; is generated by medical data processing system 500 automatically, for example, as output of one or more functions executed by medical data processing system 500; is sent to at least one requesting entity 576 for display and/or storage; and/or is otherwise utilized, generated, and/or determined in conjunction with functionality of medical data processing system 500 described herein. Some or all of the medical data metadata 610 included in/conveyed by/mapped to/generated for medical data 562 as illustrated in Figure 3A can be included in/conveyed by/mapped to/generated for any medical data 562 described herein.

Medical data metadata 610 of given medical data 562 can include, can be mapped to, and/or can otherwise indicate a medical data identifier 552, for example, uniquely identifying the given medical data 562 from other medical scans, denoting a storage location of medical data 562 for access, and/or otherwise identifying the medical data 562. The medical data identifier 552 can enabling mapping of the given medical data to corresponding patient data 564 and/or corresponding clinical trial data 566, for example, via the inclusion of medical data identifier 552 in respective entries.

Medical data metadata 610 of given medical data 562 can alternatively or additionally include, can be mapped to, and/or can otherwise indicate a patient identifier 554, for example, uniquely identifying a given patient and/or mapping the medical data 562 to corresponding patient data 564. Thus, any of the patient data 564 described herein can optionally be implemented as medical data metadata 610 for one or more medical data 562 of the corresponding patient. The patient identifier 554 can be a name of the patient and/or other unique identifier assigned to the given patient, for example, uniquely identifying the patient for other patients. Medical data metadata 610 of multiple medical data 562 optionally have a same patient identifier 554 based on being a set of different medical data collected for a same patient (e.g. multiple medical scans taken of the patient; different types of test results from multiple tests taken of the patient; etc.). Such metadata can enable various medical data to be grouped by patient, enabling multiple different medical data to be grouped together for use in training/executing a machine learning function in tandem in accordance with various functionality described herein, where some or all patient data can thus be utilized to. Alternatively or in addition, such metadata can facilitate use of addition input in training and/or executing a machine learning function, where additional features drawn from patient data for the patient (e.g. risk factors, patient history, current symptoms, etc.) are utilized as input, which can facilitate generation of more sophisticated insights as output of a corresponding machine learning model in accordance with various functionality described herein.

Medical data metadata 610 of given medical data 562 can alternatively or additionally include, can be mapped to, and/or can otherwise indicate medical data capture data 611 relating to how/when/by whom the corresponding medical data 562 was captured. For example, the medical data capture data 611 indicates time and/or date data 612 indicating when the medical data was captured/generated (e.g. by a corresponding medical device) and/or optionally when the medical data 562 was sent to/received by the medical data processing system 500. Alternatively or in addition, the medical data capture data 611 indicates provider and/or location data 613 indicating a particular place (e.g. hospital/clinic/facility) and/or person (e.g. doctor/nurse/technician) that captured the medical data (e.g. identifying a healthcare provider that ordered the corresponding test; identifying a healthcare provider that administered the corresponding test; identifying a healthcare provider that analyzed results of the corresponding test; etc.). Alternatively or in addition, the medical data capture data 611 indicates a medical device identifier and/or type 614, for example, identifying which type of medical device captured the medical data and/or indicating which particular medical device captured the medical data. Such metadata can enable various medical data to be grouped/labeled/processed in accordance with when, where, and/or how the medical data was captured when training and/or executing a corresponding machine learning model in accordance with various functionality described herein, which can aid in determining longitudinal data for a patient; aid in accounting for regional-based, location-based, provider-based, and/or machine-based disparities/biases; etc.

Medical data metadata 610 of given medical data 562 can alternatively or additionally include, can be mapped to, and/or can otherwise indicate medical data type data 615 indicating the type of medical data that the medical data value set 600 corresponds to. This can enable the corresponding values 601.1 - 601.V to be decoded/processed appropriately and/or can otherwise allow the corresponding medical data 562 to be rendered/processed. Such metadata can enable various medical data to be grouped/labeled/processed by type, for example, where different machine learning models for different medical data types are trained in accordance with various functionality described herein, and/or where multiple different types of medical data are utilized as input in tandem when training and/or executing a corresponding machine learning model to enable richer/more types of information to be processed in generating corresponding inferences.

Medical data metadata 610 of given medical data 562 can alternatively or additionally include, can be mapped to, and/or can otherwise indicate medical findings data 616 indicating findings associated with the corresponding medical data 562. The medical findings data 616 can optionally indicate model-detected finding data 617, such as detection and/or characterization of abnormalities in the medical data, an auto-detected/predicted medical condition, and/or other information generated via processing of the medical data, for example, by at least one machine learning model, for example, implemented via at least one function of the function library, which can be indicated via its corresponding function ID 559 to identify which function/which function version generated this finding data. For example, some or all of the abnormality data described previously, medical codes described previously, and/or automatically extracted information described previously can be implemented as/indicated by medical findings data 616 for the medical data generated via output of executing a corresponding machine learning function.

The medical findings data 616 can alternatively or additionally indicate human-detected finding data, such as annotations to a medical scan by a radiologist and/or medical professional; a medical report and/or text describing findings generated by a medical professional reviewing the corresponding medical data and/or corresponding patient, and/or other information. Such metadata can be processed in conjunction with various medical data, for example, as additional input, where machine learning models are trained and/or executed in accordance with various functionality described herein based on utilizing findings data as input instead of or in addition to raw values 601 of the medical data itself. Alternatively or in addition, the medical findings data can be generated as output of one or more machine learning functions, where machine learning models are executed upon medical data 562 to generate medical findings data 617 as output, for example, based on training the machine learning model with a training set of medical data having labeled medical finding data (e.g. human-detected findings data 618 and/.or other truth data utilized to train the model).

Medical data metadata 610 of given medical data 562 can alternatively or additionally include, can be mapped to, and/or can otherwise indicate model-generated prognosis data 635, which can include one or more medical outcome prognosis scores 636, each corresponding to a predicted score (e.g. a computed probability value between 0 and 1; a corresponding predicted continuous value corresponding to a predicted measurement; a corresponding predicted category of a set of two or more categories; a predicted outcome for the medical outcome; etc.) associated with a corresponding medical outcome type 637 (e.g. a corresponding type of measurement and/or determination), which was generated via execution of a corresponding medical outcome prognostication function 671 upon the given medical data as identified by function ID 579. For example, multiple medical outcome prognosis scores 636.1 - 636.Q corresponding to a plurality of different types of medical outcomes 637.1 - 637.Q are mapped to the medical data 562 based on corresponding functions (e.g. medical outcome prognostication functions 671 denoted by function IDs 579.1 - 579.Q) each having been performed upon the medical data 562 as input to render the corresponding medical outcome prognosis score 636 as output. For example, the model-generated prognosis data 635 is generated via medical outcome prognostication system and/or the medical outcome prognosis score 636 is utilized by prognosis-based trial arm assignment system 508 to assign a corresponding patient to either a control group or investigational in a corresponding clinical trial.

Medical data metadata 610 of given medical data 562 can alternatively or additionally include, can be mapped to, and/or can otherwise indicate medical outcome truth data 639 which can include one or more outcome data 638 for one or more medical outcome types 637.1 - 637.Q. For example, the outcome data 638 indicates an actual outcome (e.g. actual corresponding measurement/result/etc.) for the medical outcome type. This outcome data 638 can be human-logged, medical device-generated and/or determined in conjunction with conducting of a medical test, for example, in conjunction with a medical trial, and/or observing a corresponding patient. For example, the outcome data for a given medical outcome type 637.1 is utilized as training data to train a corresponding model (e.g. for a corresponding medical outcome prognostication function 671 of the function library) based on mapping the medical data 562 to this truth medical outcome prognosis score 636 as output. The outcome data can be utilized in the form of an outcome score (e.g. a binary representation of whether or not an event medical occurred, such as values of strictly 1 or 0 in the case where the model is trained to output values between 1 and 0; a continuous value corresponding to a measured value, where the model is trained to output such corresponding continuous values as predicted measurements; a discrete, categorical value corresponding to an outcome category having two or more categories, where the model is trained to output such corresponding discrete values as predicted categories; etc.). In such cases where the medical data 562 is utilized training data, model-generated prognosis data 635 is optionally not generated and/or is optionally generated only as validation data in testing the corresponding model. Alternatively or in addition, the medical data 562 is processed after the model is trained, where model-generated prognosis data 635 is generated to predict the medical outcome for the medical outcome type, and where the medical outcome truth data 639 is later supplied once the medical outcome is determined for the respective patient (e.g. as endpoint status data in a corresponding clinical trial). This information can be utilized to find inconsistencies in the model performance and/or can be utilized to retrain the model/improve model performance over time.

As used herein, a medical outcome of a given medical outcome type 637 can correspond to a corresponding measurement and/or observable result as defined by the given medical outcome type 637. Some or all given medical outcome types 637 described herein can correspond to any heath care outcome measures, clinical trial endpoints, and/or other measurable outcomes that are observable/measurable for a given patient. Actual, measured outcome data 638 for such a given medical outcome type can have one or more corresponding values (e.g. collected measurements via human observation and/or via at least one medical device/medical test) representative of the actual medical outcome of the medical outcome type. Medical outcome prognosis scores 636 for a given medical outcome type can have one or more corresponding values representative of a prediction for such measurements of the medical outcome of the medical outcome type, and/or can further include and/or indicate one or more probability values/confidence values corresponding to the given prediction.

Various medical outcome types 637 can be implemented as categorical outcomes corresponding to medical outcomes with two or more discrete categories. Some or all medical outcome types 637 implemented as categorical outcomes can optionally be implemented as binary outcomes corresponding to medical outcomes with exactly two discrete categories (e.g. an event either occurred or did not occur). Some or all medical outcome types 637 implemented as categorical outcomes can be implemented as non-binary categorical outcomes with three or more discrete categories. The categorical outcomes can correspond to ordered categories (e.g. a plurality of pain categories increasing with pain) and/or can correspond to unordered categories (e.g. one of a set of type of conditions observed). In some embodiments, multiple different categorical outcomes can be observed as a medical outcome, where the medical outcome indicates a subset of the set of possible categories that were observed, and/or where some or all different combinatorically defined subsets of the set of possible categories are thus possible medical outcomes.

Various medical outcome types 637 can alternatively or additionally be implemented as continuous outcomes corresponding to medical outcomes measured on a continuous scale. Some or all medical outcome types 637 implemented as continuous outcomes can optionally be implemented as event-time outcomes, such as mortality time/measured time to death or measured time to other defined, non-morality-based events. Some or all medical outcome types 637 implemented as continuous outcomes can optionally be implemented as non-event-time outcomes, where the continuous outcomes correspond to other types of continuous measurements (e.g. size measurements; weight measurements; mass measurements; blood pressure measurements; etc.

Various medical outcome types 637 can alternatively or additionally be implemented as count-based measurements and/or frequency based measurements (e.g. number of occurrences of a given event within a specified time frame; average number of occurrences of a given event within a specified time frame as measured across multiple such time frames; etc.).

In some embodiments, the medical outcome of a given medical outcome type is a binary value when the given medical outcome type corresponds to an observable event that either occurs or does not occur. For example, the medical outcome type corresponds to survival within/mortality by a certain time/certain amount of time from a current time/given starting time (e.g. 1 year, 2 years, 5 years, etc.), with medical outcomes denoting whether or not patient death occurs by the given time. As another example, the medical outcome type corresponds to whether or not a given, defined response was observed with medical outcomes denoting whether or not the given response was observed. In such embodiments, the binary event can correspond to a threshold measurement value, where a collected measurement is compared to the threshold to determine whether the binary event occurred (e.g. was the measured mortality time less than or greater than 1 year; is the measured nodule diameter more or less than 1 cm in diameter; etc.). Corresponding measured outcome data 638 can be implemented to include/indicate binary values (e.g. 1 or 0, or other predetermined values) denoting whether or not the event occurred (e.g. 1 denotes event occurred; 0 denotes event did not occur). Corresponding medical outcome prognosis scores 636 can similarly be implemented to include/indicate binary values (e.g. 1 or 0) denoting whether or not the event is predicted to occur. Corresponding medical outcome prognosis scores 636 can alternatively or additionally be implemented to include/indicate probability values (e.g. values between 0 and 1) denoting a probability by which the event is predicted to occur.

Alternatively or in addition, the medical outcome of a given medical outcome type is one of a set of predefined discrete values when the given medical outcome type corresponds to one of a set of discrete categories. In some embodiments, the event can correspond to a set of threshold measurement values defining a plurality of interval-based categories, where a collected measurement is compared to the set of thresholds to determine which category the measurement falls within (e.g. was the measured mortality time less than 1 year, between 1 and 2 years, between 2 and 5 years, or more than 5 years; is the measured nodule diameter less than 1mm, between 1mm and 10mm, or more than 10 mm in diameter; etc.). The measurement can optionally correspond to a user supplied rating (e.g. rate pain on a scale from 1 to 5). Corresponding measured outcome data 638 can be implemented to include/indicate categorical values (e.g. integer values/other labels corresponding to the set of different events, optionally preserving ordering when the categories are order-based) denoting which category was observed/measured to have occurred (and/or which subset of categories was determined to have occurred, in the case where a given label corresponds to a particular subset of a set of possible subsets of categorical events occurring). Corresponding medical outcome prognosis scores 636 can similarly be implemented to include/indicate a predicted category by indicating a predicted one of the of set of discrete values (e.g. the integer value/other label corresponding to the predicted events) denoting which category is predicted to occur (and/or which set of categories is predicted to occur, in the case where a given label corresponds to a particular subset of a set of possible subsets of categorical events occurring). Corresponding medical outcome prognosis scores 636 can alternatively or additionally be implemented to include/indicate probability values (e.g. values between 0 and 1) denoting a probability by which the predicted category is predicted to occur, and/or by which every category is predicted to occur (e.g. for a set of four categories, a set of four probability values are outputted denoting probabilities for each event, the four probabilities summing to one, and/or the greatest of which denoting the category predicted as most likely to occur).

Alternatively or in addition, the medical outcome of a given medical outcome type is a continuous value corresponding to a given measurement when the given medical outcome type corresponds to a given measurement. Corresponding measured outcome data 638 can be implemented to include/indicate a continuous value denoting the measured value for the corresponding measurement (e.g. a numeric value denoting the measurement output of a medical device and/or otherwise indicating the measured value). Corresponding medical outcome prognosis scores 636 can similarly be implemented to include/indicate a predicted value for the measurement. Corresponding medical outcome prognosis scores 636 can alternatively or additionally be implemented to include/indicate confidence values, variance data, and/or distribution data indicating probability of/amount of deviation from this amount that is expected.

Alternatively or in addition, the medical outcome of a given medical outcome type is a value corresponding to a given count/frequency when the given medical outcome type corresponds to a count and/or frequency. Corresponding measured outcome data 638 can be implemented to include/indicate a value denoting the measured count/frequency for the corresponding measurement (e.g. an integer count, a corresponding value indicating count per unit time, etc.). Corresponding medical outcome prognosis scores 636 can similarly be implemented to include/indicate a predicted value for the count and/or frequency. Corresponding medical outcome prognosis scores 636 can alternatively or additionally be implemented to include/indicate confidence values, variance data, and/or distribution data indicating probability of/amount of deviation from this amount that is expected.

Medical data metadata 610 of given medical data 562 can alternatively or additionally include, can be mapped to, and/or can otherwise indicate training set data 620 denoting which training sets the given medical data has been included in and/or otherwise indicating which models have been trained based on the given medical data and/or some or all of its respective medical scan metadata 610. For example, the training set data 620 indicates a set of function identifiers denoting which corresponding functions (e.g. inference functions applying corresponding machine learning models) were trained via a training set that included the given medical data 562.

Figure 3B presents an embodiment of information that is included in, conveyed by, mapped to, generated for, and/or otherwise associated with given patient data 564 for a given patient (e.g. a given individual, such as a given human, animal, organism, and/or object having medical data 562 and/or being a prospective and/or confirmed participant in one or more clinical trials and/or other scientific experiments). Some or all features of patient data of Figure 3B can implement any embodiment of patient data 564 described herein.

Given patient data 564 can include, can be mapped to, and/or can otherwise indicate a patient identifier 554, for example, uniquely identifying a given patient and/or enabling mapping of the given patient to corresponding medical data 562 and/or corresponding clinical trial data 566, for example, via the inclusion of patient identifier 554 in respective entries.

Given patient data 564 can alternatively or additionally include, can be mapped to, and/or can otherwise indicate a medical data set 630 indicating a set of one or more medical data 562 captured for the patient. For example, this medical data 562 is mapped to the patient via a corresponding set of medical data identifiers 552.1 - 552.W. Thus, any of the medical data 562 and/or corresponding medical data metadata 564 (e.g. medical findings in the medical data and/or corresponding reports/annotations, etc.) described herein can optionally be implemented as patient data 564 for a corresponding patient.

Given patent data 564 can alternatively or additionally include, can be mapped to, and/or can otherwise indicate other relevant data 640 regarding the patient. The other relevant data 640 can include demographic data 641; medical history data 642; family history data 643; and/or risk factor data 644. For example, the other relevant data 640 can indicate one or more of: birthdate; age; sex/gender; weight; height; residence address; geographical location of birthplace, current residence, and/or past residences; travel history; occupation and/or exposure to corresponding occupational health hazards; current medications and/or medication history; allergies/adverse reactions to medications and/or other foods/products; past and/or current medical procedures/treatment plans; history of diseases/injuries/other medical conditions; family history of diseases/injuries/other medical conditions; past and/or current symptoms and/or medical conditions; past and/or current clinical trials; prior therapy/treatment; measurements/test results of one or more medical tests; Eastern Cooperative Oncology Group (ECOG) status; smoking frequency/pack years/other smoking status; recreational drug usage; sexual partners/activity; cardiac and pulmonary toxicity, TNM (Tumor, Nodes and Metastases); histology; PD-L1 Expression; TNM stage; performance status (PS), such as pretreatment performance status; other medical status; and/or other relevant data indicating current and/or past health/associated risk factors/etc. for the patient Some or all of the other relevant data 640 can be compiled from corresponding collected information for the patient, for example, collected from at least one health care provider; at least one insurance company; at least one pharmacy; at least one questionnaire completed by the patient; and/or other data providers 575. Some or all of the other relevant data 640 can be collected based on output of at least one machine learning function performed upon the patient data and/or some or all corresponding medical data, such as medical finding data 616 (e.g. measurements of nodules detected in medical scans, etc.).

Given patent data 564 can alternatively or additionally include, can be mapped to, and/or can otherwise indicate model-generated prognosis data 635 indicating a set of one or more medical outcome prognosis scores 636.1 - 636.R, for example, each corresponding to a predicted outcome for a given medical outcome type 637 and/or each generate via execution of a corresponding function denoted via function identifier 579. The model-generated prognosis data 635 can be implemented in a same or similar fashion as discussed in conjunction with Figure 3A, for example, where this model-generated prognosis data 635 is indicated in the patient data based on being generated via processing a given medical data 562.

In some embodiments, rather than a given medical outcome prognosis score 636 of a model-generated prognosis data 635 being generated via processing a given medical data 562 as discussed in conjunction with Figure 3A, multiple medical data 562 and/or consideration of other factors regarding the patient as a whole (e.g. some or all other relevant data 640) can be processed via a corresponding machine learning model to generate a corresponding medical outcome prognosis score 636 for the patient. In some cases, these holistic medical outcome prognosis scores 636 are reflected in the medical scan metadata for one or more corresponding medical data of the patient based on the medical data being mapped to the corresponding patient data. Alternatively or in addition, some or all medical outcome prognosis scores 636 for medical data 562 can correspond to scores generated by processing the given medical data 562, and/or can correspond to historical scores generated when those medical data 562 were processed (e.g. rather than a more current score generated from more recent medical data 562). A given holistic/current medical outcome prognosis score 636 for a patient is optionally generated as a function of one or more medical outcome prognosis score 636 generated individual given medical data for the patient (e.g. as a function of/inherently factoring in differences in time from when the medical data was collected and/or different types of medical data that some or all different ones of the multiple medical data correspond to). A given holistic/current medical outcome prognosis score 636 is optionally generated as a function of processing the multiple medical data collectively as input (e.g. optionally labeled by relative time differences and/or data type) to generate a single medical outcome prognosis score 636 for a given outcome type.

In some embodiments, as conditions change over time (e.g. as time passes, new medical data is received, etc.) a patient's medical outcome prognosis score 636 for a given outcome type can be updated (e.g. regenerated via the function given the most recent information and/or given passage of time). In such cases, the medical outcome prognosis score 636 is optionally updated/replaced for the given outcome type and/or a historical logging of medical outcome prognosis scores 636 for the given outcome type can be maintained (e.g. to show how prediction of the outcome changes over time as new information is added, to enable retraining of more sophisticated models, etc.). In cases where a patient's status is tracked over time to ultimately determine whether the patient succumbs to the respective medical outcome or not, corresponding outcome data 638 can be mapped to a given medical outcome (e.g. instead of or in addition to one or more medical outcome prognosis scores 636 generated for this medical outcome previously, which can enable retraining of one or more models accordingly).

Given patent data 564 can alternatively or additionally include, can be mapped to, and/or can otherwise indicate a clinical trial set 645 indicating a patient's prospective and/or confirmed participation in one or more past, ongoing, and/or future clinical trials (and/or any scientific experiments).

The clinical trial set 645 can indicate a corresponding trial identifier 556 for one or more such clinical trials, for example, uniquely identifying the corresponding clinical trial and/or otherwise mapping the patient data to corresponding clinical trial data 566, where some or all information of the corresponding clinical trial data 566 can thus be considered patient data 564 for the given patient.

The clinical trial set 645 can indicate trial arm assignment data 631 for each clinical trial, for example, denoting whether the given patient is participating in a control group or investigational group for the clinical trial. Such information can be stored securely and/or be accessible via controlled permissions, for example, to ensure the patient and/or double-blind processes associated with conducting the trial cannot view this information, for example, to ensure that the clinical trial is conducted in an unbiased fashion and/or in a fashion required by one or more governmental/company-based regulations regarding conducting of clinical trials and/or conducting of the scientific process.

The trial arm assignment data 631 can optionally indicate that the given patient is not participating in the trial at all (e.g. the patient was a prospective participant, but excluded from the trial for one or more reasons, for example, based on model-generated data such as medical outcome prognosis data and/or other medical finding data, or any other patient data 564 and/or other reasons rendering the patient ineligible, undesirable for the trial, and/or otherwise not selected. In sone cases, such reasons for exclusion can be indicated in corresponding patient data 564 and/or corresponding trial data 566.

The clinical trial set 645 can indicate endpoint measurement data 632 for each clinical trial, for example, denoting one or more measurements and/or results for the primary endpoint of the trial. In cases where the endpoint has not yet been reached, (e.g. not enough time has passed; the trial is not complete). The endpoint measurement data 632 can indicate it is not yet known as to whether or not the patient has reached the endpoint (e.g. has a NULL value, is not yet logged, etc.)

In some embodiments, the primary endpoint of the given trial is based on and/or is the same as a medical outcome type of a given medical outcome prognosis score 636 generated for the patient. For example, the given medical outcome prognosis score 636 for this medical outcome type matching the primary endpoint of the trial is utilized to generate the trial arm assignment data 631 and/or to otherwise group the patient into either the control trial arm and/or investigational trial arm via a randomization algorithm, for example, via the prognosis-based trial arm assignment system 508.

Alternatively or in addition, in cases where the endpoint measurement data 632 for a given trial has been determined, medical outcome truth data 639 for the patient and/or one or more medical data 638 can be updated accordingly for a medical outcome type 637 corresponding to the primary endpoint of the trial. For example, this updating is performed automatically by the medical data processing system 500 based on the endpoint measurement data 632 for the clinical trial being recorded for the patient. This can enable assessment of accuracy of a corresponding model responsible for generating a corresponding medical outcome prognosis score 636 and/or can be utilized to retrain of the corresponding model.

While not illustrated, other test results; recorded measurements; etc. for the patient in conjunction with conducting the trial can optionally be indicated for the given trial in the given patient's patient data.

Figure 3C presents an embodiment of information that is included in, conveyed by, mapped to, generated for, and/or otherwise associated with given clinical trial data 566 for a given clinical trial and/or any other scientific experiment. Some or all features of clinical trial data 566 of Figure 3C can implement any embodiment of clinical trial data 566 described herein.

Given clinical trial data 566 can include, can be mapped to, and/or can otherwise indicate a clinical trial identifier 556, for example, uniquely identifying a given clinical trial 556 and/or enabling mapping of the given clinical to corresponding medical data 562 and/or corresponding patient data 564, for example, via the inclusion of clinical trial identifier 556 in respective entries.

Given clinical trial data 566 can alternatively or additionally include, can be mapped to, and/or can otherwise indicate a primary endpoint 651, which can be implemented as and/or otherwise indicate a given medical outcome type 637.x that implements and/or is associated with meeting of the primary endpoint 651 for the trial.

The primary endpoint 651 can indicate and/or correspond to an endpoint and/or outcome (e.g. having given medical outcome type 637.x) that is determined for each study participant (e.g. each patient) of the given clinical trial. For example, the corresponding medical outcome type 637.x is configured to access the effect of a corresponding treatment (e.g. drug and/or process), such as its effectiveness in treating (e.g. curing, mitigating negative effects of, etc.) of a corresponding medical condition.

The given medical outcome type 637.x defining the primary endpoint 651 a given clinical trial is optionally one of the medical outcome types 637 of medical outcome prognosis scores 636 generated via performance of a corresponding medical outcome prognostication function automatically performed by the medical data processing system 500. In particular, some or all medical outcome prognostication functions can be automatically trained by the medical data processing system 500 to generate medical outcome prognosis scores 636 specifically for medical outcome types 637 correspond to primary outcomes of clinical trials, for example, to improve the process of assigning patients to control or investigational trial arms of the clinical trial via a randomization algorithm based on stratifying patients by their medical outcome prognosis scores 636 due to being intentionally trained to be indicative of the primary outcome of the trial. Such embodiments are described in further detail in conjunction with Figures 4A - 6K. In other embodiments, the given medical outcome type 637.x of a given clinical trial is optionally different from/unrelated to the medical outcome types 637 of some or all medical outcome prognosis scores 636.

Given clinical trial data 566 can alternatively or additionally include, can be mapped to, and/or can otherwise indicate a participant set 650, which can indicate a plurality of prospective and/or confirmed participants (e.g. people, animals, organisms, or objects) for the given clinical trial and/or other scientific experiment/study.

Participant set 650 can indicate a plurality of patient identifiers 554.1 - 554.S for a plurality of prospective/confirmed participants of the given clinical trial. Each of the plurality of participants can be uniquely identified by their corresponding patient ID 554, which can optionally map some or all corresponding patient data 564 for participant in the participant set 650. Alternatively, the patient ID 554 is implemented as another identifier for clinical study participants.

Participant set 650 can indicate trial arm assignment data 631 for each participant, which can indicate assignment of the given participant to either a control arm of the clinical trial or an investigational/experimental arm of the clinical trial. In some embodiments, the clinical trial has multiple investigational groups and/or multiple control groups, has subgroups for the investigational arm and/or the control arm, and/or has a total number of groups that exceeds two, where the trial arm assignment data 631 indicates which of the three of more groups the participant is assigned to. In some embodiments, some participants of participant set 650 are prospective participants that were not selected for the study (e.g. were deemed ineligible, the study became full, were less desirable than other candidates, and/or other reasons), and can optionally have trial arm assignment data 631 indicating they are not participating in the study.

Participant set 650 can indicate endpoint measurement data 632 for each participant, which can indicate the primary endpoint measured/determined for the corresponding participant, once measured/determined. For example, endpoint measurement data 632 for a given patient indicates the endpoint status data measurement/one or more values of the corresponding medical outcome type 637.x, for example, collected once the trial duration has elapsed and/or rendering completion of the trial for the patient based on the corresponding condition being detected. The endpoint measurement data can optionally indicate status for measurement of the endpoint, such as whether the endpoint condition has yet been met/measured (e.g. the endpoint condition has not yet been measured because the trial is still ongoing, corresponding condition has not occurred). The endpoint measurement data 632 can optionally indicate partial measurements for the primary outcome (e.g. measurements/counts taken so far that will contribute to the final measurement for the primary endpoint as measurements/counts continue to be collected). The endpoint measurement data 632 is optionally NULL/not included until the trial is complete and/or the corresponding endpoint measurement data has been collected.

In some embodiments, corresponding measurement/values for endpoint measurement data 632 can be implemented as (e.g. can be utilized to automatically populate/determine) outcome data 638 for the given medical outcome type 637.x, for example, for one or more corresponding medical data 652 of the patient, which can be utilized to measure model accuracy in generating corresponding medical outcome prognosis scores 636 for this medical outcome type 637.x and/or can be utilized as new training data to retrain/improve performance of a corresponding model.

Given clinical trial data 566 can alternatively or additionally include, can be mapped to, and/or can otherwise indicate a patient assignment method data 682, which can define how participants be dispersed between the control group and the investigational group. The patient assignment method data 682 can indicate stratification factors 683 (e.g. one or more factors by which patients be stratified) and/or can indicate one or more randomization techniques 684 (e.g. a permuted block technique and/or other randomization technique, which can optionally incorporate one or more of, alternatively or in addition to the permuted block technique: a simple randomization technique, a block randomization technique, a stratified randomization technique, and/or a covariate adapted randomization technique).

The patient assignment method data 682 can alternatively or additionally indicate a randomization algorithm, for example, that is specified via function identifier 559 of a corresponding function of function library 572. In particular, the stratification factors 683 and/or randomization techniques 684 are applied via the corresponding randomization algorithm. This randomization algorithm can inherently apply the stratification factors 683 and/or randomization techniques, for example, based on being explicitly defined/configured to do so for all cases. Alternatively, different stratification factors 683 and/or randomization techniques 684 can be configured (e.g. automatically and/or based on user input) for different clinical trials, where the corresponding randomization algorithm handles the randomization techniques 684 and/or stratification factors 683 as function input and/or is otherwise executed via applying the specified randomization techniques 684 and/or stratification factors 683.

The trial arm assignment data 631.1 - 631.S for the set of participants can thus be generated based on applying the corresponding patient assignment method data 682 (e.g. performing the randomization techniques 684 while applying the corresponding stratification factors 683, optionally via execution of a corresponding function by the medical data processing system 500 as denoted by function identifier 559). For example, the patient assignment method data 682 dictates that first, participants are stratified into a plurality of stratification factor-based groups via stratification factor data 683, and that second, each of the plurality of stratification factor-based groups are separately processed via randomization techniques 684 to equally disperse participants in each of the plurality of group randomly between the control group and the investigational group (e.g. via permuted block technique).

In some embodiments, as discussed in further detail herein, a stratification factor indicated by stratification factor data 683 for some or all given clinical trials is implemented as medical outcome prognosis scores 636 for medical outcome type 637.x that are generated for the set of participants (e.g. based on processing their corresponding medical data 562 collected prior to conducting the trial), and/or some function of medical outcome prognosis scores 636 for medical outcome type 637.x that are generated for the set of participants. Thus, patients are stratified in conjunction with randomizing patients into the control arm and investigational arm via a metric configured to be indicative of the primary endpoint 651 for the trial: the medical outcome prognosis scores 636 having medical outcome type 637.x matching that of the primary endpoint 651.

In some embodiments, the medical outcome prognosis score 636 of medical outcome type 637.x the sole stratification factor of the patient assignment method data 682 of a given clinical trial (e.g. sex and/or age are additional stratification factors 683). In other embodiments, the medical outcome prognosis score 636 of medical outcome type 637.x is one of a plurality of stratification factors 683 of the patient assignment method data 682 of a given clinical trial (e.g. sex and/or age are additional stratification factors 683). In other embodiments, medical outcome prognosis score 636 of medical outcome type 637.x is not a stratification factor of the patient assignment method data 682 of a given clinical trial, where other stratification factors are utilized instead (e.g. sex and/or age). For example, the stratification factors can be implemented as and/or based on one or more of: Histology; PD-L1 Expression; Sex / Gender; Smoking Status; Prior Therapy; TNM Stage; Performance Status; Age; and/or Race / Ethnicity.

Figure 3D presents an embodiment of information that is included in, conveyed by, mapped to, generated for, and/or otherwise associated with a given function entry 571 denoting a function for execution by medical data processing system 100, and/or any computing device receiving and/or storing the corresponding function definition, for example, in conjunction with executing application data associated with the medical data processing system 100. Some or all features of function entry 571 of Figure 3D can implement any embodiment of function entry 571 566 described herein. Some or all functions that are trained and/or executed as described herein can have corresponding data (e.g. model parameters, etc.) stored as a corresponding function entry 571, for example, in function library 572.

A function entry 571 can include/indicate a corresponding function identifier (e.g. function name) which can uniquely distinguish the function entry 571 from other function entries; can denote a callable name of the function that can be denoted in a function call to execute the function (e.g. in a command entered via user input), and/or can otherwise be mapped to the function and/or indicate the function.

Some or all function entries 571 can correspond to machine learning models, for example, trained and/or execution via artificial intelligence techniques and/or machine learning techniques. Some or all function entries 571 can correspond to other types of function that optionally do not implement artificial intelligence techniques and/or machine learning techniques.

A function entry 571 can alternatively or additionally include/indicate corresponding function definition data 660. The function definition data 660 can define how the corresponding function is executed and/or how corresponding function is configured.

The function definition data 660 can include/indicate/be implemented based on model parameter data 661 for a corresponding model (e.g. a machine learning model and/or artificial intelligence model trained via artificial intelligence/machine learning). For example, the function definition data 660 stores values for a plurality of tuned weights/tuned parameters of a corresponding type of model. As a particular example, the function definition data 660 stores values for a plurality of tuned weights/tuned parameters of a corresponding convolutional neural network, such as a three-dimensional convolutional neural network. Alternatively or in addition, the function definition data 660 stores values for a plurality of tuned parameters defining a computer vision model, for example, that is applied to medical scans/other image data of medical data 652 when executed. As another example, the function definition data 660 stores values for a plurality of tuned parameters defining a natural language processing model, for example, that is applied to report data/medical history data/other text data of medical data 652 when executed.

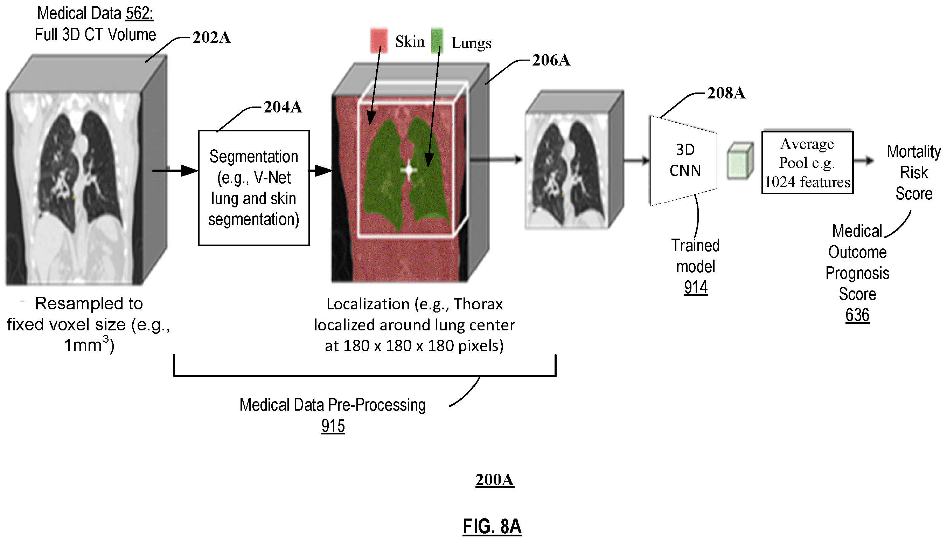

As a particular example, the model parameter data 661 defines a corresponding model 112, such as a 3DCNN model and/or any embodiment of model 112 described herein. For example, the function definition data 660 for one or more functions performed as described herein train, execute, and/or are otherwise performed based on applying a corresponding model 112, for example with some or all features and/or functionality discussed in conjunction with Figure 1B and/or one or more of Figures 8A - 9R.

The function definition data 660 can alternatively or additionally include/indicate/be implemented based on an input data type 662 indicating input processed via the corresponding function. For example, the input data type 662 for a given function is a particular type of medical data 652; a particular portion of a given type of medical data 652; a set of multiple medical data of one or more types 652, and/or one or more portions of a set of multiple medical data 652; etc. As a particular example, the input data type 662 includes a set of values 601 of one or more medical data value sets 600 of one or more medical data types (e.g. as indicated by medical data type data 615). As another particular example, the input data type 662 includes one or more medical outcome prognosis scores 636. In some embodiments, the input having input data type 662 can optionally be implemented as a feature vector (e.g. and/or any ordered set of values, such as values 601 of one or more medical scan 652; corresponding medical finding data 616 for the one or more medical data 652; corresponding medical data capture data 611 one or more medical data 652; corresponding other relevant data 640 for a corresponding patient, etc.) utilized as input to a corresponding machine learning model. The input having input data type 662 can otherwise be processed by the function as defined by the function definition 660 to render generation of corresponding output. In some embodiments, the input data type 662 for a given function indicates multiple different types of medical data 652, such as multiple different types of medical images of a given patient, be utilized as input, for example, in conjunction with implementing a multi-modal model.

The function definition data 660 can alternatively or additionally include/indicate/be implemented based on an output data type 663 indicating output generated via the corresponding function. For example, the output data type 662 for a given function is a particular type of medical data 652; a particular portion of a given type of medical data 652; a set of multiple medical data of one or more types 652, and/or one or more portions of a set of multiple medical data 652; etc. As a particular example, the output data type 663 is model-detected finding data 617. As another particular example, the output data type 663 includes at least one medical outcome prognosis score 636. As another particular example, the output data type 662 includes trial arm assignment data 631 for some or all patients of a participant set 650. The output having output data type 663 can otherwise be generated by the function as defined by the function definition 660 via processing of corresponding input.

The function definition data 660 can alternatively or additionally include/indicate/be implemented based on executable instruction data 670, for example, indicating executable code and/or other instructions that can be executed upon given input to render the corresponding output, for example, via applying the model parameter data 661.

A function entry 571 can alternatively or additionally include/indicate a corresponding version identifier 664, for example, indicating a corresponding version of the respective function. For example, a given type of machine learning model is improved over time (e.g. retrained as new data is available and/or to improve accuracy of the model), and each model has different versions. This can enable identifying which data is generated by older vs. most recent version, can enable reverting to use of older versions of functions, and/or can otherwise enable tracking, storage, and/or usage of multiple function versions.

A function entry 571 can alternatively or additionally include/indicate training set data 665, for example, indicating data identifiers 552, 554, and/or 556 for corresponding medical data 562, corresponding patient data 564, and/or corresponding clinical trial data 566, and/or otherwise indicating which data was utilized as training data in training a corresponding model (e.g. was utilized to tune the model parameters of model parameter data 661). This can be utilized to enable retraining of models utilizing some or all training data, enable removal/adding of new training data when new model versions are trained, etc. In some embodiments, the function entry 571 does not correspond to a machine learning model and/or otherwise does not indicate training set data 665, for example, based on not being generated via a training set.

Figure 3E presents an example embodiment of a set of function entries 571 of function library 562, indicating a set of functions that can be executed via medical data processing system 500 in accordance with enabling some or all functionality described herein.

The set of function entries 571 can include/indicate one or more medical outcome prognostication functions 671.1 - 671.D₁. Some or all medical outcome prognostication functions 671 can be configured to generate medical outcome prognostication data 636 (e.g. a corresponding value or set of values, such as a corresponding predicted measurement and/or corresponding probability/confidence/variance/distribution data associated with the predicted measurement) having a given medical outcome type 637 for a given patient as a function of input data having input data type 662 corresponding to: some or all values 601 of one or more medical data 652 (e.g. one or more medical images, etc.) of a given patient; values indicated in corresponding medical data metadata 610 for the one or more medical data 652 of the given patient, such as medical finding data 616 for the one or more medical data 652 and/or information indicated in corresponding medical data capture data 611 of one or more medical data 652; corresponding other relevant data 640 for the corresponding patient; and/or other input.

In some embodiments, different ones of the D₁ medical outcome prognostication functions 671 can correspond to: generate medical outcome prognostication data for 636 different medical outcome type 637; process different input data types 662 (e.g. different types of medical data) to generate medical outcome prognostication data for 636 a given medical outcome type 637; process same input data types 662 to generate medical outcome prognostication data for 636 a same medical outcome type 637, but being implemented via different machine learning model types, for example, with different corresponding types of parameters configured in model parameter data 661 and/or different corresponding executable instructions to apply the different models differently; process same input data types 662 to generate medical outcome prognostication data for 636 a same medical outcome type 637 via a same type of machine learning model, but having different versions (e.g. different version identifiers 664 based on being improved over time via retraining a corresponding model indicated by model parameter data 661, for example, rendering different values for the set of model parameters indicated by model parameter data 661); and/or functions with other differences.

Some or all medical outcome prognostication functions can be implemented via corresponding machine learning models (e.g. having tuned parameters indicated in model parameter data 661 defining the corresponding model). For example, a given medical outcome prognostication functions 671 is implemented via a set of tuned models indicated by model parameter data 661 that were generated via training the corresponding machine learning model, for example, via execution of a model training function 673 upon corresponding training set data 665.

The training set data 665 utilized to train such a machine learning model can include a plurality of training data each having corresponding input/independent variable values and/or one or more corresponding output/dependent variable values of historical data. The corresponding input/independent variable values of each training data can correspond to historical data of input data type 662 of the respective medical outcome prognostication functions 671 (e.g. some or all values 601 of one or more medical data 652 of a given historical patient; medical finding data 616 for the one or more medical data 652 of the historical patient; corresponding medical data capture data 611 one or more medical data 652 of the historical patient; corresponding other relevant data 640 for the historical patient, etc.). The one or more corresponding output/dependent variable values can correspond to corresponding historical data of output data type 662 of the respective medical outcome prognostication function 671, such as truth data for the medical outcome for the corresponding historical patient, having medical outcome type 637 for which the corresponding medical outcome prognostication function 671 is configured to generate its medical outcome prognostication data 636. For example, this truth data for the medical outcome for a corresponding historical patient is implemented as/is based on outcome data 638 of medical outcome truth data 639 for the historical patient, for example, indicated in their patient data 654 and/or in medial data metadata 661 of medical data 562 captured for the historical patient. As another example, this truth data for the medical outcome for a corresponding historical patient is implemented as/is based on endpoint measurement data 632 for the historical patient collected in their participation in a past clinical trial having a primary endpoint having medical outcome type 637 for which the corresponding medical outcome prognostication function 671 is configured to generate its medical outcome prognostication data 636.

The set of function entries 571 can alternatively or additionally include/indicate at least one trial arm assignment function 672. The one or more trial arm assignment functions 672 can be configured to generate trial arm assignment data 631 for a given patient (and/or for a given set of patients, such as all patients in a participant set 650 processed in tandem) as a function of input data having input data type 662 corresponding to: stratification factors 683; and/or randomization techniques 684. Alternatively or in addition, these stratification factors 683 and/or randomization techniques 684 are predetermined for the trial arm assignment function 672, for example, in corresponding function definition data 660 (e.g. executable instruction data 670). As a particular example, the randomization technique 684 can be implemented as permuted block randomization algorithm, for example, based on permuted block randomization being specified in the input to the function and/or the function being configured to always apply permuted block randomization. In some embodiments, the one or more trial arm assignment functions 672 can be configured to generate trial arm assignment data 631 for a given patient (and/or for a given set of patients, such as all patients in a participant set 650 processed in tandem) as a function of input data having input data type 662 corresponding to a medical outcome prognosis score for the given patient/for each patient in the given set of patients, for example, where the medical outcome prognosis score 636 is applied by the trial arm assignment functions 672 as the stratification factor of stratification factor data 683 to which the one or more randomization techniques is applied 684.

The set of function entries 571 can alternatively or additionally include/indicate at least one model training function 673. The one or more model training functions 673 can be configured to generate a trained machine learning model, such as a trained machine learning model implemented by a corresponding medical outcome prognostication function 671 and/or other function of function library (e.g. function trained to generate model-detected finding data 617; etc.) . For example, the output data type 663 of the model training function 673 is a set of tuned parameters defining a corresponding type of model, where this set of tuned parameters is stored/applied as model parameter data 661 of a corresponding medical outcome prognostication function 671 that is trained via the model training function 671 from corresponding input. The input data type 662 can correspond to a set of training data The input data type 662 can alternatively or additionally configuration of the model generation (e.g. model type; configurable instructions regarding model training/optimization/validation; etc.). In some embodiments, multiple different types of model training functions 673 are implemented, for example, where different model training functions 673 are implemented to generate different types of machine learning models; are implemented to train models (e.g. a same type of model) to be applied different types of input data (e.g. different types/portions of medical data 652 and/or corresponding medical data metadata 610 and/or patient data 654); and/or are generated to predict different types of output, which can include one or more types medical outcome prognosis scores 636, one or more types of model-detected finding data 617, and/or any other automatically generated data and/or output of machine learning functions described herein.

In some embodiments, a given model training function 673 can be configured to train a corresponding machine learning model (e.g. implemented by a corresponding function such as a medical outcome prognostication function 671 and/or any other function applying corresponding tuned parameters of the corresponding machine learning model as model parameter data 661) having a machine learning model type implemented as and/or based on: an anomaly detection model, a decision tree, a model utilizing association rules, an expert system, a knowledge-based system, a computer vision model, a natural language processing model, an artificial neural network, a convolutional neural network, a regression model (e.g. logistic regression model, linear regression model, nonlinear regression model, etc.) such as a three-dimensional convolutional neural network, a support vector machine, a Bayesian network, a genetic algorithm-based model, a feature-learning based model, a sparse dictionary learning-based model, a preference learning-based model, a deep learning-based model, a K-Nearest-Neighbors model, a K-Means model, and/or any other type of model trained via supervised learning techniques, unsupervised learning techniques, and/or any other machine learning techniques and/or artificial intelligence techniques.

In some embodiments, some or all medical outcome prognostication functions 671 and/or some or other functions applying a corresponding trained machine learning model are not trained via a model training function 673 executed by the medical data processing system 500. For example, some or all medical outcome prognostication functions 671 and/or some or other functions of function library 562 applying a corresponding trained machine learning model are trained elsewhere and/or their model parameter data 661 is otherwise determined/received/accessed/configured via user input and/or via an automatic process/etc.

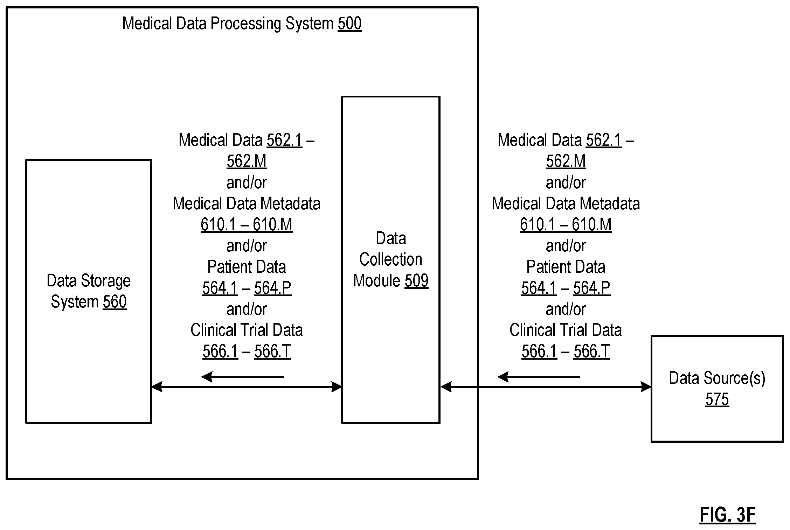

Figure 3F illustrates an embodiment of medical data processing system 500 that implements a data collection system 509 operable to collect some or all data stored in data storage system 560 from one or more data sources 575. For example, the data collection system 509 is operable to: generate requests for/instructions to send one or more types of data described herein; send these requests/instructions to one or more data sources 575; receive data from the one or more data sources 575 (e.g. in response to the requests and/or instructions, or otherwise receive data generated and/or sent by the data sources 575); extract relevant data from the received data and/or format the received data; and/or otherwise process the data for storage; and/or store the data (e.g. raw received data; extracted data; formatted data; otherwise processed data) in data storage system 560.

In some embodiments, this data that is generated and/or sent by data sources 575; and/or that is requested, received, processed, and/or stored by a data collection system 509; can include: some or all portions of some or all medical data 562.1 - 562.M, such as some or all values 601 of medical data value set 600 of some or all medical data 562.1 - 562.M, for example, in accordance with a corresponding file format and/or other formatting of the corresponding type of medical data; some or all portions of some or all medical data metadata 610.1 - 610.M, for example, in accordance with a corresponding file format and/or other formatting of the corresponding type of medical data (e.g. time and/or data 612; provider and/or location data 613 medical device ID and/or type 614; medical data type data 615; any other medical data capture data 611; human-detected finding data 618 and/or any other medical findings data 616; medical outcome truth data 639 such as outcome data 638 for one or more medical types; and/or any other metadata/information regarding the corresponding medical data 654 and/or corresponding patient 564); ; some or all portions of some or all patient data 564.1 - 564.P (e.g. demographic data 641l medical history data 642; family history data 643; risk factor data 644 and/or other relevant data 640; trial IDs for clinical trials the patient is a candidate for, is currently participating in, and/or participated in in the past; etc.; corresponding endpoint measurement data 632 for these trials, for example, for storage as outcome data 638 for corresponding medical data metadata 610, etc.); some or all portions of some or all clinical trial data 566.1 - 566.T, (e.g. primary endpoint 651; patients of participant set 650 and/or their corresponding endpoint measurement data 632, once collected; stratification factors 683, randomization techniques 684, and/or other instructions/data regarding participant assignment method data 682; etc.; and/or any other data described herein.

In some embodiments, this data that is generated and/or sent by data sources 575; and/or that is requested, received, processed, and/or stored by a data collection system 509 is collected over time, where different data is requested and/or received separately over time. For example, different medical data 652 and/or corresponding medical data metadata 610, and/or different portions of given medical data 652 and/or corresponding medical data metadata 610 is received at different times based on being requested separately and/or based on having been captured/generated/received by data source(s) 575 at different times (e.g. different patients have medical data collected on different dates; a same patient has different medical data collected over time; a given patient has medical data captured at one time and medical outcome data determined at a later time; the medical data processing system requests medical data meeting different criteria at different times in conjunction with training/executing a particular function and/or satisfying a particular request by a requesting entity; etc.); different patient data 564 and/or different portions of given patient data 654 is received at different times based on being requested separately and/or based on having been requested separately and/or based on having been generated/received by data source(s) 575 at different times (e.g. different patients have patient data collected on different dates; a same patient has different medical data collected over time; a given patient has medical data 652, relevant data 640 and/or candidacy/participation in a clinical trial determined within a first temporal period and has endpoint measurement data (e.g. medical outcome data) determined at a later time after the first temporal period; etc.); different clinical trial data 566 and/or different portions of given clinical trial data 566 is received at different times based on being requested separately and/or based on having been requested separately and/or based on having been generated/received by data source(s) 575 at different times (e.g. different clinical trials are initiated/completed on different dates; a given clinical trial has different patients identified as candidates/confirmed participants on different dates while the clinical trial is ongoing; the status of endpoint measurement data changes during the trial, where a given patient is identified as a participant of the trial and is assigned to a corresponding trial arm, such as either a control arm or an investigational arm, during a first temporal period, and the endpoint measurement data 632 is measured for the patient during a second temporal period after the first temporal period based on treating/observing the patient in conjunction with their assigned trial arm; etc.)

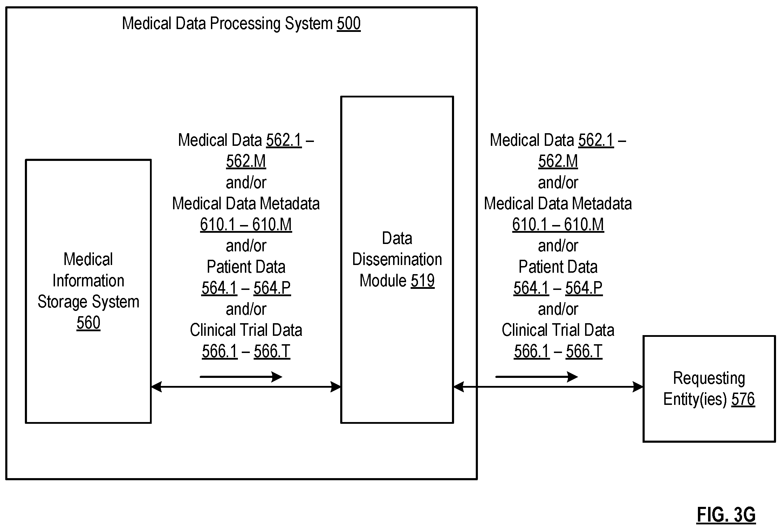

Figure 3G illustrates an embodiment of medical data processing system 500 that implements a data dissemination system 519 operable to send/otherwise communicate some or all data stored in data storage system 560 to one or more requesting entities 576. For example, the data dissemination system 519 is operable to: receive requests for/instructions to send one or more types of data described herein; facilitate processing of these requests/instructions and/or otherwise acquiring the data determined to be sent to the requesting entities based on fetching corresponding data from the data storage system 560 and/or facilitating generation of the corresponding data, for example, via execution of at least one function of function library 572 and/or via initiating corresponding functionality of at least one subsystem 501; and/or communicating the data to the one or more requesting entities 576 (e.g. for display/storage/further processing and/or transmission by requesting entities 576) based on transmitting the data to the requesting entity via network 550 and/or transmitting corresponding instructions/ for display/storage of this data; facilitating display of the corresponding data via a display device of requesting entity 576; facilitating storage of the corresponding data via a via memory resources of requesting entity 576; etc.). Some or all of this data communicated to the requesting entities 576 is data that was generated by the medical data processing system, for example, via execution of at least one function of function library 572 and/or via implementing functionality of at least one subsystem 501.

In some embodiments, this data that is requested and/or otherwise determined to be communicated to requesting entity(ies) 576; and/or that is generated, processed, sent, and/or otherwise communicated by a data collection system 509; can include: some or all portions of some or all medical data 562.1 - 562.M and/or some or all portions of some or all medical data metadata 610.1 - 610.M; some or all patient data 564.1 - 564.P; and/or some or all portions of some or all clinical trial data 566.1 - 566.T (e.g. for one or more patients, a medical outcome prognosis score 636 for one or more medical outcome types 637 and/or model-detected finding data 618 and/or any other medical findings data 616 for corresponding medical data 562; for one or more clinical trials, trial arm assignment data 631 for one or more participants; any other data generated by the medical data processing system 500 as described herein; any other data that was optionally not generated by the medical data processing system 500 itself but was instead received by and/or extracted from data received from data sources 575, for example, for display/storage in conjunction with corresponding data generated by the medical data processing system 500; any function entries 571 for any functions to be executed by the requesting entity itself, such as model parameter data 661 for one or more functions trained by the medical data processing system 500; and/or any other data described herein.

In some embodiments, this data that is generated and/or sent by data sources 575; and/or that is generated/communicated by a data dissemination system 519 is generated/communicated over time, where different data is requested, generated, and/or communicated separately over time. For example, different data is generated/communicated at different times based on being requested by the same or different requesting entity at different times; different data is generated/communicated at different times based on being generated from other data that is collected/stored by data collection entity 509 at different times; different medical outcome prognosis scores 636 for different medical outcome types 637 is generated/communicated for a same patient at different times based on their participation in different trials with different corresponding primary endpoints at different times, based on being generated via processing corresponding medical data and/or other data that is collected at different times, and/or based on being updated over time for the patient as new medical data/other corresponding data is collected/received by the medical data processing system 500; different medical outcome prognosis scores 636 are generated/communicated for different patients at different times based on their participation in a same or different trials starting at different times, and/or based on being generated via processing corresponding medical data and/or other data that is collected at different times for the different patients; different trial arm assignment data 631 being generated/communicated for different patients at different times based on their participation in same or different trials starting at different times, and/or based on corresponding different medical outcome prognosis scores 636 utilized to generate the different trial arm assignment data 631 being generated at different times; different trial arm assignment data 631 being generated/communicated a same patient at different times based on their participation in different corresponding trials starting at different times, and/or based on corresponding medical outcome prognosis scores 636 (e.g. of different medical outcome types 637) utilized to generate the different trial arm assignment data 631 being generated at different times (e.g. based on starting the trials at different times and/or being generated via processing corresponding medical data and/or other data that is collected at different times for the given patient); different model parameter data 661/corresponding functions being trained/generated at different times for different model, for example, in conjunction with new training data being updated over time and/or different types of models being generated to generate different medical outcome prognosis scores 636 for different medical outcomes based on different primary endpoints of clinical trials conducted over different corresponding temporal periods; etc.)

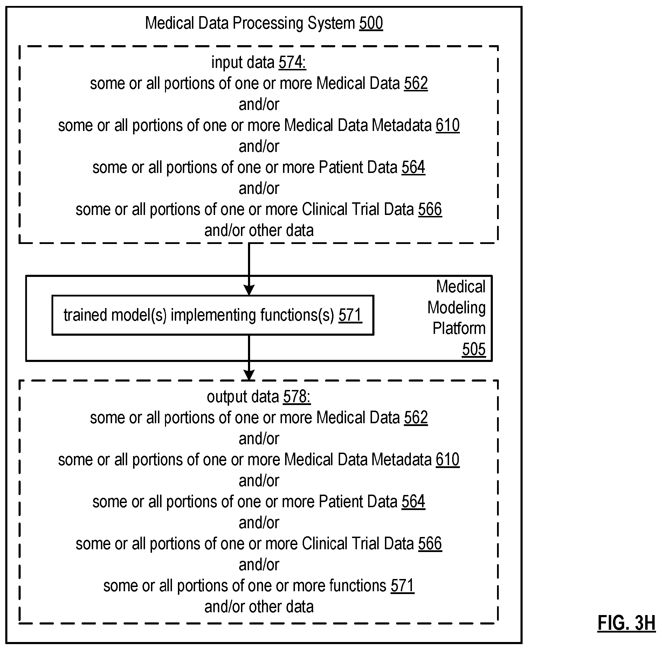

Figure 3H is a schematic block diagram of a medical data processing system 500 that implements a medical modeling platform 505. For example, medical modeling platform 505 can be implemented as a subsystem 501 and/or can otherwise be implemented via processing and/or memory resources of medical data processing system 500. Some or all features and/or functionality of medical data processing system 500 of Figure 3H can implement any embodiment of medical data processing system 500 described herein.

Implementing medical modeling system 505 can include applying a medical modeling platform 505 to different types/combinations of one or more types of input data 574 at different given times (e.g. for different given patients or different given clinical trials) to generate respective output 578 of the same or different one or more types. Implementing medical modeling system 505 can alternatively or additionally include applying a medical modeling platform 505 to generate different types/combinations of one or more types of output data 578 at different given times (e.g. for different given patients or different given clinical trials) via processing respective input data 574 of the same or different one or more types. Implementing medical modeling system 505 can alternatively or additionally include implementing different types/combinations of one or more types of functions 571 at different given times (e.g. for different given patients or different given clinical trials) to generate respective output 587 from given input 574.

In some embodiments, some or all of one or more models can be implemented as multi-modal models, for example, implemented to be applied to various different types of input included in input data 574. In some embodiments, different applications of the one more models can include applying different types of input data 574. In such cases, such multi-modal models can optionally be considered as execution of multiple different functions 571 upon respective input independently or in tandem, with these different functions 571 optionally being designated to handle different types of input (e.g. multiple unimodal models implementing a set of different functions 571 are applied in parallel to different overlapping or non-overlapping portions of input data 574 to generate respective output, and/or a further function 571 is implemented as a fusion model that further processes this output of the set of functions to generate its own output). For example, different sets of information is available for different patients, and available information is utilized as input, even if some patient input data is more complete/includes more information than others, and respective output is generated accordingly.

Alternatively or in addition, some or all of one or more models can be implemented as multi-output models, for example, implemented to generate various output, where different applications of the one more models can include generating same or different combinations of one or more outputs included in the output data 578.

In some embodiments, any training and/or execution of various functions 571 described herein can be implemented via a medical modeling platform 505. In particular medical modeling platform can implement training and/or execution of one or more functions 571 upon given input data 574 to generate respective output data 578, and/or to facilitate training/retraining of functions 571. In some embodiments, performance of any of the functions 571 described herein can be in accordance with applying one or more models trained via artificial intelligence and/or machine learning techniques. In some embodiments, any prediction data, prognosis data, and/or inference data generated via one or more corresponding functions 571 can be generated via medical modeling platform. In some embodiments, any functions 571 described herein can be trained/retrained via medical modeling platform 571.

Any such model of models implemented via medical modeling platform 505 can implement some or all features and/or functionality of modeling platform of Figs 8A - 9R and/or any embodiment of modeling platform described herein. Any such model of models implemented via medical modeling platform 505 can be implemented via some or all features and/or functionality of AI platform 588, for example, that is implemented by/as medical data processing system. Any such model or models described herein can be implemented via one or more convolutional neural networks, models implementing generative artificial intelligence, and/or any other types of models.

Figures 4A - 4I illustrate embodiments of a medical data processing system 500 that generates trial arm assignment data 631 for participants of a clinical trial as a function of medical outcome prognosis scores 636 implements a medical outcome prognostication model training system 506, medical outcome prognostication system 507, and/or a prognosis-based trial arm assignment system 508.

In some embodiments of enabling some or all features and/or functionality of the medical data processing system 500 (e.g. of the medical outcome prognostication training system 506; of the medical outcome prognostication system 507; and/or of the prognosis-based trial arm assignment system 508), given medical outcome type 637 is configured (e.g. automatically selected by the medical data processing system 500) to, based on a given clinical trial having the at least one medical outcome type 637 as its primary endpoint 601: have a corresponding medical outcome prognostication function 671 trained to predict the corresponding medical outcome type 637; have a corresponding trained medical outcome prognostication function 671 performed upon medical data 562 and/or other relevant information 640 for a set of prospective participants of the clinical trial to generate medical outcome prognosis scores 636 of the corresponding medical outcome type 637 for this set of prospective participants; and/or to have this set of prospective participants grouped into at least one control group for the clinical trial and into at least one investigative group (i.e. experimental group and/or treatment group) for the clinical trial, via a randomization algorithm, as a function of these medical outcome prognosis scores 636 (e.g. where the medical outcome prognosis scores 636 are utilized as a patient stratification factor in applying the randomization algorithm).

Such functionality can improve one or more technological fields, such as fields of medicine, healthcare, medical research, medical product development, medical treatment, medical devices, healthcare products, and/or pharmaceuticals. In particular, the use of medical outcome prognosis scores 636 in randomizing patients for clinical trials can improve the effectiveness of the corresponding clinical trial by stratifying patients more accurately, for example, by inherently accounting for more factors that contribute to the primary endpoint via use of these factors as input to a corresponding machine learning model that generates corresponding medical outcome prognosis scores for this primary endpoint. Thus, different trial arms can be randomized more effectively (e.g. more equally distributing patients likely vs. unlikely to exhibit one or more corresponding outcomes being measured between the control group and experimental groups of the clinical trial), which can improve the accuracy of the clinical trial, which can render improvements to the effectiveness of corresponding medical treatments/products being tested (e.g. renders pharmaceutical compounds and/or medical devices that are more effective in treating one or more medical conditions).

Furthermore, in some cases where this single score is utilized to stratify patients rather than multiple factors, a smaller number of patients are required to complete the trial, which can improve the technology of fields of medicine and/or pharmaceuticals based on improving the speed by which clinical trials are conducted, enabling faster scientific discovery of treatment of diseases/research advancement of corresponding medical conditions and their corresponding treatment. For example, randomizing over more factors can ensure that more conditions are accounted for, but can result in more complicated patient assignment and can require larger numbers of participants and/or more specific searching for participants meeting certain requirements to ensure all factors are balanced evenly. Meanwhile, computing a single, predictive score characterizing predicted outcome of the trial, for example, explicitly or inherently a function of multiple such factors based on a richness of one or more corresponding medical data (e.g. medical image data, DNA sequencing data, RNA transcription data, proteomic data, genomic data, metabolomic data, metagenomic data, phenomics data, transcriptomic data, medical test data, and/or other medical data, for example, comprising hundreds, thousands, and/or millions of individual values) can reduce the number of participants necessary based on reducing the number of factors being randomized, while still inherently accounting for some or all of these factors based on the impact of these factors being preserved in the overall predictive score.

Such improvements to the technology of medicine and/or pharmaceuticals can be enabled through the implementing of custom computing technology that is enabled via the training of machine learning functions upon a training set of data (e.g. rich medical data, such as medical image data, DNA sequencing data, and/or medical test data, labeled with a known medical outcome) via one or more artificial intelligence techniques and/or machine learning techniques, for example, rendering particular, tuned parameters of a corresponding machine learning model configured to predict a corresponding medical outcome. These sophisticated machine learning models can then be applied to new input (e.g. for participants of a clinical trial) to predict the corresponding primary outcome. Such improvements to the technology of medicine and/or pharmaceuticals require the training and use of a corresponding machine learning model, for example, based on analysis of the corresponding training data being infeasible for performance by the human mind (e.g. the raw data of the medical data, such as its hundreds, thousands, and/or millions of data points, are not easily consumable by the human mind; the human mind not being feasibly able process hundreds or thousands of training data to generate a corresponding model; the human mind not being feasibly able to identify relationships between various values/attributes of the input and/or not being feasibly able to relate various values/attributes of the input to their impact on the medical outcome, and thus not being able to tune a set of parameters (e.g. optionally dozens, hundreds, or thousands of weights) defining a corresponding model that can accurately predict medical outcome; the human mind not being able to predict medical outcome with the same accuracy as a trained model; and/or the human mind not being able to perform processing required to train and/or execute a corresponding model in a feasible amount of time, even when assisted by pen and paper.

As a particular example, while a human may be inclined to stratify patients by simple, easily-observable factors that may affect medical outcome (e.g. age, sex, etc.), the medical outcome prognosis scores generated by the model are generated via processing of many more data points indicative of factors that are not observable by a human and/or in a way that is not feasible for performance by the human mind. Yet the use of these model-generated medical outcome prognosis scores, instead of or in addition to more easily observable factors such as patient age and/or sex, to stratify participants of a clinical trial can greatly improve the ability to conduct the clinical trial quickly and/or via fewer patients and/or can improve the accuracy of trial results based on patients being more evenly distributed by pre-disposition to exhibit the corresponding medical outcome being tested as the primary outcome, which renders more effective corresponding medical products which improves the corresponding technology of these medical products as discussed previously.

For example, a medical outcome type corresponds to a mortality metric (e.g. amount of time until death, whether or not the patient died within a specified amount of time, etc.), for example, for use in conducting and/or otherwise based on at least one clinical trial for a treatment for an often fatal disease (e.g. a type of cancer) having mortality/survival as its primary endpoint. A corresponding medical outcome prognosis score 636 for such a medical outcome type can indicate a corresponding predicted morality metric. For example, medical outcome prognosis scores 636 for this type are generated via a corresponding medical outcome prognostication function 671 trained to output this predicted mortality metric as a function of at least one corresponding medical data, such as medical image data. In particular, this medical outcome prognostication function 671 was optionally trained via a set of medical data of the corresponding type for other historical patients having logged truth data for this mortality metric, where this mortality metric truth data was optionally utilized as training output data mapped to the corresponding medical data utilized as training input data in training a corresponding machine learning model. Such medical outcome prognosis scores 636 can be generated for prospective participants of such clinical trials for treatment of the fatal disease via processing of their medical data (e.g. medical image data) via this corresponding medical outcome prognostication function 671, where these prospective participants are assigned to either the control or investigative group of this clinical trial based on their medical outcome prognosis scores 636, indicative of the predicted mortality metric and thus indicative of the predicted primary outcome for this clinical trial.

As another example, a medical outcome type corresponds to a hospitalization metric (e.g. time until hospitalization, whether or not hospitalization occurred within a specified amount of time, count of hospitalizations in a specified amount of time, etc.), for example, induced by a corresponding condition (e.g. heart failure), for example, for use in conducting and/or otherwise based on at least one clinical trial for a treatment for a medical condition (e.g. one or more types of cardiovascular events) inducing this type of hospitalization (e.g. heart failure hospitalization) having this measure of hospitalization as a primary endpoint. A corresponding medical outcome prognosis score 636 for such a medical outcome type can indicate a predicted value for the hospitalization metric. For example, medical outcome prognosis scores 636 for this type are generated via a corresponding medical outcome prognostication function 671 trained to output this predicted hospitalization metric as a function of at least one corresponding medical data, such as medical image data. In particular, this medical outcome prognostication function 671 was optionally trained via a set of medical data of the corresponding type for patients having truth data for this hospitalization metric, where this hospitalization metric truth data was optionally utilized as training output data mapped to the corresponding medical data utilized as training input data in training a corresponding machine learning model. Such medical outcome prognosis scores 636 can be generated for prospective participants of such clinical trials for treatment of the medical condition inducing hospitalization via processing of their medical data (e.g. medical image data) via this corresponding medical outcome prognostication function 671, where these prospective participants are assigned to either the control or investigative group of this clinical trial based on their medical outcome prognosis scores 636, indicative of the predicted hospitalization metric and thus indicative of the predicted primary outcome for this clinical trial.

As another example, a medical outcome type corresponds to count metric (e.g. count, frequency, whether this count or frequency exceeds a specified threshold, etc.) of negative effects (e.g. migraines, seizures, etc.) experienced, for example, within a specified amount of time, for example, for use in conducting and/or otherwise based on at least one clinical trial for a treatment for a medical condition inducing these negative effects (e.g. migraines, seizures) having number of such negative effects as a primary endpoint. A corresponding medical outcome prognosis score 636 for such a medical outcome type can indicate predicted counts of such effects. For example, medical outcome prognosis scores 636 for this type are generated via a corresponding medical outcome prognostication function 671 trained to output this predicted count metric of negative effects as a function of at least one corresponding medical data, such as medical image data. In particular, this medical outcome prognostication function 671 was optionally trained via a set of medical data of the corresponding type for other historical patients having logged truth data for this count metric, where this count metric truth data was optionally utilized as training output data mapped to the corresponding medical data utilized as training input data in training a corresponding machine learning model. Such medical outcome prognosis scores 636 can be generated for prospective participants of such clinical trials for treatment of the negative effects via processing of their medical data (e.g. medical image data) via this corresponding medical outcome prognostication function 671, where these prospective participants are assigned to either the control or investigative group of this clinical trial based on their medical outcome prognosis scores 636, indicative of the predicted count metric and thus indicative of the predicted primary outcome for this clinical trial.

As another example, a medical outcome type correspond to a medically-defined scale-based metric, for example, corresponding a score in accordance with a corresponding scale defined in a corresponding medical field, for example, after a specified amount of time has passed, for use in use in conducting and/or otherwise based on at least one clinical trial for a treatment for a medical condition (e.g. Alzheimer's disease) whose severity and/or existence is measured based on this corresponding score. A corresponding medical outcome prognosis score 636 for such a medical outcome type can indicate predicted scores for such a medically-defined scale. For example, the medical outcome type corresponds to an ADAS-Cog score and/or a Mini-Mental State Examination (MMSE) score, and the corresponding medical outcome prognosis score 636 corresponds to a predicted ADAS-Cog score and/or predicted MMSE score, for use in conducting a clinical trial testing a treatment for Alzheimer's disease and/or dementia. For example, medical outcome prognosis scores 636 for this type are generated via a corresponding medical outcome prognostication function 671 trained to output this predicted ADAS-Cog score and/or predicted MMSE score as a function of at least one corresponding medical data, such as medical image data. In particular, this medical outcome prognostication function 671 was optionally trained via a set of medical data of the corresponding type for other historical patients having logged truth data scores for this scale, where this scale score truth data was optionally utilized as training output data mapped to the corresponding medical data utilized as training input data in training a corresponding machine learning model. Such medical outcome prognosis scores 636 can be generated for prospective participants of such clinical trials for treatment of the corresponding medical condition (e.g. Alzheimer's disease and/or dementia) via processing of their medical data (e.g. medical image data) via this corresponding medical outcome prognostication function 671, where these prospective participants are assigned to either the control or investigative group of this clinical trial based on their medical outcome prognosis scores 636, indicative of the predicted score for this scale, and thus indicative of the predicted primary outcome for this clinical trial.

As another example, a medical outcome type corresponds to a weight-change metric (e.g. amount of weight change over a specified amount of time; percentage of weight change over a specified of time; final weight; whether or not amount/percentage of weight exceeds a specified threshold; etc.) for example, for use in conducting and/or otherwise based on at least one clinical trial for a weight-loss study. A corresponding medical outcome prognosis score 636 for such a medical outcome type can indicate predicted value for the weight-change metric. For example, medical outcome prognosis scores 636 for this type are generated via a corresponding medical outcome prognostication function 671 trained to output this predicted weight-change metric response as a function of at least one corresponding medical data. In particular, this medical outcome prognostication function 671 was optionally trained via a set of medical data of the corresponding type for other historical patients having logged truth data for this weight-change metric, where this weight-change metric truth data was optionally utilized as training output data mapped to the corresponding medical data utilized as training input data in training a corresponding machine learning model. Such medical outcome prognosis scores 636 can be generated for prospective participants of such clinical trials for weight loss via processing of their medical data via this corresponding medical outcome prognostication function 671, where these prospective participants are assigned to either the control or investigative group of this clinical trial based on their medical outcome prognosis scores 636, indicative of the predicted weight-change metric and thus indicative of the predicted primary outcome for this clinical trial.

As another example, a medical outcome type corresponds to an objective tumor response, for example, in accordance with Response Evaluation Criteria in Solid Tumors (RECIST) criteria, for example, for use in for use in conducting and/or otherwise based on at least one clinical trial for treating tumors/treating at least one type of cancer. A corresponding medical outcome prognosis score 636 for such a medical outcome type can indicate a predicted objective tumor response (e.g. as categorical output, indicating one of: Stable Disease; Partial Response; Progressive Disease; Stable Disease). For example, medical outcome prognosis scores 636 for this type are generated via a corresponding medical outcome prognostication function 671 trained to output predicted objective tumor response as a function of at least one corresponding medical data, such as medical image data. In particular, this medical outcome prognostication function 671 was optionally trained via a set of medical data of the corresponding type for other historical patients having logged truth data for this objective tumor response metric, where this truth data was optionally utilized as training output data mapped to the corresponding medical data utilized as training input data in training a corresponding machine learning model. Such medical outcome prognosis scores 636 can be generated for prospective participants of such clinical trials for treatment of the tumors/cancer via processing of their medical data (e.g. medical image data) via this corresponding medical outcome prognostication function 671, where these prospective participants are assigned to either the control or investigative group of this clinical trial based on their medical outcome prognosis scores 636, indicative of the predicted objective tumor response and thus indicative of the predicted primary outcome for this clinical trial.

As another example, a medical outcome type corresponds to a metric for measured change in a corresponding medical condition exhibited by a patient (e.g. change in size, change in frequency, change in count, etc.) for example, for use in for use in conducting and/or otherwise based on at least one clinical trial for treating this medical condition. A corresponding medical outcome prognosis score 636 for such a medical outcome type can indicate a predicted metric denoting a predicted measured change. For example, medical outcome prognosis scores 636 for this type are generated via a corresponding medical outcome prognostication function 671 trained to output predicted measured change response as a function of at least one corresponding medical data, such as medical image data. In particular, this medical outcome prognostication function 671 was optionally trained via a set of medical data of the corresponding type for other historical patients having logged truth data for this measured change metric, where this measured change truth data was optionally utilized as training output data mapped to the corresponding medical data utilized as training input data in training a corresponding machine learning model. Such medical outcome prognosis scores 636 can be generated for prospective participants of such clinical trials for treatment of the corresponding medical condition via processing of their medical data (e.g. medical image data) via this corresponding medical outcome prognostication function 671, where these prospective participants are assigned to either the control or investigative group of this clinical trial based on their medical outcome prognosis scores 636, indicative of the predicted change and thus indicative of the predicted primary outcome for this clinical trial.

As another example, a medical outcome type corresponds to a metric for pain (e.g. magnitude of pain, frequency of pain; change in pain; etc., for example, of a particular type and/or in a particular bodily location, for example, as reported by the patient and/or otherwise collected/determined, for example, in accordance with a corresponding scale/metric for measuring pain), for example, for use in for use in conducting and/or otherwise based on at least one clinical trial for treating (and/or for training a corresponding medical condition inducing) this type of pain/ and/or pain occurring at this location (e.g. chest pain). A corresponding medical outcome prognosis score 636 for such a medical outcome type can indicate a corresponding predicted pain metric. For example, medical outcome prognosis scores 636 for this type are generated via a corresponding medical outcome prognostication function 671 trained to output predicted measured pain as a function of at least one corresponding medical data, such as medical image data. In particular, this medical outcome prognostication function 671 was optionally trained via a set of medical data of the corresponding type for other historical patients having logged truth data for this pain metric, where this measured change truth data was optionally utilized as training output data mapped to the corresponding medical data utilized as training input data in training a corresponding machine learning model. Such medical outcome prognosis scores 636 can be generated for prospective participants of such clinical trials for treatment of the corresponding pain/corresponding medical condition inducing the pain via processing of their medical data (e.g. medical image data) via this corresponding medical outcome prognostication function 671, where these prospective participants are assigned to either the control or investigative group of this clinical trial based on their medical outcome prognosis scores 636, indicative of the predicted pain metric and thus indicative of the predicted primary outcome for this clinical trial.

As another example, a medical outcome type corresponds to a biomarker, for example, indicative of a corresponding medical condition and/or measured in conducting a corresponding test associated with testing for and/or measuring severity of a medical condition, for example, for use in for use in conducting and/or otherwise based on at least one clinical trial for treating this medical condition. A corresponding medical outcome prognosis score 636 for such a medical outcome type can indicate a corresponding predicted biomarker. For example, medical outcome prognosis scores 636 for this type are generated via a corresponding medical outcome prognostication function 671 trained to output this predicted biomarker as a function of at least one corresponding medical data, such as medical image data. In particular, this medical outcome prognostication function 671 was optionally trained via a set of medical data of the corresponding type for other historical patients having logged truth data for this biomarker, where this measured biomarker truth data was optionally utilized as training output data mapped to the corresponding medical data utilized as training input data in training a corresponding machine learning model. Such medical outcome prognosis scores 636 can be generated for prospective participants of such clinical trials for treatment of the corresponding medical condition via processing of their medical data (e.g. medical image data) via this corresponding medical outcome prognostication function 671, where these prospective participants are assigned to either the control or investigative group of this clinical trial based on their medical outcome prognosis scores 636, indicative of the predicted biomarker and thus indicative of the predicted primary outcome for this clinical trial.

As another example, a medical outcome type corresponds to a quality of life (QoL) assessment metric (e.g. metrics relating to levels of anxiety, depression, mobility, pain/ discomfort, usual activities, self-care, and/or other factors indicative of quality of life; and/or metrics relating to duration of quality of life, for example, treated as a binary condition having a measurable/predicted duration, where the medical outcome type optionally corresponds to a metric corresponding to quality of life years), for example, for use in conducting and/or otherwise based on at least one clinical trial for a treatment for a medical condition (e.g. cancer) where assessing quality of life is valuable in identifying whether a corresponding treatment is effective/worthwhile (e.g. rather than simply assessing effect on mortality), and/or otherwise having this measure of QoL as a primary endpoint. A corresponding medical outcome prognosis score 636 for such a medical outcome type can indicate a predicted value for the QoL metric. For example, medical outcome prognosis scores 636 for this type are generated via a corresponding medical outcome prognostication function 671 trained to output this predicted QoL metric as a function of at least one corresponding medical data, such as medical image data. In particular, this medical outcome prognostication function 671 was optionally trained via a set of medical data of the corresponding type for patients having truth data for this QoL metric, where this QoL metric truth data was optionally utilized as training output data mapped to the corresponding medical data utilized as training input data in training a corresponding machine learning model. Such medical outcome prognosis scores 636 can be generated for prospective participants of such clinical trials for treatment of the medical condition inducing QoL via processing of their medical data (e.g. medical image data) via this corresponding medical outcome prognostication function 671, where these prospective participants are assigned to either the control or investigative group of this clinical trial based on their medical outcome prognosis scores 636, indicative of the predicted QoL metric and thus indicative of the predicted primary outcome for this clinical trial.

In some or all such embodiments, a medical outcome type can correspond to a metric implemented as any given future binary, categorical, and/or continuous metric, for example, corresponding to a future outcome after a particular amount of time has passed (e.g. from the present time). For example, this future metric corresponds to a clinical endpoint of a clinical trial having a corresponding particular duration (e.g. 1 year, 2 years, 5 years), where this metric is only measured/determined once the particular duration has elapsed. A corresponding medical outcome prognosis score 636 for such a medical outcome type can indicate a corresponding predicted metric expected to be exhibited after this particular duration has passed, for example, from a time that corresponding medical data, utilized as input to a corresponding medical outcome prognostication function 671, was generated/collected (e.g. starting date of a corresponding medical scan of the patient, where this medical scan is input to the medical outcome prognostication function 671), and/or from a time that the corresponding medical outcome prognostication function 671 was performed. In particular, this medical outcome prognostication function 671 was optionally trained via a set of medical data of the corresponding type for other historical patients having logged truth data for this future outcome (e.g. corresponding to a measured outcome that was measured the predetermined amount of time after corresponding historical medical data was collected), where this measured future outcome truth data was optionally utilized as training output data mapped to this corresponding historical medical data utilized as training input data in training a corresponding machine learning model. Such medical outcome prognosis scores 636 can be generated for prospective participants of clinical trials for treatment of the corresponding medical conditions via processing of their medical data (e.g. medical image data) via this corresponding medical outcome prognostication function 671, where these prospective participants are assigned to either the control or investigative group of this clinical trial based on their medical outcome prognosis scores 636, indicative of the predicted future outcome and thus indicative of the predicted primary outcome for this clinical trial, for example, in accordance with the duration of the clinical trial.

Figure 4A illustrates an embodiment of a medical data processing system 500. Some or all features and/or functionality of the medical data processing system 500 of Figure 4A can implement any embodiment of medical data processing system 500 and/or scientific data processing system 1500 described herein.

The medical data processing system 500 can generate trial arm assignment data 631.1 - 631.P for a plurality of participants 1-P of a participant set 650 of a clinical trial. The trial arm assignment data 631.1 - 631.P can be generated via a prognosis-based trial arm assignment system 508, based on the prognosis-based trial arm assignment system 508 processing medical outcome prognosis scores 636.1 - 636.P for a given medical outcome type 637.x (e.g. that is the same as, related to, and/or indicative of the primary outcome of the clinical trial). The medical outcome prognostication scores 636.1 - 636.P can be generated via a medical outcome prognostication system 507, based on the medical outcome prognostication system 507 processing participant data 712.1 - 712.P of the P participants of the participant set 650, and/or based on a medical outcome prognostication function 671.x for the given medical outcome type 637.x (e.g. the medical outcome prognostication function 671.x is performed upon each participant data 712 to generate a corresponding medical outcome prognosis score 636 for a given participant). The medical outcome prognostication function 671.x can be generated via a medical outcome prognostication model training system 506, based on the medical outcome prognostication model training system 506 processing a training set 701 that includes a plurality of training data 701.1 - 701.K.

Some or all of process of generating trial arm assignment data 631.1 - 631.P can be repeated over time, for example, for different multiple trials having same or different primary outcomes. For example, for another clinical trial with the same primary outcome, the same medical outcome prognostication function 671.x is re-performed upon a different set of participant data 712.1 - 712.P for this other clinical trial to generate corresponding medical outcome prognosis scores 636.1 - 636.P for these participants, which can be similarly processed via prognosis-based trial arm assignment system 508 to generate corresponding trial arm assignment data 631.1 - 631.P for this different trial. As another example, for another clinical trial with the same primary outcome, a different medical outcome prognostication function 671.x (e.g. updated version of medical outcome prognostication function 671.x, for example, based on retraining the medical outcome prognosis function 671.x, optionally upon truth data gathered via a prior trial for which trial arm assignment data 631.1 - 631.P was optionally generated via using the prior version of the medical outcome prognostication function 671.x, or other updated training data/updated training process) is re-performed upon a different set of participant data 712.1 - 712.P for this other clinical trial to generate corresponding medical outcome prognosis scores 636.1 - 636.P for these participants, which can be similarly processed via prognosis-based trial arm assignment system 508 to generate corresponding trial arm assignment data 631.1 - 631.P for this different trial. As another example, for another clinical trial with a different primary outcome, a different medical outcome prognostication function 671.x2 is performed upon a different set of participant data 712.1 - 712.P to generate corresponding medical outcome prognosis scores 636.1 - 636.P for these participants, which can be similarly processed via prognosis-based trial arm assignment system 508 to generate corresponding trial arm assignment data 631.1 - 631.P for this different trial.

Note that the value of P may be the same or different for different clinical trials. The set of participants of a given clinical trial can be overlapping with or entirely distinct from the set of participants of one or more other given clinical trials. Some or all different clinical trials for which trail arm assignment data is generated can occur at the same time, in overlapping time frames, or across different, non-overlapping times. Some or all participants can have multiple different medical outcome prognosis scores 636 generated for their participant data 712 based on participating in multiple trials having with different corresponding medical outcome types 637.x as their clinical endpoint. Note that the same or different participant data 712 for a given participant can be used to generate different medical outcome prognosis scores 636 for different clinical trials.

Some or all functionality of Figure 4A can be performed, in part or in its entirety, by other entities. For example, some or all functionality of the prognosis-based trial arm assessment system 508 is implemented via a trial participant assignment entity 587, for example, based on the trial participant assignment entity 587 receiving the medical outcome prognosis scores 636.1 636.P, for example, mapped to corresponding participant identifiers, where the trial arm assignment data 631.1 - 631.P is generated by trial participant assignment entity 587.

As another example, some or all functionality of medical outcome prognostication model training system 506 is implemented via an AI platform 586 (e.g. third party AI platform), where the medical outcome prognostication function 671 is trained, in part or in its entirety, by AI platform 586. For example, a generative AI model or other AI platform generates a medical outcome prognostication function 671 (e.g. in response to a request sent from medical data processing system 500 for execution by the AI platform, for example, where the request indicates training set 701 and/or indicates desired model features, such as model type, output label, etc.). In some embodiments, this medical outcome prognostication function 671 generated by AI platform corresponds to an initial version of the model, where this model is retrained/fine-tuned by medical outcome prognostication model training system 506 implemented by the medical data processing system 500 (e.g. based on the AI platform sending the model, such as corresponding tuned model parameters, to the medical data processing system 500 for further processing). In some embodiments, this medical outcome prognostication function 671 generated by AI platform corresponds to a retrained version of the model, where the model is trained in part (e.g. an initial model is generated) by medical outcome prognostication model training system 506 implemented by the medical data processing system 500, and where the AI platform retrains/fine-tunes the model (e.g. based on the AI platform receiving the initial model and/or training from the medical data processing system 500 for further processing).

As another example, some or all functionality of medical outcome prognostication system 507 is implemented via an AI platform 586 (e.g. third party AI platform), where medical outcome prognosis scores 636 are generated, in part or in their entirety, by AI platform 586. For example, a generative AI model or other AI platform performs the medical outcome prognostication function 671 (e.g. in response to a request sent from medical data processing system 500 for execution by the AI platform, for example, where the request indicates the medical outcome prognostication function 671 trained by the medical data processing system 500; indicates a request for medical outcome prognostication function 671 to be trained and also executed by AI platform; and/or indicates the participant data 712 for processing by the AI platform, and where the AI platform generates a medical outcome prognosis score 636 from the participant data 712 of each participant accordingly.

Figure 4B illustrates an embodiment of generating trial arm assignment data 631.1 - 631.P for use by/for communication to a corresponding medical product manufacturing and/or testing processing system 597, for example, that is responsible for conducting a corresponding clinical trial and/or that is manufacturing a medical product to be tested via a corresponding clinical trial. The trial arm assignment data 631.1 - 631.P can be displayed and/or stored by medical product manufacturing and/or testing processing system 597, (e.g. via a corresponding display device and/or via corresponding memory resources), for example to enable the corresponding medical product manufacturing and/or testing entity to use the trial arm assignment data 631.1 - 631.P in facilitating conducting of the corresponding clinical trial (e.g. treat patients with an actual treatment vs. placebo based on their assignment into either a control group or experimental group of the clinical trial).

In some embodiments, generating and communicating the trial arm assignment data 631.1 - 631.P to a given medical product manufacturing and/or testing processing system 597 can be based on receiving corresponding data from the given medical product manufacturing and/or testing processing system 597. For example, the given medical product manufacturing and/or testing processing system 597 sends information (e.g. in conjunction with a corresponding request for the trial arm assignment data 631.1 - 631.P) indicating: the primary endpoint 651 (e.g. which medical outcome type 537 is utilized); some or all participant data 712.1 - 712.P (e.g. the corresponding medical data and/or other information implemented as participant data 712.1 - 712.P, or other identifying information such as patient identifiers and/or medical data identifiers that are utilized by medical data processing system to access the corresponding data, for example, in medical data storage 561 and patient storage 563 accordingly). The primary endpoint and/or participant data 712.1 - 712.P for the given clinical trial can otherwise be determined by the medical data processing system 500 to enable the medical data processing system 500 to generate the trial arm assignment data accordingly.

The medical data processing system 500 can thus process a determination to generate trial arm assignment data for a given clinical trial and/or thus process a determination to communicate this trial arm assignment data to a corresponding medical product manufacturing and/or testing processing system 597 (e.g. where this determination is made in response to a corresponding request received from the medical product manufacturing and/or testing processing system 597 or another requesting entity 576; where this determination is otherwise made via a predetermination, preset schedule, accessing/processing corresponding instructions, processing user input, etc.) based on performing some or all of the process of Figure 4A, for example, by determining and using the participant data 712.1 - 712.P determined for the participant set 650 of this given trial, and by determining the particular medical outcome type 637.x that corresponds to the primary endpoint 651 of the given trial and performing the corresponding medical outcome prognostication function 671.x to generate medical outcome prognosis scores 636 for the corresponding medical outcome type 637.x accordingly.

Note that as illustrated in Figure 4B, processing a determination to generate trial arm assignment data for a given clinical trial and/or processing a determination to communicate this trial arm assignment data to a corresponding medical product manufacturing and/or testing processing system 597 optionally does not include training the medical outcome prognostication function 671.x via medical outcome prognostication model training system 506, for example, based on this medical outcome prognostication function 671.x having already been trained prior to determining to generate trial arm assignment data for a given clinical trial (e.g. the medical outcome prognostication function 671.x was trained prior to this trial being initiated and/or communicated to medical data processing system 500, and/or this medical outcome prognostication function 671.x was optionally already used to generate trial arm assignment data for participants of other clinical trials, for example, administered via /associated with the same or different medical product manufacturing and/or testing processing system 597, and will again be performed to generate trial arm assignment data for participants of this given clinical trial).

Alternatively or in addition, a processing a determination to generate trial arm assignment data for a given clinical trial and/or processing a determination to communicate this trial arm assignment data to a corresponding medical product manufacturing and/or testing processing system 597 optionally does include first training the medical outcome prognostication function 671.x via medical outcome prognostication model training system 506, for example, based on this medical outcome prognostication function 671.x not yet having been trained prior to determining to generate trial arm assignment data for a given clinical trial, and/or based on this medical outcome prognostication function 671.x being updated/retrained based on updated training data and/or other more current information/model structuring/etc.

Figure 4C illustrates an embodiment of a medical product 777 that was developed for commercial production and/or sale via a corresponding process that includes generation and use of trial arm assignment data to conduct a corresponding clinical trial, for example, based on employing some or all functionality of medical data processing system 500 of Figure 4A and/or 4B. For example, the medical product 777 of Figure 4C can be a pharmaceutical compound (e.g. medication for injection to and/or consumption by a patients, such as pills and/or a liquid administered as a drug, vitamins, a vaccine, etc.); a medical device (e.g. a stint, a device implanted into a patient's body, a device used by a corresponding patient in conjunction with treating a corresponding medical condition, etc.); a product used in conjunction with administering a medical treatment for a medical condition; a healthcare product, and/or any other medical product. For example, medical product 777 is tested via a corresponding clinical trial for which trial arm assignment data 631.1 - 631.P is generated to dictate how patients be assigned to trial arms as a function of their predicted medical outcome for the primary outcome.

In particular, trial arm assignment data 631.1 - 631.P generated for a set of participants of a participant set 650 of a clinical trial (e.g. via some or all of the process of Figure 4A and/or 4B) can be processed via a clinical trial conducting process 746 of the medical product 777 that includes at least one control arm and at least one experimental trial arm (e.g. a corresponding entity, such as a corresponding medical product manufacturing and/or testing entity, conducts the clinical trial based on assigning participants of the participant set to respective trial arms as dictated by the trial arm assignment data). As a particular example, a placebo for medical product 777 is administered to participants assigned to the at least one control trial arm of the corresponding clinical trial conducting process 746 and/or the medical product 777 is not administered to patients assigned to at least one control trial arm, while the medical product 777 is administered to participants assigned to at least one experimental trial arm, enabling comparison of the effects of the medical product 777 (e.g. as measured via primary outcome for the participants over the course of the trial) to determine effectiveness of the medical product in treating a corresponding medical condition and/or to determine whether the medical product is approved/safe for widespread use (e.g. commercial or otherwise widespread production and/or sale, for example, for over-the-counter purchase and/or for prescription use when prescribed by a medical professional/healthcare provider), for example, based on whether or not clinical trial results 772 meet corresponding regulation-mandated acceptance criteria 779 (e.g. as required by the FDA and/or another regulatory entity). For example, commercial production/sale 747 of the medical product 777 is performed to render production, sale, and/or use of the corresponding medical product 777 (e.g. by a corresponding medical product manufacturing and/or testing entity) based on the clinical trial results 772 meeting the regulation-mandated acceptance criteria 779.

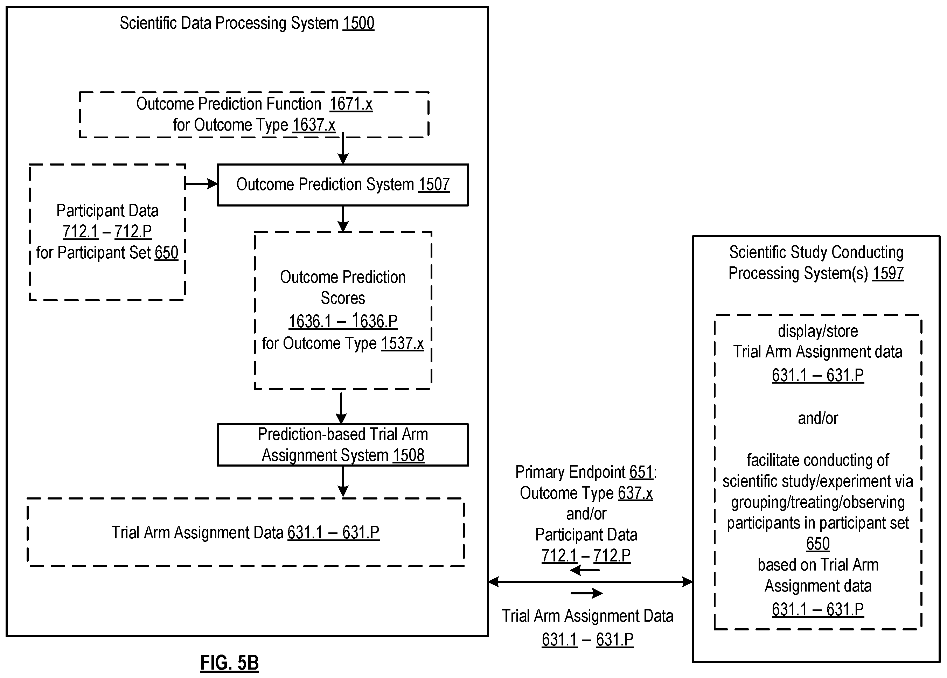

Other types of products and/or processes (e.g. any products or processes of any scientific field described herein) can be developed via similar means, for example, for commercial use, widespread production, etc., even if these products and/or processes are non-medical in nature. For example, trial arm assignment data 631.1 - 631.P generated by scientific data processing system 1500 and/or otherwise generated via functionality of scientific data processing system 1500 described herein (e.g. as illustrated in Figure 5A and/or 5B) can similarly be processed via a scientific study conducting process for the corresponding product and/or process to generate scientific study results. As a particular example, a placebo for the product/process is administered to participants (e.g. corresponding people, animals, organisms, and/or objects being tested) assigned to the at least one control trial arm of the corresponding clinical trial conducting process 746 and/or the product is not administered to participants assigned to at least one control trial arm, while the product/process is administered to participants assigned to at least one experimental trial arm, enabling comparison of the effects of the product/process (e.g. as measured via primary outcome for the participants over the course of the scientific study) to determine effectiveness of the product/process; to determine whether the product/process is approved/safe for commercial use/widespread use/use by humans (e.g. in conjunction aerial and/or automotive vehicle testing for aerial vehicle and/or automotive products; in conjunction with construction of buildings and/or infrastructure; in conjunction with growing/producing agricultural goods; in conjunction with any product/service sold for use by humans that must be deemed safe for use, etc.); to determine whether the product/process is commercially advantageous (e.g. expected to make money/render profit/increase sales for a corresponding company) and are thus deemed worth implementing/manufacturing/selling; etc.

Figure 4D illustrates an embodiment of a medical outcome prognostication model training system 506. Some or all features and/or functionality of the medical outcome prognostication model training system 506 of Figure 4D can implement the medical outcome prognostication model training system 506 of Figure 4A and/or any embodiment of medical outcome prognostication model training system 506 described herein.

The medical outcome prognostication model training system 506 can request/receive/access/otherwise determine a training set 701 that includes a plurality of training data 702.1 - 702.K. For example, some or all of the training data 701 is accessed via data storage system 560 and/or is received from data sources 575. The medical outcome prognostication model training system 506 can generate training set 701 based on generating at least one request to data storage system 560 and/or at least one data source 575 for corresponding training data and/or portions of training data (e.g. medical data meeting particular criteria; patient data meeting particular criteria; etc.). For example, the training set 701 is built to include training data 702 having types of medical data that is known/expected to be available for participants in at least one upcoming clinical trial for which the model will be executed to enable generation of medical outcome prognosis scores and/or corresponding trial arm assignment data. As another example, the training set 701 is built to include training data 702 having outcome data (e.g. truth data of medical outcomes for historical patients) of a given medical outcome type 637.x (e.g. survival/morality, hospitalization, particular scores indicative of severity of a medical condition, or any other medical outcome type described herein) that is known/expected to be utilized as a primary endpoint (and/or secondary endpoint and/or surrogate endpoint) for at least one upcoming clinical trial for which the model will be executed to enable generation of medical outcome prognosis scores and/or corresponding trial arm assignment data. The medical outcome prognostication model training system 506 can otherwise receive/determine this training set 701.

Each training data 702.1 - 702.K can correspond to a corresponding historical patient, where the training set 701 thus reflects data for K historical patients. In some embodiments, multiple training data 702 can optionally correspond to different data of a same patient.

The historical patients can be considered historical based on having known outcome data 638 of the corresponding medical outcome type 637.x for which a corresponding medical outcome prognostication function 671.x is to be trained to generate corresponding medical outcome prognostication scores 636. For example, the outcome data 638 is indicated in corresponding medical data metadata 610 and/or patient data 564 of the corresponding patient, and/or otherwise corresponds to truth data based on being detected via a medical professional, and/or optionally based on being detected via execution of another machine learning model, for example, trained and/or executed by medical data processing system and/or otherwise trained to detect medical outcomes in medical data and/or other patient data. In some embodiments, no outcome data 638 is included and/or the outcome data is not implemented as an output feature 706 (e.g. based on the corresponding model being trained via an unsupervised learning process, such as via a clustering algorithm, for which output labels are not supplied).

Each training data 702 can thus include one or more values 704 denoting the corresponding outcome data 638 of the corresponding historical patient, implemented as output features 706 (e.g. corresponding values which the model will be trained to generate as inference data/predictions, in accordance with a supervised training process). Each training data can further include one or more values 703.1 - 703.W of a corresponding input feature set 705 (e.g. mapped to the corresponding value 704 for the corresponding output feature 706, and/or thus corresponding to the input data type for which the medical outcome prognostication function will be configured to process as input to generate corresponding predictions of outcome data 638 as its medical data prognosis scores 636, once trained. Examples of input feature set 705 are discussed in conjunction with Figures 4F - 4H.

In some embodiments, temporal bounds (e.g. number of days, months, or years, or another temporal range) from the time that medical data/other data of input feature set 705 was collected to the time that the corresponding outcome data 638 was measured/determined can be indicated in the training data 702 (e.g. as a corresponding value 703) and/or can be a requirement for building the training data 701. For example, the outcome data 638 can indicate a corresponding measurement/category of outcome that was measured within a fixed amount of time from the corresponding medical data (e.g. each outcome data 638 indicates whether or not the patient survived 1 year after a corresponding medical scan was captured; each outcome data 638 indicates whether or not the patient was hospitalized within 2 years after a corresponding medical scan was captured; each outcome data 638 indicates a measurement captured between 1 and 2 years after a corresponding medical scan was captured; etc.) As another example, the outcome data 638 can indicate a corresponding measurement indicating when a corresponding event happened from the time the medical scan was captured. For example, first outcome data 638 indicates a value of 4.5 based on a corresponding patient dying 4.5 years after a corresponding medical scan was captured; while second outcome data 638 indicates a value of 2 based on a corresponding patient dying 2 years after a corresponding medical scan was captured; a reserved value optionally denotes the case where the event has not occurred (e.g. patient is still alive), etc. This can render generation of a model that predicts medical outcome as the corresponding temporal period, for example, from when the corresponding medical data was captured (e.g., training data indicates a measurement captured between 1 and 2 years, and the model generates medical outcome prognostication scores 636 indicating predictions from this measurement 1-2 years into the future from the time the corresponding medical data was captured). The corresponding patients can be considered historical based on their medical data having been captured at a time prior to current time by at least this temporal bound, as their medical outcome is known (e.g. a medical outcome indicating an outcome measured 3 years after a medical scan was captured thus requires that this medical scan is at least 3 years old).

Requirements regarding temporal bounds can be employed in building the corresponding training set (e.g. all outcome data must be captured within a particular time frame relative to when the medical scan was captured, such as between 1 and 3 years from when the medical scan was captured). Such requirements regarding temporal bounds can be generated based on a length of time associated with a corresponding clinical trial, and/or multiple models corresponding to prediction of the same medical outcome with different temporal bounds can be generated (e.g. one model predicts mortality 1 year out; another model predicts mortality 2 years out; and another model predicts mortality 5 years out; etc.) where a model is selected for use in generating medical outcome prognostication scores for generating the trial arm assignment data for a particular clinical trial based on selecting a model having a same and/or closest temporal bounds employed as a length of the clinical trial/amount of time until primary endpoint is measured/determined (e.g. the model configured to predicts mortality 5 years out is applied for clinical trial with a primary endpoint corresponding to mortality within 5 years, while the model configured to predicts mortality 1 year out is applied for clinical trial with a primary endpoint corresponding to mortality within 1 year; etc.)

In some embodiments, participant requirements (e.g. age, sex, patient history, whether or not the patient is undergoing a particular treatment, whether or not the patient has a particular medical condition, etc.) that is known and/or expected to be utilized to select participants of at least one clinical trial is utilized to identify/request training data for the historical patients that will be utilized to build the training set. For example, a training set of medical data captured for a plurality of patients of a particular age range, gender, and/or having a corresponding type/severity of a given medical condition (e.g. threshold number of tumors; threshold tumor size) is utilized to build a corresponding training set corresponding to only historical patients meeting these conditions, where the corresponding model is executed to generate medical outcome prognostication data for a corresponding clinical trial where all participants are required to be of the corresponding age range, the corresponding gender, and/or express the corresponding given medical condition. This can render more accurate models adapted specifically for predicting medical outcome in the types of patients that a given clinical trial is conducted upon.

A model training module 710 can be implemented to process the training set 701 to generate model parameter data 661 defining the corresponding trained machine learning model, for example, by performing a corresponding model training function 673 (e.g. stored in function library 572). The model parameter data 661 can include a plurality of tuned parameters 711.1 - 711.Y, for example, that are: optimized and/or otherwise tuned over multiple iterations of a corresponding training process, for example, until a convergence condition is reached; that are calculated in accordance with at least one equation and/or algorithm performed via a corresponding training process; and/or that are otherwise generated/configured to define the corresponding model. For example, the plurality of tuned parameters 711.1 - 711.Y are implemented as weights of a corresponding neural network (e.g. a convolutional neural network, such as a three-dimensional convolutional neural network as discussed in the examples of model 112 as discussed in conjunction with Figure 1B and/or some or all of Figures 8A - 8S; are implemented as coefficient values defining a corresponding linear and/or non-linear function to be applied to input feature sets to render a corresponding medical outcome prognosis score 636; and/or other parameters defining the corresponding model, for example, in conjunction with a corresponding type of machine learning model implementing the corresponding medical outcome prognostication function 671.

The corresponding model parameter data 661 and/or other function definition data for the corresponding medical outcome prognostication function 671.x can be sent to function library 572 for future use (e.g. for use by medical outcome prognostication system 507 as discussed in conjunction with Figure 4H). The corresponding model parameter data 661 defining the corresponding medical outcome prognostication function 671.x can otherwise be stored and/or utilized to then enable any execution of medical outcome prognostication function 671.x described herein.

Some or all of this process of Figure 4D can be performed multiple times to render generation of multiple different medical outcome prognostication functions 671 for use by the medical data processing system, and/or to render updating/retraining of a same given medical outcome prognostication functions 671.x for use by the medical data processing system over time.

Figure 4E illustrates an embodiment of a medical outcome prognostication system 507. Some or all features and/or functionality of the medical outcome prognostication system 507 of Figure 4E can implement the medical outcome prognostication system 507 of Figure 4A and/or any embodiment of medical outcome prognostication system 507 described herein.

The medical outcome prognostication system 507 can request/receive/access/otherwise determine a plurality of participant data 712.1 - 712.P, for example, corresponding to a set of participants of a participant set 650 for a corresponding clinical trial. For example, some or all of the plurality of participant data 712.1 - 712.P is accessed via data storage system 560 and/or is received from data sources 575 (e.g. is received from a corresponding entity conducting the clinical trial, such as medical product manufacturing and/or testing system). The medical outcome prognostication system 507 can optionally generate or otherwise determine participant data 712.1 - 712.P based on generating at least one request to data storage system 560 and/or at least one data source 575 for corresponding participant data and/or portions of participant data and (e.g. medical data for particular participants meeting particular criteria; patient data for particular participants meeting particular criteria; etc.). For example, the participant data 712.1 - 712.P is built to include types of medical data available for participants in at least one upcoming clinical trial for which a model as trained to be executed to enable generation of medical outcome prognosis scores and/or corresponding trial arm assignment data (e.g. participant data 712.1 - 712.P is built to include types of medical data included in training data 702 of training set 701 that was processed to train the corresponding medical outcome prognostication function 671.x as discussed in conjunction with Figure 4C). The medical outcome prognostication system 507 can otherwise receive/determine this participant data.

Each participant data 712.1 - 712.P can correspond to a corresponding participant of the clinical trial (e.g. candidate being considered for participation or confirmed participant). The participant data 712 can each include pre-trial and/or pre-treatment data, corresponding to data collected before treatment is administered and/or collected before the trial began (e.g. a medical image and/or test results captured prior to the trial commencing. For example, the outcome data 638 of these patients is not yet known, and is thus predicted via execution of the corresponding medical outcome prognostication function 671. As a particular example, some or all of the participant data 712.1 - 712.P (e.g. corresponding medical images or other medical data) was captured more recently that some or all training data and/or an amount of time from the current date that this medical data is captured is not as old as the temporal bounds applied in building the training data, and thus medical outcome having this temporal bound from the medical data capture date is thus not yet known.

Each participant data 712 can thus include one or more values 703.1 - 703.W of a corresponding input feature set 705 (e.g. the same number/types of values as the values 703.1 - 703.W utilized in input feature sets 702 of the training data utilized to train the corresponding model). Unlike the training data 704, each participant data 712 optionally does not include outcome data 638 of the corresponding participant implemented as output features 706 and/or otherwise has not output features, based on the outcome data 638 not yet being known and/or based on the function 671.x being performed to predict this outcome data 638 in generating medical outcome prognostication score 636.

An inference data generator module 720 can be implemented to generate a medical outcome prognosis score 636 for each participant as a function of their participant data 712. For example, the inference data generator module 720 executes medical outcome prognostication function 671.x and/or otherwise applies the model parameter data 661 that was generated via training of the model, for example, as discussed in conjunction with Figure 4D. In particular, the medical outcome prognostication system 507 can call, access, and/or receive the corresponding medical outcome prognostication function 671.x and/or corresponding model parameter data 661, for example, based on accessing/utilizing the corresponding function entry in function library 572.

The medical outcome prognosis scores 636.1 - 636.P can be implemented as predicted values of medical outcome 638 (e.g. predicted continuous value, predicted category, predicted binary value indicating whether or not the outcome is predicted to happen, etc.) and/or can indicate a probability value of one or more corresponding possible outcomes (e.g. one or more different categories and/or probability that the corresponding outcome (e.g. death/hospitalization) occurs, for example within corresponding temporal bounds, for example, that were utilized to train the model as discussed previously (e.g. predicts measurement/category of outcome 3 years from the time the corresponding input medical data of participant data 712 was collected, based on the training data 702 having outcome data 638 indicating the outcome 3 years (and/or optionally more than three years) from the time the corresponding historical medical data of the training data 702 was collected). The medical outcome prognosis scores 636 can be implemented via some or all features and/or functionality of medical outcome prognosis scores 636 of Figure 3A, 3B and/or otherwise embodiments of medical outcome prognosis scores 636 described herein.

The medical outcome prognosis scores 636.1 - 636.P can be sent to data storage system 560 for storage. The medical outcome prognosis scores 636.1 - 636.P can alternatively or additionally be communicated directly to prognosis-based group assignment system 508 for use in generating trial arm assignment data for the set of participants, for example, as discussed in conjunction with Figure 4A and/or as discussed in further detail in conjunction with Figure 4I. The medical outcome prognosis scores 636.1 - 636.P can alternatively or additionally be sent/otherwise communicated to requesting entity 576 for display/use (e.g. these scores are displayed via a display device and/or the requesting entity 576 uses these scores to perform its own participant assignment to control and investigative groups, or are used for different purposes, such as any other application of this information as described herein).

Figures 4F - 4H illustrate examples of a given input feature set 705.i for a given patient i. For example, patient i is a historical patient where feature set 705.i is included in training data 702.i, for example, as discussed in conjunction with Figure 4D; and/or patient i is a prospective participant of a clinical trial, where feature set 705.i is included in participant data 712.i, for example, as discussed in conjunction with Figure 4E. Some or all features and/or functionality of the input feature set 705.i of Figure 4F, 4G, and/or 4H can implement any input feature set 705 of any training data 702 of training set 701 utilized to train a given medical outcome prognostication function 671.x for example, as discussed in conjunction with Figure 4D. Some or all features and/or functionality of the input feature set 705.i of Figure 4F, 4G, and/or 4H can similarly implement any input feature set of any participant data 712 upon which this given medical outcome prognostication function 671.x is executed to generate corresponding medical outcome prognosis scores 636, for example, as discussed in conjunction with Figure 4H.

The values 703.1 -701.W of a given input feature set 705 can indicate same type of information across all training data 702.1 - 702.K of a given training set 701 utilized to train a given medical outcome prognostication function 761.x and/or across all participant data 712 of a participant set 650 upon which the given medical outcome prognostication function 761.x is executed. This structuring applied across all training data and corresponding participant data utilized as function input can be implemented via some or all features of any of the example embodiments illustrated in Figures 4F - 4H.

Figure 4F illustrates an example embodiment where the plurality of values 703.1 -703.W of input feature set 705.i are implemented as a plurality of values 601.1 -601.V of corresponding medical data 652 for the corresponding historical patient i. For example, the plurality of values 601.1 -601.V of corresponding medical data 652 optionally constitute all of the plurality of values 703.1 -703.W of input feature set 705.i, where W = V.

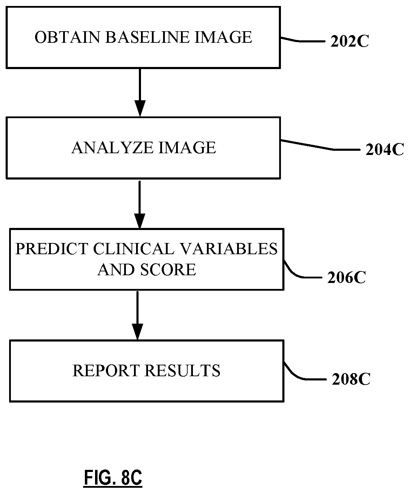

The corresponding medical data 652 can be of a given type, where a corresponding medical outcome prognostication function 761.x is trained via training data 702.1 - 702.K all having input feature set 705 implemented as the values 601.1 -601.V of medical data 652 having this type, and/or where this corresponding medical outcome prognostication function 761.x is further executed upon patient data having input feature set 705 implemented as the values 601.1 -601.V of medical data 652 having this type. As a particular example, the 601.1 -601.V of medical data 652 are implemented as a plurality of pixel values, density values, and/or voxel values of a corresponding medical scan, such as a plurality of pixel values, density values, and/or voxel values of one or more slices of the medical scan, based the type of medical data being used corresponding to medical scans (e.g. of a particular modality and/or anatomical region such as CT scans captured of a bodily region that includes the lungs, for example, as discussed in the examples of some or all of Figures 8C - 8S).

While not illustrated, an input feature set can optionally include a selected proper subset of values 601.1 -601.V of medical data 652 and/or can optionally include a set of pre-processed values generated from raw data values 601.1 -601.V of medical data 652. For example, the corresponding medical data 652 is pre-processed for use in a corresponding training data 702 and/or corresponding patient data 712.

Figure 4G illustrates an example embodiment where the plurality of values 703.1 -703.W of input feature set 705.i are implemented as a plurality of values 601.1 -601.V of multiple corresponding medical data 652.i.1 - 652.i.Z for the corresponding historical patient i. For example, a first plurality of values 601.1.1 -601.1.V1 correspond to first medical data 652 of the patient i (e.g. of a first particular type), a second plurality of values 601.2.1 -601.2.V2 correspond to second medical data 652 of the patient i (e.g. of a second particular type, with optionally different types or number of values from the first particular type).

The medical data 652.i.1 - 652.i.Z can include any set of medical data of same or different types. For example, the medical data 652.i.1 - 652.i.Z is Z medical data of a same type or different that was captured at different times (e.g. in predetermined intervals), indicative of change/rate of change in the corresponding patient/prospective participant. As another example, the medical data 652.i.1 - 652.i.Z can include medical data of different types that were optionally captured at a same time/within a same temporal period, or captured over time (e.g. in predetermined intervals). For example, the set of Z medical data includes different types of medical scans of same or different modality/same or different anatomical region; one or more medical data of the set of Z medical data are medical images/scans, while one or more other medical data of the set of Z medical data are non-image medical data (e.g. test results of a test administered on organic matter of the patient, etc.; measurement captured by a non-imaging medical device; any type of medical data described herein; etc.); all medical data 1-Z are non-image medical data; etc.

Figure 4H illustrates an example embodiment where the plurality of values 703.1 -703.W of input feature set 705.i include at least one additional value 707 that is not a value 601 of corresponding medical data, in addition to the values 601 of a set of medical data 1-Z (e.g. multiple medical data, or optionally a single medical data 652 as illustrated in Figure 4F) For example, the at least one additional value 707 provides additional information regarding patient and/or the one or more medical data 652.1 - 652.Z.

For example, the at least one additional value 707 is based on (e.g. includes/indicates/was generated as a function of/etc.) any values of patient data 654 and/or medical data metadata 610. As a particular example, the at least one additional value 707 is based on information indicated in other relevant data 640 as described herein, such as patient age, sex, medical history, current medications/treatments, etc. Alternatively or in addition, the at least one additional value 707 is based on information indicated in medical data capture data 611 of some or all of the medical data 652.1 - 652.Z (e.g. date captured, type of data, identifier of location/device collecting the data, etc.). Alternatively or in addition, the at least one additional value 707 is based on information indicated in medical findings data of some or all of the medical data 652.1 - 652.Z (e.g. model-detected finding data 617 generated via execution of a corresponding machine learning model implemented via another function in function library 572 upon the corresponding medical data 652; and/or human-detected finding data 618). Alternatively or in addition, the at least one additional value is based on commonly utilized stratification factors for clinical trials. Alternatively or in addition, the at least one additional value 707 indicates and/or based on data/factors for the given participant that optionally include one or more of: Histology; PD-L1 Expression; Sex / Gender; Smoking Status; Prior Therapy; TNM Stage; Performance Status; Age; and/or Race / Ethnicity. For example, some or all of these example factors are optionally included/indicated in any embodiment of patient data 654 and/or medical data metadata 610.

Figure 4I illustrates an embodiment of a prognosis-based trial arm assignment system 508. Some or all features and/or functionality of the prognosis-based trial arm assignment system 508 of Figure 4I can implement the prognosis-based trial arm assignment system 508 of Figure 4A and/or any embodiment of prognosis-based trial arm assignment system 508 described herein.

The prognosis-based trial arm assignment system 508 can request/receive/access/otherwise determine a plurality of medical outcome prognosis scores 636.1 - 636.P, for example, generated via medical outcome prognostication system 507 as discussed in conjunction with Figure 4E and/or or otherwise corresponding to a set of participants of a participant set 650 for a corresponding clinical trial. For example, some or all of the plurality of medical outcome prognosis scores 636.1 - 636.P is accessed via data storage system 560 (e.g. based on having been stored by medical outcome prognostication system 507 once generated) and/or is optionally received directly from medical outcome prognostication system 507. The prognosis-based trial arm assignment system 508 can optionally generate or otherwise determine medical outcome prognosis scores 636.1 - 636.P based on generating at least one request to data storage system 560 for medical outcome prognosis scores 636.1 - 636.P. The prognosis-based trial arm assignment system 508 can otherwise receive/determine the medical outcome prognosis scores 636.1 - 636.P.

A trial arm assignment module 730 can be implemented to generate trial arm assignment data 631 for each participant as a function of their medical outcome prognosis score 636. For example, the trial arm assignment module 730 executes a trial arm assignment function 672 and/or can apply stratification factor data 683, for example, indicating that the participants be stratified by at least their medical outcome prognosis scores 636. Examples of implementing trial arm assignment module 730 are discussed in conjunction with Figures 6A - 6K.

The trial arm assignment function 672 can apply predetermined and/or configured stratification factor data 683 and/or randomization techniques 684 for the clinical trial. The stratification factor data 683 and/or randomization techniques 684 and/or identifier 559 of trial arm assignment function 672 can be reflected in clinical trial data to denote how the participants were assigned to trial arms as participant assignment method data 682, and/or this participant assignment method data 682 can dictate how trial arm assignment function 672 be performed.

Each trial arm assignment data 631 can indicate which trial arm the corresponding patient is assigned to (e.g. whether they are assigned to the control arm or the experimental arm, and/or which control group or which experimental group they are assigned to if multiple control groups and/or multiple experimental groups). One or more of the P participants are optionally not assigned to a trial arm, for example, based on being deemed ineligible for the trial and/or not being selected for the trial (e.g. as a function of their participant data 712, their medical outcome prognosis score 636, and./or other factors). As a particular example, patients having medical outcome prognosis scores indicating a time to death is expected to be less than a duration of the trial with at least a threshold probability are optionally automatically excluded from selection for participation in the trial. In other embodiments, all P patients are assigned to a trial arm of the clinical trial.

The trial arm assignment data 631.1 - 631.P can be sent to data storage system 560 for storage, for example, in clinical trial data 566 for the corresponding clinical trial. The trial arm assignment data 631.1 - 631.P can alternatively or additionally be communicated directly to a requesting entity 576, such as one or more medical product manufacturing and/or testing processing systems 597 of one or more medical product manufacturing and/or testing entities 577 and/or other entity/corresponding computing device 700 associated with conducting the clinical trial.

The trial arm assignment data 631.1 - 631.P can be generated, communicated, and/or stored in conjunction with strong blinding procedures, such as double-blind procedures. For example, the trial arm assignment data 631.1 - 631.P is stored/communicated to requesting entity 576 securely and/or in a fashion where entities involved in conducting the trial that would be bias by knowledge of trial arm assignment in observing the participants cannot view/access the corresponding trial arm assignment data 631.1 - 631.

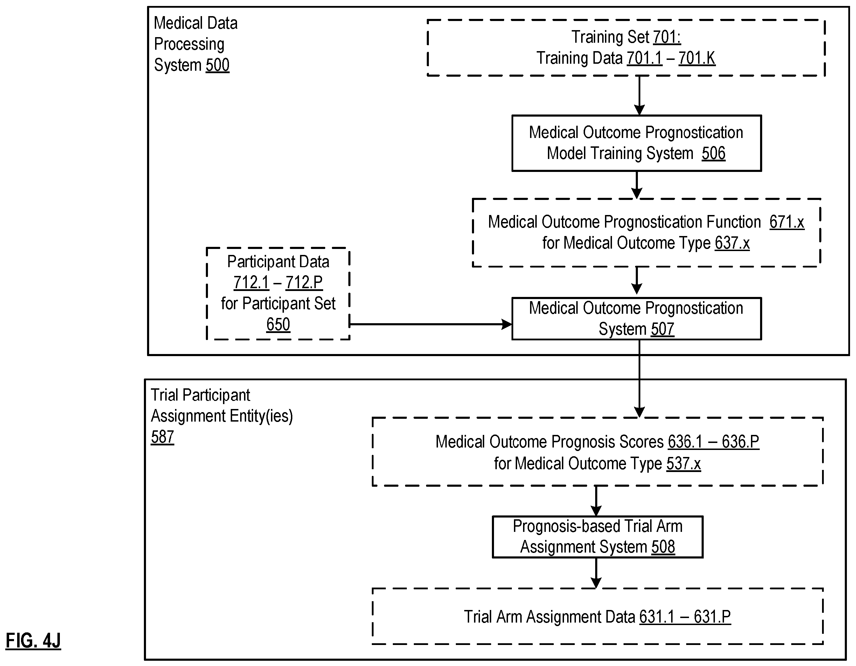

Figure 4J is a schematic block diagram of a medical data processing system 500 communicating with at least one trial participant assignment entity 587 in accordance with various embodiments. Some or all features and/or functionality prognosis-based trial arm assignment system 508 as described herein can be implemented via trial participant assignment entity 587 instead of or in addition to being implemented by the medical data processing system 500. For example, the medical data processing system 500 sends the medical outcome prognosis scores 636.1 - 636.P to the trial participant assignment entity 587, for example, in conjunction with corresponding participant identifiers and/or other information utilized to stratify participants for randomization, and the trial participant assignment entity 587 generates the trial arm assignment data 631.1 - 631.P accordingly. The trial participant assignment entity 587 can optionally communicate trial arm assignment data 631.1 - 631.P back to the medical data processing system 500 to then be communicated by the medical data processing system 500 to the corresponding requesting entity/entity conducting the trial, or the trial participant assignment entity 587 can optionally communicate trial arm assignment data 631.1 - 631.P to the requesting entity/entity conducting the trial directly.

Figures 5A - 5H illustrate embodiments of a scientific data processing system 1500 that generates trial arm assignment data 631 for participants of a scientific study as a function of outcome prediction scores 1636 based on implementing an outcome prediction model training system 1506, and outcome prediction system 1507, and/or a prediction-based trial arm assignment system 1508. Some or all features and/or functionality of the outcome prediction model training system 1506, the outcome prediction system 1507, and/or the prediction-based trial arm assignment system 1508 of Figure 5A can implement some or all features and/or functionality of the medical outcome prognostication model training system 506, the medical outcome prognostication system 507, and/or the prognosis-based trial arm assignment system 508 of some or all of Figures 4A - 4I.

In some embodiments of enabling some or all features and/or functionality of the scientific data processing system 1500 (e.g. of the outcome prediction training system 1506; of the outcome prediction system 1507; and/or of the prediction-based trial arm assignment system 1508), given outcome type 1637 is configured (e.g. automatically selected by the medical data processing system 500) to, based on a given scientific study having the at least one outcome type 1637 as its primary endpoint 601: have a corresponding outcome prediction function 1671 trained to predict the corresponding outcome type 1637; have a corresponding trained outcome prediction function 1671 performed upon scientific data 1562 and/or other relevant information 1640 for a set of prospective participants of the scientific study to generate outcome prediction scores 1636 of the corresponding outcome type 637 for this set of prospective participants; and/or to have this set of prospective participants grouped into at least one control group for the scientific study and into at least one investigative group (i.e. experimental group) for the scientific study, via a randomization algorithm, as a function of these outcome prediction scores 1636 (e.g. where the outcome prediction scores 1636 are utilized as a stratification factor in applying the randomization algorithm).

Such functionality can improve one or more technological fields for same or similar reasons as discussed in conjunction with Figures 4A - 4I. However, such improvements can be expanded to additional technological fields beyond those corresponding to medicine, healthcare, and/or pharmaceuticals based on application of the corresponding approaches to non-medical/non-healthcare scientific studies. For example, such technology can improve any scientific field for which a corresponding product, process, treatment, and/or hypothesis is tested, such as any of technological fields corresponding to engineering physical sciences, and/or other scientific fields. Such technology can alternatively improve the technological field of conducting scientific studies. In particular, the use of predicted outcome prognosis scores 1636 in randomizing participants for clinical trials can improve the effectiveness of the corresponding scientific study by stratifying participants more accurately, for example, by inherently accounting for more factors that contribute to the primary endpoint via use of these factors as input to a corresponding machine learning model that generates corresponding outcome prediction scores for this primary endpoint. Thus, different trial arms can be randomized more effectively (e.g. more equally distributing patients likely vs. unlikely to exhibit one or more corresponding outcomes being measured between the control group and experimental groups of the scientific study), which can improve the accuracy of the scientific study, which can render improvements to the effectiveness of corresponding products/processes being tested (e.g. renders better engineering of corresponding technological products, better user experience in using the technological product), and/or renders better understanding of a corresponding scientific field, which can yield improvements in technological fields relating to the corresponding scientific field based on this enhanced scientific knowledge.

Furthermore, in some cases where this single score is utilized to stratify participants rather than multiple factors, a smaller number of participants are required to complete the trial, which can improve corresponding scientific technologies based on improving the speed by which scientific studies are conducted, enabling faster scientific discovery/research advancement of corresponding scientific/technological fields. For example, randomizing over more factors can ensure that more conditions are accounted for, but can result in more complicated participant assignment and can require larger numbers of participants and/or more specific searching for participants meeting certain requirements to ensure all factors are balanced evenly. Meanwhile, computing a single, predictive score characterizing predicted outcome of the study, for example, explicitly or inherently a function of multiple such factors based on a richness of one or more corresponding scientific data (e.g. test data via corresponding scientific equipment, measurements, image data, and/or other scientific data, for example, comprising hundreds, thousands, and/or millions of individual values) can reduce the number of participants necessary based on reducing the number of factors being randomized, while still inherently accounting for some or all of these factors based on the impact of these factors being preserved in the overall predictive score.

Such improvements to the technology of medicine and/or pharmaceuticals can be enabled through the implementing of custom computing technology that is enabled via the training of machine learning functions upon a training set of data (e.g. rich scientific data, such as image data and/or test data, labeled with a known outcome) via one or more artificial intelligence techniques and/or machine learning techniques, for example, rendering particular, tuned parameters of a corresponding machine learning model configured to predict a corresponding medical outcome. These sophisticated machine learning models can then be applied to new input (e.g. for participants of a scientific study) to predict the corresponding primary outcome. Such improvements to the technology of scientific fields require the training and use of a corresponding machine learning model, for example, based on analysis of the corresponding training data being infeasible for performance by the human mind (e.g. the raw data of the scientific data, such as its hundreds, thousands, and/or millions of data points, are not easily consumable by the human mind; the human mind not being feasibly able process hundreds or thousands of training data to generate a corresponding model; the human mind not being feasibly able to identify relationships between various values/attributes of the input and/or not being feasibly able to relate various values/attributes of the input to their impact on the outcome, and thus not being able to tune a set of parameters (e.g. optionally dozens, hundreds, or thousands of weights) defining a corresponding model that can accurately predict the outcome; the human mind not being able to predict the outcome with the same accuracy as a trained model; and/or the human mind not being able to perform processing required to train and/or execute a corresponding model in a feasible amount of time, even when assisted by pen and paper.

As a particular example, while a human may be inclined to stratify participants by simple, easily-observable factors that may affect outcome (e.g. age, sex, etc.), the outcome prediction scores generated by the model are generated via processing of many more data points indicative of factors that are not observable by a human and/or in a way that is not feasible for performance by the human mind. Yet the use of these model-generated outcome prediction scores, instead of or in addition to more easily observable factors such as participant age and/or sex, to stratify participants of a scientific study can greatly improve the ability to conduct the scientific quickly and/or via fewer participants and/or can improve the accuracy of study results based on patients being more evenly distributed by predisposition to exhibit the corresponding outcome being tested as the primary outcome, which renders more effective corresponding products/processes which improves the corresponding technology of these products/ processes as discussed previously.

Figure 5A illustrates an embodiment of a scientific data processing system 1500 that generates trial arm assignment data 631.1 - 631.P for a plurality of participants 1-P of a participant set 650 of a scientific study. The generation of trial arm assignment data 631.1 - 631.P by scientific data processing system 1500 as illustrated in Figure 5A can be the same as or similar to the generation of trial arm assignment data 631.1 - 631.P by medical data processing system 500 of Figure 4A, where the trial arm assignment data 631.1 - 631.P can indicate assignment of participants to trial arms of any scientific experiment/study that is not necessarily medical/healthcare related.

The trial arm assignment data 631.1 - 631.P can be generated via a prediction-based trial arm assignment system 1508, based on the prediction-based trial arm assignment system 1508 processing outcome prediction scores 1636.1 - 1636.P for a given outcome type 1637.x (e.g. that is the same as, related to, and/or indicative of the primary outcome of the scientific study). The outcome prediction scores 1636.1 - 1636.P can be generated via an outcome prediction system 1507, based on the outcome prognostication system 1507 processing participant data 712.1 - 712.P for the P participants of the participant set 650, and/or based on a outcome prediction function 1671.x for the given outcome type 1637.x (e.g. the outcome prediction function 1671.x is performed upon each participant data 712 to generate a corresponding outcome prediction score 1636 for a given participant). The outcome prediction function 1671.x can be generated via an outcome prediction model training system 1506, based on the outcome prediction model training system 1506 processing a training set 701 that includes a plurality of training data 701.1 - 701.K.

This process can be applied for different participant sets of different scientific studies, for example, in a same or similar fashion as discussed in conjunction with Figure 4A. For example, the same or different medical outcome prognostication functions are performed upon participant data 712 of different participant sets 650 of different scientific studies over time to generate corresponding trial arm assignment data for these different scientific studies.

Some or all functionality of Figure 5A can be performed, in part or in its entirety, by other entities. For example, some or all functionality of the prediction-based trial arm assessment system 1508 is implemented via a trial participant assignment entity 587, for example, based on the trial participant assignment entity 587 receiving the outcome prediction scores 1636.1 1636.P, for example, mapped to corresponding participant identifiers, where the trial arm assignment data 1631.1 - 1631.P is generated by trial participant assignment entity 587.

As another example, some or all functionality of outcome prediction model training system 1506 is implemented via an AI platform 586 (e.g. third party AI platform), where the outcome prediction function 1671 is trained, in part or in its entirety, by AI platform 586. For example, a generative AI model or other AI platform generates an outcome prediction function 1671 (e.g. in response to a request sent from medical data processing system 500 for execution by the AI platform, for example, where the request indicates training set 701 and/or indicates desired model features, such as model type, output label, etc.). In some embodiments, this outcome prediction function 671 generated by AI platform corresponds to an initial version of the model, where this model is retrained/fine-tuned by outcome prediction model training system 1506 implemented by the scientific data processing system 1500 (e.g. based on the AI platform sending the model, such as corresponding tuned model parameters, to the scientific data processing system 1500 for further processing). In some embodiments, this outcome prediction function 1671 generated by AI platform corresponds to a retrained version of the model, where the model is trained in part (e.g. an initial model is generated) by outcome prediction model training system 1506 implemented by the scientific data processing system 1500, and where the AI platform retrains/fine-tunes the model (e.g. based on the AI platform receiving the initial model and/or training from the scientific data processing system 1500 for further processing).

As another example, some or all functionality of outcome prediction system 1507 is implemented via an AI platform 586 (e.g. third party AI platform), where outcome prediction scores 1636 are generated, in part or in their entirety, by AI platform 586. For example, a generative AI model or other AI platform performs the medical outcome prognostication function 1671 (e.g. in response to a request sent from medical data processing system 500 for execution by the AI platform, for example, where the request indicates the outcome prediction function 671 trained by the medical data processing system 500; indicates a request for outcome prediction function 1671 to be trained and also executed by AI platform; and/or indicates the participant data 712 for processing by the AI platform, where the AI platform generates an outcome prediction score 1636 from the participant data 712 of each participant accordingly.

Figure 5B illustrates an embodiment of generating trial arm assignment data 631.1 - 631.P for use by/for communication to a corresponding scientific study conducting processing system 1597, for example, that is responsible for conducting a corresponding scientific study and/or that is producing a product/service and/or conducting research that is being tested via the corresponding scientific study The trial arm assignment data 631.1 - 631.P can be displayed and/or stored by scientific study conducting processing system 1597, (e.g. via a corresponding display device and/or via corresponding memory resources), for example to enable the corresponding medical product manufacturing and/or testing entity to use the trial arm assignment data 631.1 - 631.P in facilitating conducting of the corresponding scientific study (e.g. apply an experimental procedure to participants vs. placebo based on their assignment into either a control group or experimental group of the scientific study).

Some or all features and/or functionality of the scientific data processing system 1500 and/or the scientific study conducting processing system 1597 Figure 5B can be implemented in a same or similar fashion as the medical data processing system 500 and/or medical product manufacturing and/or testing processing system 597, respectively, of Figure 4B, for example, by further facilitating generation and communication of trial arm assignment data 631.1 - 631.P for a scientific study that is optionally non-medical, for example, based on participant data 712.1 - 712.P that is non-medical (e.g. includes non-medical scientific data 1562).

Figure 5C illustrates an embodiment of a scientific outcome prediction model training system 1506. Some or all features and/or functionality of the scientific outcome prediction model training system 1506 of Figure 5C can implement the scientific outcome prediction model training system 1506 of Figure 5A and/or any embodiment of scientific outcome prediction model training system 1506 described herein. Some or all features and/or functionality of the scientific outcome prediction model training system 1506 of Figure 5C can implement the medical outcome prognostication model training system 506 of Figure 4D and/or any embodiment of medical outcome prognostication model training system 506 described herein, for example, where functionality is extended to processing training data 702 that optionally includes non-medical scientific data 1562 and/or indicates outcome data 638 for non-medical outcome types 1637. Outcome prediction function 1671.x can be implemented in a same or similar fashion as medical outcome prognostication function 671, for example, differing based on being trained on non-medical data and/or operable to predict non-medical outcomes. Medical outcome prognostication function 671 can be implemented as a type of outcome prediction function 1671.

Figure 5D illustrates an embodiment of an outcome prediction system 1507. Some or all features and/or functionality of the outcome prediction system 1507 of Figure 5D can implement the outcome prediction system 1507 of Figure 5A and/or any other embodiment of outcome prediction system 1507 described herein. Some or all features and/or functionality of the outcome prediction system 1507 of Figure 5D can implement the medical outcome prognostication system 507 of Figure 4E and/or any embodiment of medical outcome prognostication system 507 described herein, for example, where functionality is extended to processing participant data 712 that optionally includes non-medical scientific data 1562 and/or to generate outcome prediction scores 1638 predicting values/probability of outcome types 1637 that are optionally non-medical outcome types 1637. Outcome prediction function 1671.x can be implemented in a same or similar fashion as medical outcome prognostication function 671, for example, differing based on being trained on non-medical data and/or operable to predict non-medical outcomes. Medical outcome prognostication function 671 can be implemented as a type of outcome prediction function 1671, where medical outcome prognostication score 636 is implemented as a type of outcome prediction score 1636.

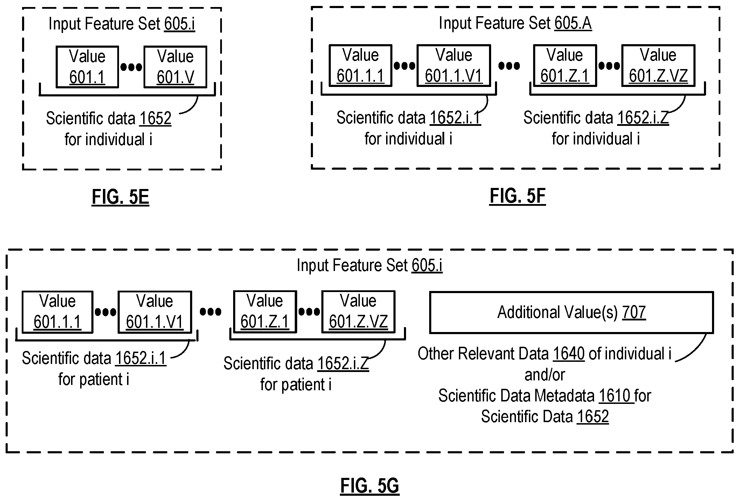

Figures 5E - 5G illustrate examples of a given input feature set 705.i for a given individual i. Some or all features and/or functionality of input feature set 705.i of Figure 5E, 5F, and/or 5G can be implemented in a same or similar fashion as input feature set 705.i of Figure 4D, 4E, and/or 4F, for example, differing based on input feature set 705 including values 601 of scientific data 1652 that is non-medical and/or additional values 707 that optionally correspond to non-medical other relevant data 1640 and/or scientific metadata for non-medical scientific data. For example, medical data 562 is implemented as a type of scientific data 1652.

For example, individual i is a historical individual where feature set 705.i is included in training data 702.i, for example, as discussed in conjunction with Figure 4D and/or as illustrated in conjunction with Figure 5C; and/or individual i is a prospective participant of a scientific study, where feature set 705.i is included in participant data 712.i, for example, as discussed in conjunction with Figure 4E and/or as illustrated in conjunction with Figure 5D. Some or all features and/or functionality of the input feature set 705.i of Figure 5E, 5F, and/or 5G can implement any input feature set 705 of any training data 702 of training set 701 utilized to train a given outcome prediction function 1671.x for example, as discussed in conjunction with Figure 4D and/or as illustrated in Figure 5C. Some or all features and/or functionality of the input feature set 705.i of Figure 5E, 5F, and/or 5G can similarly implement any input feature set of any participant data 712 upon which this given outcome prediction function 1671.x is executed to generate corresponding outcome prediction scores 1636, for example, as discussed in conjunction with Figure 4H and/or as illustrated in Figure 5D.

Figure 5H illustrates an embodiment of a prediction-based trial arm assignment system 1508. Some or all features and/or functionality of the prediction-based trial arm assignment system 1508 of Figure 5H can implement the prediction-based trial arm assignment system 1508 of Figure 5A and/or any embodiment of prediction-based trial arm assignment system 1508 described herein. Some or all features and/or functionality of the prediction-based trial arm assignment system 1508 of Figure 5H can implement the prognosis-based trial arm assignment system 508 of Figure 4I and/or any embodiment of the prognosis-based trial arm assignment system 508 described herein, for example, where functionality is extended to assigning participants to trial arms of scientific studies that are optionally non-medical based on predictive scores for a type of outcome that is optionally non-medical. The trial arm assignment function 672 and/or trial arm assignment module 730 of Figure 5H can be implemented in a same or similar fashion as trial arm assignment function 672 and/or trial arm assignment module 730 of Figure 4I, for example, where stratification factor data 683 indicates participants be stratified by outcome prediction scores 1636. Examples of implementing trial arm assignment module 730 are discussed in conjunction with Figures 6A - 6K.

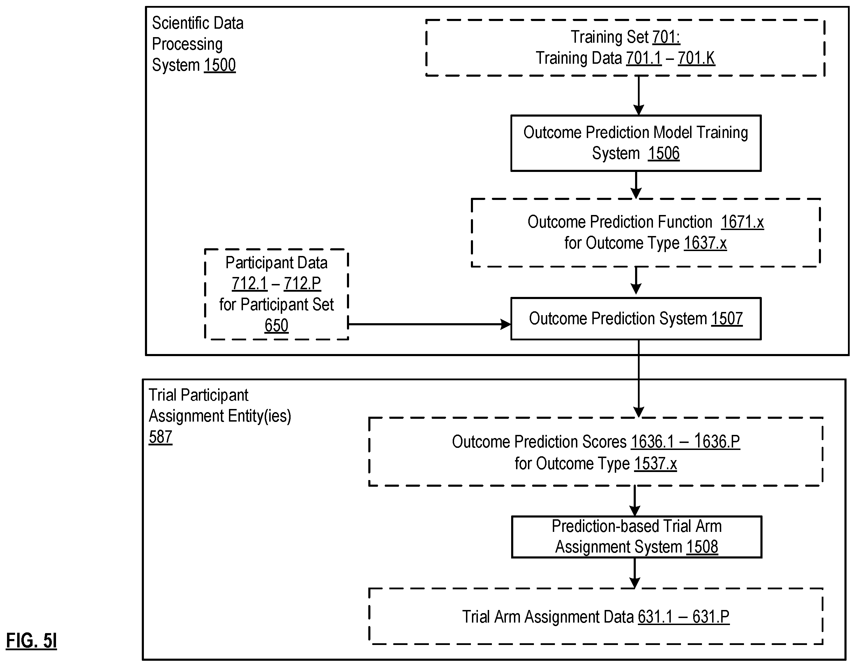

Figure 5I is a schematic block diagram of a scientific data processing system 1500 communicating with at least one trial participant assignment entity 587 in accordance with various embodiments. Some or all features and/or functionality prediction-based trial arm assignment system 1508 as described herein can be implemented via trial participant assignment entity 587 instead of or in addition to being implemented by the scientific data processing system 1500. For example, the scientific data processing system 1500 sends the outcome prediction scores 1636.1 - 1636.P to the trial participant assignment entity 587, for example, in conjunction with corresponding participant identifiers and/or other information utilized to stratify participants for randomization, and the trial participant assignment entity 587 generates the trial arm assignment data 631.1 - 631.P accordingly. The trial participant assignment entity 587 can optionally communicate trial arm assignment data 631.1 - 631.P back to the medical data processing system 500 to then be communicated by the medical data processing system 500 to the corresponding requesting entity/entity conducting the trial, or the trial participant assignment entity 587 can optionally communicate trial arm assignment data 631.1 - 631.P to the requesting entity/entity conducting the trial directly.

Figures 6A - 6K illustrate example embodiments of randomizing participants of clinical trials/other scientific studies via a randomization algorithm 800 performed by a trial arm assignment module 730 that processes medical scan prognosis scores 636 and/or other scientific prediction scores 1636. In particular, participants can be stratified as a function of their medical scan prognosis scores 636 and/or other scientific prediction scores 1636 as part of executing a corresponding randomization process.

Some or all features and/or functionality of the trial arm assignment module 730 of Figures 6A- 6K can implement the trial arm assignment module 730 Figure 4I, and/or Figure 5H, for example, in conjunction with implementing a corresponding prognosis-based trial arm assignment module 508 and/or corresponding prediction-based trial arm assignment module 1508, respectively, for example, as discussed in conjunction with Figure 4A and/or 5A, respectively.

Some or all features and/or functionality of the trial arm assignment module 730 of Figures 6A- 6K can implement the trial arm assignment module 730 of Figure 2O and/or 2P, for example, illustrative of processes performed by a corresponding trial participant assignment entity 587 (e.g. performed by human and/or computing resources of trial participant assignment entity 587, where some or all functionality of trial arm assignment module 730 of Figures 6A- 6K is performed automatically, via human intervention, of via a combination of both by trial participant assignment entity 587.

Some or all features and/or functionality of the processes of assigning patients to trial arms can implement the trial arm assignment function 672 and/or any embodiment of a randomization algorithm and/or corresponding randomization techniques 684 described herein.

As discussed previously, random assignment of individuals participating in a clinical trial/other scientific study to corresponding trial arms via a randomization algorithm that stratifies via medical outcome prognosis score and/or outcome prediction score can improve the accuracy of corresponding trial results and thus improve technological fields of medical product, treatments, and/or scientific products/hypothesis being tested, where these improvements are reliant on the use of specialized equipment (e.g. a trained machine learning model). In some embodiments, some or all of this corresponding random assignment is performed in a fashion that cannot be entirely performed via humans alone based on medical outcome prognosis score and/or outcome prediction score not being able to be generated via the human mind.

In some embodiments, some or all portions of performing randomization algorithm performed in conjunction with performing trial arm assignment function 672 cannot be feasibly performed by the human mind. For example, it is not feasible for the human mind to perform any random/pseudo-random process without inducing corresponding biases and/or otherwise inducing pattern-based/non-random assignment, unless an outside means of random number generation is utilized (e.g. separate from processing occurring within the human mind alone, even if aided by pen and paper). Automatic performance of the randomization algorithm in performing trial arm assignment function 672 by the medical data processing system 500 and/or scientific data processing system 1500 further improves the technology of conducting clinical trials, and the technologies of the corresponding treatments/products/scientific fields being tested/studied, based on ensuring the trial is conducted fairly via proper, randomized assignment being performed.

In some embodiments, the randomization algorithm in performing trial arm assignment function 672 is performed automatically by the medical data processing system 500 and/or scientific data processing system 1500 without human intervention. This can further improve the technology of conducting clinical trials, and the technologies of the corresponding treatments/products/scientific fields being tested/studied, based on aiding in ensuring that the trial is conducted via proper strong blinding procedures, such as double-blind trial arm assignments, without human intervention/knowledge of corresponding groupings of individuals into trial arms, which could sacrifice integrity of the corresponding clinical trial/scientific study. For example, automated of prognosis-based trial arm assignment system 508 and/or prediction-based trial arm assignment system 1508 to assign participants to trial arms can aid in concealing the assignments from humans that observe/administer the corresponding trial, which can be preferred over having such humans perform the random assignment, which could render these humans as not being able to also observe/treat corresponding participants in the trial based on violation of strong blinding requirements.

In other embodiments, the randomization algorithm in performing trial arm assignment function 672 is performed, at least in part, via human intervention by one or more humans. For example, human intervention is utilized to identify predetermined random assignment to trial group, for example, in accordance with implementing a permuted block randomization process. As another example, human intervention is applied in accordance with assigning participants in accordance with any type of randomization technique, which can optionally incorporate one or more of: a simple randomization technique, a block randomization technique, a stratified randomization technique, a covariate adapted randomization technique, or a permuted block randomization technique,

Human intervention can be applied in other ways, for example, while still preserving double-blinding requirements.

Figure 6A illustrates an embodiment of a prognosis-based trial arm assignment system 508 that implements trial arm assignment module 730 to assign participants 1-P of a participant set 650 to a plurality of trial arms 805.1 - 805.Y as a function of medical outcome prognosis scores 636.1 - 636.P via performance of a randomization algorithm 800, rendering a corresponding plurality of participant sets 803.1 - 803.Y. Some or all features and/or functionality of the prognosis-based trial arm assignment system 508 of Figure 6A can implement the prognosis-based trial arm assignment system 508 of Figure 4I and/or 4A.

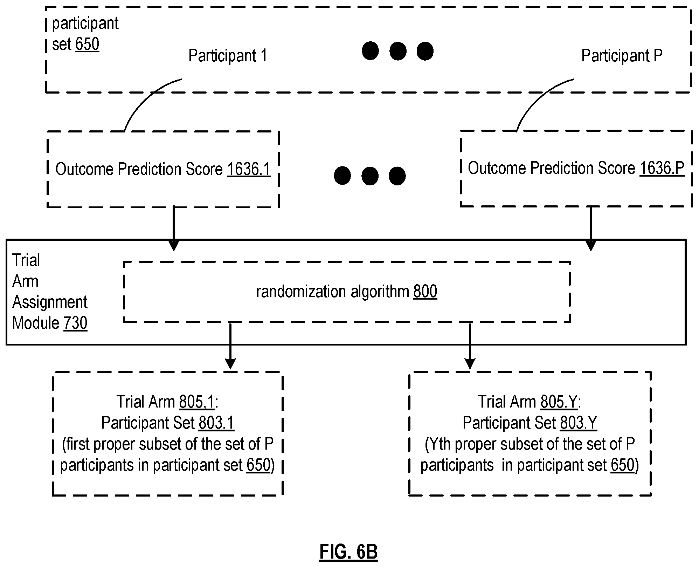

Figure 6B illustrates an embodiment of a prediction-based trial arm assignment system 508 that similarly implements trial arm assignment module 730 to assign participants 1-P of a participant set 650 to a plurality of trial arms 805.1 - 805.Y a function of outcome prediction scores 1636.1 - 1636.P via performance of a randomization algorithm 800, rendering a corresponding plurality of participant sets 803.1 - 803.Y. Some or all features and/or functionality of the prediction-based trial arm assignment system 1508 of Figure 6B can implement the prediction-based trial arm assignment system 1508 of Figure 5H and/or 5A.

The participant set 650 can be considered a full participant set (e.g. all prospective/confirmed participants for the trial) while participant sets 803.1 - 803.Y are each a corresponding subset of the full participant set 650, corresponding to only ones of the participants 1-P assigned to the corresponding trial arm 805.

Each of the plurality of participant sets 803.1 - 803.Y generated via trial arm assignment module 730 of Figure 6A and/or 6B can correspond to non-null proper subsets of the participant set 650.For example, none of the participant sets 803.1 - 803.Y include all of the participants 1-P of participant set 650, and/or each of the participant sets 803.1 - 803.Y includes at least one of the participants 1-P of participant set 650.

Alternatively or in addition, each of the plurality of participant sets 803.1 - 803.Y generated via trial arm assignment module 730 of Figure 6A and/or 6B can be non-null subsets of the participant set 650. For example, none of the participant sets 803.1 - 803.Y include all of the participants 1-P.

Alternatively or in addition, the plurality of participant sets 803.1 - 803.Y generated via trial arm assignment module 730 of Figure 6A and/or 6B can be mutually exclusive. For example, each participant is included in exactly one (or optionally none) of the participant sets 803.1 - 803.Y via corresponding assignment to a corresponding exactly one (or optionally none) of the plurality of trial arms 805.1 - 805.Y.

Alternatively or in addition, the plurality of participant sets 803.1 - 803.Y generated via trial arm assignment module 730 of Figure 6A and/or 6B can be collectively exhaustive with respect to the participant set 650. For example, all participants are included in one of the participant sets 803.1 - 803.Y based on being assigned to one (or optionally none) of the plurality of trial arms 805.1 - 805.Y. Alternatively, in some embodiments, the plurality of participant sets 803.1 - 803.Y are optionally not collectively exhaustive with respect to the participant set 650 (e.g. at least one participant is not included any the participant sets 803.1 - 803.Y based on being determined to not participate in the trial, for example, based on being deemed ineligible and/or a non-ideal candidate for the trial, the trial being determined to be full/complete, etc.).

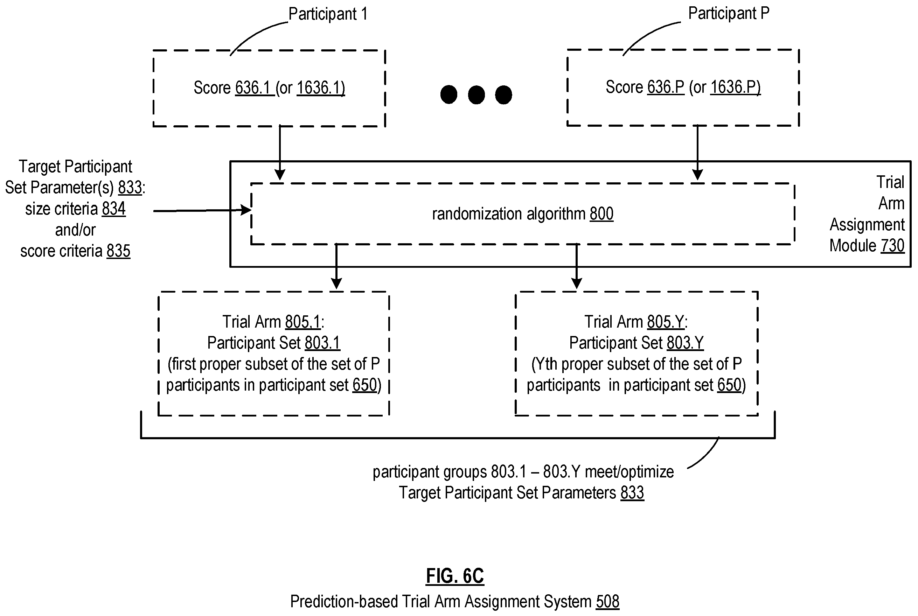

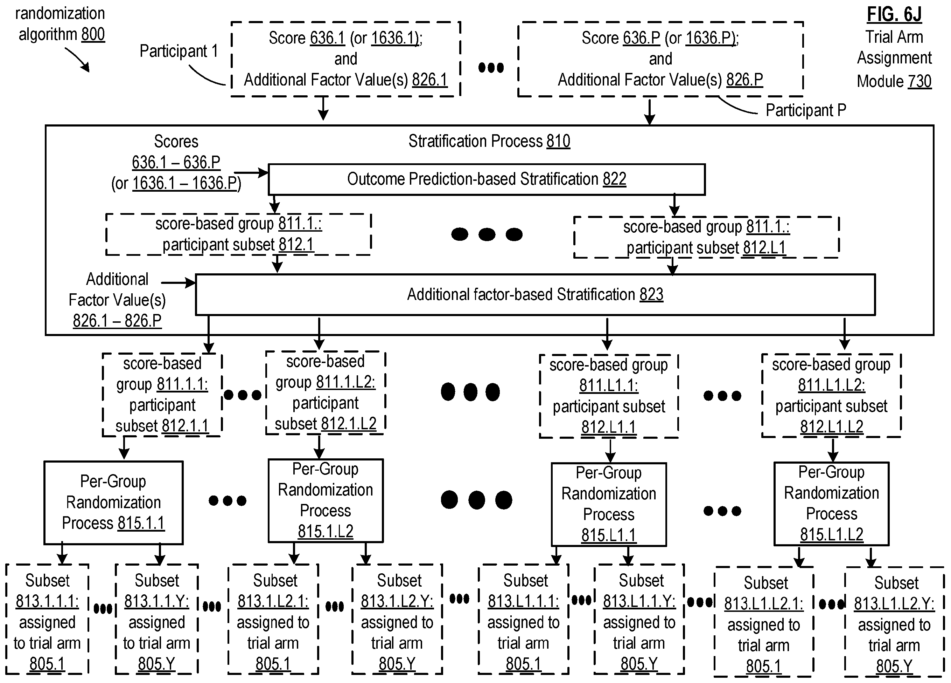

Alternatively or in addition, the plurality of participant sets 803.1 - 803.Y are generated via trial arm assignment module 730 of Figure 6A and/or 6B in accordance with meeting/optimizing particular criteria. For example, the outputted plurality of participant sets 803.1 - 803.Y meet (and/or were generated based on optimizing) this criteria. Such embodiments are discussed in conjunction with Figure 6C and Figure 6J.

These participant sets 803.1 - 803.Y generated via trial arm assignment module 730 of Figure 6A and/or 6B can indicate groups of participants assigned to different trial arms 805 can be identified/implemented via the trial arm assignment data 631.1 - 631.P, for example, of Figure 4I and/or Figure 5H. For example, participant set 803.1 corresponds to the participants of participant set 650 having corresponding trial arm assignment data 631 indicating assignment to trial arm 805.1; participant set 803.2 corresponds to the participants of participant set 650 having corresponding trial arm assignment data 631 indicating assignment to trial arm 805.2; etc.

These participant sets 803.1 - 803.Y generated via trial arm assignment module 730 of Figure 6A and/or 6B are optionally not generated all at once, and are optionally generated over time. For example, as discussed previously, a given trial/study is conducted over a period over time where different participants optionally start the trial separately, at separate times. Thus, participant sets 803.1 - 803.Y are optionally built over time as different participants are identified for participation over the course of the trial/study. As a particular example, a first given participant identified as a candidate for the trial can be processed via trial arm assignment module prior to some or all other participants being identified for the trial. This first given participant has corresponding trial arm assignment data 631 generated by trial arm assignment module 730 indicating their assignment to a given trial arm (e.g. trial arm 805.1), and are thus included in the participant set 803 (e.g. participant set 805.1). At a later time, for example, after this first participant's participation in the trial via the corresponding trial arm (e.g. corresponding treatment/experimentation being administered), and/or optionally after the first participant has completed their respective participation in the trial, a second given participant is identified as a candidate for the trial and similarly has corresponding trial arm assignment data 631 generated by trial arm assignment module 730 indicating their assignment to a given trial arm (e.g. the trial arm 805.1, or a different trial arm 805 such as trial arm 805.2), and are thus included in the participant set 803 (e.g. participant set 805.1, or a different trial arm 805 such as trial arm 805.2).

In other embodiments, participant sets 803.1 - 803.Y can instead be generated all at once, for example, once the participant set 650 is finalized. For example, all participants have their scores 636 and/or 1636 processed by trial arm assignment module once all P participants are identified to render generation of participant sets 803.1 - 803.Y based on generation of all trial arm assignment data 631.1 - 631.P, where the trial can then commence for all participants with respect to their assigned trial arms once these participant sets 803.1 - 803.Y are identified. Such embodiments can optionally enable collective processing of scores, for example, in accordance with meeting/optimizing parameters and/or criteria, such as target participant set parameters (e.g. as discussed in conjunction with Figure 6C and/or 6J). Note that in some embodiments, configuring participant sets accordance with meeting/optimizing such parameters and/or criteria, such as target participant set parameters, can be performed while still generating the participant groups over time based on assigning incoming participants to trial arms over time as new participants are identified.

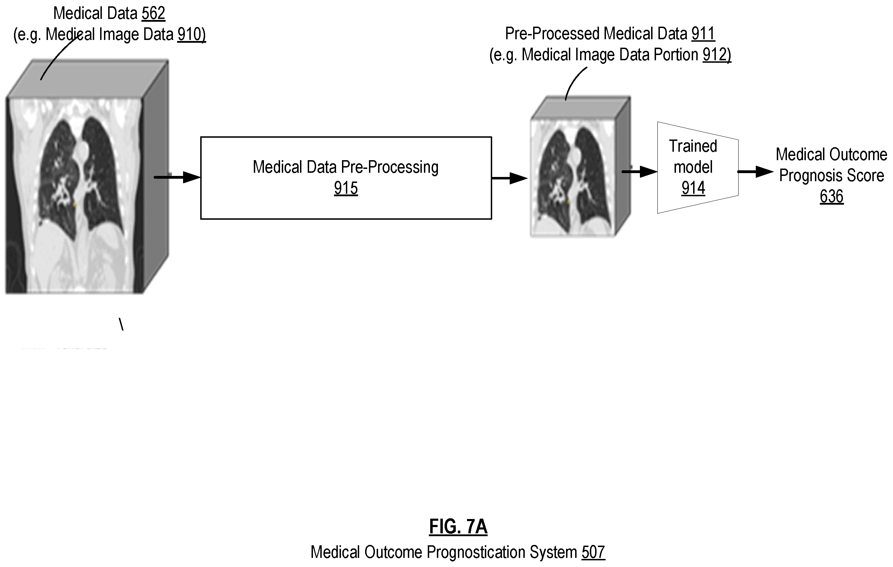

Figure 6C illustrates an embodiment of a trial arm assignment module 730 that assigns participants 1-P of a participant set 650, in accordance with applying one or more target participant set parameters 633 to a plurality of trial arms 805.1 - 805.Y as a function scores for a participant set 650 (e.g. scores 636.1 - 636.P via performance of randomization algorithm 800, for example where the trial arm assignment module is implemented via prognosis-based trial arm assignment module 508; or scores 1636.1 - 1636.P, for example, where the trial arm assignment module is implemented via prediction-based trial arm assignment module 1508). Some or all features and/or functionality of the trial arm assignment module 730 of Figure 7C can implement the trial arm assignment module 730 of Figure 7A, 7B, 4I and/or 5H, and/or can implement any embodiment of trial arm assignment module 730 described herein.

The target participant set parameters 633 can be predetermined, configured via user input, accessed in memory, received by a corresponding subsystem, automatically generated, and/or otherwise determined and/or applied. The target participant set parameters 633 can indicate size criteria 834, score criteria 835, and/or any other type of criteria.

The target participant set parameters 633 can indicate size criteria 834 corresponding to criteria relating to numbers of participants in participant sets 803 of various trial arms. In particular, the size of a given participant set 803 can correspond to the number of participants included in that participant set 803 (i.e. number of participants assigned to the corresponding trial arm 805). For example, the target participant set parameters 633 can dictate sizes of participant sets (e.g. min and/or max size) and/or dictating sizes of participant sets relative to each other (e.g. threshold difference in size; threshold variation in size across the set of trial arms; max and/or min average size across the set of trial arms; etc.) The target participant set parameters 633 can optionally indicate target size parameters and/or threshold deviation from these targets (e.g. to be applied in an optimization in accordance with also optimizing other types of parameters).

For example, the plurality of participant sets 803.1 - 803.Y are ideally a same or similar size for some or all trials/studies, for example, to render best statistical properties in evaluating results. The size criteria can indicate that all participants be equal in size (e.g. all include same number of participants) or relatively equal in size, for example, up to a given threshold difference (e.g. differ by a maximum of 5 participants; largest participant set must be no more than 110% the size of another participant set; etc.). In other embodiments, the size criteria optionally specifies non-equal sizing (e.g. corresponding fixed/desired proportions of participants across trial arms that are optionally non-equal).

In some embodiments, the plurality of participant sets 803.1 - 803.Y generated via the trial arm assignment system of Figures 6A and/or 6B are optionally equal in size and/or are similar in size (e.g. up to a configured threshold difference). For example, trial arm assignment module can be implemented to disperse participants 1-P of participant set 650 evenly, or relatively evenly, amongst the trial arms, for example, in accordance with adhering to the size criteria 834 and/or otherwise being configured to assign participants in this fashion. As a particular example, this even/relatively even dispersal of 1-P of participant set 650 across the Y trial arms 805.1 - 805.Y can be based on applying a permuted block randomization technique (e.g. where the permuted block randomization technique is implemented as some or all randomization techniques 684). In other embodiments, this even/relatively even dispersal of 1-P of participant set 650 across the Y trial arms 805.1 - 805.Y can be based on, instead of or in addition to applying the permuted block randomization technique, applying at least one other randomization technique, which can incorporate one or more of: a simple randomization technique, a block randomization technique, a stratified randomization technique, or a covariate adapted randomization technique.

As a particular example, the number of participants in some or all participant sets 803 is configured/guaranteed to be equal to, close to, and/or within a predefined threshold of the quotient P/Y for some or all trials/studies. The number of participants P is optionally configured to be a multiple of Y to guarantee equal numbers of participants in all trial arms. In embodiments where P is not a multiple of Y, the trial arms can be configured to have as equal of a number of participants as possible (e.g. all trial arms are within one participant of each other in size). Alternatively, greater variation in size is rendered, for example, based on adhering to/accounting for other criteria indicated in target participant set parameters 833 such as score criteria 835, and/or based on characteristics of the randomization algorithm. As a particular example, this greater variation in size is rendered (e.g. while still being within a predetermined threshold variation) based on achieving more even score-based distribution of patients (e.g. the set of participant sets 803. 1 - 803.Y have more similar distributions of scores, such as more similar average scores, than they could if more equal group sizes were attained).

The target participant set parameters 633 can alternatively or additionally indicate score criteria 835 corresponding to criteria relating how scores (e.g. prognosis-based medical outcome scores 636 and/or prediction-based outcome scores 1636) of participants in various participant sets 803 of various trial arms are distributed. For example, the target participant set parameters 633 can dictate average scores/distribution of participant sets (e.g. min and/or max average score) and/or dictating average scores of participant sets/distribution of scores of participant sets relative to each other (e.g. threshold difference in average score across the set of trial arms; threshold variation in average score across the set of trial arms; threshold difference in variation of score/distribution of scores of different participant sets across the set of trial arms; etc.) The target participant set parameters 633 can optionally indicate target score parameters and/or threshold deviation from these targets (e.g. to be applied in an optimization in accordance with also optimizing other types of parameters).

In some embodiments, the plurality of participant sets 803.1 - 803.Y have ideally a same or similar distribution of scores for some or all trials/studies, for example, to render best statistical properties in evaluating results. For example, participants with similar predictions for the primary endpoint are ideally evenly dispersed across trial arms, which is preferred over than having an uneven balance that could render skewed/misrepresentative trial results. For example, an uneven balance that could render skewed/misrepresentative trial results could be based on one trial arm largely made up of participants predisposed to have a first given primary endpoint/having high probabilities of a given event being observed as the primary endpoint occurring; and having another trial arm largely made up of participants predisposed to have a second given primary endpoint/having low probabilities of the given event being observed as the primary endpoint occurring.

The score criteria can thus indicate that participants be dispersed across trial arms evenly/relatively evenly by score, and/or can indicate corresponding target parameters such as: requirement/target for equal average score and/or relatively equal average score; a corresponding maximum deviation of any trial arm from average scores of other trial arms; equal average score and/or relatively equal distribution of scores; a corresponding maximum deviation of distribution of score of any trial arm from distribution (e.g. as measured by max deviation in average/variance/other statistical measures); and/or other corresponding target parameters. In other embodiments, the size criteria optionally specifies non-even distribution of participants by score (e.g. corresponding fixed/desired average score/score distributions of participants across trial arms that are optionally different).

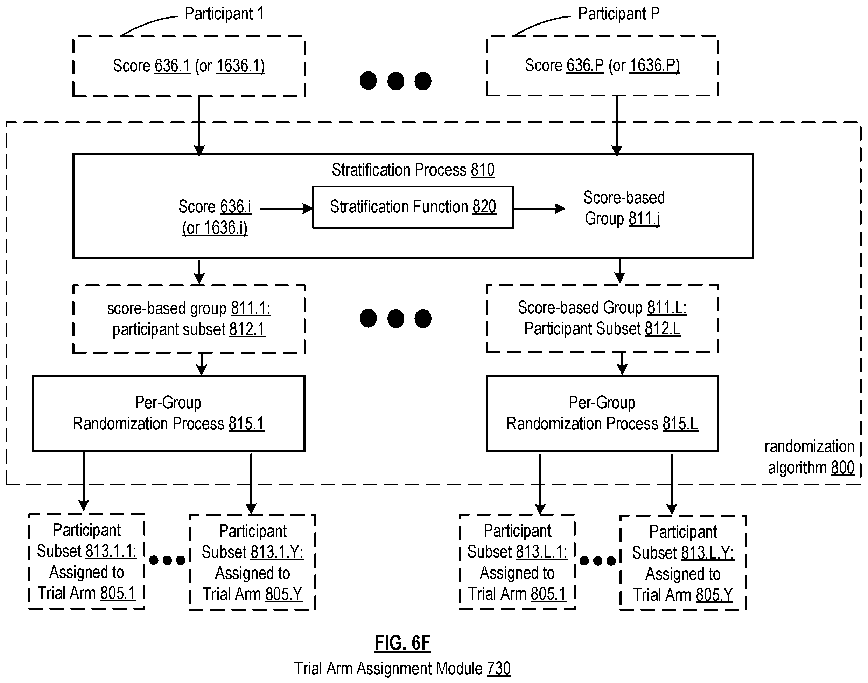

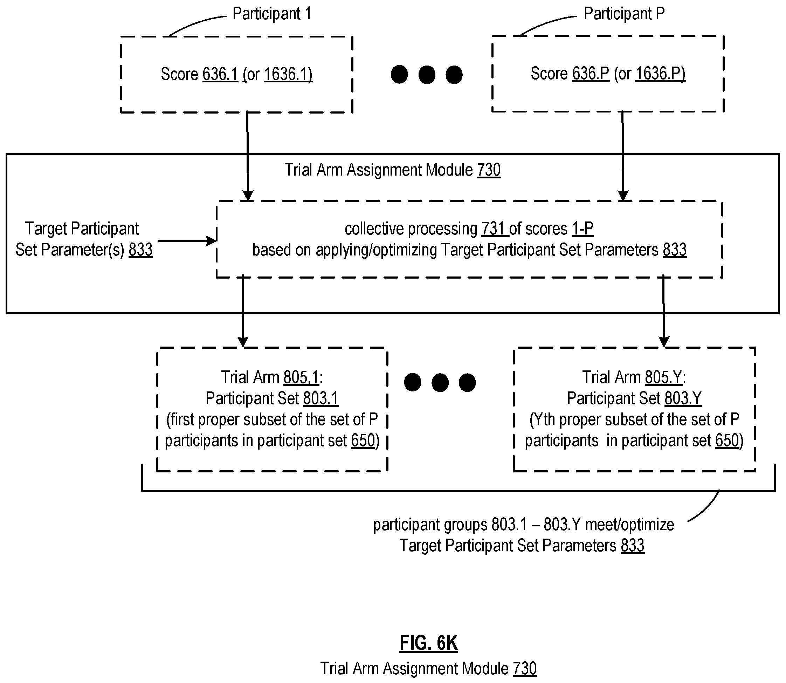

In some embodiments, the plurality of participant sets 803.1 - 803.Y generated via the trial arm assignment system of Figures 6A and/or 6B optionally have equal and/or similar score distributions. For example, trial arm assignment module can be implemented to disperse participants 1-P of participant set 650 amongst the trial arms based on stratifying participants by score 636 and/or 1636 (e.g. via some or all functionality discussed in conjunction with Figures 6F - 6J). Alternatively or in addition, trial arm assignment module can be implemented to configure participant sets 803.1 - 803.Y in accordance with minimizing differences in score distribution across all participant sets (e.g. minimizing difference in average score and/or minimizing difference in variance of score), for example, in conjunction with collectively processing the scores across participants 1 - P and/or based on applying a corresponding optimization algorithm (e.g. via some or all functionality discussed in conjunction with Figure 6K). Such equal/relatively distribution of participant scores across trial arms (e.g. in applying score parameters 835) can be achieved in accordance with also rendering equal/relatively equal sizes of the corresponding participant groups across trial arms (e.g. in applying size parameters 834).

In some embodiments, the trial arms can be configured via as equal of dispersal of scores across trial arms as possible (e.g. to achieve the closest score averages possible/most similar score distribution possible, given the set of scores 636.1 - 636.P and/or 1636.1 - 1636.P). Alternatively, greater variation in score is rendered, for example, based on adhering to/accounting for other criteria indicated in target participant set parameters 833 such as size criteria 834, and/or based on characteristics of the randomization algorithm. As a particular example, this greater variation in score average/distribution characteristics across trial arms is rendered (e.g. while still being within a predetermined threshold variation) based on achieving more evenly-sized participant sets 803.1 - 803.Y (e.g. the set of participant sets 803. 1 - 803.Y have more similar sizes, such as equal sizes or similar equal sizes, than they could if more equal score averages/distribution characteristics across trial arms were attained).

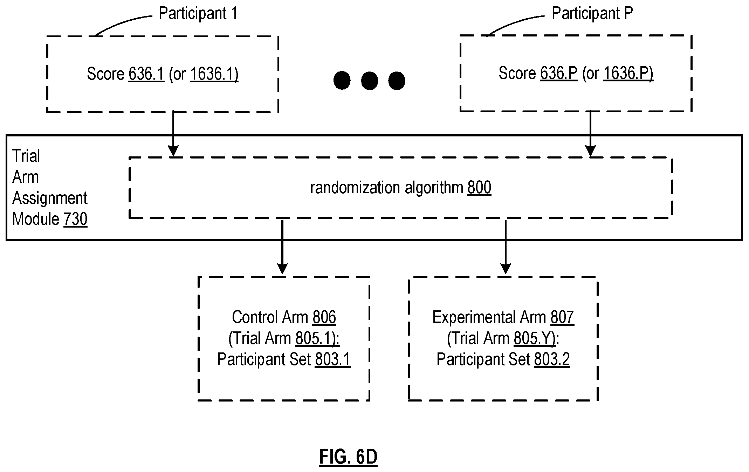

Figure 6D illustrates an embodiment of a trial arm assignment module 730 that assigns participants 1-P to either first trial arm 805.1 implemented as a control arm 806 or a second trial arm 805.2 implemented as an experimental arm 807, via dispersal of patients to two corresponding participant sets 803.1 and 803.2 accordingly. Some or all features and/or functionality of trial arm assignment module 730 of Figure 6D can implement the trial arm assignment module 730 of Figure 6A and/or 6B, and/or any embodiment of trial arm assignment module 730 described herein.

In particular, some or all embodiments of the set of trial arms 805.1 - 805.Y described herein optionally corresponds to exactly two trial arms: one control arm 806 and one experimental arm 807 (e.g. Y = 2). For example, in cases where the trial arm assignment module 730 is configured to assign equal/relatively numbers of patients to groups, participant sets 803.1 and 803.2 optionally each have exactly/close to P/2 participants.

An entity conducting the trial (e.g. medical product manufacturing and/or testing entity 577 and/or scientific study conducting entity 1577) can conduct the corresponding trial/study based on treating/processing the participants assigned to the control arm 806 and experimental group 807 differently. For example, participants in participant set 803.1 assigned to the control arm 806 are not administered a corresponding treatment/are not observed under conducting of a corresponding experimental process, while participants in participant set 803.2 assigned to the experimental arm 807 are administered a administered a corresponding treatment/are observed under conducting of a corresponding experimental process. For example, in the case of a clinical trial testing a medical product 777 (e.g. via a clinical trial conducting process 746) the medical product 777 is optionally not administered to participants in participant set 803.1 of control arm 806 (e.g. a placebo is applied), while the medical product 777 is administered to participants in participant set 803.2 of experimental arm 807. As used herein, the experimental arm can thus be implemented as a treatment arm and/or investigational arm for a corresponding clinical trial/scientific study, and the terms "experimental", "treatment", and "investigational" are interchangeably applied herein in conjunction with describing a corresponding trial arm/corresponding group of participants in a clinical trial and/or other scientific study.

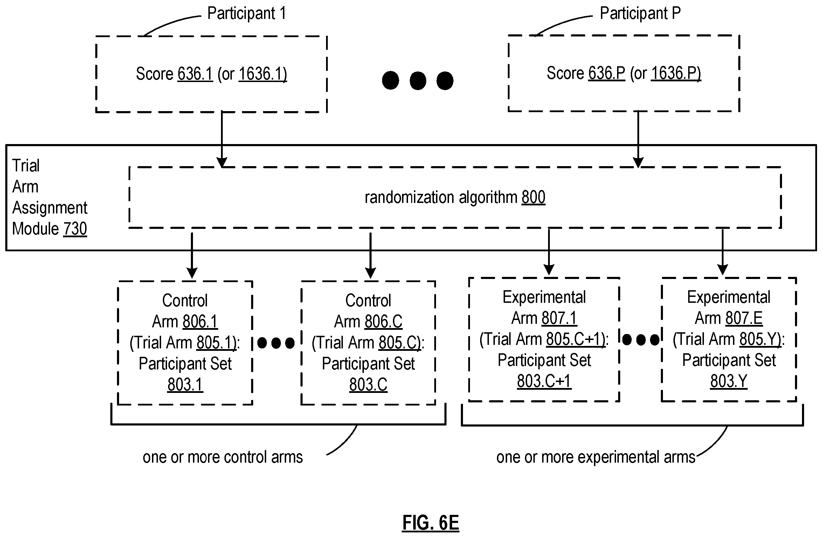

Figure 6E illustrates an embodiment of a trial arm assignment module 730 that assigns participants 1-P to one of three or more trial arms 805.1 - 805.Y (e.g. Y > 2). For example, the set of trial arms 805.1 - 805.Y includes a set of C control arms and includes a set of E experimental arms, where: C is greater than or equal to one; E is greater than or equal to one; Y is equal to C+E; and/or either C or E (or both) is strictly greater than one. Some or all features and/or functionality of trial arm assignment module 730 of Figure 6E can implement the trial arm assignment module 730 of Figure 6A and/or 6B, and/or any embodiment of trial arm assignment module 730 described herein.

For example, for some or all trials/studies for which patient arms are assigned via a trial arm assignment module as described herein, C = 1 and E = 1, where the embodiment of Figure 6D with exactly two groups is optionally implemented. As another example, for some or all trials/studies for which patient arms are assigned via a trial arm assignment module as described herein, C > 1 and E = 1, where more than one control arm is implemented and exactly one experimental arm is implemented. As another example, for some or all trials/studies for which patient arms are assigned via a trial arm assignment module as described herein, C = 1 and E > 1, where more than one experimental arm is implemented and exactly one control arm is implemented. As another example, for some or all trials/studies for which patient arms are assigned via a trial arm assignment module as described herein, C > 1 and E > 1, where more than one experimental arm is implemented and also more than one control arm is implemented. Different clinical trials/scientific studies can have same or different numbers of trial arms Y and/or can have same or different proportions of/configurations of their Y trial arms as either control arms or experimental arms.

In some embodiments, the set of control groups 806.1 - 806.C includes at least one positive control group, at least one negative control group, and/or at least one placebo control group. The set of control groups 806.1 - 806.C can include any other set of one or more types of control groups, for example, in conjunction with conducting a controlled scientific experiment.

Alternatively or in addition, in some embodiments, the set of experimental groups 807.1 - 807.E apply different forms of a corresponding treatment/experimental procedure. For example, some or all different experimental groups apply different doses of a corresponding treatment (e.g. different dosage amounts of a drug and/or other pharmaceutical compound). Alternatively or in addition, some or all different experimental groups apply different types of treatment (e.g. different pharmaceutical compounds, different procedures, etc.). The set of experimental groups 807.1 - 807.E can otherwise apply different types of treatment/experimentation, for example, in conjunction with measuring/testing different observable responses of these different forms of treatment/experimentation in generating corresponding results of the trial/study. In some cases, only a single experimental arm is implemented.

Figure 6F illustrates an embodiment of a randomization algorithm 800 performed via trial arm assignment module 730. Some or all features and/or functionality of the trial arm assignment module 730 and/or randomization algorithm 800 of Figure 6F can implement the trial arm assignment module 730 and/or randomization algorithm 800 of Figure 6A and/or 6B, and/or any other embodiment of the trial arm assignment module 730 and/or randomization algorithm described herein.

Performing randomization algorithm 800 can include performing a stratification process 810 to generate a plurality of score-based groups 811.1 - 811.L. Performing randomization algorithm 800 can include performing a corresponding per-group randomization process 815 upon each score-based group 811 to render a plurality of participant subsets 812.1 - 812.Y for each score-based group 811, corresponding to assignment of participants in each score-based group to a corresponding trial arm 805.

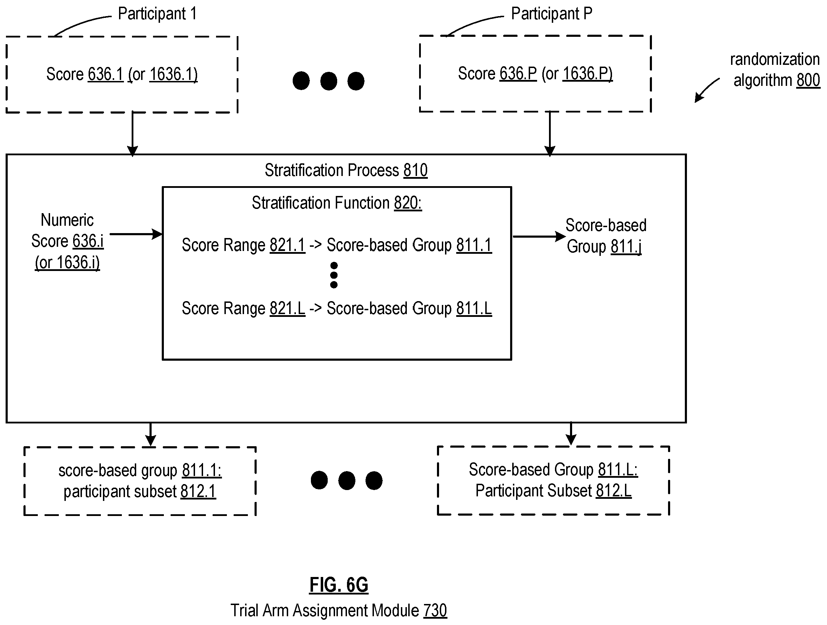

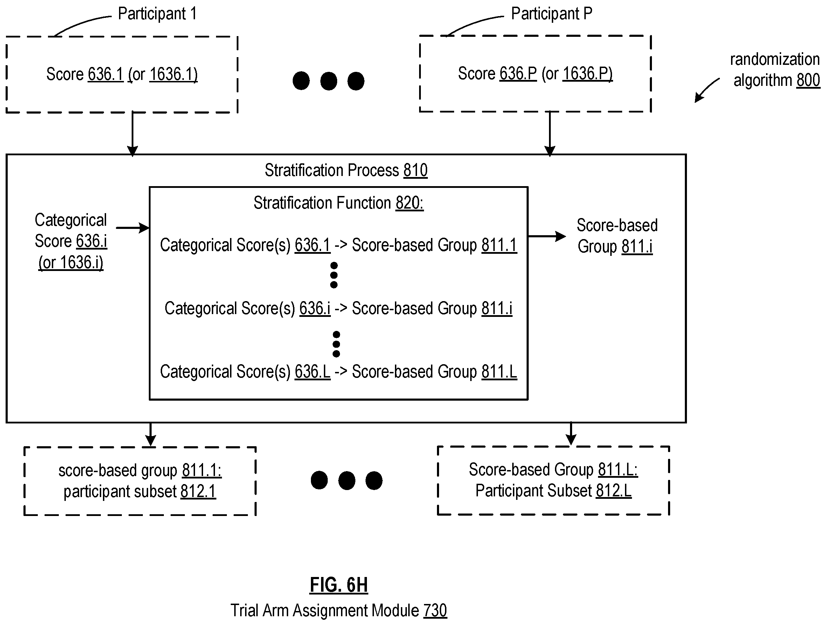

The stratification process 810 can be implemented to group each participant into one of a plurality of score-based groups 811.1 - 811.L as a function of their prognosis-based medical outcome score 636 (e.g. where trial arm assignment module 730 is implemented via a prognosis-based trial arm assignment system 508), or similarly as a function of their prediction-based medical outcome score 1636 (e.g. where trial arm assignment module 730 is implemented via a prognosis-based trial arm assignment system 508). This can include performing a corresponding stratification function 820 upon each given score 636.i or 1636.i (e.g. the score assigned to the ith participant in the participant set). Any number of two or more score-based groups can be implemented (e.g. L > 1). Examples of stratification function 820 are discussed in conjunction with Figure 6G and 6H.

In some embodiments, assignment of a given participant into a given one of the set of score-based groups 811.1 - 811.L groups via stratification function 820 is a deterministic function of their score 636, for example, where all randomization employed in trial arm assignment is later applied via the corresponding per-group randomization processes 815.1 - 815.L. For example, stratification function 820 can optionally be implemented as a deterministic mapping (e.g. many-to-one mapping) of score to score-based group. In such embodiments, any score 636/1636 having a particular value v is guaranteed to map to the same score based-group 811. This can be useful in assigning participants to groups over time, as the deterministic mapping stays constant and group assignment is not dependent on scores of other participants. Scores 636/1636 that are similar can be mapped to the same score-based group, rendering score-based groups 811 that include patients with same and similar scores.

In some embodiments, assignment of a given participant into a given one of the set of score-based groups 811.1 - 811.L groups via stratification function 820 is based on first arranging/ordering participants by score 636, and then grouping participants into score-based groups, for example, in conjunction with a desired size of each score-based group (e.g. a predetermined block size for applying permuted block randomization technique). For example, all P participants are first ordered by score (e.g. numerically) to render an ordered list. This ordered list can then be subdivided into L contiguous groups to render participant subsets 812.1 - 812.L. For example, the length of each contiguous group can be in accordance with a predetermined size (e.g. the predetermined block size), rendering assignment of the predetermined number of participants to each of the participant subset 812.1 - 812.L. The predetermined size can be the same or different for different groups. Such means of generating participant subset 812.1 - 812.L can be considered a collective processing of scores, as all participant scores must be determined prior to ordering of the scores and grouping by this ordering, and can optionally implement some or all functionality of collective processing of scores discussed in conjunction with Figure 6K.

The plurality of participant subsets 812.1 - 812.L can each be a proper subset of the plurality of participants 1-P of participant set 650 (e.g. each of the plurality of participant subsets 812.1 - 812.L does not include all participants 1-P). Alternatively or in addition, some or all of the plurality of participant subsets 812.1 - 812.L can each and/or can further be non-null (e.g. assuming that scores across participant set 650 collectively mapped to all score-based groups). Alternatively or in addition, one or more of the plurality of participant subsets 812.1 - 812.L can each and/or can be null (e.g. there were no participants in participant set 650 with scores mapping to at least one corresponding score-based groups). Alternatively or in addition, the plurality of participant subsets 812.1 - 812.L can be mutually exclusive (e.g. each participant is assigned to exactly one score-based group). Alternatively or in addition, the plurality of participant subsets 812.1 - 812.L can be collectively exhaustive with respect to the plurality of participants 1-P of participant set 650 (e.g. all participants are assigned to a score-based group).

Alternatively, some participants are not optionally assigned to a score-based group 811, for example, based on being deemed ineligible for the trial and/or not being selected. In some embodiments, the stratification process is implemented to automatically select which participants are excluded from the trial as a function of their score 636/1636. For example, one or more score-based groups 811 correspond to trial-exclusion groups, where scores mapped to these groups are deemed indicative of the patient being ineligible for the trial/otherwise not being ideal/selected for participation (e.g. scores indicating a corresponding patient will likely be deceased prior to completion of the trial generated via a corresponding medical outcome prognostication function predicting mortality, etc.). In such embodiments, the participant subsets of such exclusion groups are not assigned to trial arms via a corresponding per-group randomization process 815 based on being selected to be excluded from participation. In other embodiments, no such exclusion groups are implemented as score-based groups, where all participants in participant subsets 812.1 - 812.L are ultimately assigned to groups via respective per-group randomization processes 815 (e.g. all participants 1-P are thus assigned to a trial arm 805, or one or more participants are excluded elsewhere, such as via other evaluation prior to the stratification process).

Participants in participant subsets 812 assigned to a given score-based group 811 can be assigned to a given trial arm 805 via a corresponding per-group randomization process 815. In particular, each participant subsets 812 can be randomized separately, for example, via separately applying a same randomization technique 684. For example, each participant subset 813 is indicated by corresponding trial arm assignment data 631 generated for each participant in a corresponding participant subsets 812 of the corresponding score-based group 811.

Performance of a given per-group randomization process 815 upon a given participant subset 812 can thus render generation of a set of Y corresponding participant subsets 813.1 - 813.Y based on assignment of each participant to a corresponding trial arm. The plurality of participant subsets 813.1 - 813.Y generated via given per-group randomization process 815 performed upon a given participant subset 812 can be mutually exclusive and/or collectively exhaustive with respect to the given participant subset 812 (e.g. every participant in the given participant subset 812 is assigned to exactly one trial arm 805). Alternatively or in addition, the each participant subsets 813 generated via given per-group randomization process 815 performed upon a given participant subset 812 can each further be a proper subset of the participant subset 812 (e.g. each of the participant subsets 813.1 - 813.Y generated from a given participant subset 812 via per-group randomization process 815 include only participants from the participant subset 812, and/or do not include all participants of the participant subset 812). Alternatively or in addition, the each participant subsets 813 generated via given per-group randomization process 815 performed upon a given participant subset 812 can each further be a non-null subsets (e.g. assuming there were enough participants in the given participant subset 812 to render dispersal of participants across all trial arms 805.1 - 805.Y).

Performance of the set of per-group randomization processes 815.1 - 815.L upon the set of a participant subset 812.1 - 812.L can thus render generation of a set of YxL corresponding participant subsets 813.1.1 - 813.1.Y, ... ,813.L.1 - 813.L.Y. The plurality of L participant subsets assigned to a given trial arm can collectively constitute the full corresponding trial arm (e.g. participant subset 803.1 of trial arm 805.1 is equivalent to a union of participant subsets 813.1.1, 813.2.1, ... 813.L.1; participant subset 803.2 of trial arm 805.2 is equivalent to a union of participant subsets 813.1.2, 813.2.2, ... 813.L.2; ... ; participant subset 803.Y of trial arm 805.Y is equivalent to a union of participant subsets 813.1.Y, 813.2.Y, ... 813.L.Y). The plurality of L participant subsets 813 assigned to a given trial arm can thus each be proper subsets of the corresponding full participant subset 803 assigned to the given trial arm. The plurality of L participant subsets 813 assigned to a given trial arm can be mutually exclusive and/or collectively exhaustive with respect to the full participant subset 803 assigned to the given trial arm.

The performance of per-group randomization process 815 upon each score-based group can render adhering to size criteria 834. For example, even dispersal (dispersing in accordance with other non-even desired proportions) of the participants in each given score-based group across the set of Y trial arms 805.1 - 805.Y via a corresponding per-group randomization process 815 can render equal/near-equal sized participant subsets 803.1 - 803.Y (and/or participant subsets 813 in accordance with the other non-even desired proportions). This can further result in more accurate/less skewed clinical trial results as discussed previously, which can result in greater scientific advancement/more effective products being manufactured.

The performance of stratification process 810 prior to randomization via per-group randomization processes 815 applied separately to each resulting score-based group 811 can further render adhering to score criteria 835. For example, each per-group randomization process is implemented to evenly disperse patients across trial arms, where the collective use of these per-group randomization process across all score-based groups 811 renders even dispersal of participants having same/similar scores across the set of trial arms 805.1 - 805.Y, rendering trial arms 805.1 - 805.Y having similar score distribution characteristics for the respective participant sets 803.1 - 803.Y. This can result in more accurate/less skewed clinical trial results as discussed previously, which can result in greater scientific advancement/more effective products being manufactured.

Figure 6G illustrates an embodiment of a stratification function 820 performed via stratification process 810 that is implemented as a mapping of a set of score ranges 821.1 - 821.L to score based-groups 811.1 - 811.L. Some or all features and/or functionality of the stratification function 820 of Figure 6G can implement the stratification function 820 of Figure 6F and/or any other embodiment of stratification function 820 described herein. Some or all features and/or functionality of the trial arm assignment module 730 and/or randomization algorithm 800 of Figure 6G can implement the trial arm assignment module 730 and/or randomization algorithm 800 of Figure 6A and/or 6B, and/or any other embodiment of the trial arm assignment module 730 and/or randomization algorithm described herein.

For example, the set of score ranges 821.1 - 821.L are a set of contiguous score ranges of a full score domain that collectively constitute all of a plurality of possible numeric scores 636 and/or 1636 (e.g. where medical outcome prognosis scores 636 and/or outcome prediction scores 1636 generated via a medical outcome prognostication function and/or an outcome prediction function, respectively, are implemented as a continuous variable and/or numeric values having a corresponding ordering on a corresponding numeric scale). Each score range 821 can indicate a continuous/contiguous set of possible numeric values of the corresponding numeric scale. A given score range 821 can include one or more possible scores. For example, many possible scores are included in each score range 821 rendering a many-to-one corresponding deterministic mapping implemented as function 820.

Different score ranges can have same or different amounts of included numeric scores that map to the corresponding score-based group. For example, different score ranges are configured differently to include different numbers of possible numeric scores based on collectively constating a predetermined minimum proportion of the possible numeric scores, in accordance with their expected likelihood of occurring (e.g. probability that a given incoming score will fall within the given range), for example, based on the training set or other historical data. As a particular example, all L score ranges include sets configured to include ranges of numeric scores based on scores being expected to fall within each of the L ranges with equal probability, where some or all of the L ranges optionally correspond to different amounts of possible numeric scores (e.g. the full domain of scores ranges from 0 to 1, L is equal to two; score range 821.1 includes scores [0 - 0.8) and score range 821.2 includes scores [0.8-1], for example, where a corresponding range cut-off is automatically configured as 0.8 based on the probability of a participant having a score being between 0 and 0.8 being equal to/approximately equal to 0.5 and based on the probability of a participant having a score being between 0.8 and 1 also being equal to/approximately equal to 0.5 (e.g. based on evaluating outcome data 638 in the training set of other function utilized to generate the scores, or in historical data). Such configuration of score-ranges that are optionally unequal in size based on their relative probabilities of having scores being equal can be useful in achieving approximately equal sized score-based groups 811. In other embodiments, the ranges are automatically configured in accordance with achieving unequal probabilities of having a score fall with such ranges. Alternatively or in addition, the ranges are configured in accordance with fixed intervals (e.g. the full domain of scores ranges from 0 to 1, L is equal to four; score range 821.1 includes scores [0 - 0.25); score range 821.2 includes scores [0.25-0.5); score range 821.3 includes scores [0.5-0.75); and/or score range 821.4 includes scores [0.75-1]; regardless of any respective probability/distribution of scores across these equal-sized ranges.

Figure 6H illustrates an embodiment of a stratification function 820 performed via stratification process 810 that is implemented as a mapping of a set of categorical scores 636.1 - 636.1 to score based-groups 811.1 - 811.L. Some or all features and/or functionality of the stratification function 820 of Figure 6H can implement the stratification function 820 of Figure 6F and/or any other embodiment of stratification function 820 described herein. Some or all features and/or functionality of the trial arm assignment module 730 and/or randomization algorithm 800 of Figure 6H can implement the trial arm assignment module 730 and/or randomization algorithm 800 of Figure 6A and/or 6B, and/or any other embodiment of the trial arm assignment module 730 and/or randomization algorithm described herein.

For example, a given score-based group 811 corresponds to a given categorical score, or a set of multiple categorical scores. For example, different categories are treated separately based on not being similar/ordered in nature. As another example, multiple categories are combined, for example, based on being similar and/or based on grouping categories to render a smaller number of score-based groups L. These groupings can be optionally based on rendering as similar as possible. Alternatively, different probabilities of assignment to different score-based groups is maintained based on ensuring different categories are randomized separately. In some embodiments, L is equal to 2 based on the set of categorical scores corresponding to binary scores.

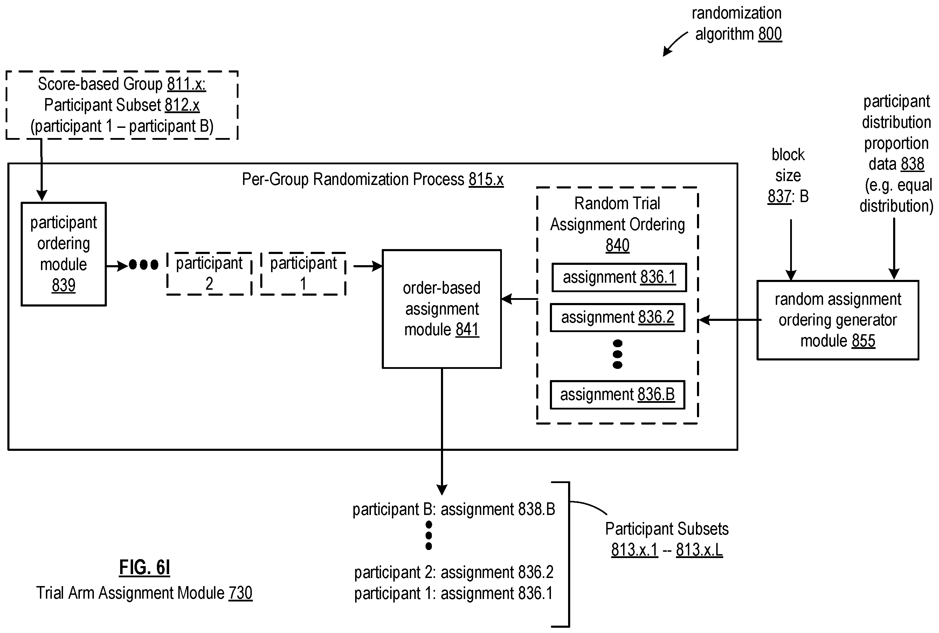

Figure 6I illustrates an embodiment of a per-group randomization process 815.x of a given per-group randomization process 815.x performed upon a given participant subset 812.x of a given score-based group 811.x, for example, having a set of B participants 1 - B. Some or all features and/or functionality of the per-group randomization process 815 of Figure 6I can implement the per-group randomization process 815 of Figure 6F and/or any other embodiment of per-group randomization process 815 described herein. Some or all features and/or functionality of the trial arm assignment module 730 and/or randomization algorithm 800 of Figure 6I can implement the trial arm assignment module 730 and/or randomization algorithm 800 of Figure 6A and/or 6B, and/or any other embodiment of the trial arm assignment module 730 and/or randomization algorithm described herein.

The per-group randomization process 815 can be performed via applying an order-based assignment module 841 to a random trial assignment ordering 840 that is generated via a random assignment ordering generator module 855 for example, implemented via the per-group randomization process 815 itself, or separately (e.g. based on being implemented prior to the trial arm assignment module 730 grouping participants, such as prior to some or all of the B participants being determined for the group based on participants being identifier over time). The random trial assignment ordering 840 can indicate a set of assignments 836.1 - 836.B in a particular order.

The random assignment ordering generator module 855 can generate random trial assignment ordering 840 in accordance with random and/or pseudorandom selection of each assignment, for example, via a random number generator and/or other random/pseudorandom process implemented via a corresponding computing device and/or automated process. The random assignment ordering generator module 855 can be implemented to generate the random assignment ordering generator module 855 to include B assignments as a function of block size 837 indicating the value of B. The random assignment ordering generator module 855 can be implemented to generate the random assignment ordering generator module 855 based on participant distribution proportion data 838. The block size 837 and/or participant distribution proportion data 838 can be predetermined, configured via user input, automatically generated, accessed in memory, received, and/or otherwise determined.

Each assignment 836 of random trial assignment ordering 840 can indicate a particular trial arm 805. A distribution of trial arms across the assignments 836.1 - 836.B can be based on the participant distribution proportion data 838 (e.g. indicating a distribution of participants across trial arms, such as an equal distribution). In the case of equal distribution, the random trial assignment ordering 840 can have a number of assignments to each trial arm equal to and/or relatively close to the quotient B/Y. Other non-equal distributions can be similarly depicted in random trial assignment ordering.

A participant ordering module 839 can order of participants of the score-based group 811. For example, the participants of participant set 812 are ordered based on an ordering of when the corresponding participants were identified, particularly if they are identified over time. As another example, the participants of participant set 812 are ordered based on an ordering of their scores being generated and/or another means of ordering the participants. The participants of participant set 812 can be ordered via any deterministic/random/arbitrary means that is independent of order-based assignment module 841.

As a particular example, the per-group randomization process 815 can be performed upon each participant subset 812 based on implementing random trial assignment ordering 840 in accordance with permuted block randomization technique. For example, the random assignment ordering generator module 855 implements a permuted block randomization technique to generate random trial assignment ordering 840 and/or the permuted block randomization technique is implemented as some or all randomization techniques 684. For example, applying permuted block randomization technique to disperse participants of a given participant subset 812 of a given score-based group 811 (e.g. a "block") can render trial arm assignments within blocks (e.g. within each participant subset 812 of each score-based group 811) being determined so that they are random in order but where desired allocation proportions (e.g. equal sized groups) are achieved exactly within each block.

As a particular example of applying a permuted block randomization technique, consider a 2-group trial with equal allocation and a block size of 6 (e.g. a given participant subset 812 of a given score-based group 811 includes 6 participants). 3 patients in each block are assigned to a first group (e.g. trial arm 805.1, such as a control trial arm) control and 3 to the second group (e.g. e.g. trial arm 805.2, such as an experimental trial arm). However, the ordering of these assignments can be random (e.g. ordering of these assignments are generated via a corresponding random/pseudorandom determined). For example, labeling trial arm 805.1 as '1" and trial arm 805.2 as '2', possible random trial assignment ordering 840 for this example might be: 122121, 212211, and/or 112122, etc.

A particular random trial assignment 840 ordering for a given score-based group 811 of a given trial can be generated via performing a random and/or pseudorandom function of the number of participants Z in a given block and the number of trial groups Y. Different random trial assignment orderings 840 can be generated for different score-based groups 811.1 - 811.L of the given trial. Random orderings having more than two groups (e.g. any number of trials Y) and/or having more than six participants (e.g. any block size B) can similarly be employed.

A random trial assignment ordering 840 utilized for a given score-based group can thus be configured via a corresponding block size 837 of B (e.g. is an ordered list of B assignments). For example, each of the score-based groups has a predetermined block size B utilized to generate the corresponding random ordering. The block size 837 can be the same or different for each score-based group. The block size 837 can be configured to be a multiple of the number of trial arms (e.g. B is a multiple of Y) to render exactly equal dispersal of participants across the trial arms.

As each block is filled (e.g. one B participants are included each score-based group), the trial can be guaranteed to have the desired allocation to each trial arm. In some embodiments, this random ordering can be predetermined via a corresponding random/pseudorandom process, and as each new participant is identified over time, they can be assigned the next trial indicted in the ordering.

This predetermined block size 837 for some or all score-based groups can optionally be determined prior to all participants of participant set being identified, for example, based on participants being identified over time. In some cases, the corresponding score-based group is not considered full/complete until a number of participants B are identified. For example, the corresponding trial is only filled once the required number of participants B is identified for each score-based group. In the case of a deterministic mapping being implemented via stratification function 820, this can optionally require that B participants be identified that have scores 636 and/or 1636 generated that map to the corresponding score-based group.

Some or all parameters dictating how score-based groups are configured in performing randomization algorithm 800 can be the same or different for different clinical trials/scientific studies. Such parameters can include: the number P of participants in participant set 650 that will participate in the trial; the number L of score-based groups 811; how scores 636 and/or 1636 are mapped to each of the L groups (e.g. where the mapping indicated via stratification function 820 is optionally configured differently for different trials); the value B of block size 837 dictating how many participants are assigned to each group 811 in participant subset 812; the number Y of trial arms; and/or other parameters.

Some or all parameters dictating how score-based groups are configured in performing randomization algorithm 800 can be automatically configured for the corresponding trial, for example, by the medical data processing system 500 and/or scientific data processing system 1500 implementing the trial arm assignment module 730. Alternatively, some or all parameters dictating how score-based groups are configured in performing randomization algorithm 800 can be configured via user input for the corresponding trial, can be predetermined for some or all iterations of randomization algorithm 700, and/or can otherwise be determined. These parameters can be configured based on and/or can be otherwise logged in corresponding clinical trial data 566 and/or corresponding scientific study data 1566. These parameters can be configured based on being configured and/or preset parameters of the randomization algorithm, for example, as function definition data of a corresponding trial arm assignment function 672 that implements the randomization algorithm 800.

In such embodiments, some or all parameters dictating how score-based groups are configured can optionally be configured such that there is a reasonable likelihood of each score based group being filled within a reasonable amount of time. For example, the set of scores (e.g. the numeric range/number of categorical scores) that map to a given score-based group are selected based on constituting a sufficient proportion of the population, such as a minimum threshold proportion. For example, proportion that a given score/score range/categorical score constitutes can be determined based on the training set utilized to train the corresponding model utilized to generate the scores 636 and/or 1636\, and/or other historical data, and/or the proportion that a given set of scores/set of ranges/set of categories constitutes can correspond to a sum of the corresponding individual proportions. The minimum threshold proportion can be predetermined, configured via user input, accessed via memory, received, automatically generated, and/or otherwise determined. The minimum threshold proportion can be different for different trials (e.g. based on the necessity to conduct the trial quickly).

The minimum threshold proportion can thus dictate how the corresponding stratification function 820 is configured. For example, the number of score-based groups L and/or the mapping of scores to each of the L groups is automatically generated via the medical data processing system 500 and/or the scientific data processing system 1500 accordingly. The particular configuration of the stratification function 820 (e.g. the number of score-based groups L; the mapping of scores to each score-based group; etc.) can be stored in function library 562 and/or identified/logged/configured in participant assignment method data 682.

As a particular example, means of maximizing effective application of the minimum threshold proportion and/or otherwise maximizing likelihood of filling all of the score-based groups with the required number of participants more quickly can include generating the mapping such that each group proportion is equal to the minimum threshold proportion. For example, if the minimum threshold proportion is 0.2, L is set as 5, where each group is mapped to scores collectively constituting 20% (or as close to 20% as possible) of all outcomes (e.g. based on historical data/the training set that trained the corresponding model). In the case where outcome is a numeric score on an ordered numeric scale, the first score range 821.1 can be configured to constitute the lowest set of contiguous scores on the ordered numeric scale collectively having proportions comprising 20% of outcomes; the second score range 821.2 can be configured to constitute the second-lowest set of contiguous scores on the ordered numeric scale collectively having proportions comprising 20% of outcomes; etc.).

As another example of configuring score-based groups such that there is a reasonable likelihood of each score based group being filled within a reasonable amount of time, in some embodiments, rather than retroactively identifying how score-based ranges/groups of different score values be mapped to the L score-based groups to render equal probabilities, the corresponding medical outcome prognostication function/outcome prediction function utilized to generate the scores 636 and/or 1636 respectively can be trained/otherwise configured to output one of a set of L labels that render assignment to one of the L score-based groups. For example, the training data utilized to train the respective model is evaluated to identify L groupings of the respective outcomes data 638 (e.g. L ranges of numerical values; L categories; etc.), for example, based on identifying corresponding ranges/groupings rendering equal proportions (e.g. the K training data in the training set has outcome data corresponding to more than L different values, and the K training data is divided into L groups of equal/relatively equal size (and/or other relatively proportions based on the corresponding L values of B if non-equal) based on grouping same/similar outcome data 638 together, such as L contiguous numeric ranges of a full scale of values of outcome data 638). For each of these identified groups of training data, the corresponding outcome data 638 can be mapped to a respective one of L labels, where these labels are utilized as the value 704 of the output feature 706 in the training data, rather than the original outcome data 638 mapping to this value. Thus, when the respective trained function trained in this fashion is applied to the participant data 712 for participants of the trial, a corresponding one of the L output labels can be generated as the score 636 and/or 1636 as function output, which is utilized to assign the participant to the corresponding one of the L groups (e.g. all participants with the same score 636 and/or 1636 are grouped together), ideally rendering participant being assigned any given group with equal probability and/or thus rendering score-based group being more likely to be filled around the same time.

As another example of configuring score-based groups such that there is a reasonable likelihood of each score based group being filled within a reasonable amount of time, the value of L is configured to be smaller (e.g. as small as possible such that participants with similar scores 636 are still grouped together and such that participants with different scores are still grouped separately). In particular, if fewer score-based groups 811.1 - 811.L are implemented, the groups can have incoming patients assigned with higher probability (e.g. particularly if and be expected to be filled faster). For example, implementing two groups (e.g. based on scores 811 corresponding to binary values or otherwise being mapped into two groups) can be preferred in filling up score-based groups more quickly than cases where L is larger. However, fewer score-based groups are implemented, this can lead to less corresponding stratification of patients by predicted outcome, which can potentially render less balanced trial arms. A trade-off can be automatically applied to configure a best value of L (for example, based on how quickly the trial need be completed, how many qualifying participants are expected, how different the predicted outcomes are (e.g. historically) and/or how many different disparate groups naturally appear in the historical data and/or training data (e.g. based on distribution of outcome data 638, such as natural peaks and/or categorical differences in values for outcome data 638), and/or other factors. The dictated number of groups L can optionally dictate configuration of the corresponding function (e.g. where exactly L labels are implemented as values 704 as discussed previously).

As another example of configuring score-based groups such that there is a reasonable likelihood of each score based group being filled within a reasonable amount of time, the value of B can be configured for each score-based group 811 based on an expected proportion of participants that will have scores 636 and/or 1636 that map to the corresponding score-based group (e.g. based on the training data and/or other historical data). For example, the expected proportions of participants that will have scores mapping to different score based groups is unequal (e.g. the score range 821/one or more categorical scores mapping to a first score-based group is expected to constitute 75% of participants, while the score range 821/one or more categorical scores mapping to a second score-based group is expected to constitute the remaining 25% of participants, where the value of B for the first is configured to be three times as large as the value of B for the first group (e.g. the first score-based group is full when 75 participants are identified, while the second score-based group is full when 25 participants are identified). This can help ensure that score-based groups having different probabilities an incoming participants being assigned are expected to become full around the same time based on adapting configuration of B for the different groups accordingly.

In some embodiments where B is predetermined for the score-based groups 811 (e.g. is fixed as the same value for all score-based groups or is different for different score based groups), the value of P can be configured accordingly, where these predetermined values of B dictate how many participants will participate in the trial based on exactly how many participants are required to fill each of the corresponding L blocks. For example, the L different values of B are summed to determine the value of P (and/or the value of P is B x L in the case where B is equal across all L groups).

As different score-based groups may fill at different times as a function of whether B participants having a score 636 and/or 1636 mapping to the corresponding score-based group have been identified from the incoming prospective participants, the filling of a given score-based group can be utilized to determine whether a prospective participant will participate in a trial. For example, a plurality of prospective participants are identified over time and are mapped to score-based groups 811 via stratification function 820 in accordance with the score 636 and/or 1636 generated from their respective participant data 712. If the given score-based group 811 a given prospective participant is mapped to is not yet full (e.g. less than B participants have been identified for this group so far), the prospective participant can be included in this given score-based group 811 accordingly as a confirmed participant of the trial that will be assigned to a trial arm based on their respective assignment 836 via per-group randomization process 815. If the given score-based group 811 a given participant is mapped to is already full (e.g. all B participants have been identified for this group so far), the prospective participant is not included in this group as it is already full, and is optionally turned away/not selected for participation in the trial based on their group being full and not having score 636 and/or 1636 that would map to other score-based groups that may still be open.

In other embodiments, rather than guaranteeing that all trial arms have exactly equal numbers of participants via identification and assignment of exactly B participants via random trial assignment ordering 840, the random trial assignment ordering can include an arbitrary number of assignments that is optionally larger than the number of patients that are ultimately assigned to a given group and/or does not necessarily need B assignments to render completion of the trial. For example, a corresponding set of less than B participants being assigned via a random trial assignment ordering of B assignments 836 generated in accordance with participant distribution proportion data 838 may render distribution of participants across trial arms not exactly evenly (e.g. if random trial assignment ordering for a block B equal to 6 has the assignments 111222 for a trial of two trial arms and only 4 patients are identified for the corresponding block, an unequal distribution is rendered where 3 participants are assigned to trial arm 1 and only one participant is assigned to trial arm 2). In cases where B is larger and/or the number of assigned participants is closer to B, this disparity can be smaller, and can optionally be acceptable for the sake of completing the trial in a more timely fashion. In some cases, this trade-off can be evaluated to configure a corresponding threshold minimum relative to B (e.g. automatically and/or via user input) that must be met to render a sufficiently even distribution (e.g. as a function of the size of B and/or the number of trials Y; for example, the minimum threshold requires that 0.9*B patients be identified for the score-based group, and up to B patients be identified for the score-based group).

Figure 6J illustrates an embodiment of a trial arm assignment module where stratification process 810 includes outcome prediction-based stratification 822 and further includes additional factor-based stratification 823. Some or all features and/or functionality of the outcome prediction-based stratification 822 and/or the additional factor-based stratification 823 of Figure 6J can implement the stratification function 820 of Figure 6F and/or any other embodiment of stratification function 820 described herein. Some or all features and/or functionality of the trial arm assignment module 730 and/or randomization algorithm 800 of Figure 6J can implement the trial arm assignment module 730 and/or randomization algorithm 800 of Figure 6A and/or 6B, and/or any other embodiment of the trial arm assignment module 730 and/or randomization algorithm described herein.

In some embodiments, participants are further stratified via stratification process 810 via one or more additional factors in addition to being stratified by score 636. For example, participants are further stratified by factors such as age, sex, etc. Additional factor-based stratification 823 can be performed in addition to outcome-prediction based stratification by score (e.g. via stratification function 820 via some or all features and/or functionality discussed previously).

Categorizing of patients by additional stratification factors can be indicated via corresponding additional factor values 826 (e.g. for a given participant, one factor value 826 for each additional factor being stratified). The additional stratification factors can be indicated/configured/logged via stratification factor data 683 for the given trial. The corresponding additional factor values 826 can correspond to values for any corresponding stratification factor, such as any types of information/corresponding valuers of the medical data metadata 610 and/or scientific data metadata 1610 described herein (e.g. any medical data capture data 611, etc.); any types of information/corresponding valuers of the other relevant data 640 of patient data 564 and/or participant data 1654 described herein (e.g. any demographic data 641; medical history data 642; family history data 643; risk factor data 644; etc.); and/or other data further categorizing participants beyond their score 636 and/or 1636 alone.

In particular, stratification by score only via predicted outcome-based stratification 822 can render stratification into L1 score-based groups 811.1 - 811.L1, for example, based dictated by L1 different groupings of possible scores 636 and/or 1636 as discussed previously. These score-based groups can be further stratified via one or more additional factors, as a function (e.g. deterministic mapping) of corresponding additional factor values for each factor determined for the participants (e.g. their age, their gender), for example, via performing additional factor-based stratification 823. Each given score-based group 811 generated via outcome prediction-based stratification 822 can be further stratified into a set of L2 groups (e.g. L2 is the number of additional categories collectively rendered by the combination of categories of each additional factor. For example, if binary identification of sex (e.g. male or female) is implemented as the only additional factor, L2 is equal to 2. As another example, if binary identification of sex is implemented as one additional factor and whether or not the participant age is: less than 30 years old; between 30 and 60 years old; or over 60 years old is the second additional factor, L2 is equal to six. Thus, the total number of score-based groups 811 generated via stratification process (e.g. where each score-based groups 811 is further based on at least one additional factor) can include L1 x L2 groups (e.g. = L1 * L2). Thus L1 x L2 per-group randomization processes 815 can each be performed accordingly on a given corresponding score-based group (e.g. in a same or similar fashion as described in conjunction with Figure 6I).

Note that such stratification by multiple factors that include stratification by score and stratification by at least one additional factor can be applied in any order - the stratification of participants first by score and second by other factors as illustrated in Figure 6J is presented for illustrative purposes of factors by which groups are stratified, and corresponding stratification by multiple factors can be achieved in any order, and/or collectively in one step. For example, the stratification function 820 of Figure 6F optionally implements both the outcome prediction-based stratification 822 and the additional factor based stratification as a deterministic mapping of a multiple values as input (e.g. input for a given participant indicates their score 636 and/or 1636, as well as one or more additional values 826 corresponding to the additional factors, where each given combination of score and the additional one or more values 826 is mapped deterministically to a given one of the L score-based groups 811).

In other embodiments, the score 636 and/or 1636 is the only stratification factor, where the L groups are based on L categories of score only. This can be ideal in reducing the number of score-based groups L, which can be helpful in filling groups more quickly (e.g. with their B participants) based on criteria for being mapped to a given group being easier to meet (e.g. participants only need a score mapping to a given group, rather than a group requiring patients having a particular score/score falling within a particular range, as well as requiring these patients meet additional criteria such as being of a particular age or having other demographic characteristics. Furthermore, corresponding factors by which an entity conducting a trial may wish to stratify can be utilized as input values 703 to the corresponding medical outcome prognostication function 671/outcome prediction function 1671 as discussed previously, and can thus be "baked-into" the corresponding scores 636 and/or 1636 (e.g. influencing the respective score based on their respective correlation/relevance in the corresponding outcome that the score predicts, as tuned via training of the corresponding model based on how/whether such correlations are present in the training data 702 utilized to train the function). Thus, stratification by score 636 and/or 1636 in cases where the scores were generated as a function of these other factors (e.g. where patient age and/or patient sex is input to the corresponding function 671 and/or 1671) can thus implicitly render a form of corresponding stratification by these other factors, for example, as relevant to indeed being predictors of the corresponding primary outcome, as reflected in the corresponding trained model. Such stratification by such scores 636 and/or 1636 as the sole stratification factor can thus improve the technology of clinical trials/scientific studies, and improve scientific advancement/effectiveness of products being tested in the respective technical/scientific fields of study, based on enabling lower values of L, making trials easier to fill, while still preserving corresponding stratification of multiple relevant factors inherently based on their influence in the corresponding single score by which participants are stratified.

Figure 6K illustrates an embodiment of a trial arm assignment module 730 that generates participant sets 803.1 - 803.Y via collective processing 731 of scores 636.1 - 636.P (and/or scores 1636.1 - 1636.P). For example, this collective processing includes applying and/or optimizing target participant set parameters 833. Such embodiments that apply collective processing 731 as illustrated in Figure 6K optionally requires that all scores 636.1 - 636.P (and/or 1636.1 - 1636.P) be generated first (e.g. all prospective participant are identified), where these participants are grouped based on collectively processing the set of P scores in tandem (e.g. in accordance with applying an optimization algorithm and/or otherwise collectively processing the scores to render participant sets 803.1 - 803.Y that meet target participant set parameters 833 and/or optimize target participant set parameters 833 (e.g. meet size criteria 834 and/or score criteria 835, and/or optimizing participant sizes and/or score distribution by performing a corresponding optimization algorithm, for example, to minimize difference in participant set sizes and/or minimize difference in score distribution characteristics of across participant sets. Some or all features and/or functionality of the trial arm assignment module 730 and/or randomization algorithm 800 of Figure 6K can implement the trial arm assignment module 730 and/or randomization algorithm 800 of Figure 6C, of Figure and/or 6B, and/or any other embodiment of the trial arm assignment module 730 and/or randomization algorithm described herein.

In some embodiments of performing the collective processing 731 to generate participant sets 803.1 - 803.C, the number of participant in some or all participant sets 803 is configured/guaranteed to be equal to, close to, and/or within a predefined threshold of the quotient P/Y for some or all trials/studies (e.g. the number of participants P is optionally configured to be a multiple of Y to guarantee equal numbers of participants in all trial arms, and/or the trial arms can be configured to have as equal of a number of participants as possible in cases P is not a multiple of Y, such as all being within one participant of each other in size). Alternatively, greater variation in size is rendered, for example, based on adhering to/accounting for other criteria indicated in target participant set parameters 833 such as score criteria 835, and/or based on characteristics of the randomization algorithm. As a particular example, this greater variation in size is rendered (e.g. while still being within a predetermined threshold variation) based on achieving more even score-based distribution of patients (e.g. the set of participant sets 803. 1 - 803.Y have more similar distributions of scores, such as more similar average scores, than they could if more equal group sizes were attained).

In some embodiments of performing the collective processing 731 to generate participant sets 803.1 - 803.C, the trial arms can be configured via as equal of dispersal of scores across trial arms as possible (e.g. to achieve the closest score averages possible/most similar score distribution possible, given the set of scores 636.1 - 636.P and/or 1636.1 - 1636.P). Alternatively, greater variation in score is rendered, for example, based on adhering to/accounting for other criteria indicated in target participant set parameters 833 such as size criteria 834, and/or based on characteristics of the randomization algorithm. As a particular example, this greater variation in score average/distribution characteristics across trial arms is rendered (e.g. while still being within a predetermined threshold variation) based on achieving more evenly-sized participant sets 803.1 - 803.Y (e.g. the set of participant sets 803. 1 - 803.Y have more similar sizes, such as equal sizes or similar equal sizes, than they could if more equal score averages/distribution characteristics across trial arms were attained).

In some embodiments of performing the collective processing 731 to generate participant sets 803.1 - 803.C, minimizing size differences across participant sets vs. minimizing difference between score average/distribution characteristics across participant sets is in accordance with a predetermined trade-off factor (e.g. one or more weighing factors weighing the importance of minimizing size differences across participant sets vs. minimizing difference between score average/distribution characteristics across participant sets). For example, this trade-off factor is predetermined, configured via user input, is automatically generated, and/or is otherwise determined. In some embodiments, performing the collective processing 731 to generate participant sets 803.1 - 803.C can be in conjunction with performing a corresponding optimization in conjunction with ha corresponding optimization algorithm (e.g. assigning participants to groups to minimize and/or maximize a particular factor that is a function of size differences across participant sets and/or difference between score average/distribution characteristics across participant sets, thus rendering minimizing each as much as possible collectively to render the minimization and/or maximization of the particular factor, for example, that inherently applies such a trade-off factor and/or corresponding weighing factors.

Consider the following particular example of randomizing patients of a clinical trial, for example, in accordance with some or all features and/or functionality of medical data processing system 500 described herein in conjunction with some or all of Figures 1A - 6K, and/or in conjunction with applying some or all features and/or functionality of the example embodiments of Figures 7A - 9R:

As an example implementing randomizing of patients of clinical trials and/or any other functionality of medical data processing system 500 described herein, automated imaging-based randomization in performed in stage I-III non-small cell lung cancer using deep learning. For example, deep learning techniques can applied to pre-treatment computed tomography imaging can generate accurate imaging-based prognostication (IPRO) scores for patients diagnosed with stage I-III non-small cell lung cancer (NSCLC), (e.g. via a corresponding medical outcome prognostication function 671, for example, that implements some or all examples of model 112 described in conjunction with some or all of Figures 7A - 9R). In this study, we explore how IPRO could be used to enhance randomization in a phase 3 NSCLC trial by increasing statistical power and complementing/replacing traditional stratification factors.

Continuing with this particular example of implementing randomizing of patients of clinical trials and/or any other functionality of medical data processing system 500 described herein, a number of patients to be randomized into a phase 3 trial includes 450 patients (e.g. the value of P of participant set 650 = 150), and/or another valuer of P can be applied.

Continuing with this particular example of implementing randomizing of patients of clinical trials and/or any other functionality of medical data processing system 500 described herein, A set of inclusion criteria can be applied, for example, to identify clinical trials meeting particular criteria for testing of the corresponding model (e.g. if a historical trial) and/or for application of the model (e.g. if an upcoming/current trial). For example, historical target enrollment of historic clinical trials can be evaluated.

Continuing with this particular example of implementing randomizing of patients of clinical trials and/or any other functionality of medical data processing system 500 described herein, such clinical trials meeting inclusion criteria can be identified based on running a corresponding query on a clinical trials dataset and/or corresponding server system (e.g. clinicaltrials.gov). As a particular example of the inclusion criteria, the inclusion criteria optionally includes some or all of: NSCLC; Stage I-III; Interventional; randomized/parallel assignment; Phase 2 or 3; start date between 01-Jan-2000 & 01-May-2021 (or other date range) and/or other inclusion criteria. In some embodiments, the phase 1/2 trials are reclassified as phase 2 trials, and/or phase 2/3 trials are reclassified as phase 3 trials. Alternatively or in addition, trials are excluded based on corresponding exclusion criteria. As a particular example of the exclusion criteria, trials are excluded based on meeting exclusion criteria corresponding to trials that are: Disease "stage IV," "stage 4," or "advanced"; "Non-randomized" or "single group assignment"; smoking cessation or lifestyle intervention trials; Phase 1; and/or Phase 4.

Continuing with this particular example of implementing randomizing of patients of clinical trials and/or any other functionality of medical data processing system 500 described herein, from the identified set of trials, trials can be further filtered based on their primary endpoint, for example, based on the primary endpoint which the corresponding medical outcome prognostication function 671 is trained to identify. As a particular example, trials are filtered with "overall survival" as the primary endpoint, for example, based on applying a corresponding medical outcome prognostication function 671 operable to predict overall survival in corresponding medical outcome prognostication scores 636 (e.g. via implementing some or all functionality of model 112 described in conjunction with some or all of Figures 7A - 9R).

Continuing with this particular example of implementing randomizing of patients of clinical trials and/or any other functionality of medical data processing system 500 described herein, in some embodiments, instead of or in addition to evaluating historical target enrollment, an expected treatment effect can be automatically determined, which can be automatically utilized to that to estimate target enrollment.

Continuing with this particular example of implementing randomizing of patients of clinical trials and/or any other functionality of medical data processing system 500 described herein, patients can be identified for inclusion in a training set 701 and/or validation dataset (e.g. in conjunction with validating a corresponding medical outcome prognostication function 671, for example, as part of the training process utilized to generate the corresponding medical outcome prognostication function 671 via implementing medical outcome prognostication model training system 506. As a particular example of identifying patients for inclusion in a training set 701 and/or validation dataset, using the cancer registry from Princess Margaret Cancer Centre (or other cancer registry), a plurality of stage I-III patients (e.g. 1,516 patients or other number of patients meeting corresponding criteria) can be identified. For example, patients are identified that were: treated between particular date range (e.g. January 1, 2004 and June 30, 2021); had received a pretreatment CT scan ≤90 days prior to first-line treatment start (and/or had other medical data 562 collected within 90 days or another data range from treatment start); have a known 5-year survival outcome (or other survival outcome/other type of medical outcome applied as outcome data 638); and/or other criteria. In some embodiments, using these patients' medical record numbers (e.g. implemented as patient identifiers and/or corresponding medical data identifiers) the institution's imaging archive can be queried to identify studies meeting the inclusion criteria (e.g. a total of 15,699 pre-treatment and follow-up CT studies, or other number of pre-treatment and follow-up CT studies, utilized to train and/or validate a corresponding model, such as model 112 and/or a corresponding medical outcome prognostication function 671, for example, configured to predict survival outcome as medical outcome type 637 of the corresponding medical outcome prognosis scores 636).

Continuing with this particular example of implementing randomizing of patients of clinical trials and/or any other functionality of medical data processing system 500 described herein, in some embodiments, a set of standard clinical trial eligibility criteria is automatically applied to narrow down the training set and/or validation set to those patients that would have qualified for a hypothetical trial. For example, 450 patients (or other number of patients) to be included in the trial are randomly selected based on meeting this set of clinical trial eligibility criteria (e.g. where these patients are excluded from the training dataset based on being utilized for validation). In some embodiments, randomization simulation using individual stratification factors and/or combinations of stratification factors is performed upon this set of 450 patients (e.g. stratifying by predicated survival outcome generating corresponding medical outcome prognosis scores 636 predicting survival outcome accordingly, and/or based on stratifying by one or more factors such as: Histology; PD-L1 Expression; Sex / Gender; Smoking Status; Prior Therapy; TNM Stage; Performance Status; Age; and/or Race / Ethnicity.

Continuing with this particular example of implementing randomizing of patients of clinical trials and/or any other functionality of medical data processing system 500 described herein, the impact of various stratification strategies can be evaluated, for example, to automatically determine which means by which to stratify patients. This evaluation can be utilized to configure some or all corresponding functionality of the trial arm assignment module 730 and/or corresponding randomization algorithm 800, such as configuring of which stratification factors 683 are applied, for example, to implement corresponding additional factor values 826 based on their evaluated effectiveness, and/or by determining whether score 636 is effective in stratifying patients (e.g. where a corresponding model is retrained accordingly based on determining to generate scores differently/stratify by corresponding predicted medical outcome differently. In some embodiments, such evaluation includes evaluating statistical power, required trial size, and/or ability to detect treatment effect sooner / more accurately (e.g. assuming enrollment of 450 and/or assuming other number of participants).

Figure 7A illustrates an example embodiment of a pipeline implemented via medical outcome prognostication system 507. Medical data 562, such as medical image data 910 or another type of medical data 562 described herein, can undergo medical data pre-processing 915 to render pre-processed medical data 911, such as pre-processed medical image data 912 in the case where medical data 562 is medical image data 910. A trained model 914 can be applied to this pre-processed medical data 911 to render generation of medical outcome prognosis score 636 (e.g. trained model 914 is defined via model parameter data 661 of a corresponding medical outcome prognostication function 671). Some or all features and/or functionality of the process illustrated Figure 7A can implement the medical outcome prognostication system 507 of Figure 4A, 4E, and/or any other embodiment of medical outcome prognostication system 507 described herein.

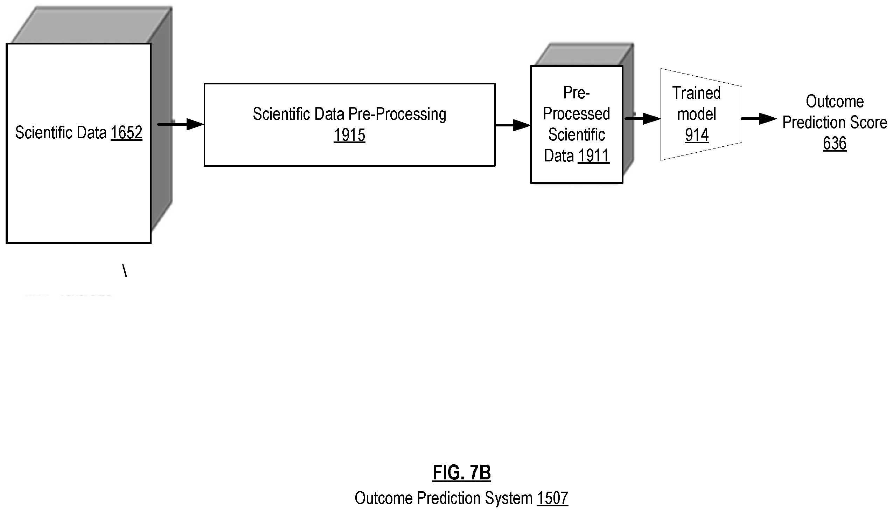

Figure 7B illustrates an example embodiment of a pipeline implemented via scientific outcome prognostication system 1507. Scientific data 562 can undergo medical data pre-processing 915 to render corresponding pre-processed scientific data 1911. A trained model 914 can be applied to this pre-processed medical data 911 to render generation of scientific outcome prognosis score 1636 (e.g. trained model 914 is defined via model parameter data 661 of a corresponding outcome prediction function 1671). Some or all features and/or functionality of the process illustrated Figure 7B can implement the outcome prediction system 1507 of Figure 5A, 5D, and/or any other embodiment of outcome prediction system 1507 described herein. Some or all features and/or functionality of the process illustrated Figure 7B can implement the medical outcome prediction system 507 of Figure 7A, where the pipeline of Figure 7B is adapted for use in the medical field, (e.g. where scientific data 564 of Figure 7B is implemented as medical image data 912 or other medical data 562), alternatively or in addition for implementation in non-medical scientific fields. Some or all features and/or functionality of the process illustrated Figure 7B can implement any embodiment of medical outcome prediction system 507 described herein.

Figure 8A is a block diagram illustrating an example, non-limiting embodiment of image-based modeling that can function or otherwise be performed by, and/or based on communicating with, medical scan processing system 500, and/or can function or otherwise be performed within the system of Figure 1B in accordance with various aspects described herein. Some or all features and/or functionality of the imaging-based prognostication (IPRO) framework 200A for image-based modeling illustrated in Figure 8A can implement the pipeline implemented by medical outcome prognostication system 507 as illustrated in Figure 7A and/or can implement any embodiment of medical outcome prognostication system 507 described herein. Some or all features and/or functionality of the pipeline for image-based modeling illustrated in Figure 8A can implement the pipeline implemented by outcome prediction system 1507 of Figure 7B and/or can implement any embodiment of outcome prediction system 1507 described herein. In particular, the imaging-based prognostication (IPRO) framework 200A can be considered a particular example embodiment of the pipeline implemented by medical outcome prognostication system 507 as illustrated in Figure 7A.

The imaging-based prognostication (IPRO) framework 200A can process 3D CT volumes 202A, such as resampling them to a fixed voxel size. For example, some or all medical data 562 described herein is implemented as 3D CT volumes 202A, where the medical image data 910 of Figure 7A is implemented as 3D CT volumes 202A. Segmentation 204A can be performed and then a localizer 206A (e.g. a thorax localizer) and a 3DCNN 208A can extract imaging features automatically along the axial, sagittal and coronal directions, such as simultaneously. As an example, the localizer 206A can limit the model input to a 3D space (of a selected size) centered on the organ of interest (e.g., lungs), thus excluding features outside of a particular region or volume (e.g., excluding features outside of the thorax, such as the abdomen, and outside of the skin, such as the CT scanner table). The automatically identified thorax region can then be fed into a three-dimensional convolutional neural network (3DCNN) which outputs at least one mortality risk score, such as one or more probability scores, such as between 0 and 1, indicating mortality at different time intervals (e.g., 1-year, 2-year, and 5-year) for a given CT scan. For example, segmentation 204A and/or localizer 206A can implement the medical data pre-processing 915 of Figure 7A. Alternatively or in addition, the 3DCNN can implement the trained model 914 of Figure 7A. Alternatively or in addition, the mortality risk score and/or probability scores can implement the medical outcome prognostication score 636.

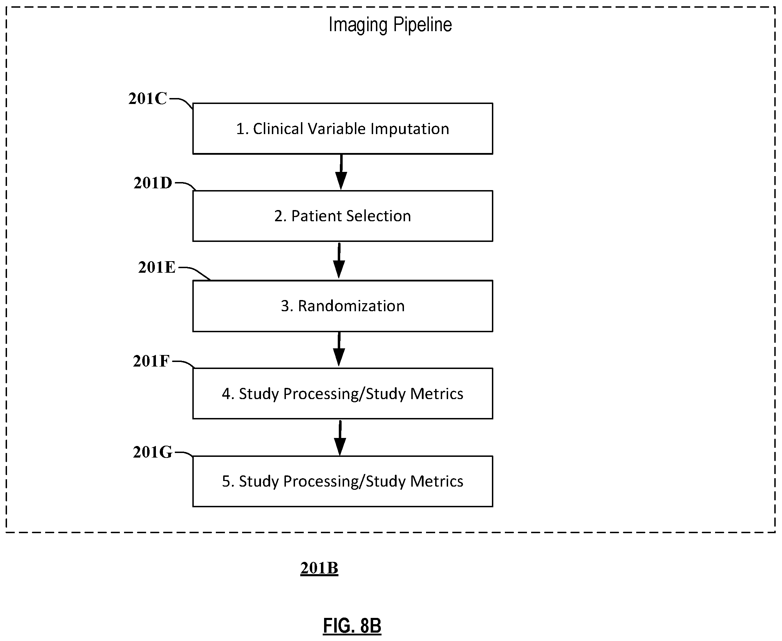

Some or all features and/or functionality of 3DCNN of Figure 8A and/or as described in conjunction with the examples of Figures 8B - 9R can implement any embodiment of medical outcome prognostication function 671 described herein (e.g. the 3DCNN is defined via corresponding model parameter data 661, for example, generated via model training performed via medical outcome prognostication model training system 506). Such a 3DCNN can be considered a particular example of a model applied via medical outcome prognostication function.

Some or all features and/or functionality of these probability scores indicating mortality at different time intervals (e.g., 1-year, 2-year, and 5-year) for a given CT scan outputted by the 3DCNN of Figure 8A and/or as described in conjunction with the examples of Figures 8B - 9R can implement any embodiment of medical outcome prognostication scores 636 described herein (e.g. can correspond to an example type of medical outcome prognostication scores 636 in the case where medical outcome type 637 corresponds to survival outcome/morality, for example, such as a probability as to whether or not the corresponding patient is to die within 1 year, 2 years, and/or 5 years). Such probability scores can be considered a particular example embodiment of medical outcome prognostication scores 636.

Some or all features and/or functionality of the IPRO framework 200A of Figure 8A and/or as described in conjunction with the examples of Figures 8B - 9R can implement any embodiment of medical outcome prognostication system 507 and/or outcome prediction system 1507 described herein, and/or can implement any other functionality performed via medical data processing system 500 and/or scientific data processing system 1500.

While the example illustrated for IPRO framework 200A processes 3D CT volumes 202A to obtain a predicted variable of a mortality risk score, in one or more embodiments the IPRO framework can also be based on 2D images (alone or in combination with 3D images) and the images can be of various types including X-ray, MRI, Ultrasound, Nuclear medicine imaging, and so forth.

The IPRO framework 200A can also provide other predicted variables (in combination with the mortality risk score or in place of it), including one or more of age, sex, weight, ECOG status, smoking status, co-morbidities, cardiac and pulmonary toxicity, TNM stage, pulmonary function, and so forth based solely on the image analysis (or in conjunction with other ingested data).

The IPRO framework 200A can be applied prospectively and/or retrospectively. For instance, predicted variables can be generated based on images ingested by a trained model for individuals where treatment in the clinical trial has not yet started or where the treatment in the clinical trial is finished (which can include clinical trials that have been completed but are being re-evaluated as to their efficacy or for planning related trials). Similarly, independent of any clinical trial, predicted variables can be generated based on images ingested by a trained model for individuals where treatment has not yet started or where the treatment has finished. In one embodiment, the modeling and analysis to generate predictive variables can be commenced during treatment where pre-treatment image(s) are available for the individual(s) and an image-based model has been trained for the organ, disease and/or treatment as described herein. For example, this can be helpful to a physician and patient in determining whether an on-going treatment should be adjusted or changed (e.g., adjusting dosage, changing treatment type, and so forth). While some of the embodiments herein describe detection and prognostication with respect to cancer and tumors, in one or more embodiment, the IPRO framework 200A can be applied to any disease, condition or medical characteristic that allows for image-based detection or evaluation. It should further be understood that the timing of application of the system and methodology can vary and can include being applied after a clinical trial(s) is over, during the clinical trial(s), and/or before the clinical trial(s) has commenced. For example, a clinical trial may have concluded and the manager of the clinical trial desires to retrospectively analyze the clinical trial. In this example, the imaging model and other functions described herein can be applied to various images that were captured at various time periods, such as pre-treatment, on-treatment and/or post-treatment images. In one or more embodiments, the imaging model and other functions described herein can be applied to some or all of the pre-treatment, on-treatment and post-treatment images, to provide an analysis of clinical trial(s), which may have already begun or may have already finished. In one or more embodiments of a retrospective analysis, the same imaging model and same functions can be applied to all (or some) of the pre-treatment, on-treatment and post-treatment images, to provide an analysis of a clinical trial(s), which has already finished.

In one or more embodiments, the IPRO framework 200A can utilize various deep learning techniques and algorithms that can analyze images. For example, different algorithms can be utilized by different models, such as based on the selected algorithm being determined to be more accurate in generating predicted variables for a particular body part or organ. In another embodiment, different algorithms can be utilized by different models being applied to the same body part or organ and the results (e.g., predicted variables at different time intervals) can be compared, such as to confirm accuracy. In yet another embodiment, different algorithms can be utilized by different models being applied to the same body part or organ, where the predicted variables at particular time intervals are selectively taken from the different models, such as based on model A being known to be more accurate at earlier time intervals and model B being known to be more accurate at later time intervals. As another example, a convolutional neural network can be utilized where the images are 2D (e.g., X-ray) while a 3DCNN can be utilized for 3D images (e.g., CT scans). In one embodiment, the best model(s) can be selected and applied according to the particular circumstances, such as the type of images, type of disease, and/or other factors that can influence model efficiency and/or accuracy. In one or more embodiments, future machine learning models that are developed, including future imaging models, can be implemented by the systems and methodologies described herein.

In one or more embodiments, the selection of a particular modeling algorithm for the IPRO framework 200A can be based on performance evaluation. For example, various algorithms can be selected and can be implemented iteratively to determine best performance, most accurate, most efficient or other performance criteria. As an example, different numbers of layers and settings can be implemented for one or different algorithms to avoid overfitting (e.g., the inclusion of dropout layers, batch normalization, and so forth) and evaluate the algorithm performance.

Clinical TNM staging can be a key prognostic factor for cancer patients (e.g., lung cancer) and can be used to inform treatment and/or monitoring. Imaging, such as radiological imaging (e.g., computed tomography), can play a central role in defining the stage of disease. As an example, deep learning applied to pretreatment CTs can offer additional, individualized prognostic information to facilitate more precise mortality risk prediction and stratification.

In one or more embodiments, the selection of the volume size for the IPRO framework 200A can be performed in a number of different ways, such as being predetermined by the algorithm and remaining the same for the organ being analyzed via determining organ sizes (from automated segmentations) from multiple datasets and selecting a size that fitted the largest organs.

In one or more embodiments, the IPRO framework 200A can perform pre-processing for images including gathering organ segmentation and extracting an organ box of a particular size (e.g., 360x360x360mm for the lungs); and/or rescaling the image such that images can be fitted into GPU(s) while retaining as much information as possible. In one or more embodiments utilizing the 3DCNN (which can be other types of machine learning models in other embodiments), a balance between the size of the image and a higher resolution for the image (which can give better performance but can make the model more prone to overfitting) can be determined and maintained. In one or more embodiments, image normalization is implemented to prevent the model from overfitting and can be determined by assessing the training loss/accuracy trend over multiple training iterations (i.e., epochs). In one or more embodiments, clipping (Hounsfield Unit) HU values between -1000 and 1000 (e.g., for thorax images) can be utilized where a range of HU values can improve performance.

In one or more embodiments, the IPRO framework 200A can analyze and reduce bias introduced into the process. For example in one embodiment, the input image(s) can be modified to remove pixels which suggest such bias (e.g., based on scanner used, hospital where acquired, and so forth).

In one or more embodiments, the IPRO framework 200A can capture and analyze multiple organs including a primary organ (e.g., exhibiting a tumor) and a secondary organ (which may or may not be exhibiting a tumor). As an example, the IPRO framework 200A may utilize multiple "arms" in the 3DCNN to learn features from various body parts. This can also include developing segmentation models to extract a 3D box encompassing the particular organ(s).

In one or more embodiments, the IPRO framework 200A can perform post-processing techniques. For example, heatmaps or activation or attention maps can be generated (e.g., utilizing GradCAM or other back propagation techniques and tools) which indicate where greatest attention is placed by the model in a particular image, and which can indicate which parts of the image were of particular importance for predicting the particular variable(s), such as survival. As an example, GradCAM activation maps were generated that indicated that an IPRO applied to the thorax learned to place outsized attention on primary lesions, where on average 54% more attention was placed on primary lesions (0.2458) compared to the average attention throughout the thorax (0.15920), which was statistically significant (p < 0.001).

In one or more embodiments, an end-to-end fully-automated framework of imaging-based prognostication can ingest images (e.g., CTs) of varying sources and imaging protocols, and can automatically analyze a 3D region encompassing one or more organs and/or their surrounding area, such as the thorax. In one or more embodiments, the IPRO can predict mortality at various time intervals (e.g., 1-year, 2-year, and/or 5-year). In one or more embodiments, the IPRO can predict other variables from the ingested image(s). In one or more embodiments, the IPRO can predict the size and/or shape of tumor(s) in the future at different time intervals. In one or more embodiments, the IPRO can perform its predictions based only on applying the trained model to the particular image, without the need for other medical/user data associated with the patient corresponding to the image. In one or more embodiments, the IPRO can perform its predictions based on applying the trained model to the particular image, in conjunction with other medical/user data (height, weight, age, gender, comorbidity, BMI, etc.) associated with the patient corresponding to the image. In one or more embodiments, IPRO can be combined with TNM staging. In one or more embodiments, the imaging analysis includes a volume surrounding the organ of interest so that the IPRO is not limited to learning prognostic features only from present lesion(s). In one or more embodiments, the IPRO can be utilized without needing or utilizing radiologists (or other users) to manually annotate regions of interest, such as primary tumors. The IPRO provides an improvement in that manual annotation is a time-consuming process, requires radiological expertise, is subject to inter-reader variability, and enforces the implication that only annotated regions of interest are correlated with outcomes.

Figure 8B is a block diagram illustrating an example, non-limiting embodiment of a modeling platform process 201B that employs image-based modeling (e.g., as described with respect to Figure 8A) to facilitate one or more clinical trials. Modeling platform process 201B can function or otherwise be performed by, and/or based on communicating with, medical scan processing system 500, and/or can function or otherwise be performed within the system of Figure 1B in accordance with various aspects described herein. Process 201B includes clinical variable imputation at 201C which can be performed utilizing captured images, such as CT scans. At 201D, patient selection for the clinical trial (e.g., eligibility) can be determined. At 201E, randomization can be determined for eligible candidates that have consented to participate, such as being randomized between an investigational arm (e.g., receives the trial treatment) and a control arm (e.g., does not receive the trial treatment but which can include receiving the standard of care treatment). At 201F, image processing can be performed and study metrics generated such as ingesting images (e.g., follow-up images after trial treatment begins) and performing quality control for the images. At 201G, an analysis can be performed according to the generated predictions from the model being applied to the images (e.g., follow-up images after trial treatment begins). As an example, the analysis allows for managing the clinical trial, including generating predicted variables (e.g., survival data that can be used to generate KM curves including predicted KM curves at different future time intervals) and providing access to the various predictions that have been made, as well as changes that have occurred to the predictions (e.g., between baseline/pre-treatment and/or between follow-up images).

Figure 8C is a block diagram illustrating an example, non-limiting embodiment of the clinical variable imputation 201C that employs image-based modeling (e.g., as described with respect to Figure 8A) to facilitate one or more clinical trials and that can function or otherwise be performed by, and/or based on communicating with, medical scan processing system 500, and/or can function or otherwise be performed within the system of Figure 1B in accordance with various aspects described herein. Process 201C includes obtaining baseline/pre-treatment images at 202C for each of the potential candidates for clinical trial(s). For example, the radiology department of a particular facility for each candidate can transmit or upload the baseline/pre-treatment images to the modeling platform. At 204C, the baseline/pre-treatment images can be analyzed by the image-based platform according to a trained image-based model (e.g., a model trained as described with respect to FIGs. 1, 2A or elsewhere herein). The training of the image-based model can be based on various datasets (public and/or private sources) that are relevant to the clinical trial (e.g., same disease, same organ, and so forth) which may or may not include images of healthy or otherwise disease-free individuals. In one or more embodiments, the datasets can be of individuals that received the standard of care treatment and/or of individuals that have not received any treatment. The analysis of the baseline/pre-treatment images can include quality control and pre-processing, including de-identification, segmentation and so forth. At 206C, clinical variables and/or scores can be predicted according to the trained image-based model (which may be only based on the baseline/pre-treatment image or may be in conjunction with other medical/user data ingested by the model). As an example and based on the submitted baseline/pre-treatment CT scans of the participants, the modeling platform can predict specific clinical variables, including, but not limited to: age, sex, ECOG status, smoking status, competing mortality risk, cardiac and pulmonary toxicity/AE, TNM stage (including relevant Tumor, Lymph Node and Metastasis classifications), pulmonary function and/or IPRO mortality risk score. At 208C, reporting or otherwise access to the results of the analysis can be provided by the modeling platform. For example, the output of the model can be provided to the referring physician (e.g., oncologist) via an official report. This information can also be provided to other relevant entities, such as the clinical manager or sponsor of the clinical trial.

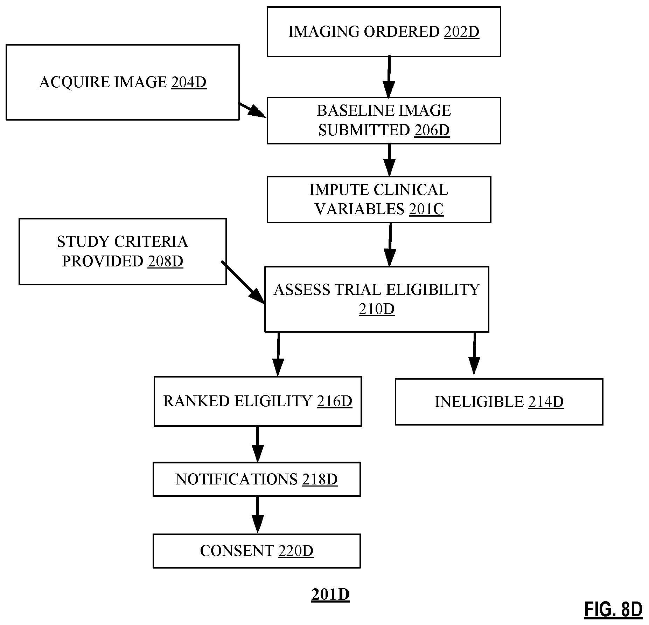

Figure 8D is a block diagram illustrating an example, non-limiting embodiment of the patient or candidate screening 201D for a clinical trial(s) that employs image-based modeling (e.g., as described with respect to Figure 8A) to facilitate one or more clinical trials and that can function or otherwise be performed by, and/or based on communicating with, medical scan processing system 500, and/or can function or otherwise be performed within the system of Figure 1B in accordance with various aspects described herein. Process 201D includes ordering (e.g., by a candidate's physician) or acquiring images at 202D, 204D which will serve as baseline/pre-treatment images for the candidates. As described herein, the baseline/pre-treatment images can be pre-treatment images of various types including 2D images or 3D images (e.g., CT scans). At 206D, baseline/pre-treatment images can be submitted. For example, the image can be ingested by the model, such as an upload from the imaging department of a facility. At 201C, one or more clinical variables can be imputed (such as described with respect to Figure 8C). At 208D, study criteria for the clinical trial can be obtained by the modeling platform. For example, study inclusion/exclusion criteria can be incorporated into the modeling platform from various sources, such as from public databases (e.g., clinicaltrials.gov). In one embodiment, the exclusion criteria can include specific anatomical features that are deemed or defined as being ineligible for the clinical trial, such as a lesion that is greater than a particular size. In one embodiment, this exclusion criteria can be applied by the modeling platform according to image analysis that determines the lesion size. At 210G, clinical trial eligibility can be assessed by the modeling platform. For example, using imputed variable(s) for each candidate, and comparing those to the study criteria, patients can be assessed by the modeling platform for trial eligibility. This assessment can be performed with or without user intervention. As described herein, the imputed criteria can include mortality risk scores, as well as other data that is determined from the model based on the image and/or is determined from data provided for the particular candidate.

In one embodiment, there can be multiple clinical trials that are seeking candidates (e.g., managed/commenced by a same entity or different entities). In this example, the modeling platform can determine eligibility for one, some or all of the multiple clinical trials. In one embodiment, where a candidate is eligible for more than one clinical trial, the modeling platform can analyze a best fit trial or otherwise rank the clinical trials from the perspective of the best candidates for a particular trial and/or from the perspective of the best trials for a particular candidate, based on various factors which may or may not be derived from the imputed variable(s) and/or the study criteria.

At 214D, the modeling platform can determine those candidates that are ineligible for the clinical trial(s), such as a candidate that is not eligible for any clinical trials. This determination can be performed with or without user intervention. At 216D, the modeling platform can determine those candidates that are eligible for the clinical trial(s). This determination can be performed with or without user intervention. In one embodiment, ranked eligibility can be performed by the modeling platform based on assessment of ongoing trials and imputed patient data. In one embodiment, the eligibility determination can include ranking candidates for the clinical trial, such as based on predicted risk mortality score, a number of criteria of the study criteria that are satisfied by the particular candidate, or other factors. At 218D, notification can be provided or otherwise generated for eligible candidates. For example, a notification can be sent to a referring physician of the candidate and/or to the candidate indicating that the candidate is eligible for ongoing clinical trial(s) or study(ies). At 220D, consent can be obtained from the candidate, such as a written consent to participate in the particular clinical trial.

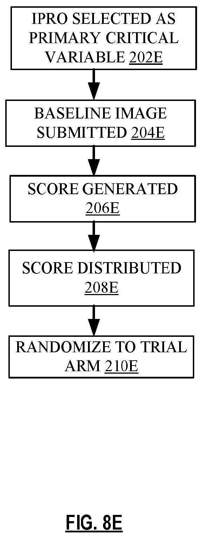

Figure 8E is a block diagram illustrating an example, non-limiting embodiment of randomization 201E for a clinical trial that employs image-based modeling (e.g., as described with respect to Figure 8A) to facilitate one or more clinical trials and that can function or otherwise be performed by, and/or based on communicating with, medical scan processing system 500, and/or can function or otherwise be performed within the system of Figure 1B in accordance with various aspects described herein. Process 201E includes selecting or otherwise determining (e.g., according to user input) a primary critical variable(s) (e.g., IPRO mortality risk score) that is to be utilized for randomization. In one embodiment, the IPRO mortality risk score can be the sole critical variable or can be used in combination with other selected primary critical variables. At 204E, baseline/pre-treatment images are submitted (e.g., as described with respect to Figure 8C and/or Figure 8D). At 206E, the primary critical variable is generated based on the baseline/pre-treatment image, such as determining an IPRO mortality risk score from applying model 112 to the baseline/pre-treatment image for the candidate (e.g., as described with respect to Figure 8C and/or Figure 8D).

At 208E, the modeling platform can distribute the primary critical variable, such as the IPRO mortality risk score. For example, the modeling platform can provide IPRO mortality risk score to study staff and/or to integrated randomization software (e.g., Interactive Voice/Web Response System (IxRS), Interactive Response Technology (IRT)). At 210E, the candidate can be randomized to a trial arm according to the primary critical variable and an analysis of balancing the trial arms, such as an investigational arm and a control arm. As an example, a candidate can be randomized automatically to a trial arm by the modeling platform per a pre-defined randomization scheme. The scheme can include balancing the primary critical variables among the investigational and control arm, and/or balancing other candidate criteria amongst the arms. In one embodiment, the IPRO mortality risk score can be included in the randomization determination (e.g., balancing between trial arms) in addition to other stratification factors (e.g., smoking, histology, TMN stage, age, prior treatment, etc.). In one embodiment, a balanced stratification can be achieved by the modeling platform utilizing a single IPRO factor (e.g., the IPRO mortality risk score). In one embodiment, the randomization is performed by the modeling platform and is based on achieving a distribution of predicted survival outcomes before the treatment commences that are equal or within a threshold of each other for the investigational and control trial arms as determined from the predictions generated from applying the image-based model to the baseline/pre-treatment CT scans (or other images). In one embodiment, the randomization can be performed by the modeling platform according to only the predicted variable(s) (e.g., without relying on the imputed variables). In another embodiment, the randomization can be performed by the modeling platform according to the predicted variable(s) in combination with other criterion, such as one or more of the imputed variables (e.g., age, sex, weight, ECOG status, smoking status, competing mortality risk, cardiac and pulmonary toxicity, TNM stage, pulmonary function, or a combination thereof) which can be determined from image analysis and/or determined from other information.

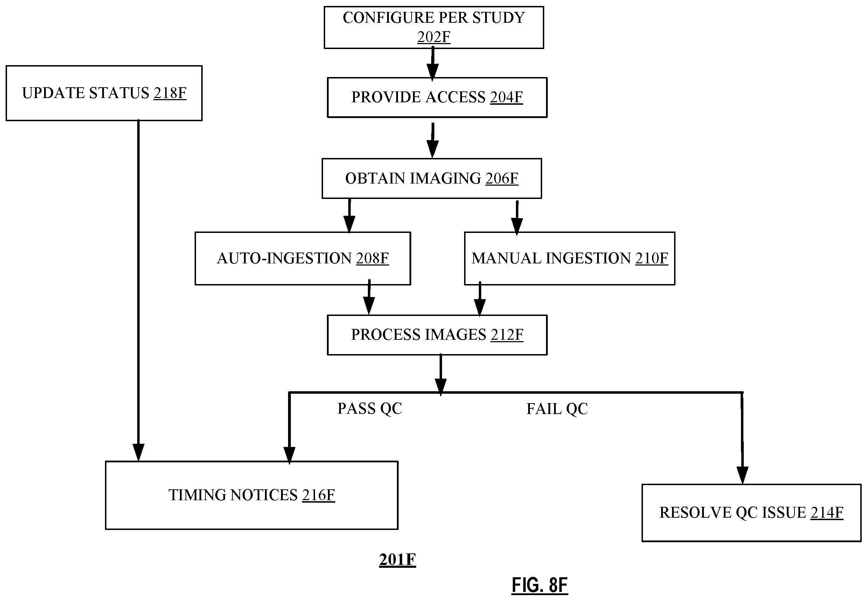

Figure 8F is a block diagram illustrating an example, non-limiting embodiment of image processing and study metrics 201F for a clinical trial that employs image-based modeling (e.g., as described with respect to Figure 8A) to facilitate one or more clinical trials and that function or otherwise be performed by, and/or based on communicating with, medical scan processing system 500, and/or can function or otherwise be performed within the system of Figure 1B in accordance with various aspects described herein. Process 201F includes configuring the modeling platform according to parameters or requirements of the particular clinical trial at 202F, providing access and/or selective access to various entities, individuals and/or teams at 204F (e.g. as described with respect to Figure 1B), and obtaining imaging at 206F (e.g., follow-up images) such as according to protocol (described in the clinical trial or otherwise defined). For instance, follow-up CT scans may be required every 4-6 weeks or at other time intervals for each of the candidates after the treatment has commenced. At 208F and 210F, automatic or manual ingestion of the images by the modeling platform can occur to enable application of the image-based model. For example, automatic ingestion can include CT scans being pulled from a site Picture Archiving and Communication System (PACS) to the modeling platform via an online web application. As another example, manual ingestion can include CT scans being submitted to the modeling platform via an online web application. At 212F, the images can be processed by the modeling platform. For example, quality control and/or de-identification, as well as other pre-processing steps can be performed on each of the images.

At 214F, if it is determined that the image did not satisfy the quality control requirements then the particular issue can be resolved. For example, a site can be automatically contacted to resolve queries regarding the particular image. This can include a request for re-capturing the CT scan or other remedial action to assist the image in passing the requirements. This step is repeated until the image passes the quality control requirements. At 216F, if it is determined that the image did satisfy the quality control requirements then timing notices can be generated with respect to the particular candidate and/or with respect to the clinical trial. For example, based on expected images (per the protocol), the modeling platform can inform or otherwise indicate to the user/viewer when images are expected, as well as a percent completed per timepoint. In this example, the user/viewer can be one or more individuals of the clinical manager, sponsor, or pharmaceutical company associated with management of the clinical trial.

In one embodiment at 218F, the modeling platform can update status for the particular candidate. For example, the modeling platform can integrate with EDC (or other study systems) to update patient status.

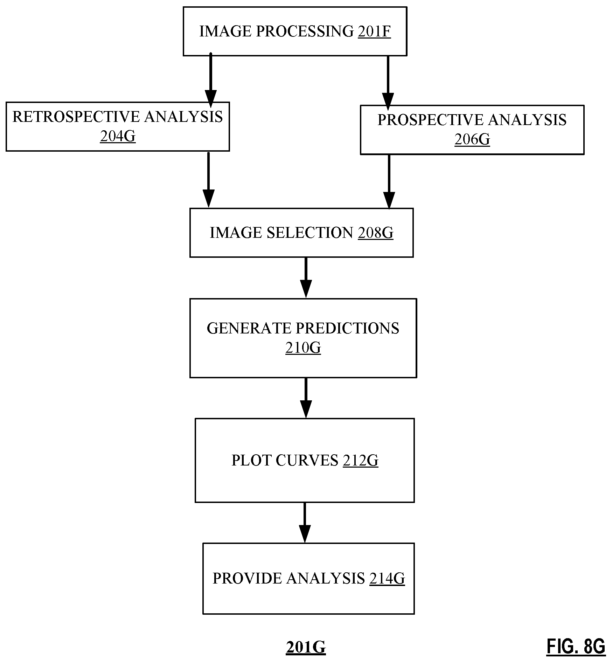

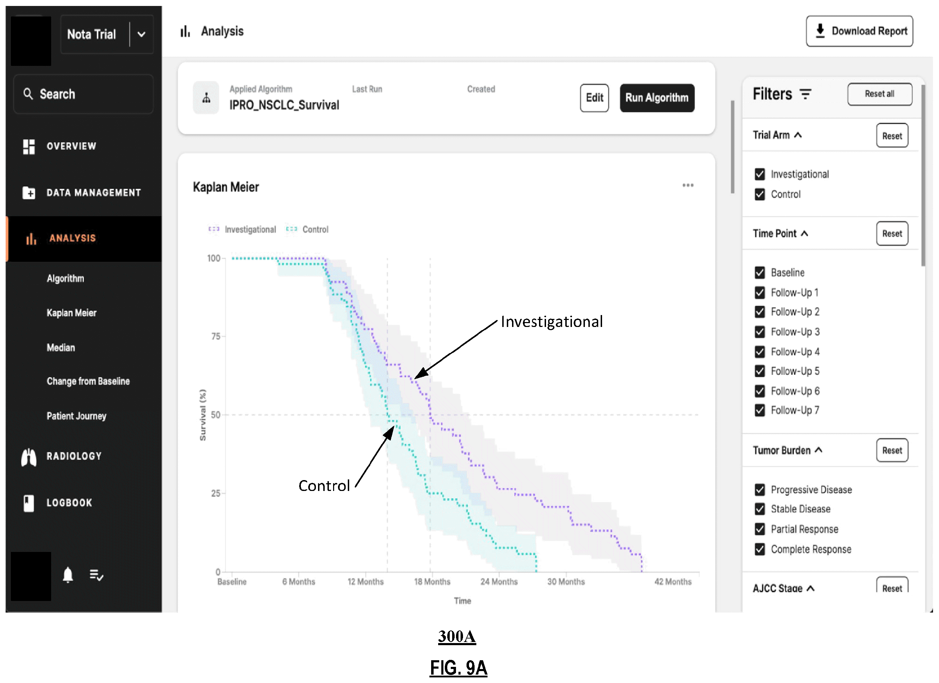

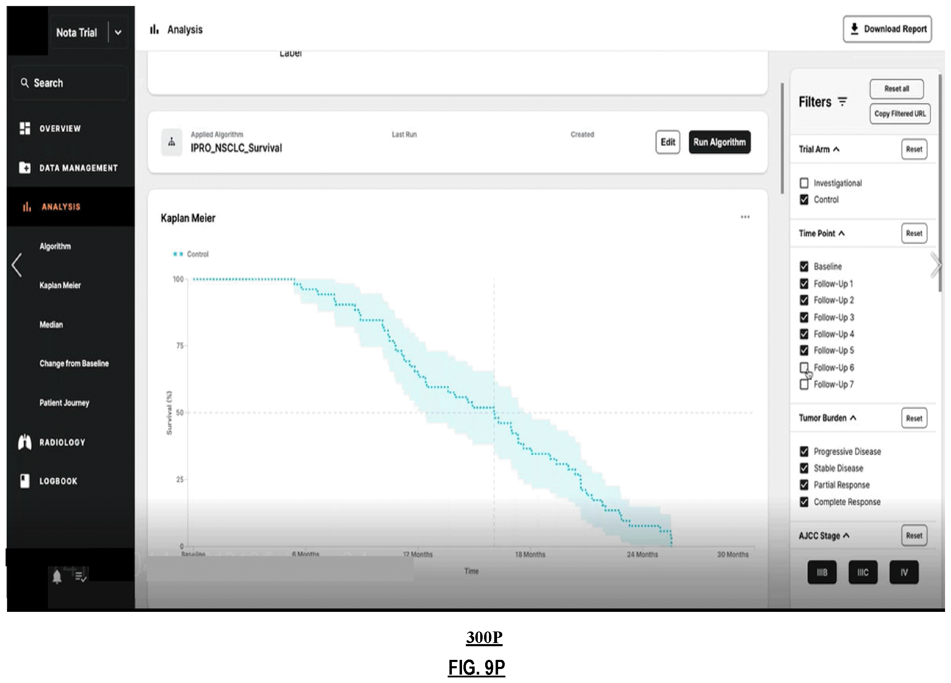

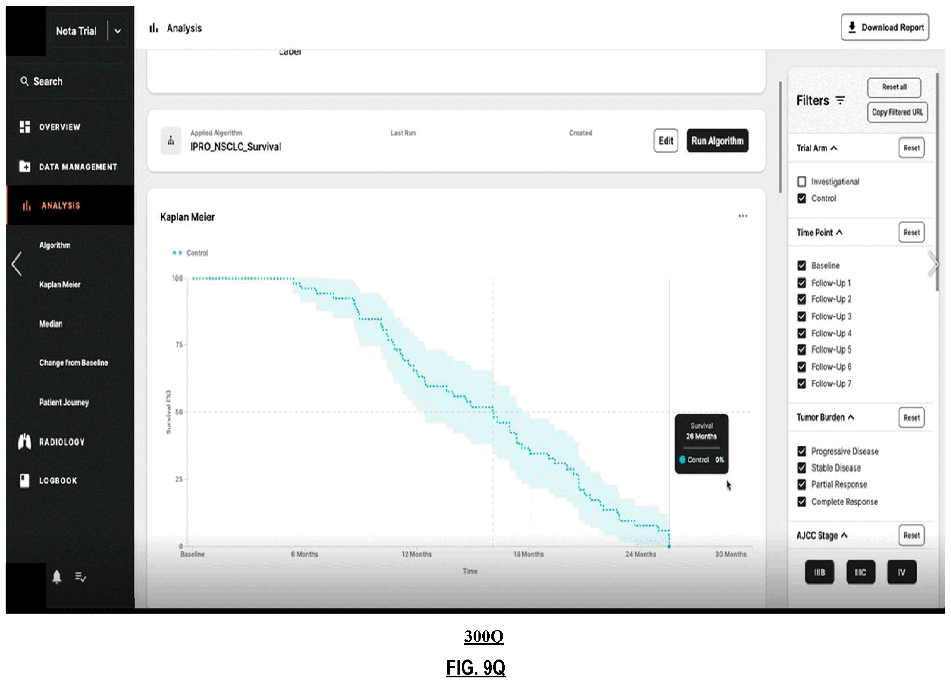

Figure 8G is a block diagram illustrating an example, non-limiting embodiment of an analysis 201G for a clinical trial that employs image-based modeling (e.g., as described with respect to Figure 8A) to facilitate one or more clinical trials and that function or otherwise be performed by, and/or based on communicating with, medical scan processing system 500, and/or can function or otherwise be performed within the system of Figure 1B in accordance with various aspects described herein. Process 201G can begin with the image processing 201F which can be a retrospective analysis 204G, for example some or all of the sets of images (e.g., baseline/pre-treatment and/or follow-up images) are available (e.g., the clinical trial has already begun or has already ended) or can be a prospective analysis 206G, for example the trial is commencing or is on-going and only some of the sets of images (e.g., baseline/pre-treatment and/or follow-up images) are available. At 208G, image selection can be provided. As an example, a user/viewer can determine which of baseline/pre-treatment or follow-up image(s) are to be utilized in the analysis. For instance, using a "study day" timeline, images can be selected to be included or excluded in the analysis (see Figure 9A). At 210G, predictions can be generated or otherwise obtained (e.g., from a data storage where the predictions had already been generated and stored by the image-based model). For example, based on selected and/or available data, survival, IPRO score, tumor size and tumor response predictions are generated or otherwise obtained). At 212G, representations of the data can be generated, such as curves, graphs, and so forth. For example, the predicted KM curves can be developed and plotted against the actual KM curve as well as other standard statistical models. At 214G, the analysis can be provided. For example, the final analysis can include: a comparison of the modeling platforms predictions vs other models; based on prospective Go/No Go criteria, a determination of when the program should be accelerated or considered futile; and/or baseline population (by arm) analysis.

In one embodiment, the analysis is performed retrospectively as described herein, to identify a sub-population of an investigational arm that had a significant improvement (e.g., improvement in survival above a particular threshold) so that the treatment can be focused on the particular sub-population (e.g., individuals with similar characteristics as the sub-population). As an example, the identified sub-population can be examined for common, similar or otherwise correlated characteristics (physiological, behavioral, etc.) and a subsequent clinical trial can be run utilizing these common characteristics as study criteria for eligibility of candidates.

While for purposes of simplicity of explanation, the respective processes are shown and described as a series of blocks in Figures 8A-8G, it is to be understood and appreciated that the claimed subject matter is not limited by the order of the blocks, as some blocks may occur in different orders and/or concurrently with other blocks from what is depicted and described herein. Moreover, not all illustrated blocks may be required to implement the methods described herein. Further, the processes described in Figures 8A-8G can be performed in whole or in part by one or more devices, such as computing devices 700, and/or any other devices with respect to FIG. 1B or other devices described herein. In one or more embodiments, the processes described in Figures 8A-8G can be performed in whole or in part retrospectively or prospectively.

### Example 1:

A retrospective study was performed providing an end-to-end deep learning approach in which the entire thorax of individual lung cancer patients was automatically evaluated to generate an IPRO score. Using publicly available pretreatment CTs split across a 5-fold validation, an assessment was performed as to how IPRO compares to and complements TNM staging for purposes of 1-year, 2-year, and 5-year mortality risk predictions in the withheld validation set. IPRO's ability to stratify patients across and within TNM stages was evaluated. The distribution of known prognostic clinical variables like age, sex, TNM stage, and histology across IPRO's risk deciles was reviewed and the amount of attention placed on lung lesions was quantified. It was determined in this Example 1 that CT imaging features were predictive of mortality risk when quantified using deep learning technologies (e.g., IPRO) which can enhance image-based prognostication and risk stratification in lung cancer patients.

A fully-automated IPRO technique was developed using deep learning to predict 1-year, 2-year, and 5-year mortality from pretreatment CTs of stage I-IV lung cancer patients. Using 6 publicly available datasets from The Cancer Imaging Archive, a retrospective five-fold cross validation was performed using 2,924 CTs of 1,689 patients, of which 1,212 had available TNM staging information. Association of IPRO and TNM staging with patients' actual survival status from the date of CT acquisition was compared, and an "Ensemble" risk score that combines IPRO and TNM staging via generalized linear regression was assessed. IPRO's ability to stratify patients within individual TNM stages using hazard ratios and Kaplan-Meier curves was also evaluated. In this Example 1, the IPRO showed similar prognostic power (C-Index 1-year: 0.70, 2-year: 0.69, 5-year: 0.67) compared to that of TNM staging (C-Index 1-year: 0.68 , 2-year: 0.70, 5-year: 0.69) at 1 and 2 years but underperformed TNM staging in predicting 5-year mortality. The Ensemble risk score yielded superior performance across all time points (C-Index 1-year: 0.76, 2-year: 0.76, 5-year: 0.75). IPRO stratified patients within TNM stages, discriminating between highest and lowest risk quintiles in stages I (HR: 7.44), II (HR: 5.51), III (HR: 3.93), and IV (HR: 1.57). This Example 1 illustrated that IPRO showed potential for enhancing imaging-based prognostication and risk stratification in lung cancer patients.

Lung cancer remains a leading cause of cancer death in North America and worldwide. The TNM staging system is used to classify the anatomic extent of cancerous tissue. This system helps to discriminate between patients in distinct groups, called TNM stages, and informs management of patients with cancer. In patients with lung cancer, TNM staging is a key prognostic factor, driving treatment and monitoring decisions. Radiological imaging, particularly computed tomography, plays a central role in defining the stage of disease. Analysis of CTs currently relies upon manual localization, classification, and measurement of nodules and is subject to inter- and intra-observer variability. More precise prognostication, as shown by the results of this Example 1 (and other embodiments described herein), can help clinicians make personalized treatment decisions that can, for example, spare a "low"-risk patient from aggressive treatment that might increase the risk of adverse effects, or, conversely, more proactively treat and monitor a "high"-risk patient.

CNNs, which are a form of deep learning, may be able to identify and quantify complex features in images that are not readily discernible to the naked eye. The use of CNNs to derive mortality risk prediction in patients with lung cancer, which rely upon manual steps, such as segmenting the primary lesion, or placing seed points or bounding boxes over regions of interest, would be inefficient. A fully automated approach, in which a system would analyze the entire thorax in a CT, may complement traditional TNM staging of lung cancer patients and provide greater prognostic power in an easily accessible manner.

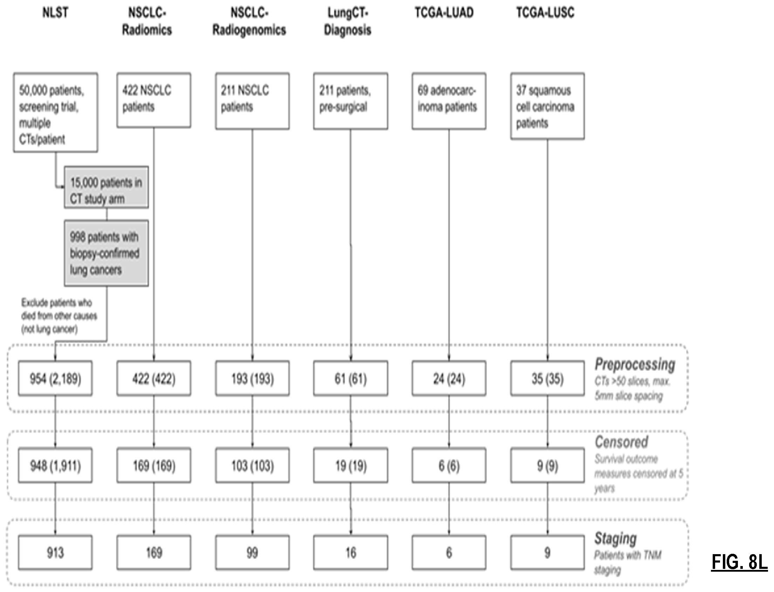

In the Example 1, publicly available pretreatment CTs of lung cancer patients were identified that also contained survival outcomes. Imaging data and associated clinical information were obtained from six datasets made available in The Cancer Imaging Archive (TCIA) (Table 1). A total of 1,689 patients were selected that had a biopsy confirmed lung cancer diagnosis, survival information, and at least one pretreatment axial CT. Mortality and CT acquisition dates were used to compute survival time and status at specified censoring dates (i.e., 1 year, 2 years, and 5 years). Cases that were lost to follow-up prior to a given censoring date were excluded from training and validation (see Figure 8L).

**Table 1: Patient characteristics in six experimental datasets.**

| Dataset | Number of Patients | Number of CTs | Gender (Male/Female) | Median Age (min, max) |
|---|---|---|---|---|
| NLST | 954 | 2,189 | 570 / 384 | 63 (55, 74) |
| NSCLC Radiomics | 422 | 422 | 290 / 132 | 68 (34, 92) |
| NSCLC Radiogenomics | 193 | 193 | 124 / 69 | 69 (24, 87) |
| TCGA-LUSC | 35 | 35 | 21 / 14 | 72 (39, 83) |
| TCGA-LUAD | 24 | 24 | 9 / 15 | 69 (42, 84) |
| LungCT Diagnosis | 61 | 61 | -- | -- |
| Total | 1,689 | 2,924 | 1,014 / 614 | 68 (24, 92) |

Given that some patients had multiple pretreatment CTs, validation was limited to only the final (i.e., most recent) pretreatment CT to assess the performance of IPRO and TNM staging. Multiple TNM staging types (e.g., clinical and pathological) and TNM staging editions (e.g., both the 6th and 7th edition of the AJCC staging system) were sometimes available for a given patient. Clinical TNM staging was prioritized over pathological TNM staging and used the most recent AJCC staging edition available for a given patient. Cases that were missing TNM staging were included in training but excluded from validation. Table 2 provides an overview of the distribution of TNM stages and survival status amongst the 5-year validation dataset, which contained 1,212 patients (605 alive, 607 deceased) with a median age of 64 (range: 43, 88) and in which 62% were male.

**Table 2: Number of patients in 5-fold validation set by clinical TNM stage and outcome at 1 year, 2 years, and 5 years post image acquisition.**

| Time from image acquisition | Number of Patients by Stage survived (deceased) | | | | Total |
|---|---|---|---|---|---|
| | I | II | III | IV | |
| 1 year | 556 (15) | 94 (21) | 272 (60) | 164 (30) | 1,086 (126) |
| 2 years | 523 (48) | 72 (43) | 184 (148) | 115 (79) | 894 (318) |
| 5 years | 438 (133) | 47 (68) | 81 (251) | 39 (155) | 605 07) |

In the Example 1, scanning protocols were varied between sites and cases (e.g., radiation dose, use of contrast, slice spacing, anatomic regions included); as such all CTs were preprocessed to standardize model inputs and improve model generalizability. This included resampling each CT to 1mm slice thickness and pixel spacing, and clipping Hounsfield Unit values at -1,000 to 1,000. Any CTs with greater than 5mm slice thickness or fewer than 50 slices were excluded.

As shown in Figure 8A, the IPRO framework consisted of a thorax localizer and a 3DCNN that extracted imaging features automatically along the axial, sagittal and coronal directions, simultaneously. The thorax localizer consisted of an algorithm that limited the model input to a 3D space (36cm x 36cm x 36cm in size) centered on the lungs, thus excluding features outside of the thorax (e.g., abdomen) and outside of the skin (e.g., CT scanner table). The automatically identified thorax region was then fed into the 3DCNN which outputted probability scores between 0 and 1 indicating 1-year, 2-year, and 5-year mortality for a given CT.

The architecture of the 3DCNN was based on a neural network called InceptionNet. This architecture enabled features to be learned without being prone to overfitting, suitable for medical applications where individual data points tend to be large but the number of patients are few. To make the neural network three-dimensional, transfer learning was first applied to stabilize the network using ImageNet, and then intermediate layers were duplicated in a new temporal dimension (i.e., z-axis). The resulting architecture allowed for entire 3D CT volumes to be fed into the 3DCNN without further modifications.

A five-fold cross validation across six lung cancer datasets was performed to train and validate the IPRO which involved randomly splitting the data into 5 groups, while ensuring class balance based on survival status and TNM staging distribution. Each group was then iteratively withheld for validation while training on the remaining 4 groups until each group was used for validation. Models were trained to predict mortality as posterior probabilities between 0 (low-risk) and 1 (high-risk) at time *t,* given 3D CT volumes, where *t* = 1, 2 or 5 years. To compare the prognostic power of IPRO to that of TNM staging, generalized linear regression models were trained using solely TNM staging information in the same 5-fold cross- validation to predict *t*-year mortality. The "glm" library in R was used for training and predicting regression models on eight TNM sub-types. Ensemble models (which combined IPRO and TNM staging) were generated by training a linear regression model per fold, where the inputs were TNM staging and IPRO mortality risk scores at time *t.* Risk scores were compared with survival status at time *t* using concordance index (C-index) and area under the receiver operating characteristic curve (AUC). Pearson *r²* correlations between IPRO scores and time-to-event from date of CT acquisition were examined. Statistical significance between models was assessed using a two-sample t-test.

To assess stability of IPRO scores in a test-retest scenario, intra-class correlation coefficient (ICC) and mean absolute differences (MAD) between IPRO risk scores generated from the CTs in the RIDER dataset were evaluated. ICC of >0.90 was considered an "excellent" agreement. IPRO was used to stratify lung cancer patients, where Kaplan-Meier curves were generated per risk group. Each group was defined as a subset of the patients in the validation set sorted by ascending IPRO mortality risk scores. To quantify differences between predicted highest- and lowest-risk groups defined as quintiles (i.e., 20%) or deciles (i.e., 10%) of the patients with either the highest or lowest IPRO scores, the coxph function was used to report hazard ratio (HR) and log rank p-values. All statistical analyses were performed in R.

Associations between the outcome predictions and known prognostic clinical variables like age, sex, TNM stage, and histology across IPRO's risk deciles were explored. Gradient-weighted Class Activation Mapping (GradCAM) activation maps were generated as a visual explanation to indicate on which anatomical regions within the thorax IPRO placed attention to generate its mortality risk prediction. The middle (i.e., 8th) layer of the network was used to generate attention weights during backpropagation resulting in a 3D attention mask, offering both spatial information and relevance to the final classification layer. Attention maps were further normalized and scaled to fit the original 3D image space. Such visualizations offer insight into a subset of the features learned in the 3DCNN and the deep learning based predictions. To quantify model attention placed on lesions, CTs from a subset of patients in the validation set were interpreted by radiologists, who manually detected and volumetrically segmented lung lesions. For each CT scan, the average attention value in the thorax was calculated and compared to the average attention placed within the segmented lesions.

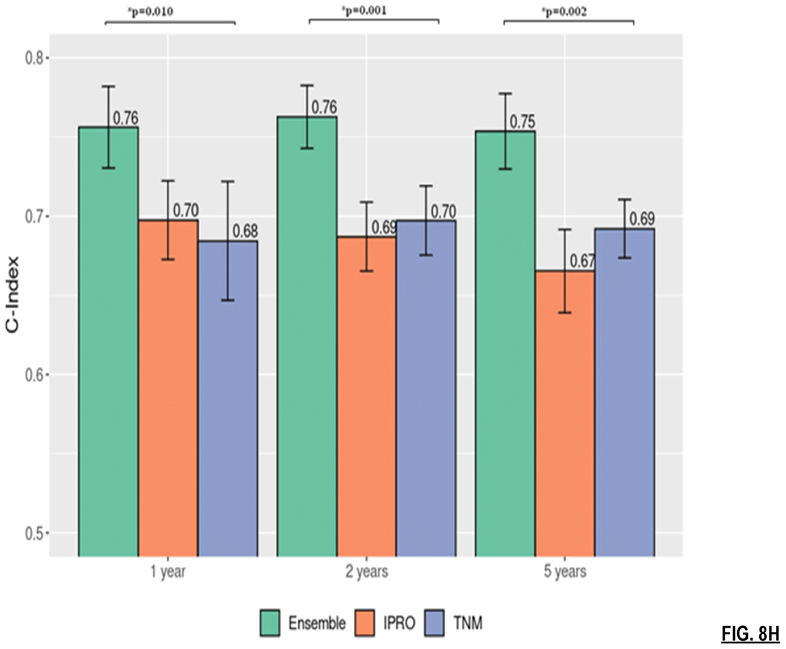

IPRO showed evidence of similar prognostic power compared to that of TNM staging in predicting 1-year and 2-year mortality but underperformed TNM staging in predicting 5-year mortality (See Figure 8H). The Ensemble model, which combines IPRO and TNM staging information, yielded significantly superior prognostic performance at all three time intervals (p ≤ 0.01) when compared to that of TNM alone. Table 3 summarizes results across metrics including C-index, AUC, and Pearson *r²*.

**Table 3: Average C-Index, AUC and r² for mortality risk prediction models across 5 folds.**

| | C-Index | | | AUC | | | Pearson r² | | |
|---|---|---|---|---|---|---|---|---|---|
| | 1 year | 2 years | 5 years | 1 year | 2 years | 5 years | 1 year | 2 years | 5 years |
| IPRO Standard Dev. | 0.697 ±0.02 | 0.687 ±0.02 | 0.665 ±0.03 | 0.714 ±0.01 | 0.716 ±0.03 | 0.706 ±0.04 | 0.159 ±0.03 | 0.174 ±0.03 | 0.178 ±0.03 |
| TNM Standard Dev. | 0.684 ±0.04 | 0.697 ±0.02 | 0.692 ±0.02 | 0.699 ±0.03 | 0.731 ±0.03 | 0.777 ±0.02 | 0.203 ±0.03 | 0.233 ±0.04 | 0.240 ±0.05 |
| Ensemble Standard Dev. | 0.756 ±0.03 | 0.763 ±0.02 | 0.754 ±0.02 | 0.776 ±0.02 | 0.803 ±0.01 | 0.840 ±0.02 | 0.293 ±0.03 | 0.333 ±0.04 | 0.341 ±0.05 |

| | | | | | | | | | |
|---|---|---|---|---|---|---|---|---|---|
| *p<0.001 for all reported metrics | | | | | | | | | |

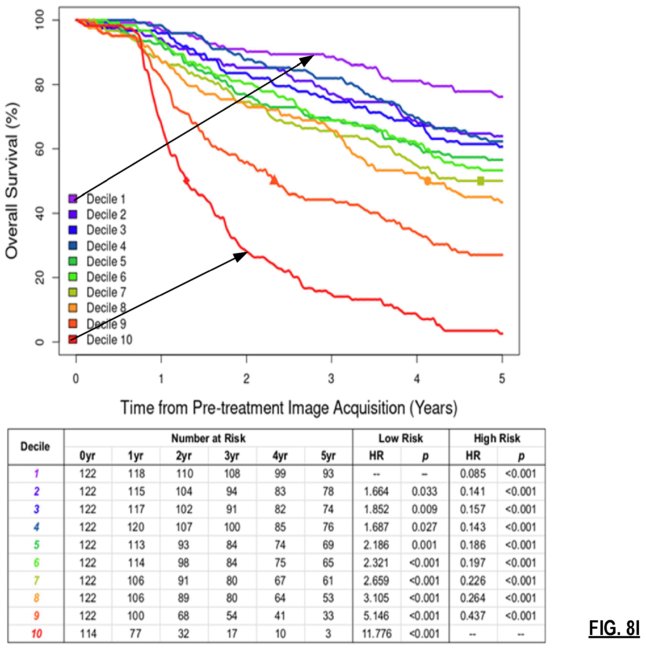

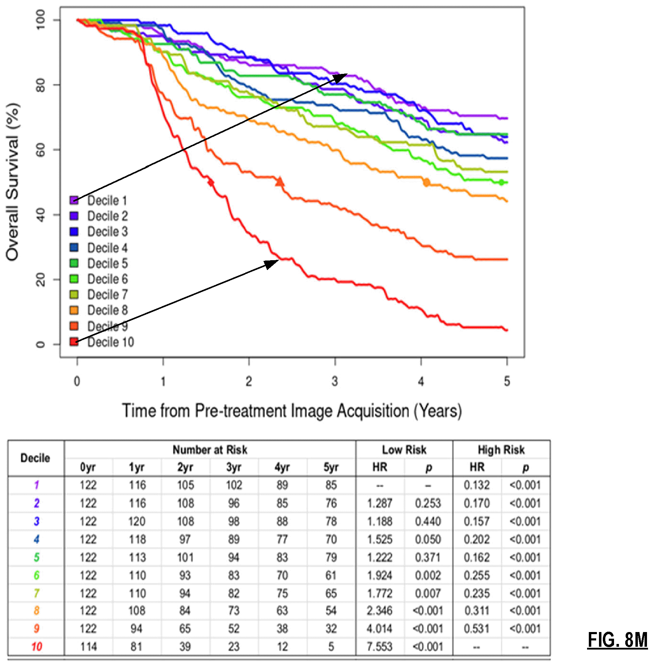

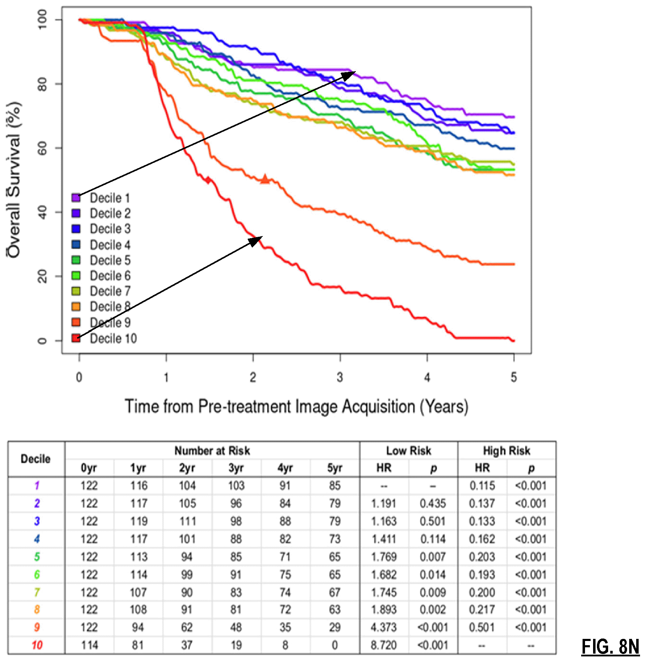

Stability assessment of IPRO using the test-retest RIDER dataset revealed strong correlations between the two consecutively acquired CTs, with average intra-class correlation coefficients of 0.87, 0.83 and 0.80 for the 1-year, 2-year and 5-year IPRO scores, respectively. Mean absolute differences between IPRO scores per RIDER patient were consistently less than 0.05 (1-year: 0.04, 2-year: 0.04, 5-year: 0.03). Kaplan-Meier curves were generated in Figure 8I and show risk stratification by IPRO deciles of all lung cancer patients (stages I-IV) included in the 5-year validation. Hazard ratios (HRs) between each decile and the highest risk group (i.e., decile 10) were statistically significant. Hazard ratios between each decile and the lowest risk decile (i.e., decile 1) were statistically significant for deciles ≥6. Kaplan-Meier curves illustrating the 1-year and 2-year IPRO deciles were generated and are shown in Figures 8M and 8N.

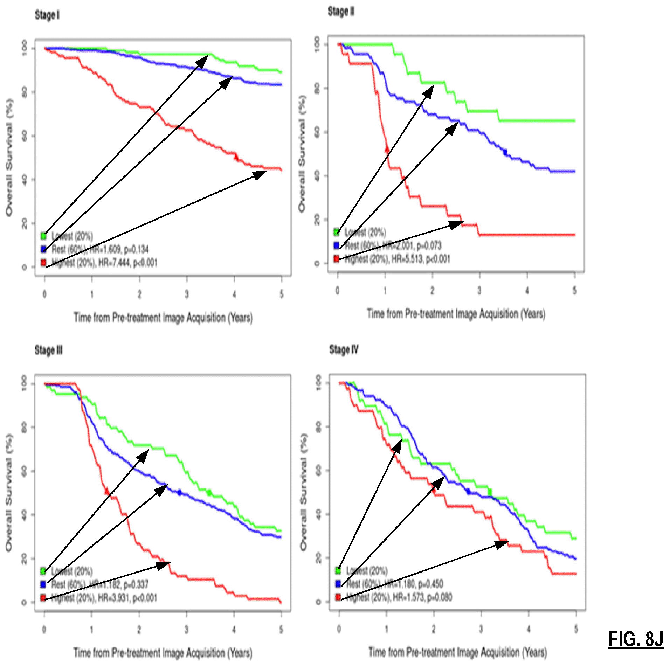

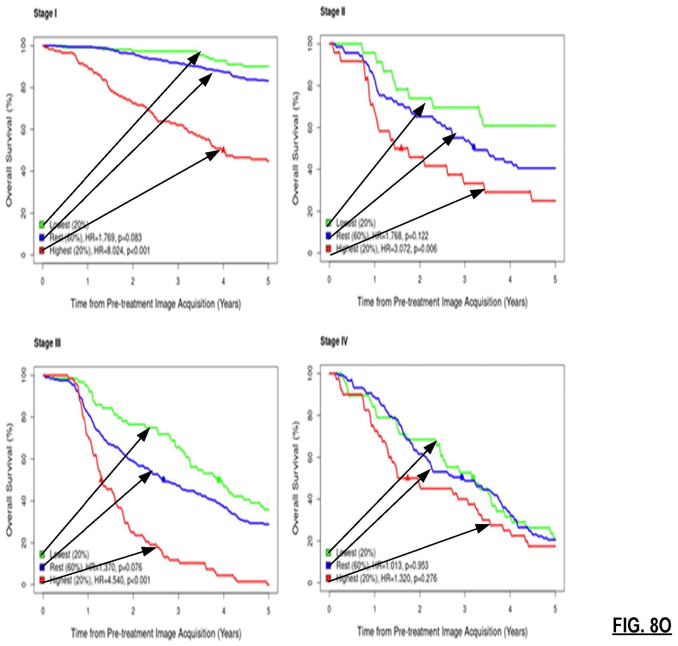

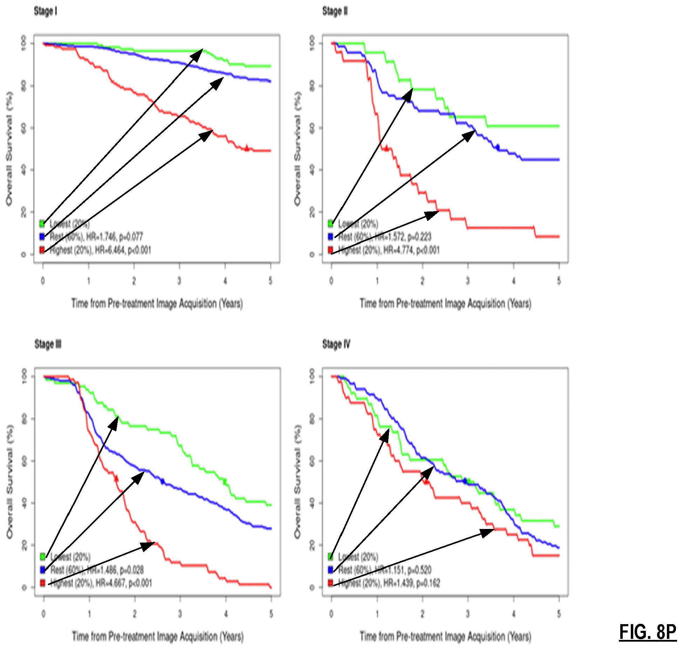

IPRO's ability to stratify patients within each TNM stage via high-risk and low-risk quintiles was assessed (see Figure 8J). Stage I patients in the highest risk IPRO quintile had a 7.4-fold (95% CI 4.0-13.8, *p* < 0.001) increased 5-year mortality hazard compared to stage I patients in the lowest risk quintile. Similarly, in stage II and stage III, patients in the highest risk IPRO quintile had a 5.5-fold (95% CI 2.4-12.7, *p* < 0.001) and 3.9-fold (95% CI 2.6-6.0, *p* < 0.001) increased 5-year mortality hazard compared to stage II and stage III patients in the lowest risk quintile, respectively. Across all TNM stages, the weakest patient stratification existed for stage IV patients where the highest risk IPRO quintile had a 1.6-fold (95% CI 0.9-2.6, *p* = 0.080) increased 5-year mortality hazard compared to stage IV patients in the lowest risk quintile. Kaplan-Meier curves were generated by TNM stage illustrating the 1-year and 2-year IPRO quintiles and are shown in Figures 8O and 8P.

To further explore IPRO's 5-year mortality predictions, the distribution of known prognostic variables including age, sex, TNM stage, and histology across the IPRO risk deciles (Table 4) was assessed. Comparing the characteristics of patients IPRO deemed lowest risk (decile 1) to those deemed highest risk (decile 10), the median age increases from 62 to 68 years and the sex composition shifts from 32.0% male in the lowest risk patients to 67.9% male in the highest risk patients. The most common histological subtype in patients comprising the lowest risk decile was adenocarcinoma (41%), while squamous cell carcinoma (38%) and large-cell carcinoma (24%) accounted for the majority of highest risk patients. Lung cancer patients diagnosed as TNM stages I & II account for 73.0% of patients in the lowest risk decile but only 29.8% of patients in the highest risk decile.

**Table 4: Distribution of known prognostic factors by IPRO risk decile including age, sex, TNM stage and histology subtype.**

| IPRO Risk Decile | Median Age | Sex* (M / F) | Stage | Histology* | | | | | | | |
|---|---|---|---|---|---|---|---|---|---|---|---|
| | | | I | II | III | IV | SqCC | AC | SCC | LCC | Other |
| 1 (low) | 62 | 39 / 83 | 83 | 6 | 16 | 17 | 12 | 50 | 12 | 3 | 45 |
| 2 | 64 | 58 / 64 | 73 | 3 | 23 | 23 | 34 | 37 | 7 | 4 | 40 |
| 3 | 63 | 56 / 64 | 68 | 8 | 23 | 23 | 22 | 45 | 14 | 3 | 36 |
| 4 | 63 | 68 / 53 | 62 | 7 | 30 | 23 | 30 | 41 | 11 | 5 | 34 |
| 5 | 64 | 82 / 40 | 60 | 7 | 27 | 28 | 23 | 50 | 19 | 3 | 27 |
| 6 | 64 | 80 / 40 | 55 | 14 | 27 | 26 | 24 | 44 | 15 | 7 | 30 |
| 7 | 65 | 100 / 16 | 51 | 15 | 33 | 23 | 32 | 45 | 10 | 5 | 24 |
| 8 | 65 | 96 / 25 | 58 | 15 | 28 | 21 | 35 | 38 | 11 | 4 | 33 |
| 9 | 67 | 84 / 32 | 44 | 23 | 45 | 10 | 34 | 41 | 3 | 12 | 26 |
| 10 (high) | 68 | 76 / 36 | 17 | 17 | 80 | 0 | 42 | 20 | 0 | 27 | 23 |

| | | | | | | | | | | | |
|---|---|---|---|---|---|---|---|---|---|---|---|
| * excludes 20 patients that are missing sex and histology information. | | | | | | | | | | | |

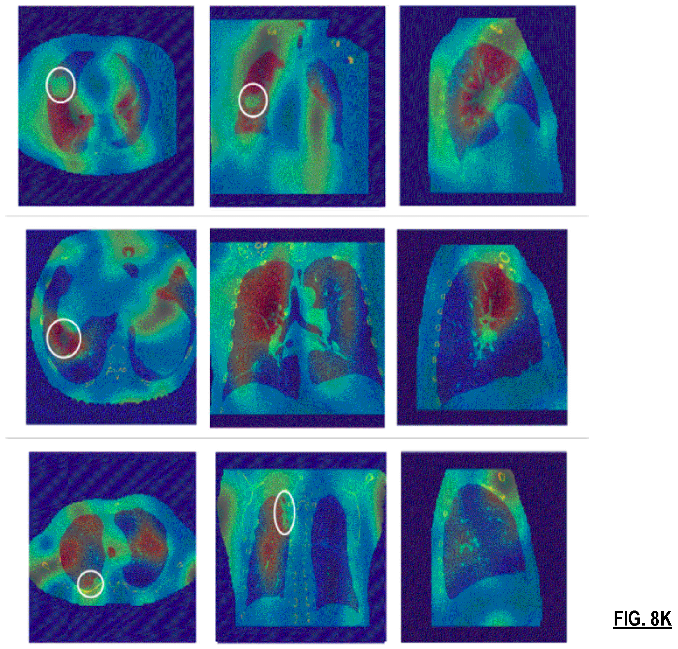

GradCAM activation maps indicated that IPRO learned to place outsized attention on lesions. On average, twice the amount of attention was placed on lesions (0.248) compared to the average attention placed on the thorax (0.120). GradCAM activation maps were reviewed to qualitatively assess on which anatomical regions within the thorax IPRO placed attention to generate the 5-year mortality risk prediction. In Figure 8K, three sample cases are provided depicting areas that received the greatest attention (red) and the least attention (blue). Hand-drawn white ellipses (not visible to IPRO) denote areas containing primary lesions.

Based on the results of this Example 1, it is demonstrated that deep learning can provide additional prognostic information based on both known and unknown features present in CTs in a quantifiable, continuous variable. The end-to-end fully-automated framework of IPRO can ingest CTs of varying sources and imaging protocols, and can automatically analyze a 3D region encompassing the thorax. IPRO predicted mortality consistently and accurately at 1-year, 2-year, and 5-year time intervals, and generated similar performance to TNM staging. By combining IPRO with TNM, the Ensemble model showed improved performance across all time intervals, suggesting that IPRO- and human-derived features are complementary. By encompassing the anatomical structures comprising the thorax, IPRO is not limited to learning prognostic features only from present lung lesion(s). This approach has the benefit of not needing radiologists to manually annotate regions of interest, such as primary tumors. Manual annotation is a time-consuming process, requires radiological expertise, is subject to inter-reader variability, and enforces the implication that only annotated regions of interest are correlated with outcomes.

In reviewing regions of the CT volume that received the greatest attention by IPRO (Figure 8K), it was determined that IPRO gravitated towards tissue comprising primary lesions, indicating that IPRO learned that this tissue has prognostic value. Given that lesion annotations were not provided during training, this showed that features used by IPRO correlate with those defined in manual image interpretation guidelines such as TNM or RECIST 1.1. More interesting are the peritumoral areas also highlighted in the attention maps (Figure 8K), indicating such areas hold additional prognostic insight. Known prognostic variables such as age and sex for patients within each risk group (Table 4) revealed that those patients in the highest risk group (decile 10) were on average 6 years older and mostly male compared to those in the lowest risk group (decile 1). Histology subtypes in decile 10 were also more likely to exhibit large cell carcinoma and squamous cell carcinoma subtypes. Given the incorporation of the entire chest in the model, not only characteristics of the tumor, lymph nodes and metastasis, other potential useful information, such as coronary artery calcification, size of the heart, body composition, or pulmonary emphysema may have been learned and used by the model. In one embodiment, training and evaluating of region-specific 3DCNNs can be performed to better derive anatomic origins of IPRO's predictions.

The primary component of IPRO is an end-to-end 3DCNN that, unlike two-dimensional neural networks that learn from features in only the XY dimension (i.e., from a single CT slice), learns a series of feature maps at multiple scales across an additional dimension (i.e., Z), capturing millions of patterns not easily discernible to the naked eye. This can help IPRO incorporate richer features like volume of tumors and features in peritumoral tissue that span multiple CT slices, rather than just a single 2D slice. This Example 1 predicts mortality risk for lung cancer patients and incorporates a wider range of pretreatment CTs from multiple datasets and sites.

Staging classification systems are not a primarily prognostic tool, but instead can be a way to provide a consistent means of communication, allowing physicians to exchange information about an individual tumor or group of tumors. Nonetheless, the anatomic extent of disease can be a major factor affecting prognosis and can help in selecting the appropriate treatment approach. Clinical trials comparing different treatment regimens for lung cancer, for example, use TNM staging categories as inclusion/exclusion criteria. In this context and based on the results of this Example 1, despite the lack of detailed information regarding tumor biology and type of treatment offered, IPRO provided at least similar prognostic insight when compared to TNM staging.

In this Example 1, IPRO was able to stratify patients within the same TNM stage. Particularly in stage I, II and III, there are clear distinctions in survival outcomes between the IPRO highest-risk and lowest-risk quintiles. While TNM staging has prognostic power, the ability to further separate high and low risk subgroups within the same stage is an improvement. In one or more embodiments described herein, studies incorporating follow up CTs during and after treatment may be used to further refine mortality prediction.

IPRO's complementary insight via predictive data such as mortality risk may intensify treatment and monitoring of high-risk patients (e.g., at the clinician's discretion), while watchful waiting approaches for low risk patients may assist in avoiding aggressive treatment that might unnecessarily increase risk of adverse effects or reduce quality of life. In one or more embodiments, the IPRO can train and validate predictive models according to larger, independent datasets, as well as in prospective studies. In one or more embodiments, the datasets for training and validation can be expanded to different stages of cancers, different ages, and/or different habits (e.g., smoking vs non-smoking). In one or more embodiments, treatment (which is a major determinant of patient prognosis after a diagnosis of lung cancer) can be incorporated or otherwise utilized by the IPRO model, which in the Example 1 described above was based exclusively on pretreatment imaging.

In this Example 1, to enable the framework to adapt to multiple scanning protocols, V-Net segmentation models were developed to identify and contour the lungs and skin automatically. Such segmentation masks were used to mask out artifacts outside the body and navigate the model to a fixed 3D box centered in the thorax to encapsulate both lungs. The V-Net was based on a deep segmentation model that has been used in medicine and can adapt to multiple organs and tissue types. In IPRO, two separate V-Net models were trained: one to identify regions encased in the body (i.e., within the skin), and the other to segment the lung air space. The skin segmentation mask was used to eliminate artifacts such as the table, blanket, etc., whereas the lung segmentation mask acted as a guide for centering the 3D box (360x360x360 pixels) to encapsulate the lungs. The 3D box centered on the lungs was further downscaled by a factor of 2 and was used as the input for the 3DCNN.

To train both V-Nets, publicly available NSCLC-Cetuximab (RTOG-0617) dataset was used, containing CTs from 490 patients, in which organs at risk including the lungs and skin were contoured for radiotherapy treatment planning. Scans were selected containing annotated regions for lung_cntr or lung_ipsi, and skin, and distributed into training and test sets as shown in Table 5. CT volumes and contours were then rescaled to a size of 100x128x128 pixels to fit into GPU memory. As a post-processing, hole filling was applied to lung segmentation masks to remove spurious regions. Performance of the both V-Nets on held-out test sets were determined and are illustrated in Table 6.

**Table 5: Number of CTs used for training lung and skin segmentation V-Net models.**

| | Train | Test |
|---|---|---|
| Lung Segmentation | 389 | 97 |
| Skin Segmentation | 384 | 95 |

**Table 6: Performance of lung and skin segmentation V-Net models. Standard deviation between scans is provided in brackets.**

| | Intersection Over Union | Sørensen-Dice coefficient |
|---|---|---|
| Lung Segmentation | 81.20 | 89.03 |
| *Standard Deviation* | *±10.81* | *±10.13* |
| Skin Segmentation | 87.91 | 92.20 |
| *Standard Deviation* | *±18.08* | *±14.43* |

3DCNN training was performed over ten epochs with a learning rate of 5e⁻⁷ and a batch size of 48. Model parallelization was used across 8 GPUs to speed up training, taking ~11 hours per fold. Five percent of the training set was allocated for the tuning set which was used to set the number of training iterations and weight decay parameters. An lr-finder open source library was used prior to training to initialize the learning rate. To encourage generalizability, Dropout was applied to the final layers of each IPRO model and a focal loss function was adopted to deal with extreme class imbalance.

To assess stability of IPRO's predictions, an independent publicly available dataset, RIDER, was used consisting of 32 patients diagnosed with lung cancer. Each patient underwent two chest CTs within 15 minutes using the same imaging protocol, therefore only minute changes were visible between scans.

Figure 9A is an illustrative embodiment of a GUI 300A. The GUI 300A can serve as an illustrative embodiment of a user interface that can be accessed or selectively accessed by various devices to provide various information to various individuals, such as patients, healthcare providers, clinical trial managers, pharmaceutical companies, and so forth. In one embodiment, access to the GUI 300A can be by way of a trial dashboard where an entity involved in multiple clinical trials can access information for each of them.

As an example, GUI 300A can be a trial view that provides access to buttons for an overview, data management, and analysis of the clinical trial. For instance, GUI 300A can provide event estimation information such as survival data (e.g., KM curves) to be generated (based on the image-based model applied to the baseline/pre-treatment and/or follow-up images of the clinical trial) and/or presented according to a selection of particular images as shown in the option "time point." The data, such as the KM curves, can be shown for the investigational arm, the control arm or both (as is depicted in Figure 9A). For example, a user/viewer can determine which of baseline/pre-treatment and/or follow-up image(s) are to be utilized in the analysis. For instance, using a "study day" timeline, images can be selected to be included or excluded in the analysis. In one embodiment, the 300A allows a viewer to toggle on or off the image predictions for any follow-up images such that if toggled on then the KM curve will include those images in the predictions.

GUI 300A depicts KM curves based on data generated from applying the image-based algorithm on images (e.g., baseline/pre-treatment and/or follow-up images) that have been ingested so far and the KM curves are the predicted KM curves based on that data. As an example, the prediction can be a time to mortality as of the date of image acquisition.

GUI 300A depicts KM curves where the investigational arm is performing (according to survival) better than the control arm which is indicative of or otherwise shows or measures the treatment effect for the clinical trial. In this example, the control arm can include digital twins for one, some or all of the actual candidates in the investigational arm, where the digital twins (and its corresponding predicted variables) are generated by the image-based model from the baseline/pre-treatment image of the particular candidate with or without incorporation of other medical user data into the modeling. In one or more embodiments, the control arm can be made of only digital twins, such as a one-to-one correspondence of digital twins with actual candidates (which are in the investigation arm). In other embodiments, the control arm may include only actual candidates; or may include actual candidates along with digital twins of actual candidates from the investigational arm. As explained herein, the analysis (which includes generating data by applying the image-based model to the baseline/pre-treatment and/or follow-up images) can be prospective such as during an on-going trial where treatment has not yet finished (e.g., predicting the treatment effect) or can be retrospective such as where the clinical trial has been completed.

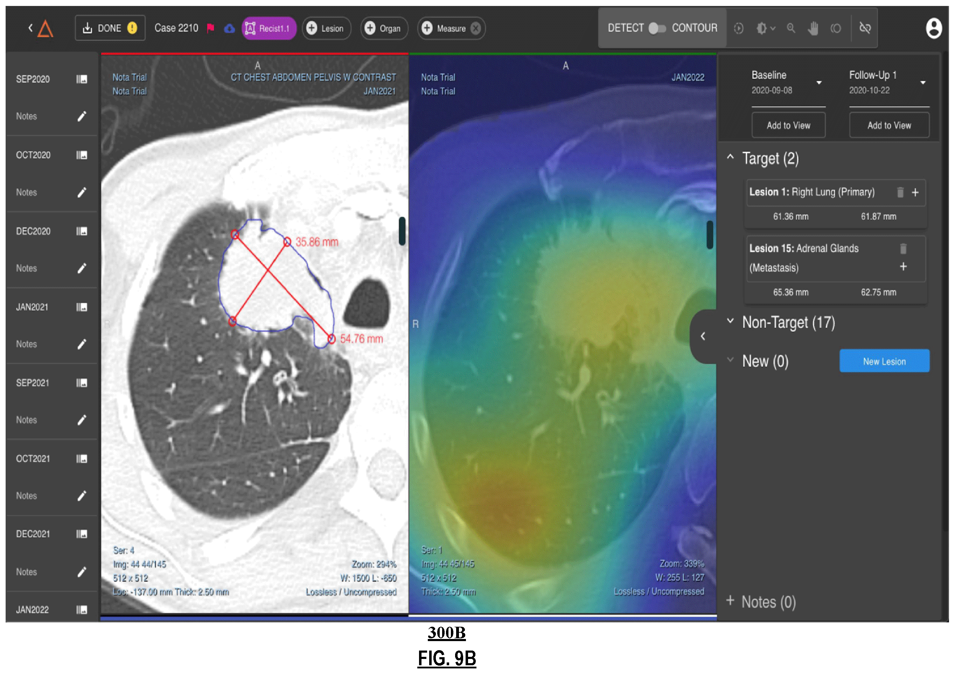

Figure 9B is an illustrative embodiment of a GUI 300B. The GUI 300B can serve as an illustrative embodiment of a user interface that can be accessed or selectively accessed by various devices to provide various information to various individuals, such as patients, healthcare providers, clinical trial managers, pharmaceutical companies, and so forth. GUI 300B can allow for image-based predictions to be generated which can in some embodiments complement traditional imaging interpretation frameworks. For instance, the GUI 300B can allow for annotations to be manually entered. In another embodiment, the annotations are generated by the image-based model. Other information can be provided, such as activation maps that indicate regions of attention in the organ according to weighting by the model.

In one or more embodiments, the modeling platform can streamline customizable imaging workflows, increase reproducibility of imaging interpretation, and/or generate (e.g., with or without user input or user assistance) annotations for ML research and biomarker discovery.

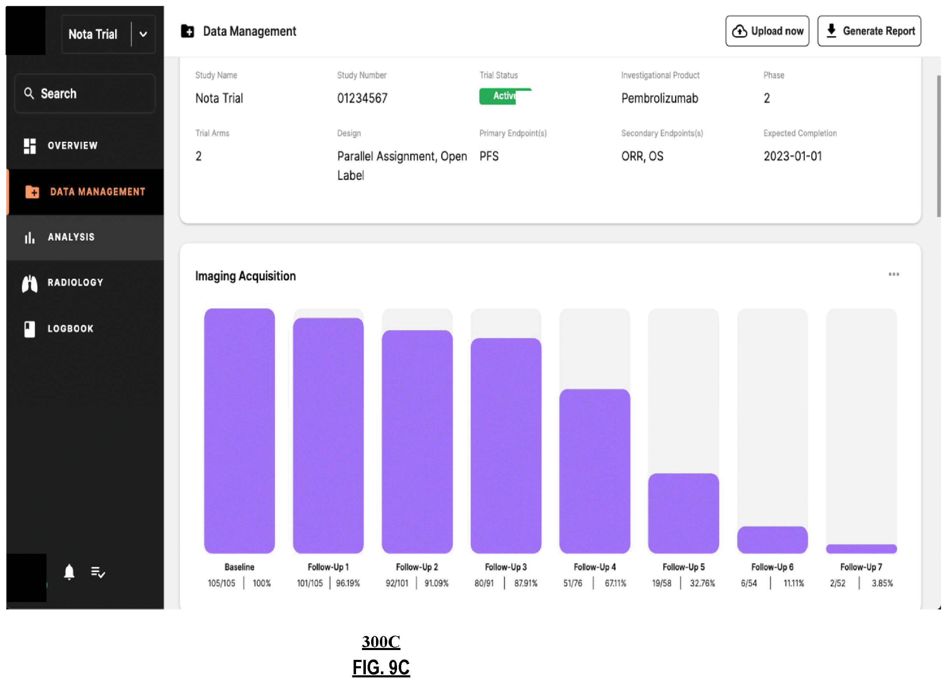

Figure 9C is an illustrative embodiment of a GUI 300C. The GUI 300C can serve as an illustrative embodiment of a user interface that can be accessed or selectively accessed by various devices to provide various information to various individuals, such as patients, healthcare providers, clinical trial managers, pharmaceutical companies, and so forth. In this example, the user/viewer can be one or more individuals of the clinical manager, sponsor, or pharmaceutical company associated with management of the clinical trial. In one embodiment, GUI 300C can be accessed via the data management button for the clinical trial which shows current image acquisition (e.g., 105 of 105 baseline/pre-treatment images acquired; 101 of 105 follow-up one images acquired, and so forth) to facilitate managing the clinical trial.

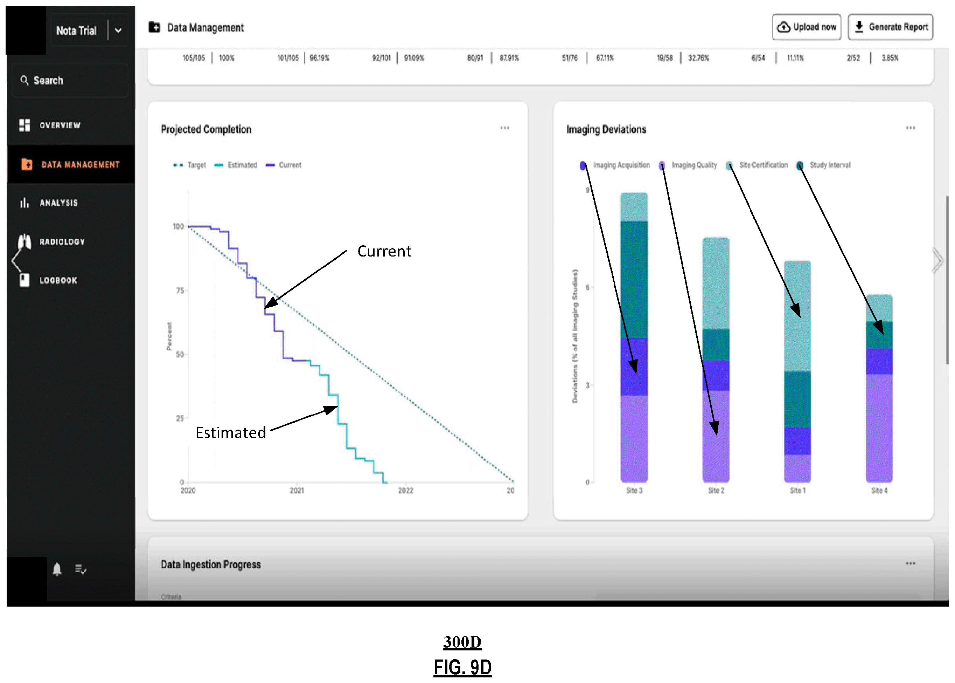

Figure 9D is an illustrative embodiment of a GUI 300D. The GUI 300D can serve as an illustrative embodiment of a user interface that can be accessed or selectively accessed by various devices to provide various information to various individuals, such as patients, healthcare providers, clinical trial managers, pharmaceutical companies, and so forth. For example, based on expected images (per the protocol), the modeling platform can inform or otherwise indicate to the user/viewer when images are expected, as well as a percent completed per timepoint. In this example, the user/viewer can be one or more individuals of the clinical manager, sponsor, or pharmaceutical company associated with management of the clinical trial. GUI 300D provides for projected completion information and further indicates for this example that about 50% of the images have been ingested. GUI 300D also provides information regarding imaging deviations, such as indicating imaging quality or incorrect format. GUI 300D can also indicate what images (or the number thereof) have been uploaded, de-identified, and/or quality controlled.

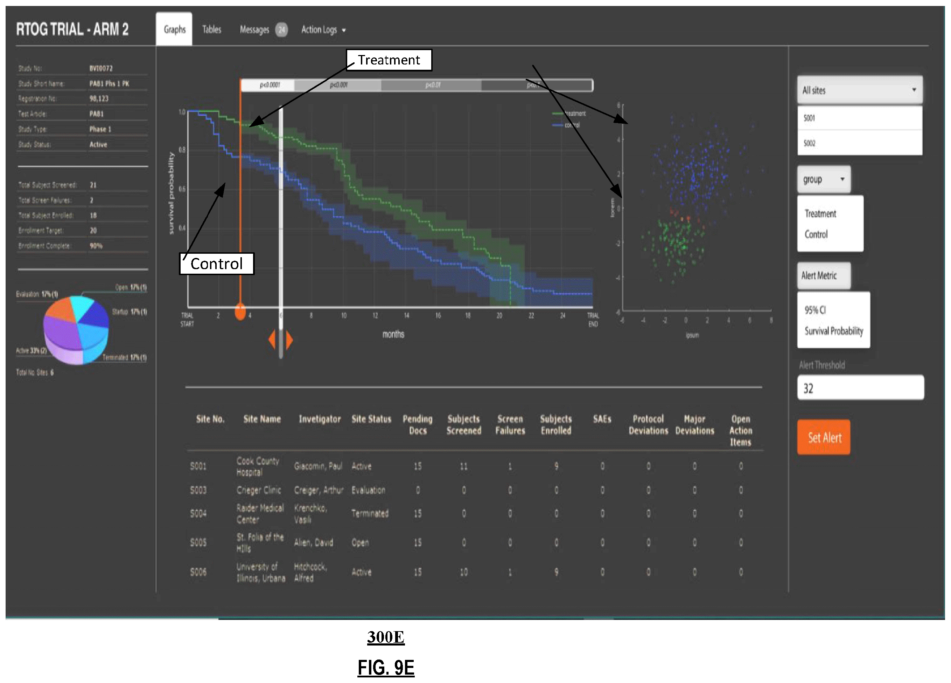

Figure 9E is an illustrative embodiment of a GUI 300E. The GUI 300E can serve as an illustrative embodiment of a user interface that can be accessed or selectively accessed by various devices to provide various information to various individuals, such as patients, healthcare providers, clinical trial managers, pharmaceutical companies, and so forth. For instance, the GUI 300E (e.g., a trial view) can show information indicating the status of the clinical trial, such as subjects screened, screen failures, subjects enrolled, which may be broken down by various criteria such as site names, investigators, and so forth. Other information, including event estimation information, survival data, KM curves, can be generated (according to predictions from applying the image-based models to the images as described herein) and presented.

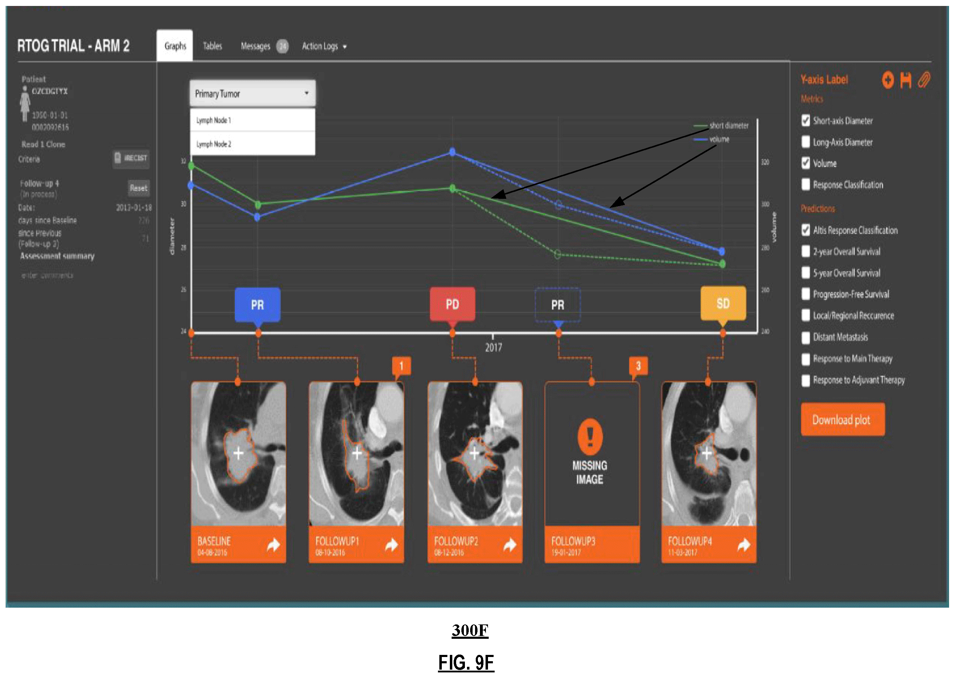

Figure 9F is an illustrative embodiment of a GUI 300F. The GUI 300F can serve as an illustrative embodiment of a user interface that can be accessed or selectively accessed by various devices to provide various information to various individuals, such as patients, healthcare providers, clinical trial managers, pharmaceutical companies, and so forth. In one embodiment, the GUI 300F can be a patient view accessed by one or more of the devices 120, 130, 135 to view patient-specific data that is related to a particular candidate without providing access to a remainder of the graphical user interface (e.g., data of other candidates). In one embodiment, the GUI 300F can include baseline/pre-treatment and follow-up images of the organ or body part that has been utilized by the model for predictions. In one embodiment, the GUI 300F allows for annotations to be made to images and/or provides for automated annotations based on determined points of interest (e.g., points of interest as determined by the image-based model).

In one embodiment, the GUI 300F can include a predicted image(s) of the organ or body part at a future time(s) that is generated based on the image-modeling of the baseline/pre-treatment and/or on-treatment images, and/or based on the predicted variables and/or the predicted on-treatment variables. As an example, the predicted image(s) of the organ or body part at the future time(s) can be generated based on predicted tumor size, predicted tumor shape, predicted growth rate, predicted tumor shape change, and/or predicted tumor location (which can be generated based on the image-modeling of the baseline/pre-treatment and/or on-treatment images). GUI 300F can be used by the healthcare provider to facilitate treatment and treatment decisions for the particular patient as described herein.

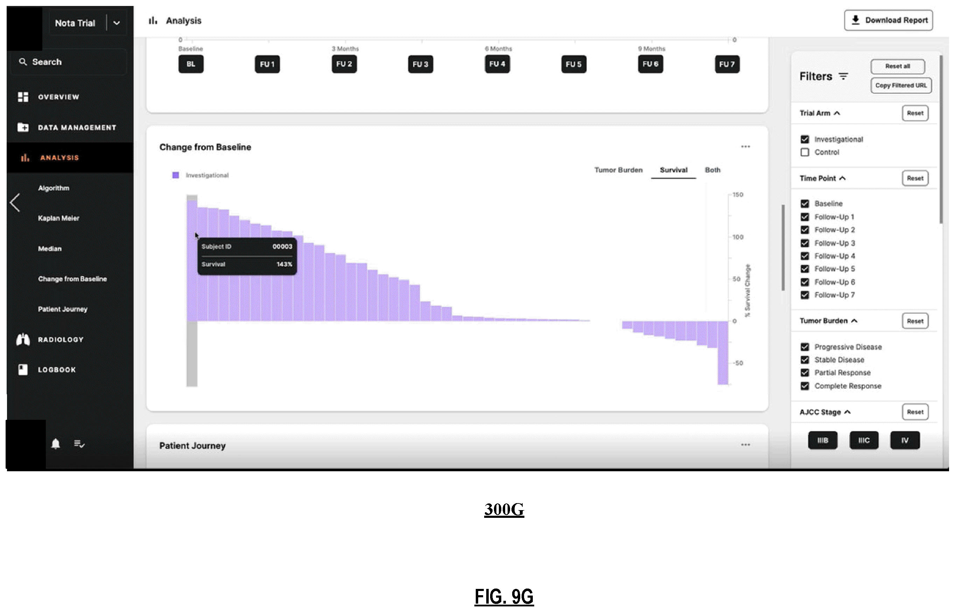

Figure 9G is an illustrative embodiment of a GUI 300G. The GUI 300G can serve as an illustrative embodiment of a user interface that can be accessed or selectively accessed by various devices to provide various information to various individuals, such as patients, healthcare providers, clinical trial managers, pharmaceutical companies, and so forth. GUI 300G provides information regarding changes to predicted survival such as for the investigational arm patients. For example, the subject ID 00003 is predicted to survive 143% longer than their baseline prediction based on applying the image-based model to the most recent image for the patient. GUI 300G can also selectively provide tumor burden information and changes from baseline such as for the investigational arm patients. In one embodiment, GUI 300G can also selectively provide predicted survival information, tumor burden information and/or changes from baseline for the control arm.

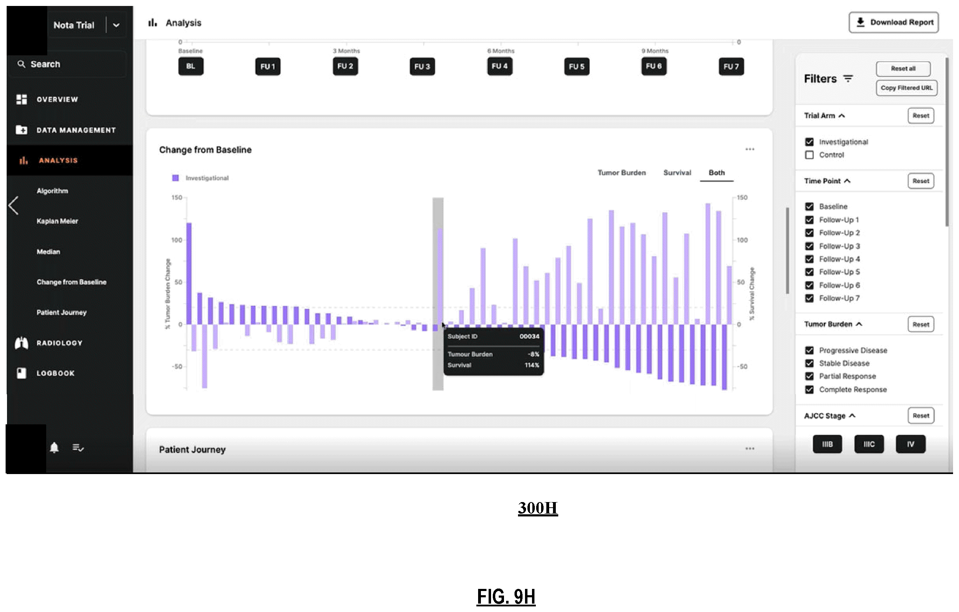

Figure 9H is an illustrative embodiment of a GUI 300H. The GUI 300H can serve as an illustrative embodiment of a user interface that can be accessed or selectively accessed by various devices to provide various information to various individuals, such as patients, healthcare providers, clinical trial managers, pharmaceutical companies, and so forth. GUI 300H provides information regarding changes to both predicted survival and tumor burden, such as for the investigational arm patients. For example, the subject ID 00034 is predicted to survive 114% longer than their baseline prediction and an 8% decrease in tumor burden based on applying the image-based model to the most recent image for the patient. In one or more embodiments, GUI 300H allows access directly into CT scans or other images of the patient whose data is being reviewed. In one embodiment, GUI 300H can also selectively provide predicted survival information, tumor burden information and/or changes from baseline for the control arm.

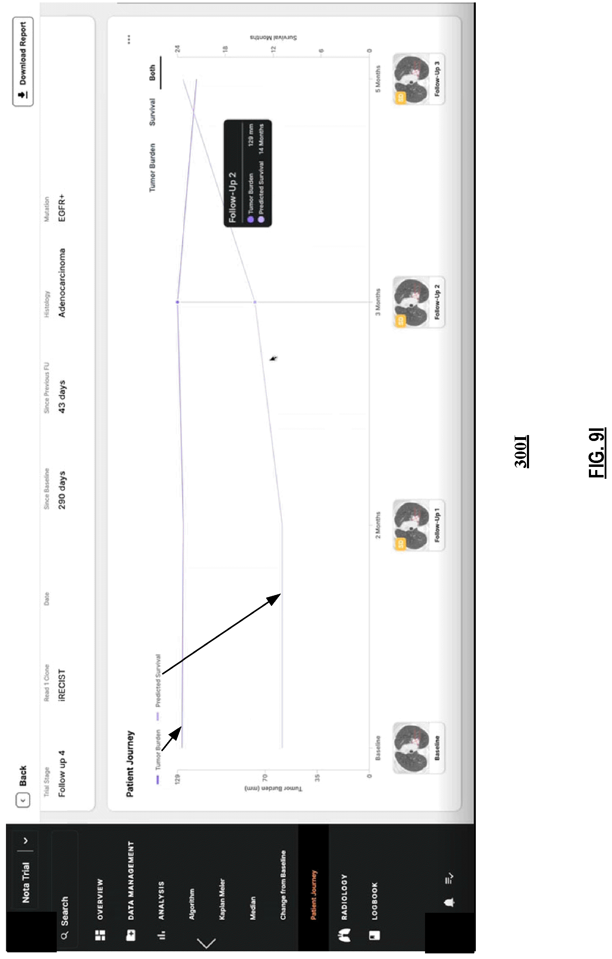

Figure 9I is an illustrative embodiment of a GUI 300I. The GUI 300I can serve as an illustrative embodiment of a user interface that can be accessed or selectively accessed by various devices to provide various information to various individuals, such as patients, healthcare providers, clinical trial managers, pharmaceutical companies, and so forth. GUI 300I provides a patient's journey with relevant data over the treatment time period including tumor burden, baseline/pre-treatment and follow-up images, and survival data. In one embodiment of GUI 300I, portions of the relevant data provided in the patient's journey is predicted data (predicted survival, predicted tumor size, and so forth).

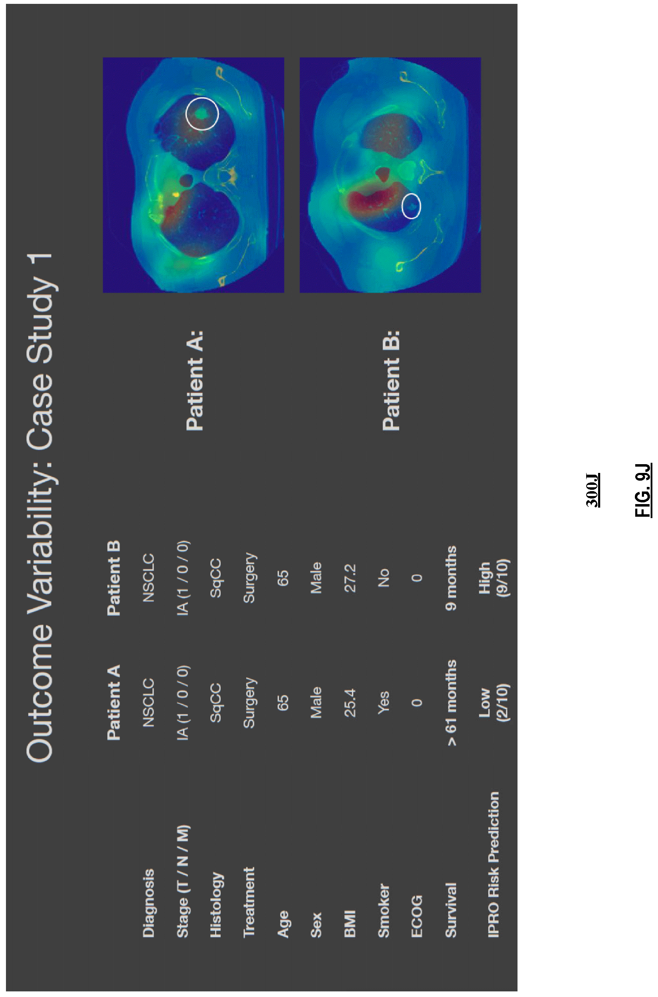

Figure 9J is a case study 300J indicating outcome variability between two patients having similar characteristics (e.g., lung squamous cell carcinoma (SqCC), stage (T/N/M) being 1A (1/0/0), age 65, males, ECOG 0, similar BMI, surgical treatment). However, patient A survived greater than 61 months while patient B survived 9 months. Consistent with the survival data, the image-based model as described herein being applied to baseline/pre-treatment images (activation maps of which are shown in Figure 9J) accurately quantifies risk for patient A as low (2/10) and risk for patient B as high (9/10).

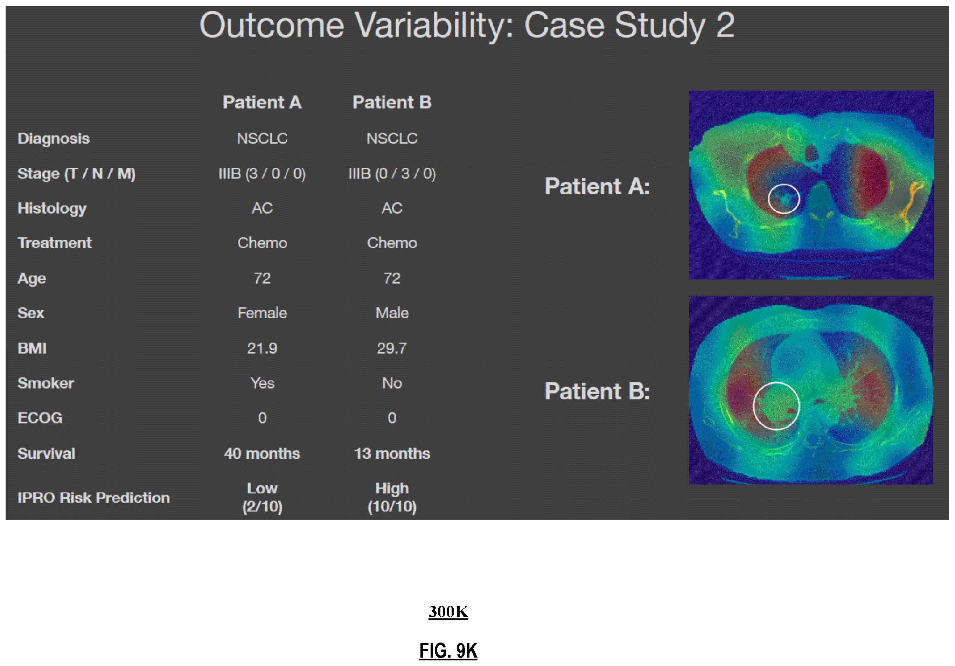

Figure 9K is a case study 300K indicating outcome variability between two patients having similar characteristics (e.g., non-small cell lung cancer (NSCLC), stage IIIB, age 72, ECOG 0, chemotherapy treatment). However, patient A survived 40 months while patient B survived 13 months. Consistent with the survival data, the image-based model as described herein being applied to baseline/pre-treatment images (activation maps of which are shown in Figure 9K) accurately quantifies risk for patient A as low (2/10) and risk for patient B as high (10/10).

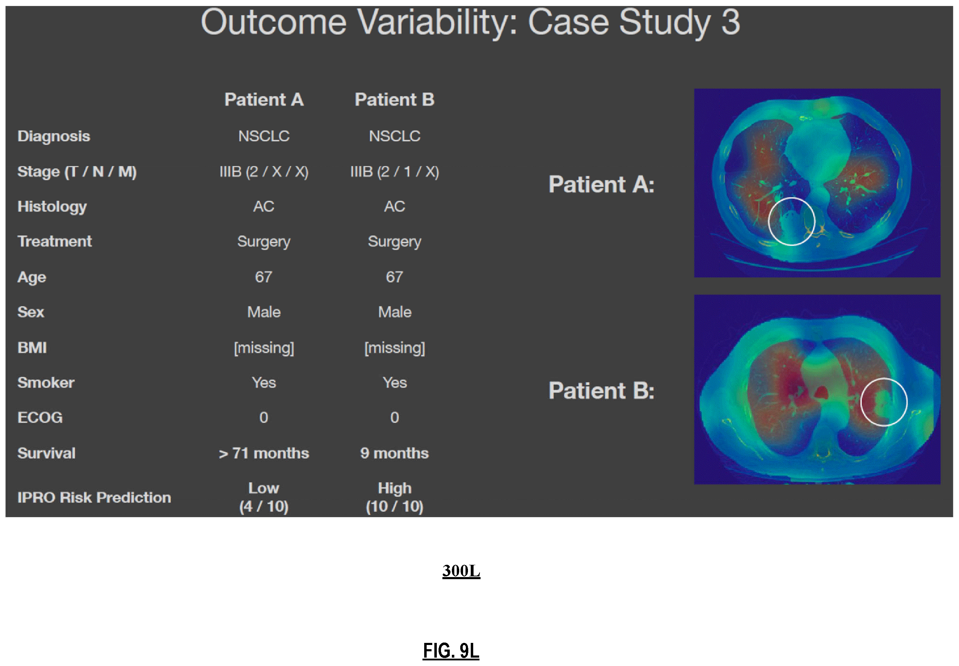

Figure 9L is a case study 300L indicating outcome variability between two patients having similar characteristics (e.g., NSCLC, stage IIIB, age 67, males, smoking history, ECOG 0, chemotherapy treatment). However, patient A survived greater than 71 months while patient B survived 9 months. Consistent with the survival data, the image-based model as described herein being applied to baseline/pre-treatment images (activation maps of which are shown in Figure 9L) accurately quantifies risk for patient A as low (4/10) and risk for patient B as high (10/10).

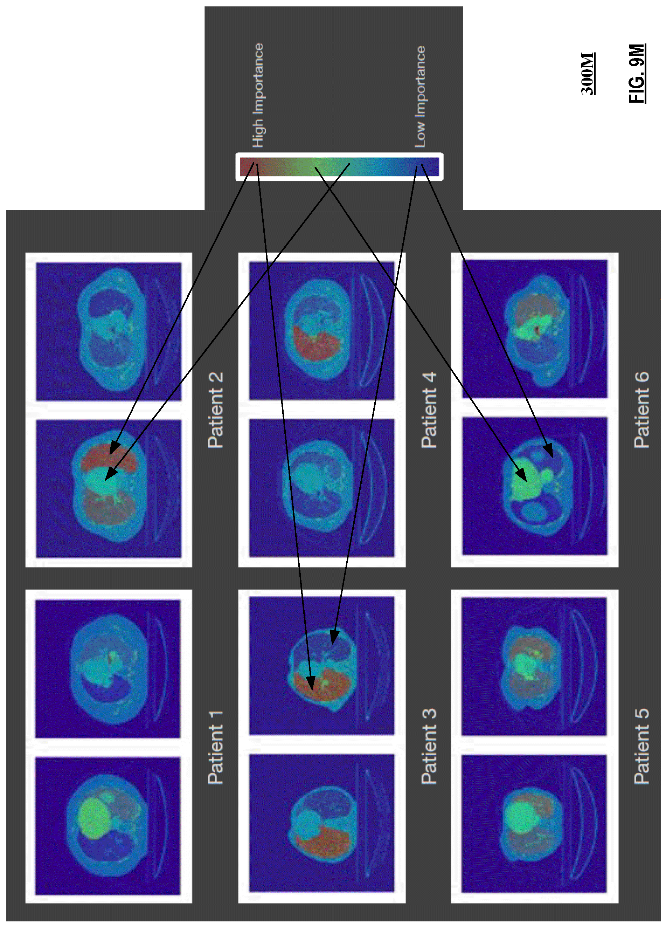

Figure 9M is an illustrative example of attention heatmaps or activation maps generated for different patients where the weighting applied by the exemplary image-based model is determined and indicated for the entire organ rather than for the particular pixels or areas within the organ (see Figure 8K). As explained herein, in one or more embodiments, activation maps can be generated by the modeling platform to indicate organ segmentation illustrating prognostic importance to the image-based model. In this example, the activation maps can indicate that the image-based model has placed attention on the correct organ(s). In other embodiments where the activation maps show weighting for particular pixels or areas within the organ (see e.g., Figure 8K), the activation maps can be generated to indicate that the image-based model has weighted tumors and peritumoral tissue heavily even though the image-based model was not trained to focus on tumors.

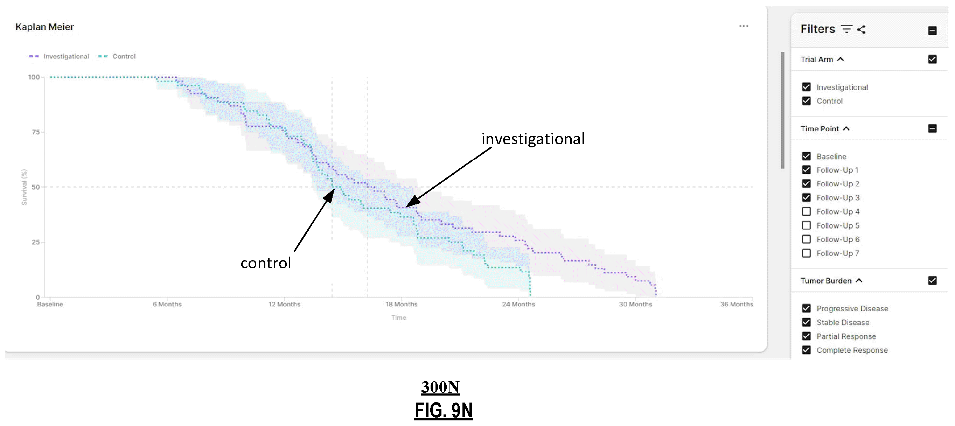

Figure 9N is an illustrative embodiment of a GUI 300N. The GUI 300N can serve as an illustrative embodiment of a user interface that can be accessed or selectively accessed by various devices to provide various information to various individuals, such as patients, healthcare providers, clinical trial managers, pharmaceutical companies, and so forth. In this example, the user/viewer can be one or more individuals of the clinical manager, sponsor, or pharmaceutical company associated with management of the clinical trial. Continuing with this example, the clinical trial has already commenced. Based on the KM curves for the investigational arm and the control arm (which have been generated according to the application of the image-based model to the baseline/pre-treatment CT scans (which is indicated by the selection mark for the baseline button under Time Point)), an imbalance in the clinical trial exists.

In some instances, a KM curve can indicate that the control arm is predicted to survive longer than the treatment arm, can occur as a result of an imperfect or erroneous randomization (e.g., healthier patients were utilized in the control arm as compared to the investigational arm). GUI 300N allows quantification and/or visualization of the error in randomization (e.g., the difference between the KM curves such as at baseline). This quantification allows the clinical managers or other entities looking at the data to better understand the results, such as at the end of the trial, when comparing the actual survival of the treatment arm and the control arm, so that the imbalance can be taken into account. As described with respect to process 201E of Figure 8E, the modeling platform also prevents or reduces such error by allowing for balanced randomization such that the investigational arm and the control arm can be properly balanced according to the predictions from application of the image-based model to the baseline/pre-treatment CT scans.

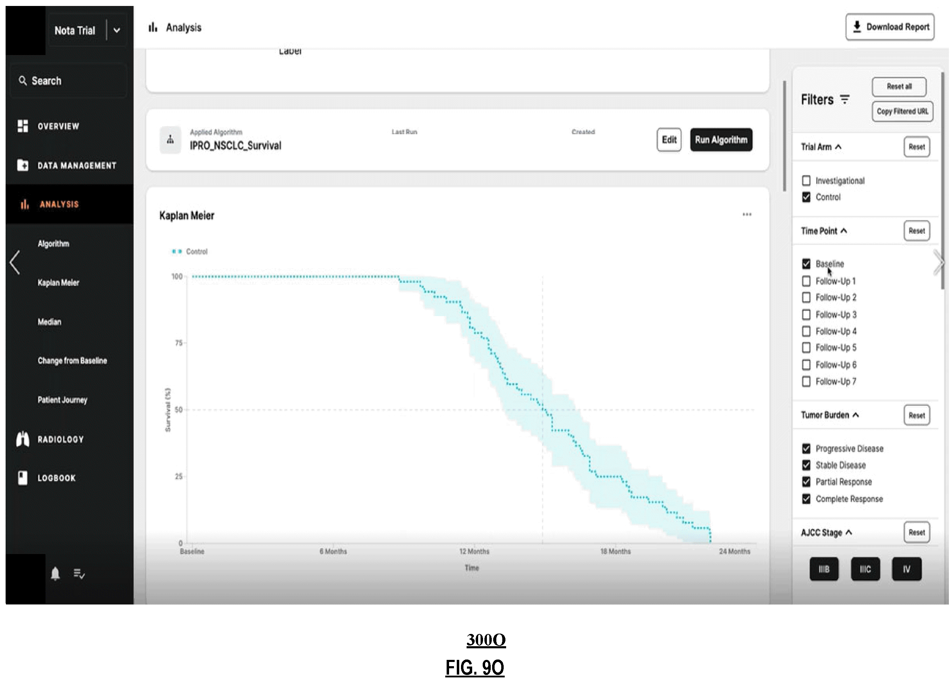

Figures 9O-9Q are illustrative embodiments of GUIs 300O, 300P, 300Q. The GUIs 300O, 300P, 300Q can serve as illustrative embodiments of user interfaces that can be accessed or selectively accessed by various devices to provide various information to various individuals, such as patients, healthcare providers, clinical trial managers, pharmaceutical companies, and so forth. GUI 300O shows a KM curve generated for the control arm according to predictions made from the application of the image-based model to the baseline/pre-treatment CT scans. As more follow-up scans are obtained and ingested, the predictions of the control arm can be updated (according to the application of the image-based model to the most recent follow-up CT scans) and the KM curves will then adjust or change as illustrated by the differences in the KM curves presented by GUI 300P (i.e., follow-up images five) as compared to GUI 300Q (follow-up images seven). Other types of event estimation information can be generated or otherwise predicted including time-to-event information, survival data, and so forth.

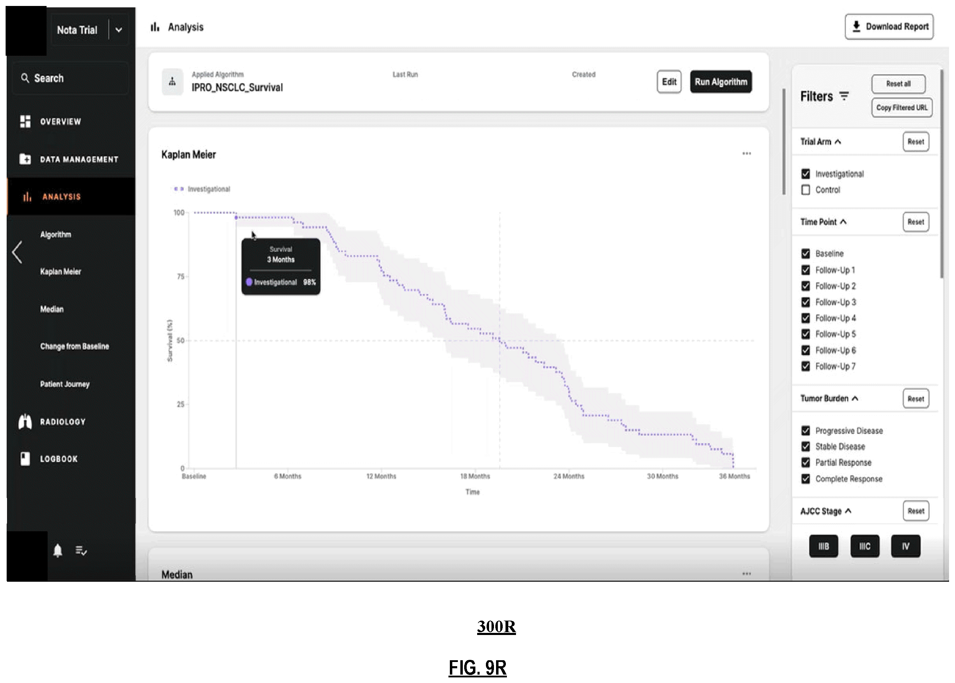

Figure 9R is an illustrative embodiment of a GUI 300R. The GUI 300R can serve as an illustrative embodiment of a user interface that can be accessed or selectively accessed by various devices to provide various information to various individuals, such as patients, healthcare providers, clinical trial managers, pharmaceutical companies, and so forth. As an example, GUI 300R can be a trial view that provides access to event estimation information such as KM curves (as well as other generated data). In this example, the KM curves are generated according to predictions that are determined by the image-based model applied to the most recent follow-up image for each patient of the clinical trial. The selection of the investigational arm causes the GUI 300R to present the KM curve for the investigational arm that was generated according to predictions made from the image-based model as applied to the follow-up seven images.

In one or more embodiments, one, some, or all of the functions described herein can be performed in conjunction with a virtualized communication network. For example, a virtualized communication network can facilitate in whole or in part providing image-based modeling and a modeling platform to assist in clinical trials, healthcare treatment or other health-related events, such as through presenting predictive variables for a treatment at different future time periods. In particular, a cloud networking architecture can leverage cloud technologies and support rapid innovation and scalability such as via a transport layer, a virtualized network function cloud and/or one or more cloud computing environments. In various embodiments, this cloud networking architecture can be an open architecture that leverages application programming interfaces (APIs); reduces complexity from services and operations; supports more nimble business models; and rapidly and seamlessly scales to meet evolving customer requirements including traffic growth, diversity of traffic types, and diversity of performance and reliability expectations. For example, the virtualized communication network can employ virtual network elements (VNEs) that perform some or all of the functions of traditional network elements such as providing a substrate of networking capability, (e.g., Network Function Virtualization Infrastructure (NFVI)) or infrastructure that is capable of being directed with software and Software Defined Networking (SDN) protocols to perform a broad variety of network functions and services.

As described previously, any of the functions performed by one or more subsystems 501 and/or any of the functions with corresponding function entries 571 of the function library 572 such as functions that correspond to medical outcome prognostication function or other outcome prediction functions, medical outcome prognostication model training functions or other model training functions; trial arm assignment functions and/or corresponding randomization algorithms; and/or any other functions described herein; can utilize, correspond to, and/or be based on artificial intelligence algorithms and/or techniques, and/or machine learning algorithms and/or techniques.

Some or all of these functions discussed herein cannot be practically trained by the human mind. Instead, such functions and/or corresponding models can be trained based on applying artificial intelligence algorithms and/or techniques, and/or machine learning algorithms and/or techniques, to a training set of medical data and/or corresponding feature vectors. Alternatively or in addition, training some or all medical scan analysis functions cannot be practically be trained by the human mind based upon: a great complexity of the resulting function and/or corresponding model; a large size of the training set utilized to train the function and/or model (e.g. the training set includes hundreds or thousands of training data to be processed); a large size of each training data in the training set (e.g. a feature set for each training data in the training set includes dozens, hundreds, or thousands of values); the model taking an infeasibly long amount of time to train utilizing only pen and paper; and/or other reasons.

Some or all of these functions discussed herein cannot be practically performed by the human mind. Instead, such functions can be performed utilizing artificial intelligence algorithms and/or techniques, and/or machine learning algorithms and/or techniques. Alternatively or in addition, such functions can be performed utilizing a model trained utilizing artificial intelligence algorithms and/or techniques, and/or machine learning algorithms and/or techniques. Alternatively or in addition, performing some or all functions cannot be practically performed by the human mind based upon: a great complexity of these functions; an accuracy rate and/or consistency of generating output being more favorable than that of a human; a speed of generating output being more favorable than that of a human; input to these functions including a feature set that includes hundreds and/or thousands of values to be processed; these functions taking an infeasibly long amount of time to perform utilizing only pen and paper; and/or other reasons.

As described previously, the medical data processing system 500 and/or scientific data processing system 1500 is operable to generate various data in conjunction with performing functionality of one or more subsystems 501, such as: generating tuned parameter data defining a machine learning model via training of the machine learning model (e.g. of a medical outcome prognostication function, a medical findings detection function, an outcome prediction function, etc.); generating medical outcome prognostication scores and/or outcome prediction scores as a function of corresponding medical data and/or scientific data; generating trial arm assignment data via execution of a corresponding randomization algorithm; and/or data generated as described herein.

Some or all of this data can be generated, and/or some or all functions of function library can be performed within a short time frame, such as within a single hour, a single minute, single second, single millisecond, and/or single microsecond. For example, the medical data processing system 500 generates, within the short time, one or more of the various data described herein for a given one or more requests, such as a requests to process data corresponding to: given medical data; a given individual (e.g. patient, clinical trial participant), a given training set; and/or a given clinical data. As another example, the medical data processing system 500 performs one or more given functions of function library 572 within the short time frame.

Generating one or more of the various types of data described herein for a given request within a short time frame, and/or performing one or more of the various functions of function library within the short time frame, cannot feasibly be performed by the human mind, for example based upon: the human mind not being able to feasibly perform one or more given functions of function library within the short time frame with an accuracy of output and/or consistency of output attained by the medical scan processing system 500; the human mind not being able to feasibly perform one or more given functions of function library within the short time frame due to the computational complexity of performing these functions; the human mind not being able to feasibly perform one or more given functions of function library within the short time frame due to processing complexity of processing a large required amount of input data to each function, such as a large number of responses and/or large amount of medical data; the human mind not being able to feasibly perform one or more given functions of function library within the short time frame due to searching for and/or retrieval of the appropriate input data from a large amount data, such as hundreds, thousands, and/or millions of different medical accounts; the human mind not being able one or more given functions within the short time frame utilizing only pen and paper; the human mind not being able to feasibly generate one or more of the various data described herein within the same short time frame utilizing only pen and paper; and/or other reasons.

Furthermore, as described previously, the medical scan processing system 500 is operable to generate some or all of this various data for multiple different requests, such as requests corresponding to different medical data, different training sets, different individuals (e.g. different patients; different prospective clinical trial participants), and/or different clinical trials. In some cases, the medical data processing system 500 and/or scientific data processing system 1500 receives requests from and/or otherwise determines to generate data for, multiple different users, such as dozens, hundreds, and/or thousands of users within a same, short time frame, such as a same second, a same minute, a same hour, or other short time frame.

The medical data processing system 500 and/or scientific data processing system 1500 can optionally implement its various processing resources of processing module 520, and/or of a given subsystem processing module 521, to generate data for multiple different users within the same time frame in a parallelized fashion, and/or to perform other functionality described herein for multiple different users within the same time frame in a parallelized fashion. For example, the medical data processing system 500 generates data for multiple different requests/medical data/portions of medical data within the same time frame as multiple processes being performed in parallel via distinct and/or shared processing resources within the same time frame. As another example, the medical data processing system 500 and/or scientific data processing system 1500 performs functions of function library 572 for multiple different requests/medical data/portions of medical data within the same time frame as multiple processes being performed in parallel via distinct and/or shared processing resources within the same time frame. As a particular example, within a given microsecond, millisecond, second, minute, hour or other short time frame, the medical data processing system 500 can perform multiple different functions of function library and/or can generate multiple different data for multiple different requests/medical data/portions of medical data in parallel as dozens, hundreds, and/or thousands of parallel processes.

The medical data processing system 500 and/or scientific data processing system 1500 can alternatively or additionally implement processing resources of processing module 520, and/or a given subsystem processing module 521, to generate data for multiple different requests/medical data/portions of medical data within the short time frame in a serialized fashion, and/or to perform other functionality described herein for multiple different requests/medical data/portions of medical data within the short time frame in a serialized fashion. For example, the medical data processing system 500 generates data for multiple different users within the short time frame as multiple processes being performed in serially within a short time frame. As another example, the medical data processing system 500 and/or scientific data processing system 1500 performs functions of function library 572 for multiple different requests/medical data/portions of medical data within the short time frame as multiple processes being performed serially within the short time frame. As a particular example, within a given microsecond, millisecond, second, minute, hour, or other short time frame, the medical data processing system 500 and/or scientific data processing system 1500 can perform multiple different functions of function library and/or can generate multiple different data for multiple different requests/medical data/portions of medical data serially as dozens, hundreds, and/or thousands of processes performed one at a time.

Generating data for multiple different users within the same time frame in a parallelized fashion or a serialized fashion, and/or performing functions for multiple different requests/medical data/portions of medical data within the same time frame as multiple processes being performed in parallel and/or serially, cannot feasibly be performed by the human mind, for example based upon: the human mind not being able to feasibly perform multiple functions of function library in parallel and/or within the short time frame with an accuracy of output and/or consistency of output attained by the medical data processing system 500 and/or scientific data processing system 1500; the human mind not being able to feasibly perform multiple functions of function library in parallel due to the computational complexity of performing these functions; the human mind not being able to feasibly perform multiple functions of function library in parallel due to processing complexity of processing a large required amount of input data to each function, such as a large number of responses and/or large amount of data portions in medical data; the human mind not being able to feasibly perform dozens, hundreds, and/or thousands of the functions of function library in parallel; the human mind not being able to feasibly generate dozens, hundreds, and/or thousands of one or more types of the various data described herein in parallel; the human mind not being able to feasibly perform dozens, hundreds, and/or thousands of these functions within a same microsecond, same millisecond, same second, same minute, same hour, or other short time frame; the human mind not being able to feasibly generate dozens, hundreds, and/or thousands of the one or more types of the various data described herein within a same microsecond, same millisecond, same second, a same minute, same hour, or other short time frame; the human mind not being able to feasibly perform dozens, hundreds, and/or thousands of these functions within the same short time frame utilizing only pen and paper; the human mind not being able to feasibly generate dozens, hundreds, and/or thousands of the various data described herein within the same short time frame utilizing only pen and paper; and/or other reasons.

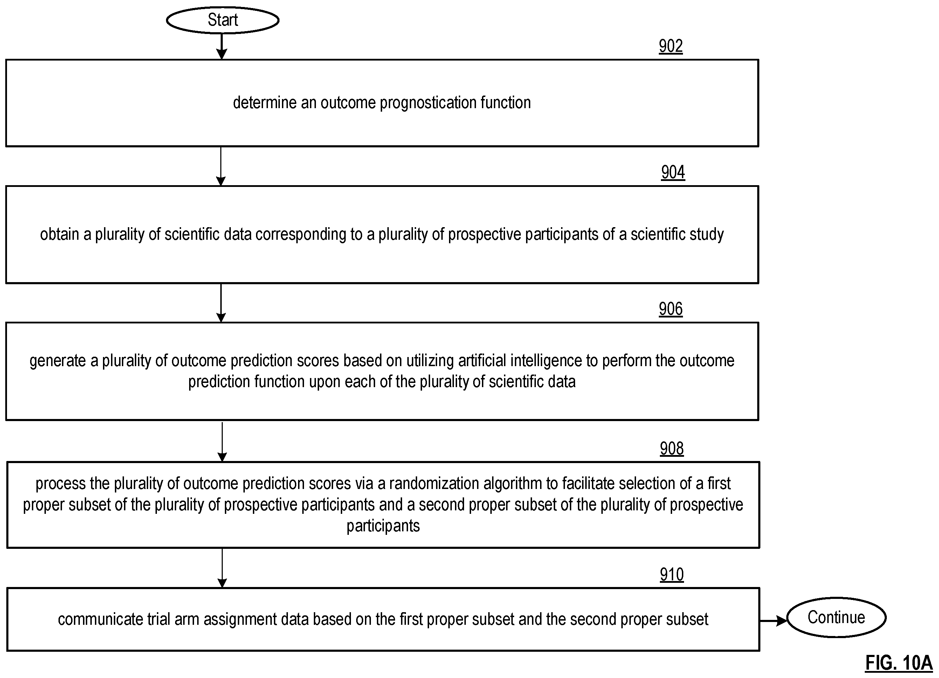

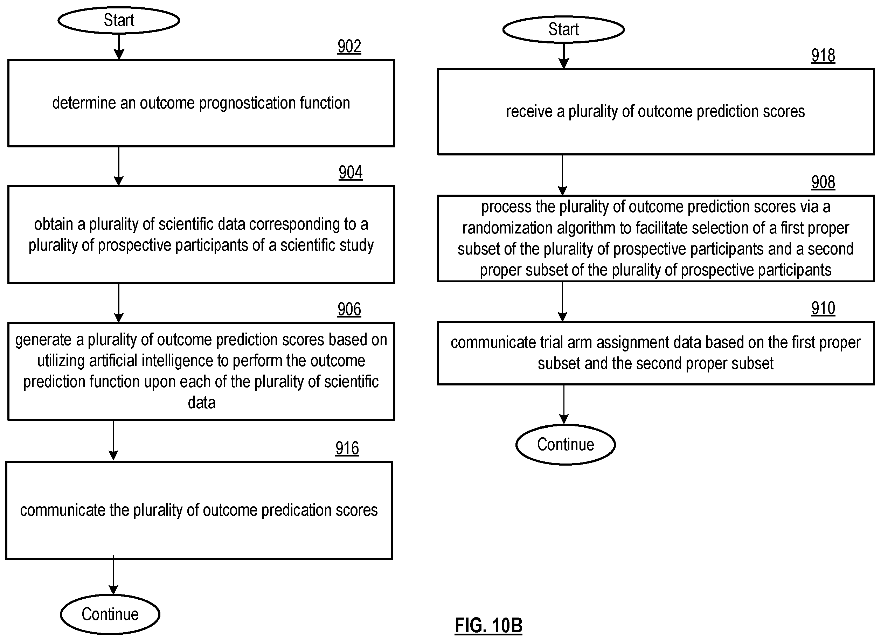

Figures 10A - 10B illustrate methods for execution by at least one processor. For example, some or all of Figure 10A and/or 10B can be executed via scientific data processing system 1500 and/or based on communicating with a scientific data processing system 1500. For example, some or all of the steps of Figure 10A and/or 10B can be performed in conjunction with implementing some or all features and/or functionality of the scientific data processing system of Figures 5A - 5H. Alternatively or in addition, some or all of the steps of Figure 10A and/or 10B can be performed in conjunction with implementing some or all features and/or functionality of any embodiment of the outcome prediction model training system 1506; the outcome prediction system 1507; and/or the prediction-based trial arm assignment system 1508 described herein. Alternatively or in addition, some or all of the steps of Figure 10A and/or 10B can be performed in conjunction with implementing some or all features and/or functionality of the trial arm assignment module 730 and/or the randomization algorithm 800 of one or more of Figures 6A - 6K, and/or any embodiment of trial arm assignment module 730 and/or the randomization algorithm 800 described herein. Alternatively or in addition, some or all of the steps of Figure 10A can be performed in conjunction by and/or via communication with one or more data sources 575, one or more requesting entities 576, and/or one or more scientific study conducting processing systems 1597 of one or more scientific study conducting entities 1577. Alternatively or in addition, some or all of the steps of Figure 10A and/or 10B can be performed in conjunction with the system 100 of Figure 1B, for example, in conjunction with implementing the imaging-based prognostication (IPRO) framework 200A and/or at least one corresponding model 112, for example, in conjunction with some or all features and/or functionality presented in Figure 8A and/or the example embodiments of Figures 8B - 9R. Alternatively or in addition, some or all of the steps of Figure 10A and/or 10B can be performed by, and/or based on communications between, any one or more computing devices 700, any one or more corresponding server systems, and/or any processing system that includes at least one processor and/or at least one memory.

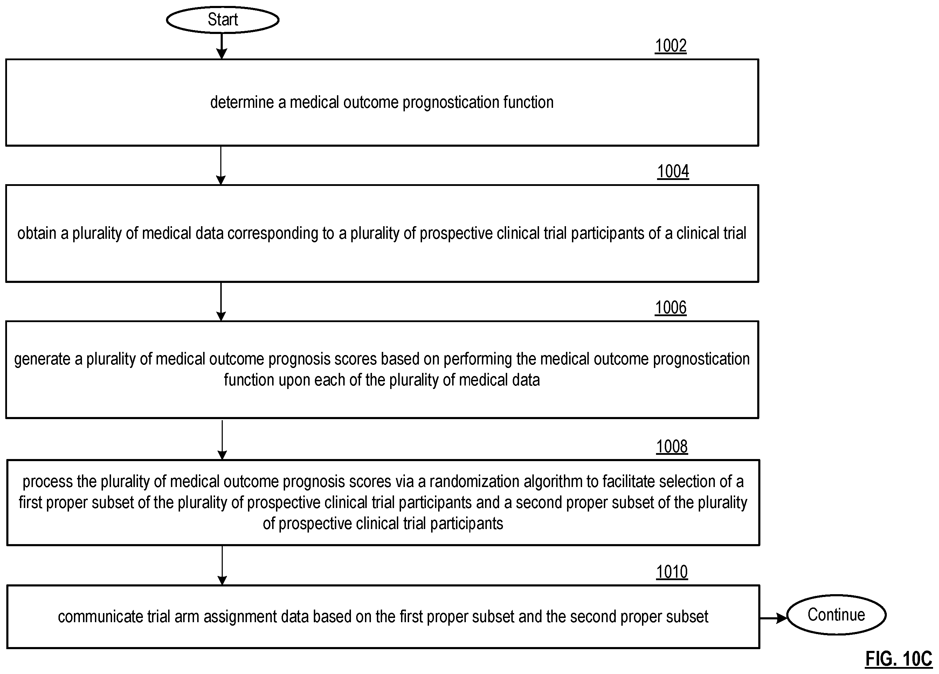

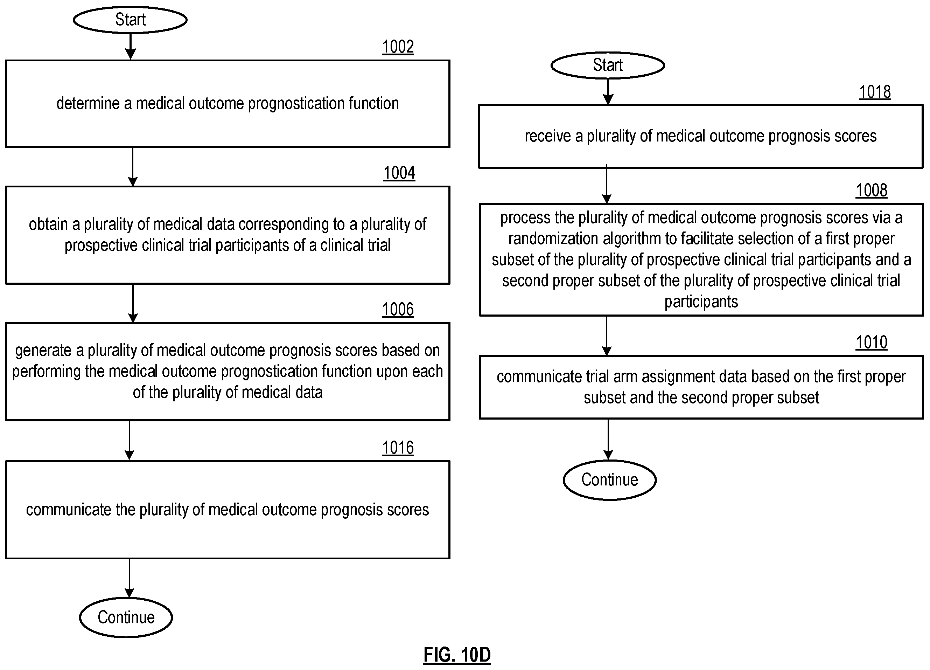

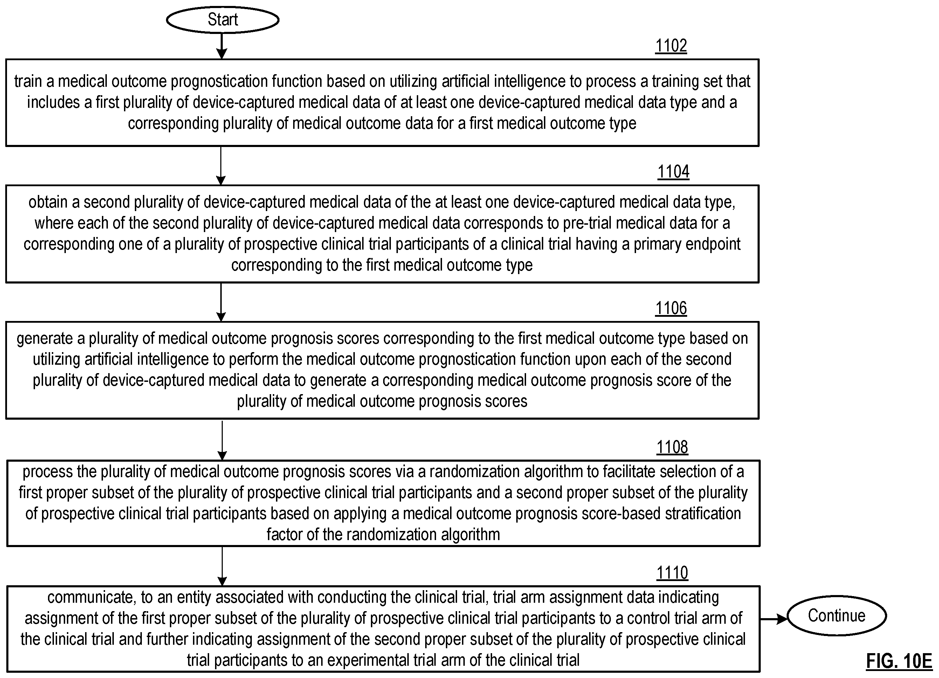

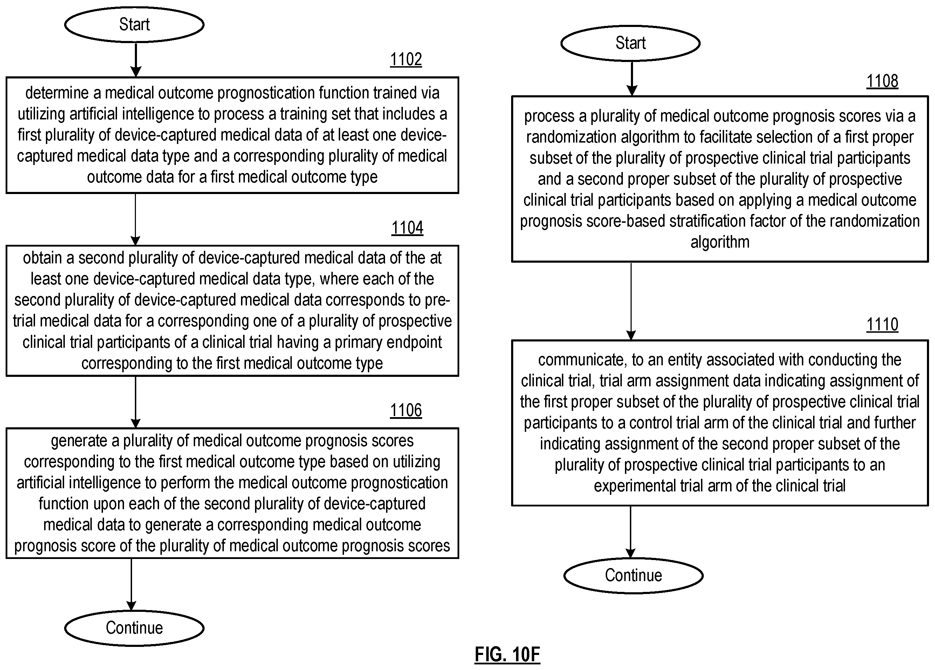

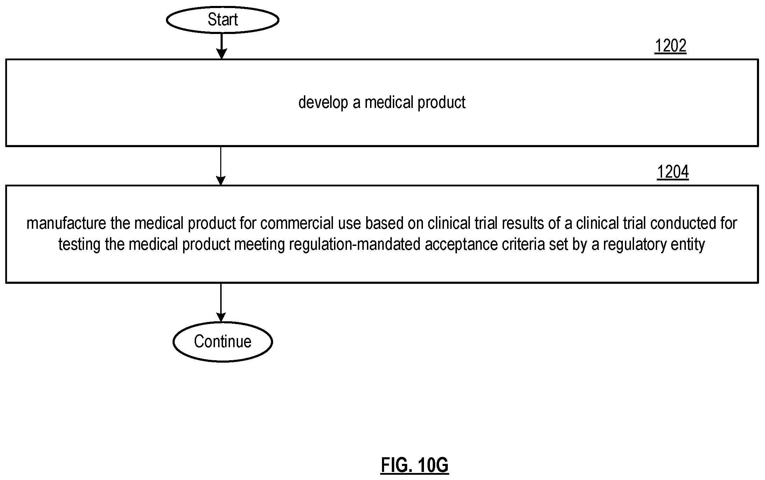

Some or all steps of the method of Figure 10A and/or 10B can performed in conjunction with performance and/or can be implemented based on performing one or more steps of any other method described herein, such as any method of Figures 8B - 8G, the method of Figure 10C, one of both methods of Figure 10D, the method of Figure 10E, one or both methods of Figure 10F, and/or the method of Figure 10G.

Step 902 includes determining an outcome prediction function. In some or all embodiments, outcome prediction function of step 902 can be implemented via some or all features and/or functionality of any embodiment of outcome prediction function 1671 described herein. In some or all embodiments, outcome prediction function of step 902 can be implemented via some or all features and/or functionality of any embodiment of model 112 described herein.

Alternatively to or in addition to any of the foregoing, step 902 is performed via implementing some or all features and/or functionality of outcome prediction model training system 1506 to train the outcome prediction function, for example, based on tuning parameters of corresponding model parameter data 661. Alternatively to or in addition to any of the foregoing, the outcome prediction function of step 902 can be determined via any other means (e.g. based on being accessed in memory, being received via the network, being generated/trained by another entity, based on being automatically trained and/or generated, for example, as a step of the method of Figure 10A, based on being configured based on user input, based on having being previously trained via a separate entity, and/or based on otherwise being determined).

Alternatively to or in addition to any of the foregoing, the outcome prediction function determined step 902 was trained based on utilizing artificial intelligence. Alternatively to or in addition to any of the foregoing, the method further includes training the outcome prediction function, for example, based on utilizing artificial intelligence to process a training set. Alternatively to or in addition to any of the foregoing, the training set includes a first plurality of scientific data of at least one scientific data type. Alternatively to or in addition to any of the foregoing, the first plurality of scientific data is a first plurality of device-captured scientific data (e.g. based on being generated/captured by/in conjunction with use of a corresponding scientific instrument/device/equipment device), and/or the at least one scientific type is at least one device-captured scientific data type. Alternatively to or in addition to any of the foregoing, training set includes a corresponding plurality of scientific outcome data for a first outcome type.

Alternatively to or in addition to any of the foregoing, some or all of first plurality of scientific data can be implemented via any embodiment of scientific data 1562 described herein, and can optionally be implemented as medical data or non-medical data. Alternatively to or in addition to any of the foregoing, some or all of the first plurality of scientific data can include a plurality of values 601. Alternatively to or in addition to any of the foregoing, some or all of the corresponding plurality of outcome data can be implemented via any embodiment of outcome data 638 described herein. Alternatively to or in addition to any of the foregoing, some or all of the first outcome type can be implemented via any embodiment of outcome type 1637 described herein.

Alternatively to or in addition to any of the foregoing, the first plurality of scientific data and/or the corresponding plurality of outcome data can be included in the training set based on being received from a data source 575. Alternatively to or in addition to any of the foregoing, the first plurality of scientific data and/or the corresponding plurality of outcome data can be included in the training set based on being stored in, accessed via, and/or received from a data storage system, such as scientific data storage 1561.

Alternatively to or in addition to any of the foregoing, the method further includes requesting the first plurality of scientific data of the training set and/or further includes requesting the corresponding plurality of outcome data (e.g. via generating a corresponding at least one request and/or via sending to the at least one request to a data storage system or a data source), where the first plurality of scientific data and/or the corresponding plurality of outcome data of the training set is obtained in response to the at least one request. Alternatively to or in addition to any of the foregoing, requesting the plurality of scientific data is based on configuring at least one request for scientific data meeting a set of parameters, where the first plurality of scientific data meets the set of parameters based on the request. Alternatively to or in addition to any of the foregoing, the set of parameters indicates a corresponding scientific data type, for example, where the first plurality of scientific data is of the at least one device-captured scientific data type based on the at least one request for the first plurality of scientific data and/or the corresponding plurality of outcome data indicating the at least one device-captured scientific data type. Alternatively to or in addition to any of the foregoing, the set of parameters indicates the first scientific outcome type, where the corresponding plurality of scientific outcome data corresponds to the first outcome type based on the at least one request for the first plurality of scientific data and/or the corresponding plurality of outcome data indicating the first scientific outcome type.

Step 904 includes obtaining a plurality of scientific data corresponding to a plurality of prospective participants of a scientific study. Alternatively to or in addition to any of the foregoing, the plurality of scientific data can be obtained based on being received from a data source 575. Alternatively to or in addition to any of the foregoing, the plurality of scientific data can be obtained based on being stored in, accessed via, and/or received from a data storage system, such as scientific data storage 1561.

Alternatively to or in addition to any of the foregoing, the plurality of scientific data obtained in step 904 is a second plurality of scientific data (e.g. distinct from the first plurality of scientific data utilized to train the outcome prediction function). Alternatively to or in addition to any of the foregoing, the plurality of scientific data is a second-plurality of device-captured scientific data of the at least one device-captured scientific data type (e.g. based on being generated/captured by/in conjunction with use of a corresponding scientific device/instrument). Alternatively to or in addition to any of the foregoing, each of the plurality of scientific data obtained in step 904 corresponds to pre-trial scientific data for a corresponding one of the plurality of prospective participants of the scientific study. Alternatively to or in addition to any of the foregoing, the scientific study has a primary endpoint corresponding to the first outcome type, for example, of the corresponding plurality of outcome data of the training set utilized to train the outcome prediction function determined in step 904.

Alternatively to or in addition to any of the foregoing, the method further includes requesting the plurality of scientific data of step 904 (e.g. via generating a corresponding at least one request and/or via sending to the at least one request to a data storage system or a data source), where the plurality of scientific data is obtained in response to the request. Alternatively to or in addition to any of the foregoing, requesting the plurality of scientific data is based on configuring at least one request for scientific data meeting a set of parameters, where the plurality of scientific data obtained in step 1004 meets the set of parameters based on the at least one request. Alternatively to or in addition to any of the foregoing, the set of parameters indicates the at least one device-captured scientific data type, for example, where the plurality of scientific data obtained in step 1004 is of the at least one device-captured scientific data type based on the at least one request for the plurality of scientific data indicating the at least one device-captured scientific data type. Alternatively to or in addition to any of the foregoing, the set of parameters indicates the plurality of prospective clinical trial participants (e.g. based on patient identifiers of the plurality of prospective clinical trial participants), for example, where the plurality of scientific data obtained in step 904 corresponds to the plurality of prospective participants based on the at least one request for the plurality of scientific data indicating the plurality of prospective participants. Alternatively to or in addition to any of the foregoing, the set of parameters indicates the clinical trial (e.g. based on a clinical trial identifier of the clinical trial), for example, where the plurality of scientific data obtained in step 904 corresponds to the plurality of prospective participants of the scientific study based on the at least one request for the plurality of scientific data indicating the scientific study.

Step 906 includes generating a plurality of outcome prediction scores corresponding to the first outcome type based on performing the outcome prediction function each of the plurality of scientific data, for example, based on the plurality of scientific data being obtained in step 906 and/or based on the outcome prediction function being determined in step 902. Alternatively to or in addition to any of the foregoing, step 906 is performed via implementing some or all features and/or functionality of outcome prediction system 1507 to perform the outcome prediction function.

Alternatively to or in addition to any of the foregoing, the plurality of outcome prediction scores correspond to the first outcome type, for example, of the corresponding plurality of outcome data of the training set utilized to train the outcome prediction function determined in step 902, and/or corresponding to the primary endpoint of the scientific study. Alternatively to or in addition to any of the foregoing, the outcome prediction function is performed in step 906 based on utilizing artificial intelligence. Alternatively to or in addition to any of the foregoing, the outcome prediction function is performed upon each of the plurality of scientific data (e.g. implemented as a second plurality of scientific data, such as a second plurality of device-captured scientific data) to generate a corresponding outcome prediction score of the plurality of outcome prediction scores. Alternatively to or in addition to any of the foregoing, each outcome prediction score of the plurality of outcome prediction scores corresponds to one of the plurality of prospective participants.

Step 908 includes processing the plurality of outcome prediction scores via a randomization algorithm to facilitate selection of a first proper subset of the plurality of prospective participants and a second proper subset of the plurality of prospective participants.

Alternatively to or in addition to any of the foregoing, facilitating selection of the first proper subset of the plurality of prospective participants and the second proper subset of the plurality of prospective participants includes automatically selecting the first proper subset of the plurality of prospective participants and the second proper subset of the plurality of prospective participants. Alternatively to or in addition to any of the foregoing, facilitating selection of the first proper subset of the plurality of prospective participants and the second proper subset of the plurality of prospective participants is based on providing the plurality of outcome prediction scores for use in a randomized selection process that applies the randomization algorithm, where this a randomized selection process is optionally performed: via an automated process; via at least one human; or via a combination of automated processes and human-conducted processes, for example, intervention with at least one automated process.

Alternatively to or in addition to any of the foregoing, the randomization algorithm is implemented via some or all features and/or functionality of any embodiment of the randomization algorithm 800 described herein. Alternatively to or in addition to any of the foregoing, step 908 is performed via implementing some or all features and/or functionality of prediction-based trial arm assignment system 1508 described herein and/or any embodiment of trial arm assignment module 730 described herein.

Alternatively to or in addition to any of the foregoing, the first proper subset and the second proper subset are mutually exclusive. Alternatively to or in addition to any of the foregoing, the first proper subset and the second proper subset are collectively exhaustive with respect to the plurality of prospective participants. Alternatively to or in addition to any of the foregoing, at least one of the plurality of prospective clinical trial participants is not included in the first proper subset or the second proper subset.

Alternatively to or in addition to any of the foregoing, performing step 908 includes processing the plurality of outcome prediction scores via the randomization algorithm to automatically select a plurality of proper subsets of the plurality of prospective clinical trial participants that includes only the first proper subset and the second proper subset. Alternatively to or in addition to any of the foregoing, performing step 908 includes processing the plurality of medical outcome prognosis scores via the randomization algorithm to automatically select a plurality of proper subsets of the plurality of prospective clinical trial participants that includes the first proper subset, the second proper subset, and at least one additional proper subset.

Alternatively to or in addition to any of the foregoing, processing the plurality of outcome prediction scores via the randomization algorithm is based on applying an outcome prediction score-based stratification factor of the randomization algorithm. Alternatively to or in addition to any of the foregoing, applying the outcome prediction score-based stratification factor includes implementing any embodiment of stratification process 810 described herein, for example, by performing any embodiment of stratification function 820 described herein.

Step 910 includes communicating trial arm assignment data based on the first proper subset and the second proper subset. Alternatively to or in addition to any of the foregoing, the trial arm assignment data is communicated to an entity associated with conducting the scientific study. Alternatively to or in addition to any of the foregoing, the trial arm assignment data is communicated to a requesting entity 575, and/or a scientific study conducting processing system 1597 of a scientific study conducting entity 1577. Alternatively to or in addition to any of the foregoing, communicating the trial arm assignment data includes at least one of: transmitting or otherwise sending the trial arm assignment data (e.g. to the entity associated with conducting the clinical trial); displaying the trial arm assignment data (e.g. via a display device of a computing device associated with the entity associated with conducting the scientific study); and/or storing the trial arm assignment data (e.g. in scientific study storage 1565 and/or other storage resources).

Alternatively to or in addition to any of the foregoing. some or all of the steps 902 - 910 can be performed by a given entity, such as the scientific data processing system 1500 itself. For example, the flow illustrated in Figure 10A is performed in its entirety by processing resources of scientific data processing system 1500.

Alternatively to or in addition to any of the foregoing, some of all of the steps 902 - 910 can be performed by separate entities, for example, where scientific data processing system 1500 performs some steps and where a third party entity or separate entity communicating with scientific data processing system 1500 performs other steps. An example of such an embodiment is illustrated in Figure 10B.

Alternatively to or in addition to any of the foregoing, a first entity can perform steps 902, 904, and 906, where the plurality of outcome prediction scores are communicated (e.g. transmitted to, displayed to, sent to, etc.) to a second entity, for example, via performance of an corresponding additional step 916 by the first entity of communicating the plurality of outcome prediction scores, for example, as illustrated in Figure 10B. Alternatively to or in addition to any of the foregoing, a second entity (e.g. the second entity to which the plurality of outcome prediction scores were communicated via the first entity in step 916) can perform steps 908 and 910, where the plurality of outcome prediction scores are processed in step 908 based on being received by the second entity via performance of a corresponding additional step 918 by the second entity of receiving the plurality of outcome prediction scores (e.g. from the first entity). As a first particular example, the first entity is implemented as the scientific data processing system 1500, and the second entity is an entity responsible for performing randomization and/or for assigning participants accordingly for scientific trials, such as trial participant assignment entity 587. As a second particular example, the first entity is implemented as an entity responsible for training, retraining and/or executing AI models to generate model output, such as AI platform 588, and the second entity is implemented as the scientific data processing system 1500.

Figures 10C - 10D illustrate methods for execution by at least one processor. For example, some or all of Figure 10C and/or 10D can be executed via medical data processing system 500 and/or based on communicating with a medical data processing system 500. For example, some or all of the steps of Figure 10C and/or 10D can be performed in conjunction with implementing some or all features and/or functionality of the medical data processing system of Figures 4A - 4I. Alternatively or in addition, some or all of the steps of Figure 10C and/or 10D can be performed in conjunction with implementing some or all features and/or functionality of any embodiment of the medical outcome prognostication model training system 506; the medical outcome prognostication system 507; and/or the prognosis-based trial arm assignment system 508 described herein. Alternatively or in addition, some or all of the steps of Figure 10C and/or 10D can be performed in conjunction with implementing some or all features and/or functionality of the trial arm assignment module 730 and/or the randomization algorithm 800 of one or more of Figures 6A - 6K, and/or any embodiment of trial arm assignment module 730 and/or the randomization algorithm 800 described herein. Alternatively or in addition, some or all of the steps of Figure 10C and/or 10D can be performed in conjunction by and/or via communication with one or more data sources 575, one or more requesting entities 576, one or more medical product testing processing systems 597 of one or more medical product testing entities 577, and/or one or more medical product manufacturing processing systems 597 of one or more medical product manufacturing entities 577. Alternatively or in addition, some or all of the steps of Figure 10C and/or 10D can be performed in conjunction with the system 100 of Figure 1B, for example, in conjunction with implementing the imaging-based prognostication (IPRO) framework 200A and/or at least one corresponding model 112, for example, in conjunction with some or all features and/or functionality presented in Figure 8A and/or the example embodiments of Figures 8B - 9R. Alternatively or in addition, some or all of the steps of Figure 10C and/or 10D can be performed by, and/or based on communications between, any one or more computing devices 700, any one or more corresponding server systems, and/or any processing system that includes at least one processor and/or at least one memory.

Some or all steps of the method of Figure 10C and/or 10D can performed in conjunction with performance and/or can be implemented based on performing one or more steps of any other method described herein, such as any method of Figures 8B - 8G, the method of Figure 10A, one of both methods of Figure 10B, the method of Figure 10E, one of both methods of Figure 10F and/or the method of Figure 10G. As a particular example, the method of Figure 10C can be implemented as a particular example of the method of Figure 10A (and/or one or both methods of Figure 10D can be implemented as particular examples of the corresponding methods of Figure 10B), for example where the outcome prediction function of Figure 10A and/or 10B is implemented as the medical outcome prognostication function of Figure 10C and/or 10D; where the plurality of scientific data of Figure 10A and/or 10B is implemented as the plurality of medical data of Figure 10C; where the scientific study of Figure 10A and/or 10B is implemented as the clinical trial of Figure 10C; where the plurality of outcome prediction scores of Figure 10A and/or 10B is implemented as the plurality of medical outcome prognosis scores of Figure 10C and/or 10D; where step 1002 of Figure 10C and/or 10D is a particular example of performing step 902 of Figure 10A and/or 10B; where step 1004 of Figure 10C and/or 10D is a particular example of performing step 904 of Figure 10A and/or 10B; where step 1006 of Figure 10C and/or 10D is a particular example of performing step 906 of Figure 10A and/or 10B; where step 1008 of Figure 10C and/or 10D is a particular example of performing step 908 of Figure 10A and/or 10B; where step 1010 of Figure 10C and/or 10D is a particular example of performing step 910 of Figure 10A and/or 10B; where step 1016 of Figure 10D is a particular example of performing step 916 of Figure 10B; and/or where step 1018 of Figure 10D is a particular example of performing step 918 of Figure 10B. The method Figure 10A and/or 10B can alternatively or additionally be implemented to process non-medical data to generate trial arm assignment data for a non-medical scientific study and/or can otherwise be performed differently from the method illustrated in Figure 10C and/or 10D.

Step 1002 includes determining a medical outcome prognostication function. In some or all embodiments, medical outcome prognostication function of step 1002 can be implemented via some or all features and/or functionality of any embodiment of medical outcome prognostication function 671 described herein. In some or all embodiments, medical outcome prognostication function of step 1002 can be implemented via some or all features and/or functionality of any embodiment of model 112 described herein. Alternatively to or in addition to any of the foregoing, step 1002 is performed via implementing some or all features and/or functionality of medical outcome prognostication model training system 506 to train the medical outcome prognostication function, for example, based on tuning parameters of corresponding model parameter data 661. Alternatively to or in addition to any of the foregoing, the medical outcome prognostication function of step 1002 can be determined via any other means (e.g. based on being accessed in memory, being received via the network, being generated/trained by another entity, based on being automatically trained and/or generated, for example, as a step of the method of Figure 10C, based on having being previously trained via a separate entity, based on being configured based on user input, and/or based on otherwise being determined).

Alternatively to or in addition to any of the foregoing, the medical outcome prognostication function determined step 1002 was trained based on utilizing artificial intelligence. Alternatively to or in addition to any of the foregoing, the method further includes training the medical outcome prognostication function, for example, based on utilizing artificial intelligence to process a training set. Alternatively to or in addition to any of the foregoing, the training set includes a first plurality of medical data of at least one medical data type. Alternatively to or in addition to any of the foregoing, the first plurality of medical data is a first plurality of device-captured medical data (e.g. based on being generated/captured by/in conjunction with use of a corresponding medical device), and/or the at least one medical type is at least one device-captured medical data type. Alternatively to or in addition to any of the foregoing, training set includes a corresponding plurality of medical outcome data for a first medical outcome type.

Alternatively to or in addition to any of the foregoing, some or all of the first plurality of medical data can be implemented via any embodiment of medical data 562 described herein. Alternatively to or in addition to any of the foregoing, some or all of the first plurality of medical data can include a plurality of values 601. Alternatively to or in addition to any of the foregoing, some or all of the corresponding plurality of medical outcome data can be implemented via any embodiment of outcome data 638 described herein. Alternatively to or in addition to any of the foregoing, some or all of the first medical outcome type can be implemented via any embodiment of medical outcome type 637 described herein.

Alternatively to or in addition to any of the foregoing, the first plurality of medical data and/or the corresponding plurality of medical outcome data can be included in the training set based on being received from a data source 575. Alternatively to or in addition to any of the foregoing, the first plurality of medical data and/or the corresponding plurality of medical outcome data can be included in the training set based on being stored in, accessed via, and/or received from a data storage system, such as medical data storage 561.

Alternatively to or in addition to any of the foregoing, the method further includes requesting the first plurality of medical data of the training set and/or further includes requesting the corresponding plurality of medical outcome data (e.g. via generating a corresponding at least one request and/or via sending to the at least one request to a data storage system or a data source), where the first plurality of medical data and/or the corresponding plurality of medical outcome data of the training set is obtained in response to the at least one request. Alternatively to or in addition to any of the foregoing, requesting the plurality of medical data is based on configuring at least one request for medical data meeting a set of parameters, where the first plurality of medical data meets the set of parameters based on the request. Alternatively to or in addition to any of the foregoing, the set of parameters indicates the at least one device-captured medical data type, for example, where the first plurality of medical data is of the at least one device-captured medical data type based on the at least one request for the first plurality of medical data and/or the corresponding plurality of medical outcome data indicating the at least one device-captured medical data type. Alternatively to or in addition to any of the foregoing, the set of parameters indicates the first medical outcome type, where the corresponding plurality of medical outcome data corresponds to the first medical outcome type based on the at least one request for the first plurality of medical data and/or the corresponding plurality of medical outcome data indicating the first medical outcome type.

Step 1004 includes obtaining a plurality of medical data corresponding to a plurality of prospective clinical trial participants of a clinical trial. Alternatively to or in addition to any of the foregoing, the plurality of medical data can be obtained based on being received from a data source 575. Alternatively to or in addition to any of the foregoing, the plurality of medical data can be obtained based on being stored in, accessed via, and/or received from a data storage system, such as medical data storage 561.

Alternatively to or in addition to any of the foregoing, the plurality of medical data obtained in step 1004 is a second plurality of medical data (e.g. distinct from the first plurality of medical data utilized to train the medical outcome prognostication function). Alternatively to or in addition to any of the foregoing, the plurality of medical data is a second-plurality of device-captured medical data of the at least one device-captured medical data type (e.g. based on being generated/captured by/in conjunction with use of a corresponding medical device). Alternatively to or in addition to any of the foregoing, each of the plurality of medical data obtained in step 1004 corresponds to pre-trial medical data for a corresponding one of the plurality of prospective clinical trial participants of the clinical trial. Alternatively to or in addition to any of the foregoing, the clinical trial has a primary endpoint corresponding to the first medical outcome type, for example, of the corresponding plurality of medical outcome data of the training set utilized to train the medical outcome prognostication function determined in step 1002.

Alternatively to or in addition to any of the foregoing, the method further includes requesting the plurality of medical data of step 1004 (e.g. via generating a corresponding at least one request and/or via sending to the at least one request to a data storage system or a data source), where the plurality of medical data is obtained in response to the request. Alternatively to or in addition to any of the foregoing, requesting the plurality of medical data is based on configuring at least one request for medical data meeting a set of parameters, where the plurality of medical data obtained in step 1004 meets the set of parameters based on the at least one request. Alternatively to or in addition to any of the foregoing, the set of parameters indicates the at least one device-captured medical data type, for example, where the plurality of medical data obtained in step 1004 is of the at least one device-captured medical data type based on the at least one request for the plurality of medical data indicating the at least one device-captured medical data type. Alternatively to or in addition to any of the foregoing, the set of parameters indicates the plurality of prospective clinical trial participants (e.g. based on patient identifiers of the plurality of prospective clinical trial participants), for example, where the plurality of medical data obtained in step 1004 corresponds to the plurality of prospective clinical trial participants based on the at least one request for the plurality of medical data indicating the plurality of prospective clinical trial participants. Alternatively to or in addition to any of the foregoing, the set of parameters indicates the clinical trial (e.g. based on a clinical trial identifier of the clinical trial), for example, where the plurality of medical data obtained in step 1004 corresponds to the plurality of prospective clinical trial participants of the clinical trial based on the at least one request for the plurality of medical data indicating the clinical trial.

Step 1006 includes generating a plurality of medical outcome prognosis scores corresponding to the first medical outcome type based on performing the medical outcome prognostication function each of the plurality of medical data, for example, based on the plurality of medical data being obtained in step 1004 and/or based on the medical outcome prognostication function being determined in step 1002. Alternatively to or in addition to any of the foregoing, step 1006 is performed via implementing some or all features and/or functionality of medical outcome prognostication system 507 to perform the medical outcome prognostication function.

Alternatively to or in addition to any of the foregoing, the plurality of medical outcome prognosis scores correspond to the first medical outcome type, for example, of the corresponding plurality of medical outcome data of the training set utilized to train the medical outcome prognostication function determined in step 1002, and/or corresponding to the primary endpoint of the clinical trial. Alternatively to or in addition to any of the foregoing, the medical outcome prognostication function is performed in step 1006 based on utilizing artificial intelligence. Alternatively to or in addition to any of the foregoing, the medical outcome prognostication function is performed upon each of the plurality of medical data (e.g. implemented as a second plurality of medical data, such as a second plurality of device-captured medical data) to generate a corresponding medical outcome prognosis score of the plurality of medical outcome prognosis scores. Alternatively to or in addition to any of the foregoing, each medical outcome prognosis score of the plurality of medical outcome prognosis scores corresponds to one of the plurality of prospective clinical trial participants.

Step 1008 includes processing the plurality of medical outcome prognosis scores via a randomization algorithm to facilitate selection of a first proper subset of the plurality of prospective clinical trial participants and a second proper subset of the plurality of prospective clinical trial participants.

Alternatively to or in addition to any of the foregoing, facilitating selection of the first proper subset of the plurality of prospective participants and the second proper subset of the plurality of prospective participants includes automatically selecting the first proper subset of the plurality of prospective participants and the second proper subset of the plurality of prospective participants. Alternatively to or in addition to any of the foregoing, facilitating selection of the first proper subset of the plurality of prospective participants and the second proper subset of the plurality of prospective participants is based on providing the plurality of medical outcome prognosis scores for use in a randomized selection process that applies the randomization algorithm, where this a randomized selection process is optionally performed: via an automated process; via at least one human; or via a combination of automated processes and human-conducted processes, for example, intervention with at least one automated process.

Alternatively to or in addition to any of the foregoing, the randomization algorithm is implemented via some or all features and/or functionality of any embodiment of the randomization algorithm 800 described herein. Alternatively to or in addition to any of the foregoing, step 1008 is performed via implementing some or all features and/or functionality of prognosis-based trial arm assignment system 508 described herein and/or any embodiment of trial arm assignment module 730 described herein.

Alternatively to or in addition to any of the foregoing, the first proper subset and the second proper subset are mutually exclusive. Alternatively to or in addition to any of the foregoing, the first proper subset and the second proper subset are collectively exhaustive with respect to the plurality of prospective clinical trial participants. Alternatively to or in addition to any of the foregoing, at least one of the plurality of prospective clinical trial participants is not included in the first proper subset or the second proper subset.

Alternatively to or in addition to any of the foregoing, performing step 1008 includes processing the plurality of medical outcome prognosis scores via the randomization algorithm to automatically select a plurality of proper subsets of the plurality of prospective clinical trial participants that includes only the first proper subset and the second proper subset. Alternatively to or in addition to any of the foregoing, performing step 1008 includes processing the plurality of medical outcome prognosis scores via the randomization algorithm to automatically select a plurality of proper subsets of the plurality of prospective clinical trial participants that includes the first proper subset, the second proper subset, and at least one additional proper subset.

Alternatively to or in addition to any of the foregoing, processing the plurality of medical outcome prognosis scores via the randomization algorithm is based on applying a medical outcome prognosis score-based stratification factor of the randomization algorithm. Alternatively to or in addition to any of the foregoing, applying the medical outcome prognosis score-based stratification factor includes implementing any embodiment of stratification process 810 described herein, for example, by performing any embodiment of stratification function 820 described herein.

Step 1010 includes communicating trial arm assignment data based on the first proper subset and the second proper subset. Alternatively to or in addition to any of the foregoing, the trial arm assignment data is communicated to an entity associated with conducting the clinical trial. Alternatively to or in addition to any of the foregoing, the trial arm assignment data is communicated to a requesting entity 575, a medical product manufacturing processing system 597 of a medical product manufacturing entity 577, and/or a medical product testing processing system 597 of a medical product testing entity 577. Alternatively to or in addition to any of the foregoing, communicating the trial arm assignment data includes at least one of: transmitting or otherwise sending the trial arm assignment data (e.g. to the entity associated with conducting the clinical trial) ; displaying the trial arm assignment data (e.g. via a display device of a computing device associated with the entity associated with conducting the clinical trial); and/or storing the trial arm assignment data (e.g. in clinical trial storage 565 and/or other storage resources, for example, as trial arm assignment data 631 of clinical trial data 566).

Alternatively to or in addition to any of the foregoing, each of the plurality of medical outcome prognosis scores indicate a corresponding probability value denoting a predicted probability that a corresponding prospective clinical trial participant will attain the first medical outcome type based on the medical outcome prognostication function being trained to predict probability of the first medical outcome type as a function of device-captured medical data having the at least one device-captured medical data type.

Alternatively to or in addition to any of the foregoing, each given medical data of the plurality of medical data corresponding to a corresponding given prospective clinical trial participant of the plurality of prospective clinical trial participants includes multiple types of medical data for the corresponding given prospective clinical trial participant of the plurality of prospective clinical trial participants, where a given corresponding medical outcome prognosis score for the given prospective clinical trial participant corresponding to the first medical outcome type is generated for the corresponding given prospective clinical trial participant based on performing the medical outcome prognostication function upon the multiple types of medical data for the corresponding given prospective clinical trial participant. Alternatively to or in addition to any of the foregoing, the given corresponding medical outcome prognosis score for the given prospective clinical trial participant corresponding to the first medical outcome type is generated for the corresponding given prospective clinical trial participant based on performing the medical outcome prognostication function upon the multiple types of medical data for the corresponding given prospective clinical trial participant based on the medical outcome prognostication function being implemented as a multi-modal model. Alternatively to or in addition to any of the foregoing, the multiple types of medical data includes two or more types of medical images of two or more corresponding different medical imaging modalities. Alternatively to or in addition to any of the foregoing, the multiple types of medical data includes any other two or more types of medical data describe herein.

Alternatively to or in addition to any of the foregoing, the randomization algorithm is performed to select the first proper subset and/or the second proper subset in accordance with at least one of: a simple randomization technique, a block randomization technique, a stratified randomization technique, a covariate adapted randomization technique, or a permuted block randomization technique. Alternatively to or in addition to any of the foregoing, the randomization algorithm is performed to select the first proper subset and/or the second proper subset based on applying a stratification function based on utilizing the first medical outcome type as a stratification factor, and further includes applying a permuted block randomization technique. Alternatively to or in addition to any of the foregoing, the randomization algorithm is performed automatically, without human intervention. Alternatively to or in addition to any of the foregoing, the randomization algorithm is performed based on human intervention.

Alternatively to or in addition to any of the foregoing, performing the randomization algorithm to automatically select the first proper subset and the second proper subset is based on determining a deterministic mapping of a plurality of possible medical outcome prognosis scores to a plurality of score-based groups. Alternatively to or in addition to any of the foregoing, the deterministic mapping indicates a mapping of each of the plurality of possible medical outcome prognosis scores to exactly one of the plurality of score-based groups. Alternatively to or in addition to any of the foregoing, the plurality of possible medical outcome prognosis scores render a full score range that includes all possible medical outcome prognosis scores generated via performance of the medical outcome prognostication function. Alternatively to or in addition to any of the foregoing, performing the randomization algorithm to automatically select the first proper subset and the second proper subset is based on segregating the plurality of prospective clinical trial participants across the plurality of score-based groups by including each of the plurality of prospective clinical trial participants in a corresponding one of the plurality of score-based groups based on determining which one of the plurality of score-based groups is mapped to by the corresponding medical outcome prognosis score generated from corresponding device-captured medical data of the each of the plurality of prospective clinical trial participants. Alternatively to or in addition to any of the foregoing, performing the randomization algorithm to automatically select the first proper subset and the second proper subset is based on applying a randomization sub-algorithm of the randomization algorithm to each of the plurality of score-based groups independently to distribute prospective clinical trial participants in a given score-based group of the plurality of score-based groups between the first proper subset and the second proper subset separately from distributing other prospective clinical trial participants in other score-based groups of the plurality of score-based groups between the first proper subset and the second proper subset.

Alternatively to or in addition to any of the foregoing, the plurality of possible medical outcome prognosis scores correspond to numeric values in the full score range. Alternatively or in addition to any of the foregoing, determining the deterministic mapping is based on determining a plurality of medical outcome prognosis score ranges to define the plurality of score-based groups. Alternatively to or in addition to any of the foregoing, the plurality of medical outcome prognosis score ranges contiguously render the full score range that includes all possible medical outcome prognosis scores generated via performance of the medical outcome prognostication function. Alternatively to or in addition to any of the foregoing, each of the plurality of medical outcome prognosis score ranges include multiple corresponding ones of the plurality of possible medical outcome prognosis scores.

Alternatively to or in addition to any of the foregoing, applying the randomization sub-algorithm of the randomization algorithm to the each of the plurality of score-based groups is based on generating a random trial assignment ordering for the each of the plurality of score-based groups. Alternatively to or in addition to any of the foregoing, the random trial assignment ordering includes an ordered plurality of assignments based on participant distribution proportion data. Alternatively to or in addition to any of the foregoing,
determining an ordering of prospective clinical trial participants in the each of the plurality of score-based groups. Alternatively to or in addition to any of the foregoing, distributing prospective clinical trial participants in the each of the plurality of score-based groups is based on applying the random trial assignment ordering to the ordering of prospective clinical trial participants.

Alternatively to or in addition to any of the foregoing, performing the randomization algorithm to automatically select the first proper subset and the second proper subset includes distributing prospective clinical trial participants between the first proper subset and the second proper subset based on minimizing a first difference between a first number of prospective clinical participants included the first proper subset and a second number of prospective clinical trial participants included in the second proper subset. Alternatively to or in addition to any of the foregoing, performing the randomization algorithm to automatically select the first proper subset and the second proper subset includes minimizing a second difference between: a first mean medical outcome prognosis score of first medical outcome type prognosis scores generated from medical data of prospective clinical trial participants included in the first proper subset; and a second mean medical outcome prognosis score of second medical outcome prognosis scores generated from second medical data of second prospective clinical trial participants included in the second proper subset.

Alternatively to or in addition to any of the foregoing, each device-captured medical data the first plurality of device-captured medical data and of the second plurality of device-captured medical data indicates a plurality of sensor data values captured by at least one medical device of at least one corresponding medical device type. Alternatively to or in addition to any of the foregoing, the medical outcome prognostication function is trained based on processing a first plurality of feature vectors that each include a first corresponding plurality of sensor data values for a corresponding one of the first plurality of device-captured medical data. Alternatively to or in addition to any of the foregoing, the medical outcome prognostication function is performed upon the second plurality of device-captured medical data based on processing a second plurality of feature vectors that each include a second corresponding plurality of sensor data values for a corresponding one of the second plurality of device-captured medical data.

Alternatively to or in addition to any of the foregoing, the at least one medical imaging modality includes a computed tomography (CT) scan modality. Alternatively to or in addition to any of the foregoing, the first plurality of medical imaging data includes a first plurality of CT scans. Alternatively to or in addition to any of the foregoing, the second plurality of medical imaging data includes a second plurality of CT scans.

Alternatively to or in addition to any of the foregoing, the at least one device-captured medical data type further corresponds to at least one anatomical region captured via the at least one medical imaging modality. Alternatively to or in addition to any of the foregoing, the at least one device-captured medical data type further corresponds to at least one image plane by which the at least one medical imaging modality is captured.

Alternatively to or in addition to any of the foregoing, the at least one device-captured medical data type includes a histological slide type; a DNA sequence data type; and/or a test strip type. Alternatively to or in addition to any of the foregoing, the device-captured medical data type includes at least one health sensor data type corresponding to health sensor data captured by a wearable device that includes at least one of: heart rate data; blood oxygen measurement data; blood pressure data; internal temperature data; respiratory rate data; pedometer data; and/or sleep cycle data. Alternatively to or in addition to any of the foregoing, the at least one device-captured medical data type corresponds to proteomic data, genomic data, metabolomic data, metagenomic data, phenomics data, and/or transcriptomic data (e.g. RNA transcriptions). Alternatively to or in addition to any of the foregoing, the at least one device-captured medical data type corresponds to any other one or more medical data types described herein.

Alternatively to or in addition to any of the foregoing, the training set includes a plurality of training data instances corresponding to a plurality of individuals. Alternatively to or in addition to any of the foregoing, each training data instance includes, for a corresponding one of the plurality of individuals, one of the first plurality of device-captured medical data captured for the corresponding one of the plurality of individuals as input feature data. Alternatively to or in addition to any of the foregoing, each training data instance includes, for a corresponding one of the plurality of individuals, exactly one of the first plurality of device-captured medical data captured for the corresponding one of the plurality of individuals as input feature data. Alternatively to or in addition to any of the foregoing, each training data instance includes, for a corresponding one of the plurality of individuals, more than one of the first plurality of device-captured medical data captured for the corresponding one of the plurality of individuals as input feature data. Alternatively to or in addition to any of the foregoing, each training data instance includes, for a corresponding one of the plurality of individuals, one of the corresponding plurality of medical outcome data determined for the corresponding one of the plurality of individuals as output label data. Alternatively to or in addition to any of the foregoing, the plurality of individuals are distinct from the plurality of prospective clinical trial participants.

Alternatively to or in addition to any of the foregoing, the input feature data of the each training data instance further includes, for the corresponding one of the plurality of individuals, demographic data for the corresponding one of the plurality of individuals (e.g. any one or more types of demographic data 641); medical history data for the corresponding one of the plurality of individuals (e.g. any one or more types of medical history data 642); patient history data for the corresponding one of the plurality of individuals (e.g. any one or more types of medical history data 643); medical report text data for the corresponding one of the plurality of individuals; or risk factor data for the corresponding one of the plurality of individuals (e.g. any one or more types of risk factor data 644). Alternatively to or in addition to any of the foregoing, the input feature data of the each training data instance further includes, for the corresponding one of the plurality of individuals: histology of the corresponding one of the plurality of individuals; PD-L1 Expression of the corresponding one of the plurality of individuals; gender of the corresponding one of the plurality of individuals; smoking status of the corresponding one of the plurality of individuals; prior therapy of the corresponding one of the plurality of individuals; TNM stage of the corresponding one of the plurality of individuals; performance status of the corresponding one of the plurality of individuals; and/or age of the corresponding one of the plurality of individuals. Alternatively to or in addition to any of the foregoing, the input feature data of the each training data instance further includes, for the corresponding one of the plurality of individuals, any one or more types of other relevant data 640 described herein. Alternatively to or in addition to any of the foregoing, the input feature data of the each training data instance further includes, for the corresponding one of the plurality of individuals, any one or more types of medical data metadata 610 described herein for corresponding device-captured medical data.

Alternatively to or in addition to any of the foregoing, the method further includes training a plurality of medical outcome prognostication functions corresponding to a plurality of different medical outcome types. Alternatively to or in addition to any of the foregoing, the medical outcome prognostication functions is one of the plurality of medical outcome prognostication functions and/or the first medical outcome type is one of the plurality of different medical outcome types. Alternatively to or in addition to any of the foregoing, the medical outcome prognostication function is selected for performance upon the second plurality of device-captured medical data for the plurality of prospective clinical trial participants of the clinical trial based on the clinical trial having the primary endpoint corresponding to the first medical outcome type. Alternatively to or in addition to any of the foregoing, the method further includes obtaining a third plurality of device-captured medical data. Alternatively to or in addition to any of the foregoing, each of the third plurality of device-captured medical data corresponds pre-treatment medical data for a corresponding one of a second plurality of prospective clinical trial participants of a second clinical trial having another primary endpoint corresponding to a second medical outcome of the plurality of different medical outcome types. Alternatively to or in addition to any of the foregoing, the method further includes selecting a second medical outcome prognostication function of the plurality of medical outcome prognostication functions for performance upon the third plurality of device-captured medical data for the second plurality of prospective clinical trial participants of the second clinical trial based on the second clinical trial having a second primary endpoint corresponding to the second medical outcome. Alternatively to or in addition to any of the foregoing, the method further includes generating a second plurality of medical outcome prognosis scores corresponding to the second medical outcome based on utilizing artificial intelligence to perform the second medical outcome prognostication function upon each of the third plurality of device-captured medical data to generate a second corresponding medical outcome prognosis score of the second plurality of medical outcome prognosis scores. Alternatively to or in addition to any of the foregoing, the method further includes processing the second plurality of medical outcome prognosis scores via the randomization algorithm to automatically select another first proper subset of the second plurality of prospective clinical trial participants and another second proper subset of the second plurality of prospective clinical trial participants based on utilizing the second medical outcome as the medical outcome prognosis score-based stratification factor of the randomization algorithm. Alternatively to or in addition to any of the foregoing, the another first proper subset and the another second proper subset are mutually exclusive. Alternatively to or in addition to any of the foregoing, the method further includes communicating second clinical trial assignment data indicating assignment of the another first proper subset of the plurality of prospective clinical trial participants to a second control trial arm of the clinical trial and further indicating assignment of the another second proper subset of the second plurality of prospective clinical trial participants to a second experimental trial arm of the second clinical trial.

Alternatively to or in addition to any of the foregoing, the first medical outcome type corresponds to at least one of: a mortality metric; a hospitalization metric; a count metric; a medically-defined scale-based metric; an objective tumor response; a metric for measured change; a metric for pain; at least one biomarker; any primary endpoint of the clinical trial and/or any other clinical trial; any secondary endpoint of the clinical trial and/or any other clinical trial; any surrogate endpoint of the clinical trial and/or any other clinical trial; any medical outcome type 637 described herein; and/or any other type of medical outcome.

Alternatively to or in addition to any of the foregoing, the entity conducts the clinical trial based on conducting the clinical trial for the first proper subset of the plurality of prospective clinical trial participants in conjunction with the control trial arm of the clinical trial to generate control trial arm result data. conducting the clinical trial for the second proper subset of the plurality of prospective clinical trial participants in conjunction with the experimental trial arm of the clinical trial to generate experimental trial arm result data. Alternatively to or in addition to any of the foregoing, the entity conducts the clinical trial based on generating corresponding clinical trial results based on the control trial arm result data and the experimental trial arm result data.

Alternatively to or in addition to any of the foregoing, the clinical trial is conducted to test a corresponding medical product. Alternatively or in addition to any of the foregoing, the corresponding medical product is commercially manufactured by a corresponding medical product manufacturing entity based on clinical trial results of the clinical trial comparing favorably to regulation-mandated acceptance criteria.

Alternatively to or in addition to any of the foregoing, the method further includes determining trial eligibility requirements for the clinical trial indicating at least one required condition. Alternatively to or in addition to any of the foregoing, the method further includes generating trial eligibility data for each of plurality of prospective clinical trial participants based on utilizing artificial intelligence to perform at least one condition detection function upon corresponding device-captured medical data to determine whether the at least one required condition is detected. Alternatively to or in addition to any of the foregoing, the method further includes identifying a third proper subset of the plurality of prospective clinical trial participants that meet the trial eligibility requirements based on each having trial eligibility data indicating detection of the at least one required condition in their corresponding medical data. Alternatively to or in addition to any of the foregoing, at least one of the plurality of prospective clinical trial participants is not included in the third proper subset based on having trial eligibility data indicating detection of the least one required condition was not detected in at least one corresponding medical data. Alternatively to or in addition to any of the foregoing, the first proper subset and the second proper subset are collectively exhaustive with respect to the third proper subset of the plurality of prospective clinical trial participants.

Alternatively to or in addition to any of the foregoing, the first medical outcome type corresponds to an event occurring within and/or after a predetermined amount of time has elapsed. Alternatively to or in addition to any of the foregoing, the clinical trial has a duration that corresponds to and/or is greater than the predetermined amount of time. Alternatively to or in addition to any of the foregoing, the medical outcome prognostication function is trained to predict occurrence of first medical outcome type within and/or after the predetermined amount of time has elapsed. Alternatively to or in addition to any of the foregoing, a given one of the first plurality of device-captured medical data of the at least one device-captured medical data type is captured at a first time for a corresponding individual, and a given corresponding medical outcome data of the corresponding plurality of medical outcome data for the first medical outcome type is measured for the corresponding individual at a second time. Alternatively to or in addition to any of the foregoing, the second time is within the predetermined amount of time from the first time and/or the second time is after the predetermined amount of time from the first time. Alternatively to or in addition to any of the foregoing, the predetermined amount of time corresponds to one of: one year, two years, five years, and/or any other duration.

Alternatively to or in addition to any of the foregoing, the first medical outcome type corresponds to at least one of: a mortality metric; a hospitalization metric; a count metric; a medically-defined scale-based metric; objective tumor response; a metric for measured change; a metric for pain; at least one biomarker; any other medical outcome type; and/or any primary outcome measured in any clinical trial.

Alternatively to or in addition to any of the foregoing. some or all of the steps 1002 - 1010 can be performed by a given entity, such as the medical data processing system 500 itself. For example, the flow illustrated in Figure 10C is performed in its entirety by processing resources of medical data processing system 500.

Alternatively to or in addition to any of the foregoing, some of all of the steps 1002 - 1010 can be performed by separate entities, for example, where medical data processing system 500 performs some steps and where a third party entity or separate entity communicating with medical data processing system 500 performs other steps. An example of such an embodiment is illustrated in Figure 10B.

Alternatively to or in addition to any of the foregoing, a first entity can perform steps 1002, 1004, and 1006, where the plurality of medical outcome prognosis scores are communicated (e.g. transmitted to, displayed to, sent to, etc.) to a second entity via performance of an corresponding additional step 1016 by the first entity of communicating the plurality of medical outcome prognosis scores, for example, as illustrated in Figure 10D. Alternatively to or in addition to any of the foregoing, a second entity (e.g. the second entity to which the plurality of medical outcome prognosis scores were communicated via the first entity in step 1016) can perform steps 1008 and 1010, where the plurality of medical outcome prediction scores are processed in step 1108, for example, based on being received by the second entity via performance of a corresponding additional step 1018 by the second entity (e.g. based on having been communicated via the first entity), for example, as illustrated in Figure 10D. As a first particular example, the first entity is implemented as the medical data processing system 500, and the second entity is an entity responsible for performing randomization and/or for assigning participants accordingly for scientific trials, such as trial participant assignment entity 587. As a second particular example, the first entity is implemented as an entity responsible for training, retraining and/or executing AI models to generate model output, such as AI platform 588, and the second entity is implemented as the medical data processing system 500.

Figures 10E - 10F illustrate methods for execution by at least one processor. For example, some or all of Figure 10E and/or 10F can be executed via medical data processing system 500 and/or based on communicating with a medical data processing system 500. For example, some or all of the steps of Figure 10E and/or 10F can be performed in conjunction with implementing some or all features and/or functionality of the medical data processing system of Figures 4A - 4I. Alternatively or in addition, some or all of the steps of Figure 10E and/or 10F can be performed in conjunction with implementing some or all features and/or functionality of any embodiment of the medical outcome prognostication model training system 506; the medical outcome prognostication system 507; and/or the prognosis-based trial arm assignment system 508 described herein. Alternatively or in addition, some or all of the steps of Figure 10E and/or 10F can be performed in conjunction with implementing some or all features and/or functionality of the trial arm assignment module 730 and/or the randomization algorithm 800 of one or more of Figures 6A - 6K, and/or any embodiment of trial arm assignment module 730 and/or the randomization algorithm 800 described herein. Alternatively or in addition, some or all of the steps of Figure 10E and/or 10F can be performed in conjunction by and/or via communication with one or more data sources 575, one or more requesting entities 576, one or more medical product testing processing systems 597 of one or more medical product testing entities 577, and/or one or more medical product manufacturing processing systems 597 of one or more medical product manufacturing entities 577. Alternatively or in addition, some or all of the steps of Figure 10E and/or 10F can be performed in conjunction with the system 100 of Figure 1B, for example, in conjunction with implementing the imaging-based prognostication (IPRO) framework 200A and/or at least one corresponding model 112, for example, in conjunction with some or all features and/or functionality presented in Figure 8A and/or the example embodiments of Figures 8B - 9R. Alternatively or in addition, some or all of the steps of Figure 10E and/or 10F can be performed by, and/or based on communications between, any one or more computing devices 700, any one or more corresponding server systems, and/or any processing system that includes at least one processor and/or at least one memory.

Some or all steps of the method of Figure 10E and/or 10F can performed in conjunction with performance and/or can be implemented based on performing one or more steps of any other method described herein, such as any method of Figures 8B - 8G, the method of Figure 10A, one of both methods of Figure 10B, the method of Figure 10C, one or both methods of Figure 10D, and/or the method of Figure 10G. As a particular example, the method of Figure 10E and/or 10F can be implemented as a particular example of the method of Figure 10C and/or of one or both methods of Figure 10D, for example, where step 1102 of Figure 10E and/or 10F is a particular example of performing step 1002 of Figure 10C; where step 1104 of Figure 10E and/or 10F is a particular example of performing step 1004 of Figure 10C and/or 10D; where step 1106 of Figure 10E and/or 10F is a particular example of performing step 1006 of Figure 10C and/or 10D; where step 1108 of Figure 10E and/or 10F is a particular example of performing step 1008 of Figure 10C and/or 10D; and/or where step 1110 of Figure 10E and/or 10F is a particular example of performing step 1010 of Figure 10C and/or 10D. In other embodiments, Figure 10E and/or 10F can be performed differently from the method illustrated in 10C and/or 10D.

Step 1102 training a medical outcome prognostication function based on utilizing artificial intelligence to process a training set that includes a first plurality of device-captured medical data of at least one device-captured medical data type and a corresponding plurality of medical outcome data for a first medical outcome type. Step 1104 includes obtaining a second plurality of device-captured medical data of the at least one device-captured medical data type, where each of the second plurality of device-captured medical data corresponds to pre-trial medical data for a corresponding one of a plurality of prospective clinical trial participants of a clinical trial having a primary endpoint corresponding to the first medical outcome type. Step 1106 includes generating a plurality of medical outcome prognosis scores corresponding to the first medical outcome type based on utilizing artificial intelligence to perform the medical outcome prognostication function upon each of the second plurality of device-captured medical data to generate a corresponding medical outcome prognosis score of the plurality of medical outcome prognosis scores. Step 1108 includes processing the plurality of medical outcome prognosis scores via a randomization algorithm to facilitate selection of a first proper subset of the plurality of prospective clinical trial participants and a second proper subset of the plurality of prospective clinical trial participants based on applying a medical outcome prognosis score-based stratification factor of the randomization algorithm. Step 1110 includes communicating, to an entity associated with conducting the clinical trial, trial arm assignment data indicating assignment of the first proper subset of the plurality of prospective clinical trial participants to a control trial arm of the clinical trial and further indicating assignment of the second proper subset of the plurality of prospective clinical trial participants to an experimental trial arm of the clinical trial.

Alternatively to or in addition to any of the foregoing. some or all of the steps 1102 - 1110 can be performed by a given entity, such as the medical data processing system 500 itself. For example, the flow illustrated in Figure 10E is performed in its entirety by processing resources of medical data processing system 500.

Alternatively to or in addition to any of the foregoing, some of all of the steps 1102 - 1110 can be performed by separate entities, for example, where medical data processing system 500 performs some steps and where a third party entity or separate entity communicating with medical data processing system 500 performs other steps. An example of such an embodiment is illustrated in Figure 10F.

Alternatively to or in addition to any of the foregoing, a first entity (e.g. the medical data processing system 500) can perform steps 1102, 1104, and 1106, for example, where the plurality of medical outcome prognosis scores are communicated (e.g. transmitted to, displayed to, sent to, etc.) to a second entity via performance of an corresponding additional step, such as step 1016 of Figure 10D. Alternatively to or in addition to any of the foregoing, a second entity (e.g. the second entity to which the plurality of medical outcome prognosis scores were communicated or that is otherwise responsible for randomizing participants of a clinical trial) can perform steps 1108 and 1110, where the plurality of medical outcome prognosis scores are processed in step 1008 based on being received by the second entity via performance of a corresponding additional step 1018 by the second entity of receiving the plurality of medical outcome prognosis scores (e.g. from the first entity). As a first particular example, the first entity is implemented as the medical data processing system 500, and the second entity is an entity responsible for performing randomization and/or for assigning participants accordingly for scientific trials, such as trial participant assignment entity 587. As a second particular example, the first entity is implemented as an entity responsible for training, retraining and/or executing AI models to generate model output, such as AI platform 588, and the second entity is implemented as the medical data processing system 500.

In various embodiments, any one of more of the various examples described herein are implemented in conjunction with performing some or all steps of Figure 10E, Figure 10F, Figure 10C, Figure 10D, Figure 10A and/or Figure 10B. In various embodiments, any set of the various examples described herein, can be implemented in tandem, for example, in conjunction with performing some or all steps of Figure 10F, Figure 10C, Figure 10D, Figure 10A and/or Figure 10B.

In various embodiments, at least one memory device, memory section, and/or memory resource (e.g., a non-transitory computer readable storage medium) can store operational instructions that, when executed by one or more processing modules of one or more computing devices of a database system, cause the one or more computing devices to perform any or all of the method steps of Figure 10F, Figure 10C, Figure 10D, Figure 10A and/or Figure 10B described above, for example, in conjunction with further implementing any one or more of the various examples described above.

In various embodiments, a medical data processing system includes at least one processor and at least one memory that stores operational instructions. In various embodiments, the operational instructions, when executed by the at least one processor, cause the database system to perform some or all steps of Figure 10E, Figure 10C, and/or Figure 10A, for example, in conjunction with further implementing any one or more of the various examples described above.

In various embodiments, the operational instructions, when executed by the at least one processor, cause the medical data processing system to: train a medical outcome prognostication function based on utilizing artificial intelligence to process a training set that includes a first plurality of device-captured medical data of at least one device-captured medical data type and/or a corresponding plurality of medical outcome data for a first medical outcome type; obtain a second plurality of device-captured medical data of the at least one device-captured medical data type, where each of the second plurality of device-captured medical data corresponds to pre-trial medical data for a corresponding one of a plurality of prospective clinical trial participants of a clinical trial having a primary endpoint corresponding to the first medical outcome type; and generate a plurality of medical outcome prognosis scores corresponding to the first medical outcome type based on utilizing artificial intelligence to perform the medical outcome prognostication function upon each of the second plurality of device-captured medical data to generate a corresponding medical outcome prognosis score of the plurality of medical outcome prognosis scores. The plurality of medical outcome prognosis scores are processed via a randomization algorithm to facilitate selection of a first proper subset of the plurality of prospective clinical trial participants and a second proper subset of the plurality of prospective clinical trial participants based on applying a medical outcome prognosis score-based stratification factor of the randomization algorithm, where the first proper subset and the second proper subset are mutually exclusive. Trial arm assignment data indicating assignment of the first proper subset of the plurality of prospective clinical trial participants to a control trial arm of the clinical trial and further indicating assignment of the second proper subset of the plurality of prospective clinical trial participants to an experimental trial arm of the clinical trial is communicated communicate to an entity associated with conducting the clinical trial,

In various embodiments of the medical data processing system, the operational instructions, when executed by the at least one processor, further cause the medical data processing system to process the plurality of medical outcome prognosis scores via the randomization algorithm to facilitate the selection (e.g. via automatic selection by the at least one processor) of the first proper subset of the plurality of prospective clinical trial participants and the second proper subset of the plurality of prospective clinical trial participants based on applying the medical outcome prognosis score-based stratification factor of the randomization algorithm.

Alternatively to or in addition to any of the foregoing embodiments of the medical data processing system, the operational instructions, when executed by the at least one processor, further cause the medical data processing system to communicate, to the entity associated with conducting the clinical trial, the trial arm assignment data indicating assignment of the first proper subset of the plurality of prospective clinical trial participants to the control trial arm of the clinical trial and further indicating assignment of the second proper subset of the plurality of prospective clinical trial participants to the experimental trial arm of the clinical trial.

Alternatively to or in addition to any of the foregoing embodiments of the medical data processing system, the operational instructions, when executed by the at least one processor, further cause the medical data processing system to communicate, to a second entity, the plurality of medical outcome prognosis scores, where the plurality of medical outcome prognosis scores are processed by this second entity, where this second entity performs the randomization algorithm to facilitate the selection of the first proper subset of the plurality of prospective clinical trial participants and the second proper subset of the plurality of prospective clinical trial participants based on applying the medical outcome prognosis score-based stratification factor of the randomization algorithm, and/or where this second entity communicates, to the entity associated with conducting the clinical trial, the trial arm assignment data indicating assignment of the first proper subset of the plurality of prospective clinical trial participants to the control trial arm of the clinical trial and further indicating assignment of the second proper subset of the plurality of prospective clinical trial participants to the experimental trial arm of the clinical trial. For example, this second entity can process the plurality of medical outcome prognosis scores and/or can generate and/or communicate the trial arm assignment data via at least one computing device, via at least one automated process, and/or via human intervention. As a particular example, the second entity corresponds to an entity (e.g. trial participant assignment entity 587) associated with randomizing the clinical trial (e.g. via an entirely automated process and/or a process conducted via human intervention).

Alternatively to or in addition to any of the foregoing embodiments of the medical data processing system, each of the plurality of medical outcome prognosis scores indicate a corresponding probability value denoting a predicted probability that a corresponding prospective clinical trial participant will attain the first medical outcome type based on the medical outcome prognostication function being trained to predict probability of the first medical outcome type as a function of device-captured medical data having the at least one device-captured medical data type.

Alternatively to or in addition to any of the foregoing embodiments of the medical data processing system, the randomization algorithm is performed to select the first proper subset and/or the second proper subset in accordance with at least one of: a simple randomization technique, a block randomization technique, a stratified randomization technique, a covariate adapted randomization technique, or a permuted block randomization technique. Alternatively to or in addition to any of the foregoing embodiments of the medical data processing system, the randomization algorithm is performed to select the first proper subset and/or the second proper subset based on applying a stratification function based on utilizing the first medical outcome type as a stratification factor, and further includes applying a permuted block randomization technique.

Alternatively to or in addition to any of the foregoing embodiments of the medical data processing system, the randomization algorithm is performed automatically, without human intervention. Alternatively to or in addition to any of the foregoing embodiments of the medical data processing system, the randomization algorithm is performed based on human intervention.

Alternatively to or in addition to any of the foregoing embodiments of the medical data processing system, performing the randomization algorithm to automatically select the first proper subset and the second proper subset is based on determining a deterministic mapping of a plurality of possible medical outcome prognosis scores to a plurality of score-based groups. Alternatively to or in addition to any of the foregoing, the deterministic mapping indicates a mapping of each of the plurality of possible medical outcome prognosis scores to exactly one of the plurality of score-based groups. Alternatively to or in addition to any of the foregoing, the plurality of possible medical outcome prognosis scores render a full score range that includes all possible medical outcome prognosis scores generated via performance of the medical outcome prognostication function. Alternatively to or in addition to any of the foregoing, performing the randomization algorithm to automatically select the first proper subset and the second proper subset is based on segregating the plurality of prospective clinical trial participants across the plurality of score-based groups by including each of the plurality of prospective clinical trial participants in a corresponding one of the plurality of score-based groups based on determining which one of the plurality of score-based groups is mapped to by the corresponding medical outcome prognosis score generated from corresponding device-captured medical data of the each of the plurality of prospective clinical trial participants. Alternatively to or in addition to any of the foregoing, performing the randomization algorithm to automatically select the first proper subset and the second proper subset is based on applying a randomization sub-algorithm of the randomization algorithm to each of the plurality of score-based groups independently to distribute prospective clinical trial participants in a given score-based group of the plurality of score-based groups between the first proper subset and the second proper subset separately from distributing other prospective clinical trial participants in other score-based groups of the plurality of score-based groups between the first proper subset and the second proper subset.

Alternatively to or in addition to any of the foregoing embodiments of the medical data processing system, the plurality of possible medical outcome prognosis scores correspond to numeric values in the full score range. Alternatively or in addition to any of the foregoing, determining the deterministic mapping is based on determining a plurality of medical outcome prognosis score ranges to define the plurality of score-based groups. Alternatively to or in addition to any of the foregoing, the plurality of medical outcome prognosis score ranges contiguously render the full score range that includes all possible medical outcome prognosis scores generated via performance of the medical outcome prognostication function. Alternatively to or in addition to any of the foregoing, each of the plurality of medical outcome prognosis score ranges include multiple corresponding ones of the plurality of possible medical outcome prognosis scores.

Alternatively to or in addition to any of the foregoing embodiments of the medical data processing system, applying the randomization sub-algorithm of the randomization algorithm to the each of the plurality of score-based groups is based on generating a random trial assignment ordering for the each of the plurality of score-based groups. Alternatively to or in addition to any of the foregoing, the random trial assignment ordering includes an ordered plurality of assignments based on participant distribution proportion data. Alternatively to or in addition to any of the foregoing, determining an ordering of prospective clinical trial participants in the each of the plurality of score-based groups. Alternatively to or in addition to any of the foregoing, distributing prospective clinical trial participants in the each of the plurality of score-based groups is based on applying the random trial assignment ordering to the ordering of prospective clinical trial participants.

Alternatively to or in addition to any of the foregoing embodiments of the medical data processing system, performing the randomization algorithm to automatically select the first proper subset and the second proper subset includes distributing prospective clinical trial participants between the first proper subset and the second proper subset based on minimizing a first difference between a first number of prospective clinical participants included the first proper subset and a second number of prospective clinical trial participants included in the second proper subset. Alternatively to or in addition to any of the foregoing, performing the randomization algorithm to automatically select the first proper subset and the second proper subset includes minimizing a second difference between: a first mean medical outcome prognosis score of first medical outcome type prognosis scores generated from medical data of prospective clinical trial participants included in the first proper subset; and a second mean medical outcome prognosis score of second medical outcome prognosis scores generated from second medical data of second prospective clinical trial participants included in the second proper subset.

Alternatively to or in addition to any of the foregoing embodiments of the medical data processing system, each device-captured medical data the first plurality of device-captured medical data and of the second plurality of device-captured medical data indicates a plurality of sensor data values captured by at least one medical device of at least one corresponding medical device type. Alternatively to or in addition to any of the foregoing, the medical outcome prognostication function is trained based on processing a first plurality of feature vectors that each include a first corresponding plurality of sensor data values for a corresponding one of the first plurality of device-captured medical data. Alternatively to or in addition to any of the foregoing, the medical outcome prognostication function is performed upon the second plurality of device-captured medical data based on processing a second plurality of feature vectors that each include a second corresponding plurality of sensor data values for a corresponding one of the second plurality of device-captured medical data.

Alternatively to or in addition to any of the foregoing embodiments of the medical data processing system, the at least one medical imaging modality includes a computed tomography (CT) scan modality. Alternatively to or in addition to any of the foregoing, the first plurality of medical imaging data includes a first plurality of CT scans. Alternatively to or in addition to any of the foregoing, the second plurality of medical imaging data includes a second plurality of CT scans.

Alternatively to or in addition to any of the foregoing embodiments of the medical data processing system, the at least one device-captured medical data type further corresponds to at least one anatomical region captured via the at least one medical imaging modality. Alternatively to or in addition to any of the foregoing, the at least one device-captured medical data type further corresponds to at least one image plane by which the at least one medical imaging modality is captured.

Alternatively to or in addition to any of the foregoing embodiments of the medical data processing system, the at least one device-captured medical data type includes a histological slide type; a DNA sequence data type; and/or a test strip type. Alternatively to or in addition to any of the foregoing, the device-captured medical data type includes at least one health sensor data type corresponding to health sensor data captured by a wearable device that includes at least one of: heart rate data; blood oxygen measurement data; blood pressure data; internal temperature data; respiratory rate data; pedometer data; and/or sleep cycle data. Alternatively to or in addition to any of the foregoing, the at least one device-captured medical data type corresponds to proteomic data, genomic data, metabolomic data, metagenomic data, phenomics data, and/or transcriptomic data (e.g. RNA transcriptions). Alternatively to or in addition to any of the foregoing, the at least one device-captured medical data type corresponds to any other one or more medical data types described herein.

Alternatively to or in addition to any of the foregoing embodiments of the medical data processing system, the training set includes a plurality of training data instances corresponding to a plurality of individuals. Alternatively to or in addition to any of the foregoing, each training data instance includes, for a corresponding one of the plurality of individuals, one of the first plurality of device-captured medical data captured for the corresponding one of the plurality of individuals as input feature data. Alternatively to or in addition to any of the foregoing, each training data instance includes, for a corresponding one of the plurality of individuals, exactly one of the first plurality of device-captured medical data captured for the corresponding one of the plurality of individuals as input feature data. Alternatively to or in addition to any of the foregoing, each training data instance includes, for a corresponding one of the plurality of individuals, more than one of the first plurality of device-captured medical data captured for the corresponding one of the plurality of individuals as input feature data. Alternatively to or in addition to any of the foregoing, each training data instance includes, for a corresponding one of the plurality of individuals, one of the corresponding plurality of medical outcome data determined for the corresponding one of the plurality of individuals as output label data. Alternatively to or in addition to any of the foregoing, the plurality of individuals are distinct from the plurality of prospective clinical trial participants.

Alternatively to or in addition to any of the foregoing embodiments of the medical data processing system, the input feature data of the each training data instance further includes, for the corresponding one of the plurality of individuals, demographic data for the corresponding one of the plurality of individuals (e.g. any one or more types of demographic data 641); medical history data for the corresponding one of the plurality of individuals (e.g. any one or more types of medical history data 642); patient history data for the corresponding one of the plurality of individuals (e.g. any one or more types of medical history data 643); medical report text data for the corresponding one of the plurality of individuals; or risk factor data for the corresponding one of the plurality of individuals (e.g. any one or more types of risk factor data 644). Alternatively to or in addition to any of the foregoing, the input feature data of the each training data instance further includes, for the corresponding one of the plurality of individuals: histology of the corresponding one of the plurality of individuals; PD-L1 Expression of the corresponding one of the plurality of individuals; gender of the corresponding one of the plurality of individuals; smoking status of the corresponding one of the plurality of individuals; prior therapy of the corresponding one of the plurality of individuals; TNM stage of the corresponding one of the plurality of individuals; performance status of the corresponding one of the plurality of individuals; and/or age of the corresponding one of the plurality of individuals. Alternatively to or in addition to any of the foregoing, the input feature data of the each training data instance further includes, for the corresponding one of the plurality of individuals, any one or more types of other relevant data 640 described herein. Alternatively to or in addition to any of the foregoing, the input feature data of the each training data instance further includes, for the corresponding one of the plurality of individuals, any one or more types of medical data metadata 610 described herein for corresponding device-captured medical data.

Alternatively to or in addition to any of the foregoing embodiments of the medical data processing system, the medical data processing system is operable to train a plurality of medical outcome prognostication functions corresponding to a plurality of different medical outcome types. Alternatively to or in addition to any of the foregoing, the medical outcome prognostication functions is one of the plurality of medical outcome prognostication functions and/or the first medical outcome type is one of the plurality of different medical outcome types. Alternatively to or in addition to any of the foregoing, the medical outcome prognostication function is selected for performance upon the second plurality of device-captured medical data for the plurality of prospective clinical trial participants of the clinical trial based on the clinical trial having the primary endpoint corresponding to the first medical outcome type. Alternatively to or in addition to any of the foregoing, the medical data processing system is operable to obtain a third plurality of device-captured medical data. Alternatively to or in addition to any of the foregoing, each of the third plurality of device-captured medical data corresponds pre-treatment medical data for a corresponding one of a second plurality of prospective clinical trial participants of a second clinical trial having another primary endpoint corresponding to a second medical outcome of the plurality of different medical outcome types. Alternatively to or in addition to any of the foregoing, the medical data processing system is operable to select a second medical outcome prognostication function of the plurality of medical outcome prognostication functions for performance upon the third plurality of device-captured medical data for the second plurality of prospective clinical trial participants of the second clinical trial based on the second clinical trial having a second primary endpoint corresponding to the second medical outcome. Alternatively to or in addition to any of the foregoing, the medical data processing system is operable to generate a second plurality of medical outcome prognosis scores corresponding to the second medical outcome based on utilizing artificial intelligence to perform the second medical outcome prognostication function upon each of the third plurality of device-captured medical data to generate a second corresponding medical outcome prognosis score of the second plurality of medical outcome prognosis scores. Alternatively to or in addition to any of the foregoing, the medical data processing system is operable to process the second plurality of medical outcome prognosis scores via the randomization algorithm to automatically select another first proper subset of the second plurality of prospective clinical trial participants and another second proper subset of the second plurality of prospective clinical trial participants based on utilizing the second medical outcome as the medical outcome prognosis score-based stratification factor of the randomization algorithm. Alternatively to or in addition to any of the foregoing, the another first proper subset and the another second proper subset are mutually exclusive. Alternatively to or in addition to any of the foregoing, the medical data processing system is operable to communicate second clinical trial assignment data indicating assignment of the another first proper subset of the plurality of prospective clinical trial participants to a second control trial arm of the clinical trial and further indicating assignment of the another second proper subset of the second plurality of prospective clinical trial participants to a second experimental trial arm of the second clinical trial.

Alternatively to or in addition to any of the foregoing embodiments of the medical data processing system, the first medical outcome type corresponds to at least one of: a mortality metric; a hospitalization metric; a count metric; a medically-defined scale-based metric; an objective tumor response; a metric for measured change; a metric for pain; at least one biomarker; any primary endpoint of the clinical trial and/or any other clinical trial; any secondary endpoint of the clinical trial and/or any other clinical trial; any surrogate endpoint of the clinical trial and/or any other clinical trial; any medical outcome type 637 described herein; and/or any other type of medical outcome.

Alternatively to or in addition to any of the foregoing embodiments of the medical data processing system, the entity conducts the clinical trial based on conducting the clinical trial for the first proper subset of the plurality of prospective clinical trial participants in conjunction with the control trial arm of the clinical trial to generate control trial arm result data. conducting the clinical trial for the second proper subset of the plurality of prospective clinical trial participants in conjunction with the experimental trial arm of the clinical trial to generate experimental trial arm result data. Alternatively to or in addition to any of the foregoing, the entity conducts the clinical trial based on generating corresponding clinical trial results based on the control trial arm result data and the experimental trial arm result data.

Alternatively to or in addition to any of the foregoing embodiments of the medical data processing system, the clinical trial is conducted to test a corresponding medical product. Alternatively or in addition to any of the foregoing, the corresponding medical product is commercially manufactured by a corresponding medical product manufacturing entity based on clinical trial results of the clinical trial comparing favorably to regulation-mandated acceptance criteria.

Alternatively to or in addition to any of the foregoing embodiments of the medical data processing system, the medical data processing system is operable to determine trial eligibility requirements for the clinical trial indicating at least one required condition. Alternatively to or in addition to any of the foregoing, the medical data processing system is operable to generate trial eligibility data for each of plurality of prospective clinical trial participants based on utilizing artificial intelligence to perform at least one condition detection function upon corresponding device-captured medical data to determine whether the at least one required condition is detected. Alternatively to or in addition to any of the foregoing, the medical data processing system is operable to identify a third proper subset of the plurality of prospective clinical trial participants that meet the trial eligibility requirements based on each having trial eligibility data indicating detection of the at least one required condition in their corresponding medical data. Alternatively to or in addition to any of the foregoing, at least one of the plurality of prospective clinical trial participants is not included in the third proper subset based on having trial eligibility data indicating detection of the least one required condition was not detected in at least one corresponding medical data. Alternatively to or in addition to any of the foregoing, the first proper subset and the second proper subset are collectively exhaustive with respect to the third proper subset of the plurality of prospective clinical trial participants.

Alternatively to or in addition to any of the foregoing embodiments of the medical data processing system, the first medical outcome type corresponds to an event occurring within and/or after a predetermined amount of time has elapsed. Alternatively to or in addition to any of the foregoing, the clinical trial has a duration that corresponds to and/or is greater than the predetermined amount of time. Alternatively to or in addition to any of the foregoing, the medical outcome prognostication function is trained to predict occurrence of first medical outcome type within and/or after the predetermined amount of time has elapsed. Alternatively to or in addition to any of the foregoing, a given one of the first plurality of device-captured medical data of the at least one device-captured medical data type is captured at a first time for a corresponding individual, and a given corresponding medical outcome data of the corresponding plurality of medical outcome data for the first medical outcome type is measured for the corresponding individual at a second time. Alternatively to or in addition to any of the foregoing, the second time is within the predetermined amount of time from the first time and/or the second time is after the predetermined amount of time from the first time. Alternatively to or in addition to any of the foregoing, the predetermined amount of time corresponds to one of: one year, two years, five years, and/or any other duration.

Alternatively to or in addition to any of the foregoing embodiments of the medical data processing system, the first medical outcome type corresponds to at least one of: a mortality metric; a hospitalization metric; a count metric; a medically-defined scale-based metric; objective tumor response; a metric for measured change; a metric for pain; at least one biomarker; any other medical outcome type; and/or any primary outcome measured in any clinical trial.

Figure 10G illustrates a method for execution by at least one processor. For example, some or all of Figure 10G can be executed via at least one medical data manufacturing entity 577, for example, in conjunction with implementing medical data manufacturing processing system 597 and/or in conjunction with communication with and/or implementing a medical data processing system 500. Alternatively or in addition, some or all of Figure 10G can be executed via at least one medical data testing entity 577, for example, in conjunction with implementing medical data testing processing system 597 and/or in conjunction with communication with and/or implementing a medical data processing system 500. For example, some or all of the steps of Figure 10G can be performed in conjunction with implementing some or all features and/or functionality of the medical data processing system of Figures 4C. Alternatively or in addition, some or all of the steps of Figure 10G can be performed in conjunction by and/or via communication with one or more data sources 575 and/or one or more requesting entities 576. Alternatively or in addition, some or all of the steps of Figure 10G can be performed in conjunction with the system 100 of Figure 1B, for example, in conjunction with implementing the imaging-based prognostication (IPRO) framework 200A and/or at least one corresponding model 112, for example, in conjunction with some or all features and/or functionality presented in Figure 8A and/or the example embodiments of Figures 8B - 9R. Alternatively or in addition, some or all of the steps of Figure 10G can be performed by, and/or based on communications between, any one or more computing devices 700, any one or more corresponding server systems, and/or any processing system that includes at least one processor and/or at least one memory.

Some or all steps of the method of Figure 10G can performed in conjunction with performance and/or can be implemented based on performing one or more steps of any other method described herein, such as any method of Figures 8B - 8G, the method of Figure 10A, the method of Figure 10C, and/or the method of Figure 10E.

Step 1202 includes developing a medical product (e.g. a pharmaceutical compound, a medical device, and/or any medical product 777). Step 1204 includes manufacturing the medical product for commercial use based on clinical trial results of a clinical trial conducted for testing the medical product meeting regulation-mandated acceptance criteria set by a regulatory entity. In some embodiments, this clinical trial is conducted based on: obtaining a plurality of device-captured medical data, where each of the plurality of device-captured medical data corresponds to pre-treatment medical data for a corresponding one of a plurality of prospective clinical trial participants of a clinical trial having a primary endpoint corresponding to the first medical outcome type; generating a plurality of medical outcome prognosis scores corresponding to the first medical outcome type based on utilizing artificial intelligence to perform the medical outcome prognostication function upon each of the second plurality of device-captured medical data to generate a corresponding medical outcome prognosis score of the plurality of medical outcome prognosis scores; processing the plurality of medical outcome prognosis scores via a randomization algorithm to automatically select a first proper subset of the plurality of prospective clinical trial participants and a second proper subset of the plurality of prospective clinical trial participants based on applying a medical outcome prognosis score-based stratification factor of the randomization algorithm, where the first proper subset and the second proper subset are mutually exclusive; conducting the clinical trial for the first proper subset of the plurality of prospective clinical trial participants in conjunction with a control trial arm of the clinical trial; and/or conducting the clinical trial for the second proper subset of the plurality of prospective clinical trial participants in conjunction with an experimental trial arm of the clinical trial. As a particular example, conducting the clinical trial for the first proper subset of the plurality of prospective clinical trial participants in conjunction with the control trial arm of the clinical trial includes not treating the first proper subset of the plurality of clinical trial participants with the medical product, and/or conducting the clinical trial for the second proper subset of the plurality of prospective clinical trial participants in conjunction with the experimental trial arm of the clinical trial includes treating the second proper subset of the plurality of clinical trial participants with the medical product.

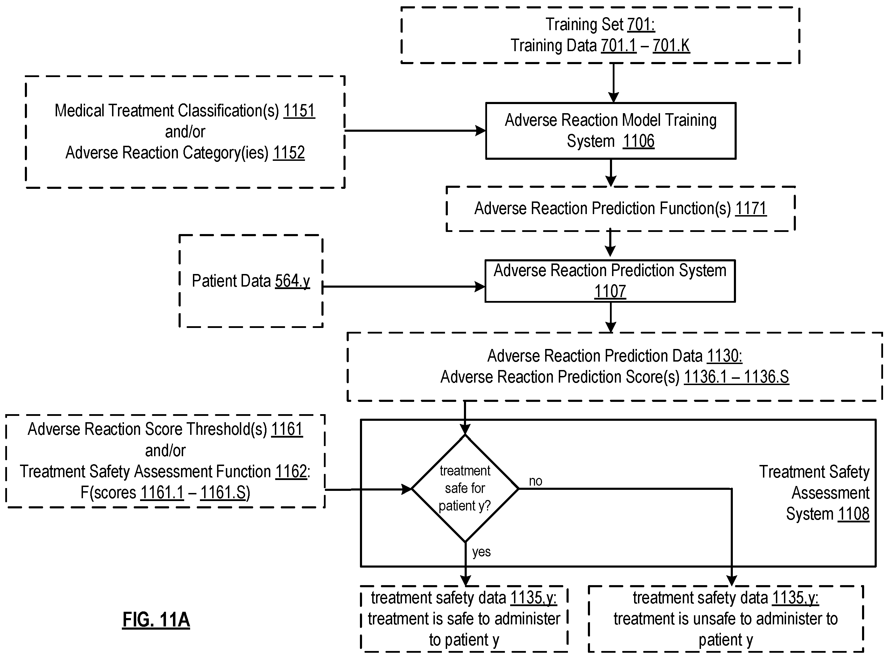

Figures 11A - 11H present embodiments of a system operable to predict one or more types of adverse reactions a patient could have to one or more types of medical treatments based on applying a trained model and/or other artificial intelligence and/or machine learning techniques to medical image data captured for the patient. Some or all features and/or functionality presented in Figures 11A - 11H can implement any embodiment of medical data processing system 500 and/or modeling platform described herein.

Consider the case where a patient is considering medical treatment, for example, to treat a medical condition exhibited by the patient. While the medical treatment would hopefully assist in the treatment of the medical condition or otherwise promoting a healthy wellbeing for the patient, the medical treatment could potentially induce at least one adverse reaction (e.g. adverse side effects), which would render use of the medical treatment unsafe or undesirable for the patient. While such adverse reactions can be linked to particular risk factors. As used herein, risk factors for adverse reaction to medical treatment can include and/or be based on: age, gender, weight, ethnicity, past or present medical condition(s), past or present use of other particular medication(s)/medical treatment(s), past or present exposure to unsafe substances and/or unsafe practices, such as smoking history, inflammation levels, hormone levels, genetics, diet, liver function, kidney function, any other patient data and/or risk factors discussed herein, and/or any other risk factors), some underlying conditions that may render a patient more susceptible to adverse reactions can be less well-defined and/or can be unknown to a given patient/corresponding medical professional, for example, due to being more difficult to detect and/or due to being more obscure.

Figures 11A - 11H present improvements to the technology of medicine, medical imaging and patient care in medical environments by presenting embodiments of a system operable to automatically predicting whether a patient will exhibit an adverse reaction to medical treatment based on processing at least one medical image of the patient, and/or other medical data and/or patient data of the patient. The medical image can be processed via a model trained to predict adverse reaction to medical treatment based on processing medical image data. For example, such a model is trained and/or applied via some or all features and/or functionality regarding processing of medical data and/or implementing modeling platform and/or one or more subsystems of medical data processing system 500 described herein. This functionality can be useful in evaluating whether it is safe/desirable to administer a medical treatment to a patient, based on whether or not adverse reaction is expected as indicated by output of the model, and/or optionally based on how severe the adverse reaction is expected to be. This information can enable a patient, and/or a medical provider caring for the patient, to determine whether the patient be treated with a medical treatment. This information can further aid in determining whether patients be included in clinical trials testing such medical treatments, to avoid inclusion of patients expected to have adverse reactions to the medical treatment being tested or for whom the medical treatment being tested is otherwise deemed unsafe/undesirable as dictated by model output. Use of such a system can thus be useful in reducing the number of patients that encounter adverse reactions to medical treatments, improving overall patient health. Use of such a system can furthermore improve the development of new medical treatments, such as new pharmaceutical compounds, by improving the conducting of corresponding clinical trials to reduce the number of trial participants that encounter adverse reactions to medical treatments based on excluding prospective participants automatically determined to be more likely to exhibit these adverse reactions.

As used herein, a given medical treatment can correspond to a single, or any combination of at least one: pharmaceutical compound, medication, drug, vaccination, supplement, medical procedure, dietary regimen, exercise regimen, and/or any product or process to be administered to and/or performed by the patient, for example, configured to treat a corresponding medical condition, physical condition, and/or mental condition, and/or configured to otherwise promote a healthy wellbeing. In some embodiments, the medical treatment can be implemented as and/or can include application/use of any embodiment of medical product 777 described herein. As used herein, a given medical treatment can have corresponding treatment plan, which can include usage/application instructions, such as count/amount (e.g. dosage) per use/application, frequency of usage/application, duration of usage/application, or other instructions dictating how/how much/how often/for how long the medical treatment be applied. As used herein, a given medical treatment and/or its corresponding usage/application instructions can be configured on a per-patient basis, or can be common to some or all patients (e.g. all patients in an investigative group of a clinical trial, all patients with a given medical condition and/or given age/weight/gender/other demographic and/or medical history, etc.).

A given medical treatment can optionally be characterized by one of a plurality of medical treatment classifications 1151 that distinguish different types of medical treatments. As used herein, medical treatment classifications 1151 can include: at least one curative treatment classification, at least one preventative treatment classification, at least one palliative treatment classification, at least one chemotherapy classification, at least one radiation therapy classification, at least one immunotherapy classification, at least one vaccine therapy classification, at least one stem cell transplantation classification, at least one blood transfusion classification, at least one surgery classification, at least one targeted therapy classification, at least one biomarker testing classification, at least one hormone therapy classification, at least one hyperthermia treatment classification, at least one photodynamic therapy classification, a set of one or more classifications based on different mechanism of actions of different medical treatments, a set of one or more classifications based on different physiologic effects of different medical treatments, a set of one or more classifications based on different chemical structures of different medical treatments, a set of one or more classifications based on different therapeutic uses of different medical treatments, a set of one or more classifications based on different formulary classification of different medical treatments, a set of one or more classifications corresponding to and/or based on the Anatomical Therapeutic Chemical (ATC) classification system, a set of one or more classifications corresponding to and/or based on the United States Pharmacopeia (USP) classification system, a set of one or more classifications corresponding to and/or based on the Uniform System of Classification (USC), a set of one or more classifications corresponding to and/or based on a classification scheme generated, maintained, and/or used by a government entity and/or regulatory entity, a set of one or more classifications corresponding to cancer treatment classifications corresponding to different types of cancer treatments, a set of one or more classifications corresponding to different individual medical products implementing different medical treatments, a set of one or more classifications corresponding to different dosages, usage amounts, usage frequencies, and/or usage durations of a given medical treatment and/or a broader medical treatment classification, and/or any other one or more classifications for different types of medical treatment.

In some embodiments, some or all treatment classifications 1151 are implemented in accordance with a hierarchical structure, where some treatment classifications 1151 have corresponding sub-classifications, which themselves can be considered further treatment classifications 1151. For example, the hierarchical structure is implemented as, based on, and/or similar to the hierarchical structure of the ATC classification system, and/or includes a set of hierarchical level such as a first (top) level corresponding to organ system and/or pharmacological classification; a second level corresponding to therapeutic subgroup, a third level corresponding to pharmacological subgroup, a fourth level corresponding to chemical subgroup, and/or a fifth level corresponding to chemical substance. As another example, the hierarchical structure is implemented as, based on, and/or similar to the hierarchical structure of the USP classification system, and/or includes a set of hierarchical level such as a first (top) level corresponding to therapeutic category, a second level corresponding to pharmacologic class, and/or a third level corresponding to formulary key drug types. As another example, the hierarchical structure further includes different dosage/duration that can be applied for a given medical treatment. Some or all treatment classifications 1151 described herein can optionally include multiple treatment classifications 1151, for example, based on the hierarchical structuring or otherwise grouping multiple similar classifications (e.g. a treatment classification 1151 corresponding to cardiovascular agents includes all relevant classification subcategories, such as a set of subcategories that includes: alpha-adrenergic agonists; alpha-adrenergic blocking agents; antiarrhythmics; beta-adrenergic blocking agents, calcium channel blocking agents, diuretics, dyslipidemias, renin-angiotensin-aldosterone system inhibitors, vasodilators, and/or other cardiovascular agents, where each of the subcategories optionally further includes its own set of subcategories).

As used herein, an adverse reaction can correspond to one or more adverse drug reactions, adverse events, side effects, and/or other physical/mental reaction, for example, definitively and/or suspected to be caused by administering of medical treatment. Adverse reactions can include unwanted, negative reactions, for example, that are harmful to and/or unpleasant when encountered by a corresponding patient.

A given adverse reaction can optionally be characterized by one of a plurality of adverse reaction categories 1152 that distinguish different types of adverse reactions. As used herein, medical treatment classifications 1151 can include: at least one dose-related (e.g. augmented, predictable non-immunological, and/or intolerance) reaction, at least one non-dose related (e.g. bizarre, unpredictable, immunological, and/or allergic) reaction, at least one idiosyncratic reaction, at least one time-related (e.g. delayed) reaction, at least one withdrawal (e.g. end of use) reaction, at least one failure of therapy (e.g. failure) reaction; a set of one or more categories based on severity of the adverse reaction (e.g. mild, moderate, severe, lethal, or other scale of different severity); whether the adverse reaction is lethal or non-lethal, a set of categories corresponding to different probabilities the adverse reaction will be lethal (e.g. unlikely vs. moderately likely vs. very likely, or other scale), a set of one or more symptom categories based on how the adverse reaction is exhibited (e.g. cough, nausea, vomiting, diarrhea, constipation, headache, rash, itching, jaundice, anemia, decrease in white blood cell count, kidney damage, nerve injure, vision impairment, hearing impairment, dizziness, fatigue, confusion, and/or other symptoms); a set of one or more categories corresponding to different medical conditions induced as the adverse reaction (e.g. cancer, diabetes, etc.). a set of one or more categories based on when adverse reaction is exhibited after treatment is administered (e.g. immediately vs. days later vs. weeks later, or any time scale); a set of one or more categories based on how long the adverse reaction is exhibited (e.g. minutes vs. hours later vs. days vs. weeks vs. years, or any time scale); a set of one or more categories corresponding to different medical outcome types 637; a set of one or more categories corresponding to different clinical endpoints; and/or other categorization of various types of adverse reactions that can be caused by a given medical treatment, that can be caused by various medical treatments of a given medical treatment classification, and/or that can be caused by various types of medical treatments of various different medical treatment classifications.

As illustrated in Figure 11A, an adverse reaction model training system 1106 can be implemented to train at least one adverse reaction prediction function 1171. This can be based on processing a training set 701 that includes a plurality of training data 701.1 - 701.K. The training set 701 of Figure 11A can implement some or all features and/or functionality of any embodiment of training set 701 described herein. In some embodiments, the plurality of training data 701.1 - 701.K includes a plurality of medical data 562, such as a plurality of medical image data corresponding to any one or more medical imaging modalities, and/or some or all of a plurality of corresponding patient data. In some embodiments, the plurality of training data 701.1 - 701.K is labeled with truth data indicating, for some or all corresponding medical image data and/or other medical data: one or more medical treatment classifications for particular medical treatments taken by the patient and/or one more adverse reactions induced by the corresponding patient, for example, in response to the one or more particular medical treatment. In some embodiments, the plurality of training data 701.1 - 701.K is labeled with truth data indicating, for some or all corresponding medical image data and/or other medical data: one or more particular risk factors mapped to and/or otherwise associated with one more adverse reactions induced by the corresponding patient and/or associated with adverse reaction to medical treatments falling under one or more particular medical treatment classifications.

Adverse reaction model training system 1106 can train one or more adverse reaction prediction functions 1171, each operable to generate adverse reaction prediction data for one or more particular medical treatment classifications 1151 and/or one or more particular adverse reaction categories 1152.

Training the one or more adverse reaction prediction functions 1171 can be optionally based on applying training data labeled with truth data distinguishing between adverse reaction categories of (and/or lack of) adverse reactions for the corresponding patient and/or as detected in the medical image, and/or to distinguish between medical treatment classifications of (and/or lack of) medical treatments administered the corresponding patient. This can be optionally based on applying different training data sets corresponding to different adverse reaction categories and/or different medical treatment classifications for training different corresponding adverse reaction prediction functions.

Selection of training data (e.g. for retrieval from the data storage system 560 or other location) can include configuring which corresponding medical data and/or patient data be included, for example, based on whether the corresponding medical data and/or patient data has truth data matching configured parameters, such as whether the medical data and/or patient data corresponds to a patient: having been treated with a medical treatment; having been treated with a medical treatment of a particular medical treatment classification for which one or more adverse reaction prediction function 1171 are to be trained to predict probability of adverse reaction; having been treated with no medical treatment (for example, to enable distinguishing between patients having undergone treatment vs not); having a particular medical condition to be treated via the medical treatment; having an adverse reaction (or having a corresponding medical condition that is a risk factor for the adverse reaction), for example after being treated with the medical treatment; having an adverse reaction of a particular adverse reaction category for which one or more adverse reaction prediction function 1171 are to be trained to predict (or corresponding medical condition that is a risk factor for the particular adverse reaction category), for example after being treated with the medical treatment; having no adverse reaction, for example despite being treated with the medical treatment (e.g. to enable distinguishing between whether or not the adverse reaction occurred, for example, in the case where a corresponding medical condition to be treated by the medical treatment is present); and/or other parameters. In some cases, some or all medical data (e.g. medical images) of the training data 507 are selected based on corresponding to pre-medical treatment image data (e.g. captured before the corresponding patient received medical treatment), where this medical data is optionally labeled with truth data indicating whether they did or did not exhibit an adverse reaction after receiving the medical treatment (e.g. and/or optionally labeled with truth data indicating at least one adverse reaction category of the adverse reaction, and/or optionally labeled with truth data indicating a medical treatment classification of at least one particular medical treatment administered), and/or where this medical data is optionally labeled with truth data indicating (or is processed via image processing techniques to detect evidence of) risk factors for adverse reaction (e.g. evidence of a medical condition exhibited in the medical image that corresponds to a risk factor for the adverse reaction.

In some embodiments, the system is optionally operable to automatically detect risk factors for adverse reactions based on processing the training data and identifying patterns denoting types of medical conditions, patient history, or other information exhibited in medical data or patient data having high correlation with adverse reaction to medical treatment, where these automatically detected risk factors are optionally utilized in predicting adverse reaction and/or are optionally displayed, stored, and/or communicated to a medical entity and/or requesting entity.

In some embodiments, risk factors correlating with adverse reactions is known, for example, as a predetermined mapping and/or data indicating correlation with a given risk factor to adverse reaction (and/or particular adverse reaction category(ies) to medical treatment (and/or in response to medical treatment of a particular medical treatment classification), where this information is received, stored, and/or processed in training some or all adverse reaction prediction functions 1171 to enable prediction of adverse reaction based on detection of corresponding risk factors with known correlation to these adverse reactions

A given adverse reaction prediction function 1171 can optionally be implemented via a multi-modal model (e.g. configured to process multiple different types of medical scans, medical data, and/or patient data) and/or can be implemented via a multi-output model (e.g. configured to generate multiple outputs corresponding to multiple predictions, such as predictions corresponding to multiple different types of adverse reactions, and/or predictions corresponding to adverse reaction in response to multiple different types of medical treatment). Alternatively or in addition, some or all different types of inputs and/or different types of outputs can be configured for processing/generation accordingly via applying different corresponding adverse reaction prediction functions 1171 trained by the adverse reaction model training system 1106. Alternatively or in addition, a given adverse reaction prediction function 1171 can be retrained over time.

One or more adverse reaction prediction functions 1171 trained by adverse reaction model training system 1106 can be performed upon patient data 564.y of a given patient y to generate adverse reaction prediction data 1130 as output. The adverse reaction prediction data 1130 can include one or more adverse reaction prediction scores 1136.1 - 1136.S.

In some embodiments, a given adverse reaction prediction score 1136 corresponds to a predicted score indicating probability of adverse reaction to medical treatment. For example, adverse reaction prediction score 1136 is implemented as, is based on, and/or indicates: a computed probability value between 0 and 1; a corresponding predicted continuous value corresponding to a predicted measurement of adverse reaction, for example, of a corresponding adverse reaction type; a corresponding predicted category of a set of two or more categories (e.g. whether or not an adverse reaction is expected, which of a set of adverse reaction categories is expected, whether treatment is safe or unsafe; etc.); and/or other automatically computed one or more values corresponding to other information regarding likelihood of, severity of, duration of, and/or type of adverse reaction to medical treatment.

In some embodiments, a given adverse reaction prediction score 1136 can correspond to a particular medical treatment classification (e.g. score 1136.1 corresponds to medical treatment classification 1151.1 indicating probability of patient y having an adverse reaction if administered medical treatment of medical treatment classification 1151.1; score 1136.2 corresponds to medical treatment classification 1151.2, indicating probability of patient y having an adverse reaction if administered medical treatment of medical treatment classification 1151.1).

Alternatively or in addition, a given adverse reaction prediction score 1136 can correspond to a particular adverse reaction category (e.g. score 1136.1 corresponds to adverse reaction category 1152.1 indicating probability of patient y having an adverse reaction of adverse reaction category 1 to medical treatment; score 1136.2 corresponds to adverse reaction category 1152.2, indicating probability of patient y having an adverse reaction of adverse reaction category 1152.2 to medical treatment).

Alternatively or in addition, a given adverse reaction prediction score 1136 can correspond to a particular adverse reaction category and/or can correspond to a particular medical treatment classification (e.g. score 1136.1 corresponds to medical treatment classification 1151.1 and adverse reaction category 1152.1, indicating probability of patient y having an adverse reaction of adverse reaction category 1152.1 if administered medical treatment of medical treatment classification 1151.1; score 1136.2 corresponds to medical treatment classification 1151.1 and adverse reaction category 1152.2, indicating probability of patient y having an adverse reaction of adverse reaction category 2 if administered medical treatment of medical treatment classification 1151.1; score 1136.3 corresponds to medical treatment classification 1151.2 and adverse reaction category 1152.1, indicating probability of patient y having an adverse reaction of adverse reaction category 1152.1 if administered medical treatment of medical treatment classification 1151.2; score 1136.4 corresponds to medical treatment classification 1151.2 and adverse reaction category 1152.2, indicating probability of patient y having an adverse reaction of adverse reaction category 1152.2 if administered medical treatment of medical treatment classification 1151.2; etc.).

Different adverse reaction prediction scores 1136 can be generated by the same adverse reaction prediction function operable to generate multiple output for multiple adverse reaction categories 1152 (e.g. a same adverse reaction prediction function 1171 generates both score 1136.1 and 1136.2) and/or multiple medical treatment classifications 1151 or by different adverse reaction prediction functions operable to generate output for different adverse reaction categories 1152 and/or different medical treatment classifications 1151 (e.g. a first adverse reaction prediction function 1171.1 generates score 1136.1 and a second adverse prediction function 1171.2 generates score 1136.2 based on these two functions being configured for different corresponding adverse reaction categories and/or medical treatment classifications).

In some embodiments, scores 1136 are generated for only a proper subset of possible medical treatment classifications 1151 and/or adverse reaction categories 1152 for which the one or more adverse reaction prediction functions 1171 are trained. For example, despite the one or more adverse reaction prediction functions 1171 being operable to output probability of adverse reaction for many types of medical treatment classifications, the one or more scores 1136 generated in adverse reaction data 1130 for patient y correspond to only one particular medical treatment classification (or multiple relevant medical treatment classifications that do not include every medical treatment classification), for example, based on the patient being considered as a prospective candidate for medical treatment of this medical treatment classification, and not others (e.g. the patient has a corresponding medical condition that could be treated by medical treatment of this classification; the patient is a prospective candidate for a clinical trial for medical treatment of this classification, etc.). As another example, despite the one or more adverse reaction prediction functions 1171 being operable to output probability of many different adverse reaction categories, the one or more scores 1136 generated in adverse reaction data 1130 for patient y correspond to only one particular adverse reaction category (or multiple relevant adverse reaction categories that do not include every adverse reaction category), for example, based on this category being the only relevant factor in determining whether to administer corresponding medical treatment to the patient y (e.g. this category dictates whether candidates be excluded from a clinical trial for which the patient y is a prospective candidate, while others are irrelevant to the patients ability to participate in the trial, the patient's current medical condition is severe enough such that less severe categories of adverse reaction are less relevant in decision to pursue medical treatment for patient y, etc.). Selecting which adverse reaction categories and/or medical treatment classifications have corresponding scores 1136 be generated for a given patient and/or have corresponding adverse reaction prediction functions 1171 are applied to the given patient's medical data 564.y can be configured via user input, can be automatically selected via an automated process, and/or can otherwise be determined optionally on a patient by patient basis or trial by trial basis, for example, based on which adverse reaction categories and/or medical treatment classifications are relevant for the patient and/or a corresponding clinical trial at the given time. In other embodiments, all scores are generated for the patient, even if not all adverse reaction categories/medical treatment classifications are relevant for the patient at the given time. In other embodiments, the one or more adverse reaction prediction functions 1171 is configured to only output scores for one adverse reaction category (or broadly across some or all adverse reaction categories and/or medical treatment classification (or broadly across some or all medical treatment classifications). In some embodiments, the one or more adverse reaction prediction functions 1171 is configured to only output scores for one adverse reaction category and/or medical treatment classification.

A treatment safety assessment system 1108 can be implemented to automatically process the adverse reaction prediction data 1130 to generate treatment safety data 1135.y for the patient y indicating whether or not the medical treatment (e.g. one or more particular treatments of medical treatment classification for which scores were generated and/or for which the patient is a candidate) is recommended to be administered to the patient (e.g. as binary output, textual and/or graphical output for display and/or communication to a medical professional, etc.). This can include assessing whether or not medical treatment should be administered to the patient, for example, as an automatic assessment of whether or not the medical treatment is safe (e.g. based on whether adverse reactions are expected and/or how severe these adverse reactions are expected to be, for example, relative to the severity of the condition being treated). This can include generating the treatment safety data based on comparing some or all adverse reaction predication scores 1136 to one or more corresponding predefined adverse reaction prediction score thresholds 1161 and/or based on performing a predefined function F upon some or all adverse reaction prediction scores 1136.1 - 1136.S and optionally comparing the output of this function to a corresponding predefined threshold value. For example, if an adverse reaction score 1136 (e.g. for a particular medical treatment classification) exceeds a corresponding score threshold 1161 and/or otherwise compares unfavorably to the score threshold based on the probability of the adverse reaction being too high as defined by the score threshold 1161, the treatment safety data 1135.y is generated to indicate the corresponding medical treatment (e.g. of the particular medical treatment classification) is unsafe; while if the adverse reaction score 1136 (e.g. for the particular medical treatment classification) falls below and/or compares favorably to the corresponding score threshold 1161 and/or otherwise compares favorably to the score threshold based on the probability of the adverse reaction being low enough as defined by the score threshold 1161, the treatment safety data 1135.y is generated to indicate the corresponding medical treatment (e.g. of the particular medical treatment classification) is safe.

In some embodiments, different adverse reaction categories have different thresholds. For example, such differences can be based on their severity. As a particular example, the threshold score for deeming medical treatment is safe is stricter (e.g. requiring very low probability of adverse reaction/requiring very high probability of no adverse reaction to be safe) for scores corresponding to adverse reaction categories that are more severe, such as an adverse reaction category corresponding to death and/or a condition likely to result in death, and is looser (e.g. allowing higher probability of adverse reaction/requiring less high probability of no adverse reaction to be safe) for scores corresponding to adverse reaction categories that are more severe, such as an adverse reaction category corresponding to headache or other milder reaction.

In some embodiments, different medical treatment classifications have different thresholds. For example, such differences can be based on the severity of the condition being treated and/or the expected success of the medical treatment in treating the condition. As a particular example, the threshold score for deeming medical treatment is safe is looser (e.g. allowing higher probability of adverse reaction/requiring less high probability of no adverse reaction to be safe) for adverse reactions corresponding to medical treatment classifications treating more severe medical conditions (e.g. cancer) and/or that have a higher probability of being effective (e.g. treatment is well studied, and/or has high rate of alleviating the condition), and is stricter (e.g. requiring very low probability of adverse reaction/requiring very high probability of no adverse reaction to be safe) for medical treatment classifications treating less severe medical conditions (e.g. allergies) and/or that have a lower probability of being effective (e.g. treatment is still undergoing medical trials, and/or has a lower rate of alleviating the condition).

In some embodiments, the predefined thresholds 1161 and/or predefined function F can be configured via user input and/or can be automatically generated. In some embodiments, the predefined thresholds 1161 and/or predefined function F can be configured differently (E.g. via user input or automatically) for different patients (e.g. based on the severity, such as probability of death and/or predicted medical outcome as described herein, of the current medical condition exhibited by the patient to be treated via the proposed medical treatment) and/or for different clinical trials (e.g. based on regulations regarding safety of conducting the clinical trial, other desires for candidate selection by administrators of the clinical trial, etc.).

The treatment safety data 1135.y generated for the patient can be communicated to the medical storage system, a requesting entity, or other entity (e.g. the treatment safety data for storage in patient data for the patient, for display/audio output for viewing by/dictation to the patient, the medical professional caring for the patient, a person administering a clinical trial and determining whether prospective participants are eligible.

Additional adverse reaction prediction data and/or corresponding treatment safety data can be generated for the given patient over time, for example, as a given medical condition progresses and/or new medical images are captured for processing as input, as the patient becomes eligible for/a prospective recipient of other medical treatment of other medical classification categories, as the adverse reaction prediction functions are retrained over time to become more accurate or have more specificity in identifying different types of reactions or in detecting reaction for different types of medical classifications, etc.

Additional adverse reaction prediction data and/or corresponding treatment safety data can be generated for other patients in a same or similar fashion, at the same time (e.g. for a batch of patients eligible for a clinical study of a medical treatment) or at different times (e.g. as medical scans for the patients are collected or as the patients are identified at different times for being potentially treated via medical treatment).

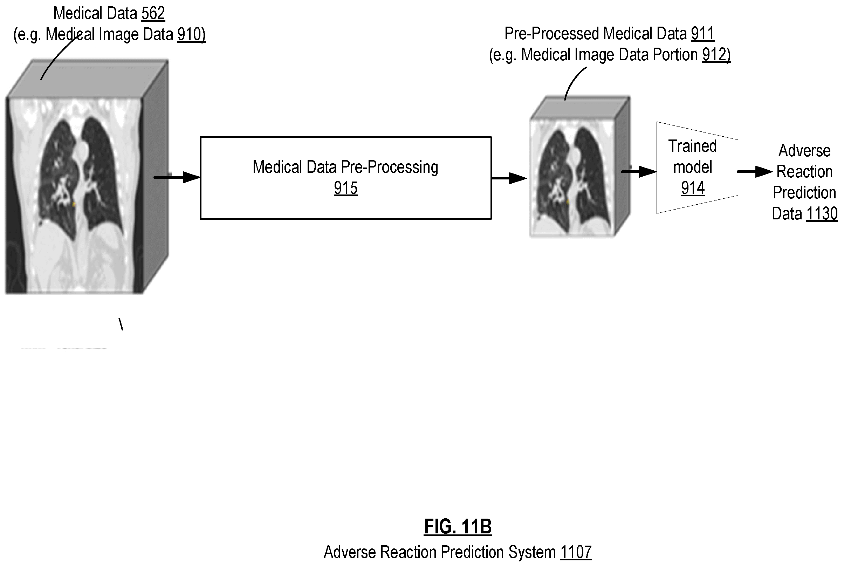

Figure 11B illustrates an embodiment of adverse reaction prediction system 1107. Some or all features and/or functionality of the adverse reaction prediction system 1107 of Figure 11B can implement any embodiment of the adverse reaction prediction system 1107 described herein. In some embodiments, the medical processing prediction system 1107, the one or more adverse reaction prediction functions 1171, and/or corresponding training of these functions via adverse reaction model training system 1106 can be implemented via some or all functionality of the modeling platform operable to process medical images discussed in conjunction with some or all features and/or functionality Figures 8A - 9R. As illustrated in Figure 11B, medical images can be processed via some or all features and/or functionality of the modeling platform discussed in conjunction with some or all of Figures 8A - 9R to generate adverse reaction prediction data 1130, alternatively or in addition to generating the medical outcome prognosis score 636 as illustrated in Figure 8A. In some embodiments, some or all embodiments of the adverse reaction prediction data 1130 and/or the underlying adverse reaction prediction scores 1136.1 - 1136.S are implemented in a same or similar fashion as the medical outcome prognosis score 636, where the corresponding medical outcome optionally corresponds to an adverse reaction to a medical treatment, and/or corresponds to another medical outcome that is a risk factor for and/or has known correlation with the adverse reaction to a medical treatment.

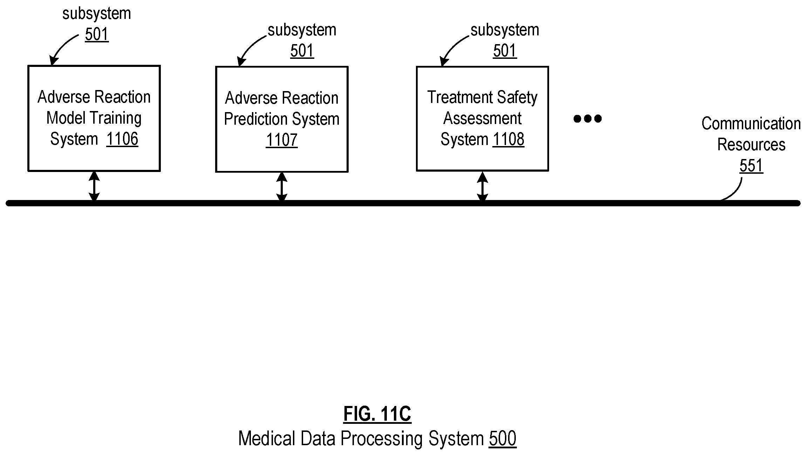

Figure 11C illustrates an embodiment of medical data processing system 500. Some or all features and/or functionality of the medical data processing system 500 of Figure 11C can implement any embodiment of the medical data processing system 500 described herein.

The medical data processing system 500 can be operable to implement some or all of the functionality of Figure 11A for example, based on implementing the adverse reaction model training system 1106, the adverse reaction prediction system 1107, and/or the treatment safety assessment system 1108 as respective subsystems 501, for example, alternatively or in addition to implementing some or all other subsystems 501 described herein.

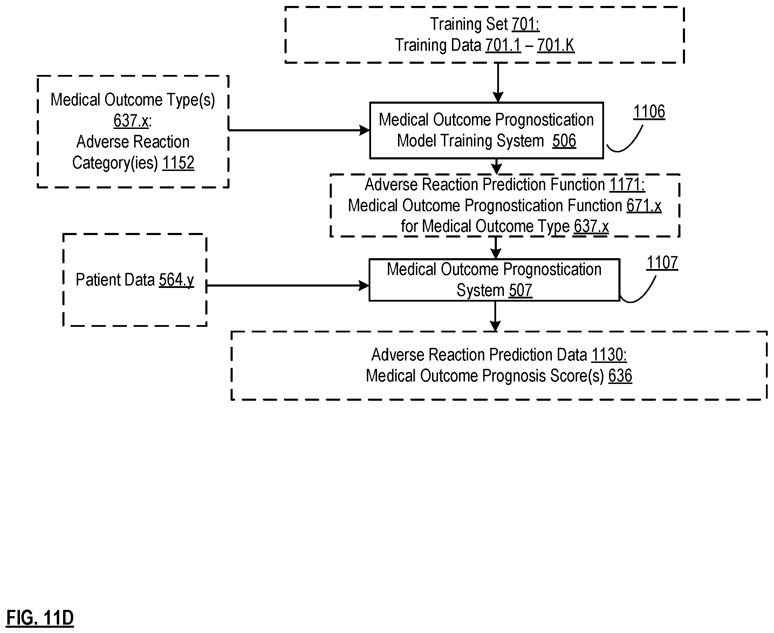

Figure 11D illustrates an embodiment of generating adverse prediction data 1130 based on implementing the adverse reaction prediction system 1107 via some or all features and/or functionality of the medical outcome prognostication system 507 and/or based on implementing adverse reaction prediction model training system 1106 via some or all features and/or functionality of the medical outcome prognostication model training system. For example, the one or more adverse reaction categories for which the adverse reaction prediction function is trained to predict adverse reaction probability can be implemented as one or more corresponding medical outcome types 637 (e.g. the medical outcome type corresponds to exhibiting of the corresponding type of adverse reaction), where the adverse reaction prediction function 1171 is optionally implemented as and/or based on at least one corresponding medical outcome prognostication function 671 configured for predicting medical outcome type 637 corresponding to adverse reaction. Thus, some or all adverse reaction prediction scores 1136 of adverse reaction prediction data 1130 generated in processing patient data 564 (e.g. a medical image and/or other medical data or patient data for a corresponding patient) can be implemented as a medical outcome prognosis score for the medical outcome type 637, as the medical outcome type 637 corresponds to the adverse reaction. In such cases, as the medical outcome prognostication function is configured to predict adverse reaction, this medical outcome optionally does not correspond to, or is not directly related to, the clinical endpoint of a clinical trial (e.g. while a clinical trial might be testing a medical treatment for a medical condition, its clinical endpoint may not correspond to a measurable event corresponding to presence of an adverse reaction to the medical treatment, but instead corresponds to a clinical endpoint measuring effectiveness of the medical treatment in treating the medical condition, such as hospitalization or death induced by the medical condition and not the adverse reaction. Some or all features and/or functionality of generating adverse prediction data 1130 and/or training adverse reaction prediction function 1171 of Figure 11D can implement any embodiment of generating adverse prediction data 1130 and/or training adverse reaction prediction function 1171 described herein.

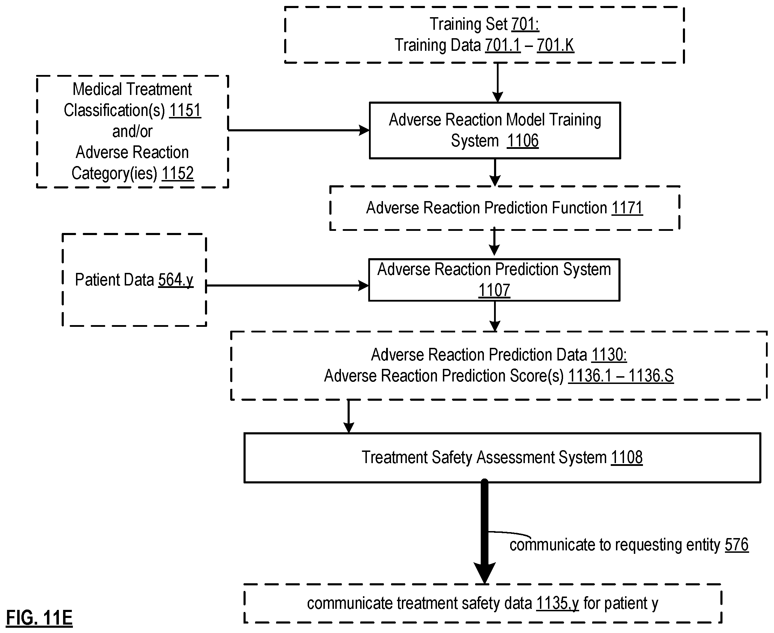

Figure 11E illustrates an embodiment where treatment safety assessment system 1108 is operable to communicate treatment safety data 1135.y for patient y to a requesting entity 576. Some or all features and/or functionality of the treatment safety assessment system 1108 of Figure 11E can implement any embodiment of the treatment safety assessment system 1108 described herein. The treatment safety data 1135.y can be communicated based on being sent to requesting entity 576, stored by requesting entity 576, displayed via a display device of requesting entity 576, or otherwise being communicated. The requesting entity 576 can be implemented via computing resources corresponding to the patient y, to a medical provider for the patient y, for an entity associated with conducting a clinical trial for which patient y is being considered for participation, and/or any requesting entity 576 described herein.

The binary information treatment safety data 1135.y (e.g. whether a given medical treatment is deemed safe for administering to the patient) can be communicated to requesting entity 576 alternatively or in addition to communicating some or all of the raw adverse reaction prediction data 1130 to the requesting entity 576 for corresponding receipt/display/storage/transmission/etc. In some embodiments, adverse reaction system 1107 communicates the adverse reaction prediction data 1130 to requesting entity 576, for example, to enable the raw data and/or actual emitted probability value to be reviewed. In some embodiments, the treatment safety assessment system is not implement, where only this raw output of adverse reaction prediction data 1130 of the adverse reaction prediction system 1130 is communicated/stored/displayed without the further processing by treatment safety assessment system 1108.

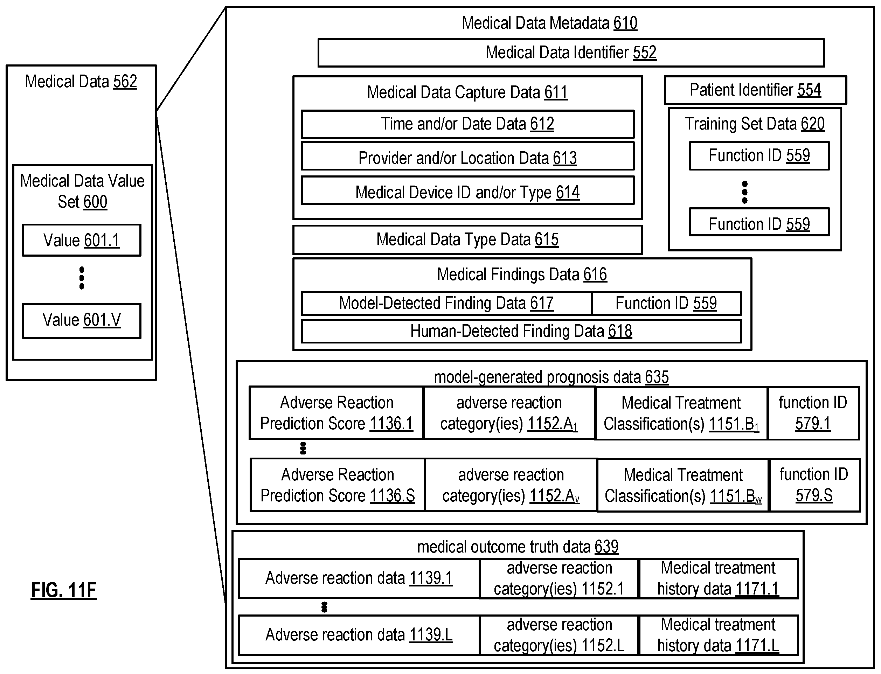

Figure 11F illustrates an embodiment of medical data metadata 610. Some or all features and/or functionality of the medical data metadata 610 of Figure 11F can implement any embodiment of medical data metadata 610 described herein. In particular, the medical data metadata 610 can include information regarding administering of medical treatments and/or corresponding adverse reactions to these treatments, alternatively or in addition to any other the other information of medical data metadata 610 of other embodiments of medical data metadata 610 described herein.

As illustrated in Figure 11F, model-generated prognosis data 635 of given medical data metadata 610 can include at least one adverse reaction prediction score 1136 generated via adverse reaction prediction function processing the corresponding medical data 562 (e.g. where the medical data 562 corresponds to medical image data). The adverse reaction category 1152 and/or medical treatment classification 1151 for each adverse reaction prediction score 1136 can optionally be indicated, and/or the function identifier 579 identifying which adverse reaction prediction function 1171 generated each adverse reaction prediction score 1136 can optionally be indicated. Note that multiple adverse reaction prediction scores 1136 generated for a given medical data can be generated by a same function 1171; can have a same adverse reaction category 1152; and/or can have a same medical treatment classification 1151.

Alternatively or in addition to the model-generated prognosis data 635, medical outcome truth data 639 of given medical data metadata 610 can include at least one adverse reaction data 1139, which can each indicate a corresponding adverse reaction category 1152 and/or medical treatment history data 1171 indicating medical treatment believed to induce the corresponding adverse reaction. The adverse reaction data 1139 can indicate whether or not an adverse reaction of the corresponding adverse reaction category was exhibited after administering of medical treatment indicated in medical treatment history data 1171, and/or can indicate further information such as the corresponding symptoms, severity, whether or not the reaction was lethal, etc. This medical outcome truth data 639 can be utilized to train and/or retrain a corresponding adverse reaction prediction function. In the case where model-generated prognosis data 635 was also generated, the medical outcome truth data 639 can be utilized to determine whether corresponding adverse reaction prediction scores 1136 were reasonable or not, and/or can be utilized to facilitate retraining of corresponding functions with corresponding function identifiers 579. In some embodiments, the medical outcome truth data includes no such information due to the corresponding patient not having received the medical treatment (e.g. based on their adverse reaction prediction score denoting treatment was unsafe, not having undergone medical treatment, and/or not yet having exhibited adverse reaction to medical treatment).

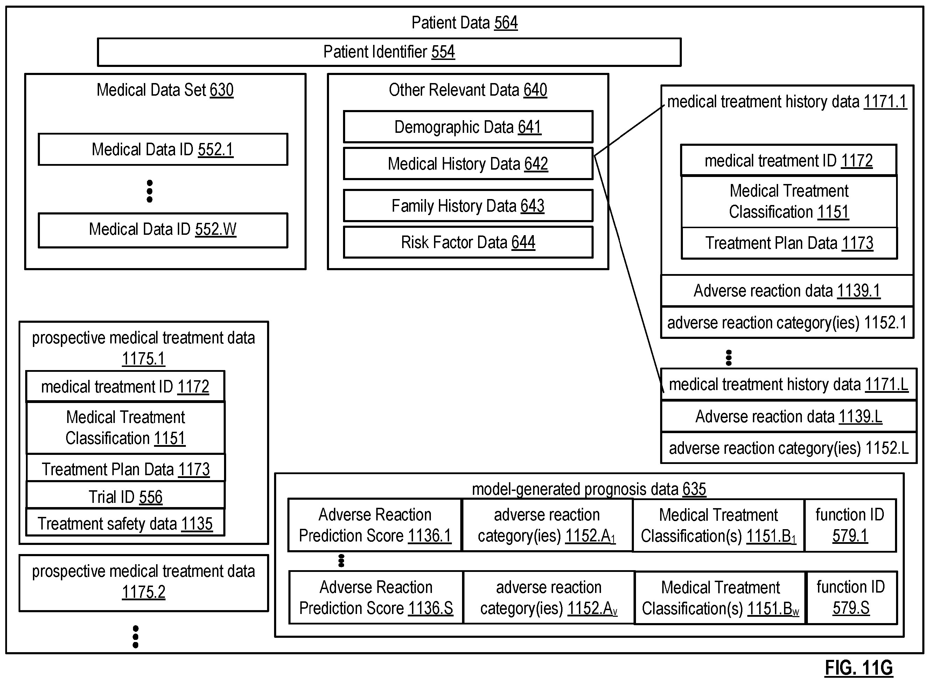

Figure 11G illustrates an embodiment of patient data 564. Some or all features and/or functionality of the patient data 564 of Figure 11G can implement any embodiment of patient data 564 described herein. In particular, the medical data metadata 610 can include information regarding administering of medical treatments and/or corresponding adverse reactions to these treatments, alternatively or in addition to any other the other information of patient data 564 of other embodiments of medical data metadata 610 described herein.

As illustrated in Figure 11G, the medical history data 642 of given patient data 564 can indicate, alternatively or in addition to other information indicated in medical history data as described herein, one or more medical treatment history data 1171 corresponding to current and/or past medical treatment administered to the patient. Given medical treatment history data 1171 can indicate a particular medical treatment via a corresponding medical treatment identifier 1172; a medical treatment classification 1151 for this medical treatment (or multiple classifications if applicable); and/or treatment plan data 1173, for example, regarding dosage/amount administered, how often, duration, and/or corresponding dates/times (e.g. relative to the current date). This can be mapped to adverse reaction data 1139.1 (e.g. whether the patient had adverse reaction to this treatment, and/or further information), and/or the adverse reaction categories for the adverse reaction, if applicable. This medical history data 642 can map to and/or otherwise be utilized to dictate medical outcome truth data 639.

Alternatively or in addition, as illustrated in Figure 11G, given patient data 564 can indicate model-generated prognosis data 635 indicating the one or more adverse reaction prediction scores 1136 generated for a given medical data (e.g. given medical image or batch of corresponding data processed at a given time), or optionally multiple different medical data (e.g. different adverse reaction prediction scores 1136 generated based on different medical images being processed, for example, at different dates as the patient is observed over time). These adverse reaction prediction scores 1136 can map to the adverse reaction prediction scores 1136 in medical scan metadata 610 for medical data upon which the corresponding adverse reaction prediction function was performed.

In some cases, the adverse reaction prediction scores correspond only to medical treatment classifications for which the patient has prospective medical treatment data 1175. In other embodiments, the adverse reaction prediction scores correspond only to other ,medical treatment classifications, including those for which the patient has no prospective medical treatment data 1175, for example, to provide more information and/or enable evaluation of other medical treatments that may be considered to be performed upon the patient at a later date.

Alternatively or in addition, as illustrated in Figure 11G, given patient data 564 can indicate prospective medical treatment data for one or more medical treatments being considered by the patient and/or corresponding medical provider to be administered. This can include medical treatment ID 1172, medical treatment classification 1151, and treatment plan data 1173, as well as trial ID 556 for a corresponding clinical trial (if applicable) and/or treatment safety data 1135 generated for the patient, for example, based on an adverse reaction prediction score 1136 for the corresponding medical treatment classification 1151 in the patient's model-generated prognosis data 635, if available. Alternatively, the model-generated prognosis data 635 is optionally not included in the patient data, for example, based on only the treatment safety data 1135 being communicated and/or stored. In cases where the prospective medical treatment of given prospective medical treatment data is ultimately administered to the patient, this prospective medical treatment data 1175 can become medical treatment history data 1171. In cases where the given prospective medical treatment data is ultimately not administered to the patient (e.g. due to treatment safety data 1135 indicating treatment is unsafe), this prospective medical treatment data 1175 can still optionally be maintained in the patient data, for example, to indicate this medical treatment classification is/was unsafe, if same or similar treatment is considered in the future.

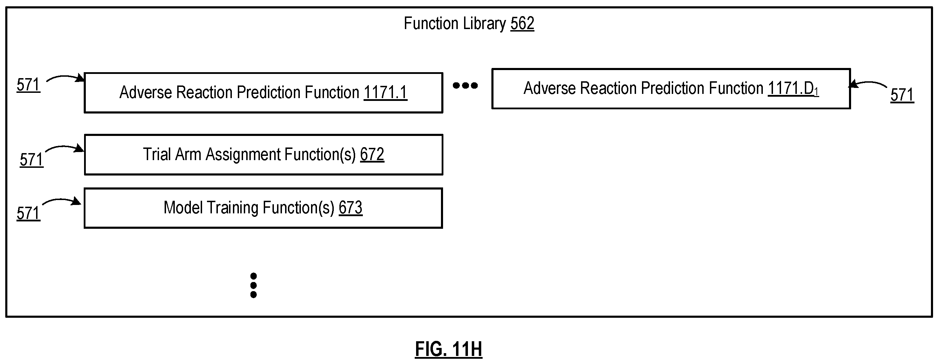

Figure 11H illustrates an embodiment of function library 562. Some or all features and/or functionality of function library 562 and/or the underlying function definitions can implement any embodiment of function library described herein. Some or all adverse reaction prediction functions 1171 trained by adverse reaction model training system 1106 described herein can optionally be implemented as functions 571 of function library 562, and/or some or all training functions implemented by any embodiment of adverse reaction model training system 1106 described herein can be implemented as model training functions 673 of function library.

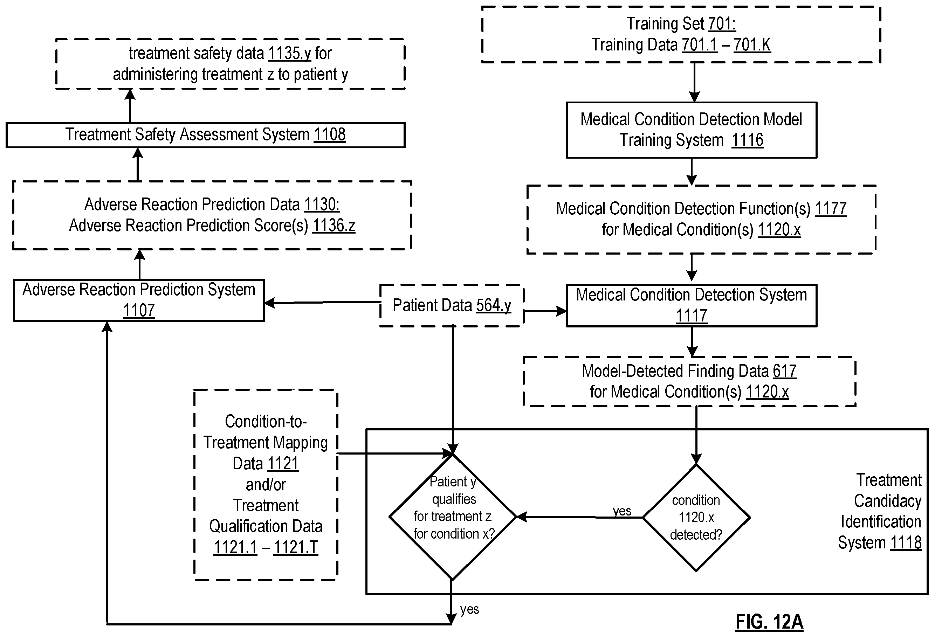

Figures 12A - 12C illustrate embodiments of a system that automatically identifies prospective medical treatments for patients based on automatically detecting corresponding medical conditions that can be treated by such medical treatments (e.g. via processing their medical image data and/or other medical data and/or patient data via artificial intelligence and/or machine learning techniques). Some or all features and/or functionality presented in Figures 12A - 12C can be implemented in tandem with some or all features and/or functionality of some or all of Figures 11A - 11H. Some or all features and/or functionality presented in Figures 12A - 12C can implement any embodiment of medical data processing system 500 and/or modeling platform described herein.

As illustrated in Figure 12A, a medical condition detection model training system 1116 can be operable to generate one or more medical condition detection functions 1177 operable to detect one or more corresponding medical conditions 1120 via processing a corresponding training set 701. Training set 701 can be implemented via some or all features and/or functionality described herein. Training set 701 can optionally include label data for some or all medical data in the training data indicating medical conditions present in corresponding patients, and/or can indicate location of anatomical features indicating presence of such a condition (e.g. location of tumors indicating presence of cancer). The medical condition detection function 1117 can be processed via medical condition detection system 1117 to process patient data 564 for a given patient (e.g. corresponding medical image data and/or other information) to generate model-detected finding data 617, which can implement any embodiment of model-detected finding data 617 described herein, and can indicate presence of (or lack of) one or more types of medical conditions. The medical condition detection function 1117 can be implemented in a same or similar fashion as medical outcome prognostication function 671 described herein and/or can implement some or all features and/or functionality of modeling platform of Figures 8A - 9R.

The model-detected finding data 617 can be processed via a treatment candidacy identification system 1118 to determine whether the patient qualifies for treatment of one or more detected medical condition 1120.x indicated in the model-detected finding data 617. This can be based on processing condition-to-treatment mapping data 1121 and/or treatment qualification data 1121.1 - 1121.T for a plurality of medical treatments 1-T. For example, the condition-to-treatment mapping data 1121 indicates recommended treatment for each of a set of medical conditions, while the treatment qualification data 1121 indicates requirements for the patient to quality for the treatment (e.g. as determined in their patient data 564, such as risk factors, whether the patient is allergic to this type of medical treatment, current medications that would conflict with the medical treatment, etc.). In this example, the condition-to-treatment mapping data 1121 indicates medical condition x maps to a given medical treatment z. Evaluating whether the patient qualifies can be based on accessing relevant portions of the patient data 564.y.

In cases where a given medical treatment is currently or soon undergoing a clinical trial, the treatment qualification data 1121 can correspond to clinical trial qualification data denoting requirements for qualifying for participation in the clinical trial. In such embodiments, the system of Figure 12A can be implemented to automatically detect prospective clinical trial candidates for ongoing/future clinical trials.

The condition-to-treatment mapping data 1121 and/or treatment qualification data 1121.1 - 1121.T can be received, accessed in memory resources, configured via user input, and/or automatically determined. Some or all of the condition-to-treatment mapping data 1121 and/or treatment qualification data 1121.1 - 1121.T can be based on known medical guidance and/or regulatory standards.

If the patient qualifies as dictated by treatment qualification data 1121 for given medical treatment z for the detected medical condition x, the adverse reaction prediction system 1107 and treatment safety can be applied to generate treatment safety data 1135 whether the medical treatment of medical treatment classification corresponding to medical treatment z is safe to administer, for example, as discussed in conjunction with Figure 11A. In some embodiments, this assessment is performed as part of the treatment candidacy identification system 1118, for example, in the case where the adverse reaction prediction system 1107 was already applied to generate adverse reaction prediction data 1130 and/or where treatment safety assessment system 1108 was already applied to generate treatment safety data 1135. As a particular example, the one or more adverse reaction prediction functions 1171 and/or the one or more medical condition detection functions 1177 are applied in tandem (e.g. as a part of applying a multi-output model), where the adverse reaction prediction data 1130 is generated in conjunction with the model-detected finding data 617.

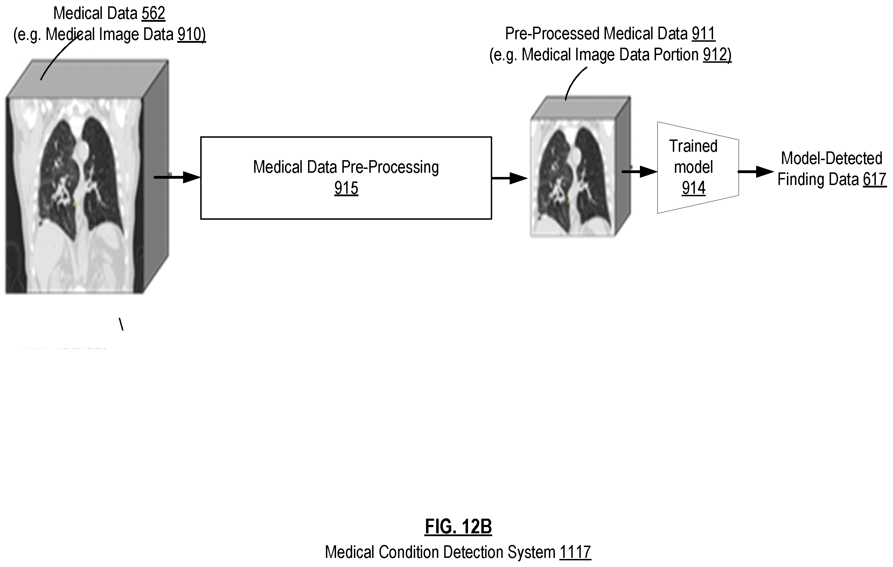

Figure 12B illustrates an embodiment of medical condition detection system 1117. Some or all features and/or functionality of the medical condition detection system 1117 of Figure 12B can implement any embodiment of the medical condition detection system 1117 described herein. In some embodiments, the medical condition detection system 1117, the one or more medical condition detection functions 1177, and/or corresponding training of these functions via medical condition detection model training system 1116 can be implemented via some or all functionality of the modeling platform operable to process medical images discussed in conjunction with some or all features and/or functionality Figures 8A - 9R. As illustrated in Figure 12B, medical images can be processed via some or all features and/or functionality of the modeling platform discussed in conjunction with some or all of Figures 8A - 9R to generate model-detected finding data 617, alternatively or in addition to generating the medical outcome prognosis score 636 as illustrated in Figure 8A and/or alternatively or in addition to generating the adverse reaction prediction score 1136 as illustrated in Figure 11B. In some embodiments, some or all embodiments of the model-detected findings data 617 are implemented in a same or similar fashion as the medical outcome prognosis score 636, where the corresponding medical outcome optionally corresponds to detected presence of a medical condition, and/or corresponds to another medical outcome that is a risk factor for and/or has known correlation with the medical condition.

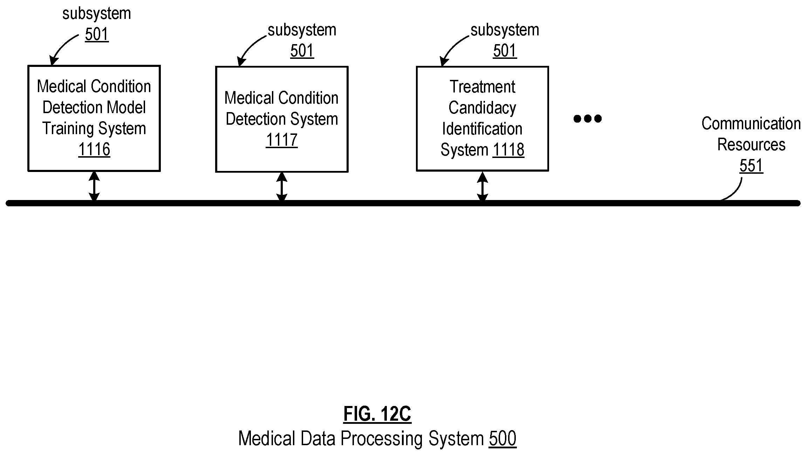

Figure 12C illustrates an embodiment of medical data processing system 500. Some or all features and/or functionality of the medical data processing system 500 of Figure 12C can implement any embodiment of the medical data processing system 500 described herein.

The medical data processing system 500 can be operable to implement some or all of the functionality of Figure 12A for example, based on implementing the medical condition detection model training system 1116, , the medical condition detection system 1117, and/or the treatment candidacy identification system 1118 as respective subsystems 501, for example, alternatively or in addition to implementing some or all other subsystems 501 described herein.

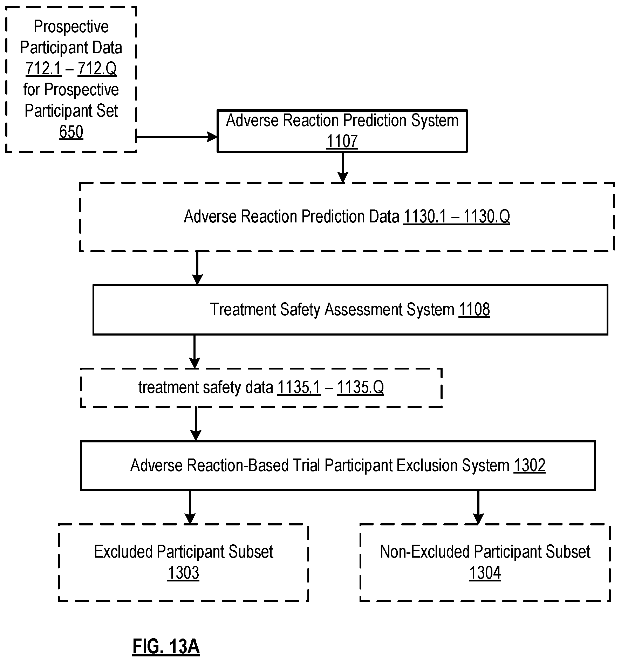

13A - 13D illustrate embodiments of a system that automatically identifies prospective medical treatments for patients based on automatically detecting corresponding medical conditions that can be treated by such medical treatments (e.g. via processing their medical image data and/or other medical data and/or patient data via artificial intelligence and/or machine learning techniques). Some or all features and/or functionality presented in Figures 13A - 13D can be implemented in tandem with some or all features and/or functionality of some or all of Figures 11A - 11H and/or 12A - 12C. Some or all features and/or functionality presented in Figures 13A - 13D can implement any embodiment of medical data processing system 500 and/or modeling platform described herein.

As illustrated in Figure 13A, adverse reaction prediction system 1107 can be utilized to generate adverse reaction prediction data 1130 for each of a plurality of prospective participants of a clinical trial based on processing corresponding prospective participant data 712.1 - 712.Q. The adverse reaction prediction system 1107 can be implemented via any features and/or functionality of adverse function prediction system 1107 described herein.

The prospective participant data 712 for the prospective participant set 650 can be implemented via any features and/or functionality of participant data 712 of participant set 615 for a clinical trial described herein. In particular, the clinical trial can be testing a given medical treatment, and/or can involve administering of the given medical treatment. Each prospective participant data 712 can be implemented to include some or all patient data 654 and/or any other relevant data (e.g. at least one medical image, and/or other information) utilized as input to at least one adverse reaction prediction function 1171.

The adverse reaction data 1130 can be generated for each given prospective participant to include one or more adverse reaction prediction scores 1136 for the given prospective participant via performance of at least one adverse reaction prediction function 1171 upon corresponding prospective participant data 712 (e.g. medical image data captured prior to the corresponding clinical trial, and/or other function input). In particular, the some or all adverse reaction scores can correspond to probability of adverse reaction to medical treatment of a given medical treatment classification that includes the given medical treatment being administered in the clinical trial.

The adverse reaction prediction data 1130.1 - 1130.Q can be processed via treatment safety assessment system 1108 to generate treatment safety data 1135.1 - 1135.Q by generating treatment safety data 1135 for each prospective participant via processing their adverse reaction prediction data 1130. The treatment safety assessment system 1108 can be implemented via some or all features and/or functionality of any embodiment of the treatment safety assessment system 1108 described herein. In particular, each treatment safety data 1135 can indicate whether it is safe to administer medical treatment of the given medical treatment classification to a corresponding prospective participant, thus indicating whether it is safe to administer the given medical treatment being administered in the clinical trial to the prospective participant.

This information can be utilized to exclude participants from the clinical trial (e.g. prior to random assignment of participants to control group vs. investigative group) based on whether it is safe to administer the given medical treatment being administered in the clinical trial. In particular, an adverse reaction-based trial participant exclusion system 1302 can be operable to generate an excluded participant subset 1303 indicating ones of the prospective participants 1-Q to be excluded from the trial, and to generate a non-excluded participant subset 1304 indicating ones of the prospective participants 1-Q to not be excluded from the trial. The excluded participant subset 1303 and the non-excluded participant subset can be mutually exclusive and/or collectively exhaustive with respect to the set of prospective participants 1-Q. In particular, the excluded participant subset 1303 can be generated based on including prospective participants with treatment safety data 1135 indicating administering of the medical treatment being administered in the clinical trial is unsafe, the non-excluded participant subset 1304 can be generated based on including prospective participants with treatment safety data 1135 indicating administering of the medical treatment being administered in the clinical trial is safe.

In some embodiments, in the case where multiple different medical treatments are administered in the clinical trial, the adverse reaction prediction data 1130 can be generated to include adverse reaction prediction scores 1136 for a set of medical treatment classifications of the multiple different medical treatments, where prospective participants are excluded from participation if any of the different medical treatments are deemed unsafe (e.g. based on comparison of the corresponding adverse reaction prediction scores to corresponding predefine score thresholds as discussed previously).

In some embodiments, the adverse reaction prediction data 1130 and/or treatment safety data 1135 can be generated based on a configured severity/type of adverse reaction that would render exclusion from the trial. As a particular example, high probability of adverse reaction categories corresponding to severe adverse reactions such as lethal/potentially lethal adverse reactions can render exclusion of participants from the trial, while high probability of adverse reaction categories corresponding to mild adverse reaction (e.g. headache or diarrhea) optionally do not exclude participants from the trial. This can be based on generating, via adverse reaction prediction system 1107 and/or processing, via treatment safety assessment system, only adverse reaction prediction scores 1136 corresponding to corresponding adverse reaction categories that should render such exclusion.

In some cases, high probability of adverse reaction considered less severe than the condition being treated are acceptable and allow participant participation, while high probability of adverse reaction considered more severe than the condition being treated are unacceptable and disallow participant participation (e.g. high risk of diarrhea is acceptable in the case where the participant is in a clinical trial for cancer treatment, while high risk of diarrhea is unacceptable in the case where the participant is in a clinical trial for a treatment for acne). Such tuning can be configured via user input and/or automatically (e.g. based on a known and/or user-configured set of relative severities of various medical conditions/symptoms).

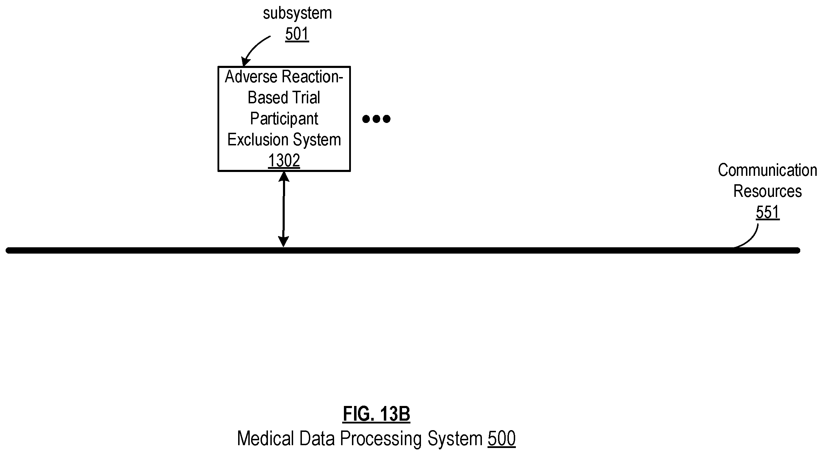

Figure 13B illustrates an embodiment of medical data processing system 500. Some or all features and/or functionality of the medical data processing system 500 of Figure 13B can implement any embodiment of the medical data processing system 500 described herein.

The medical data processing system 500 can be operable to implement some or all of the functionality of Figure 13B for example, based on implementing the adverse reaction-based trial participant exclusion system 1302, for example, alternatively or in addition to implementing some or all other subsystems 501 described herein.

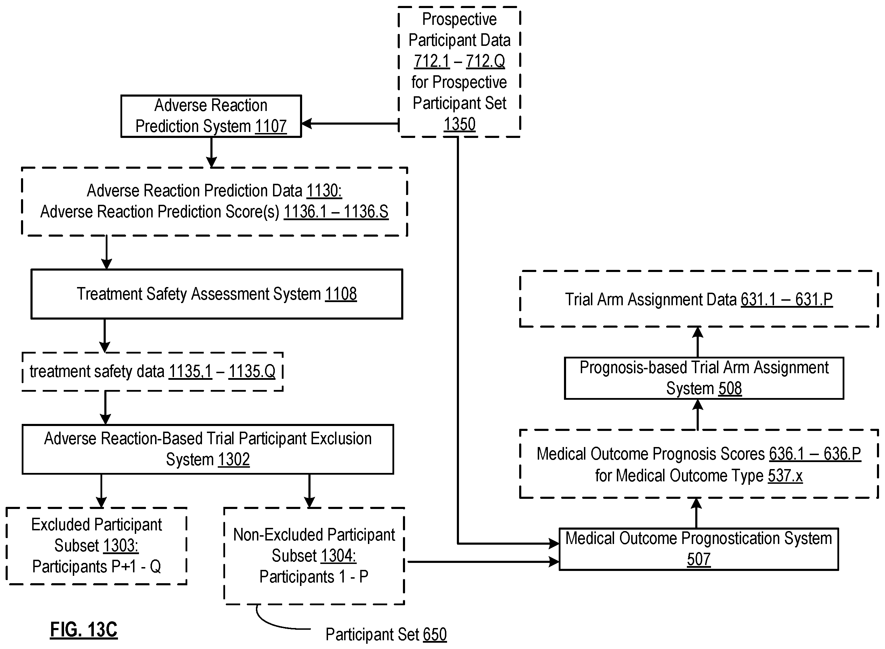

Figure 13C illustrates an embodiment of a system where the non-excluded participant subset 1304 is further processed to enable dispersal of non-excluded participants into trial arms based on medical outcome scores 636 generated via medical outcome prognostication system for the non-excluded participants. Some or all features and/or functionality of Figure 13C can implement some or all features and/or functionality of some of all of Figures 6A - 6K. In particular, a proper subset of the Q prospective participants in prospective participant set can be included in the non-excluded subset 1304, and can include the P patients 1-P that are ultimately assigned to trial arms for participation in the trial based on not being excluded due to likely adverse reaction, and/or other reasons.

The medical outcome prognostication system 507 can be implemented to generate medical outcome prognosis scores 636 for the medical outcome type corresponding to medical outcome of the corresponding clinical trial as discussed previously. This can be applied after patients likely to exhibit adverse reactions are excluded via adverse reaction-based trial participant exclusion system 1302, where medical outcome prognosis scores 636 are generated for only patients included in non-excluded participant subset 1-P as illustrated in Figure 13C. In other embodiments, the medical outcome prognostication data was optionally previously generated for some or all of the Q patients, even if some or all of these patients are ultimately excluded from participation. Thus, in either case, the prognosis-based trial arm assignment system 508 only process the medical outcome prognosis scores 636 of the patients in non-excluded participant subset to enable segregation of participants across trial arms via some or all features and/or functionality of prognosis-based trial arm assignment system 508 described herein, where excluded participants of excluded participant subset 1303 are not assigned to any trial arm, regardless of whether they have medical outcome prognosis scores 636 generated, due to being excluded from the trial.

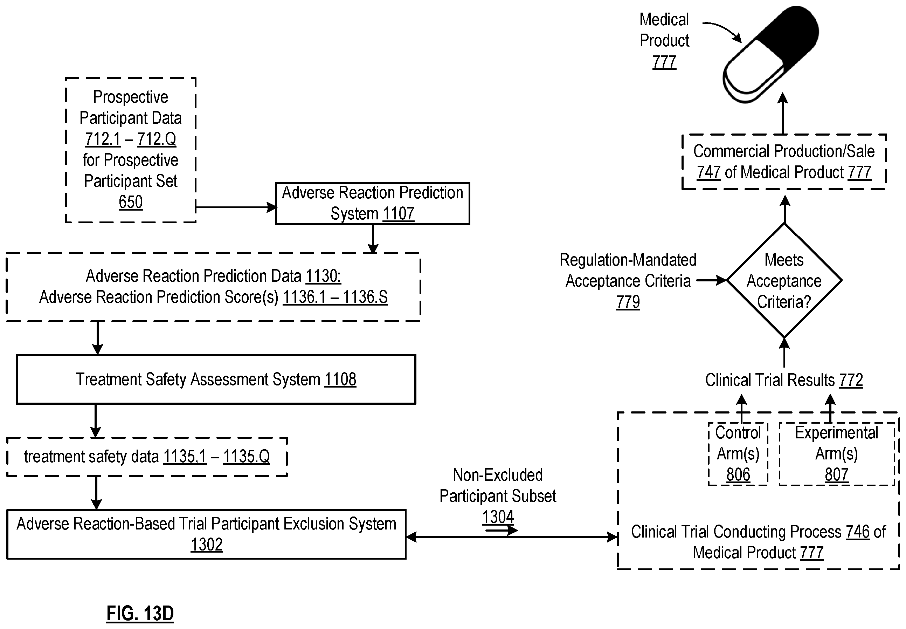

Figure 13D illustrates an embodiment of a medical product 777 developed via applying some or all features and/or functionality of the adverse reaction prediction system 1107, the treatment safety assessment system 1108, and/or the adverse reaction-based trial participant exclusion system 1302. Some or all features and/or functionality of the medical product development process of developing medical product 777 of Figure 13D can implement some or all features and/or functionality of development of a medical product 777 of Figure 4C. The medical product 777 of Figure 13D can implement any embodiment of a medical treatment described herein.

In particular, a non-excluded participant subset 1304 generated for a set of prospective participants of a participant set 650 of a clinical trial (e.g. via some or all of the process of Figure 13A - 13C) can be processed via a clinical trial conducting process 746 of the medical product 777, for example, that includes at least one control arm and at least one experimental trial arm (e.g. a corresponding entity, such as a corresponding medical product manufacturing and/or testing entity, conducts the clinical trial based on assigning only prospective participants of the non-excluded participant subset 1304, and not prospective participants of the excluded participant subset 1303, to respective trial arms, for example, as dictated by trial arm assignment data 631.1 - 631.P of the P participants in the non-excluded participant subset 1304). As a particular example, the trial is conducted via administering of the medical product 777 to ones of the non-excluded participant subset included in an investigational trial arm to determine effectiveness of the medical product, for example, in treating a corresponding medical condition and/or to determine whether the medical product is approved/safe for widespread use (e.g. commercial or otherwise widespread production and/or sale, for example, for over-the-counter purchase and/or for prescription use when prescribed by a medical professional/healthcare provider), for example, based on whether or not clinical trial results 772 meet corresponding regulation-mandated acceptance criteria 779 (e.g. as required by the FDA and/or another regulatory entity). For example, commercial production/sale 747 of the medical product 777 is performed to render production, sale, and/or use of the corresponding medical product 777 (e.g. by a corresponding medical product manufacturing and/or testing entity) based on the clinical trial results 772 meeting the regulation-mandated acceptance criteria 779. The exclusion of the participants likely to exhibit adverse reactions to the medical product 777 via various functionality discussed in conjunction with Figures 11A - 13C can improve the efficiency and/or effectiveness of the clinical trial, which can improve the technical field of medicine by enabling such products to be utilized readily to treat corresponding medical conditions, for example, more quickly.

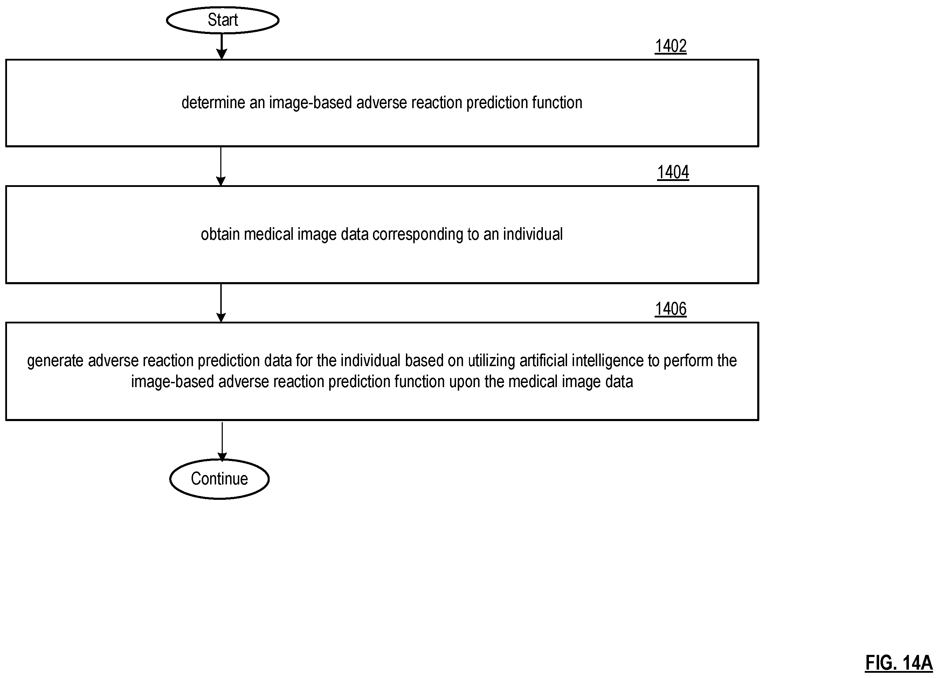

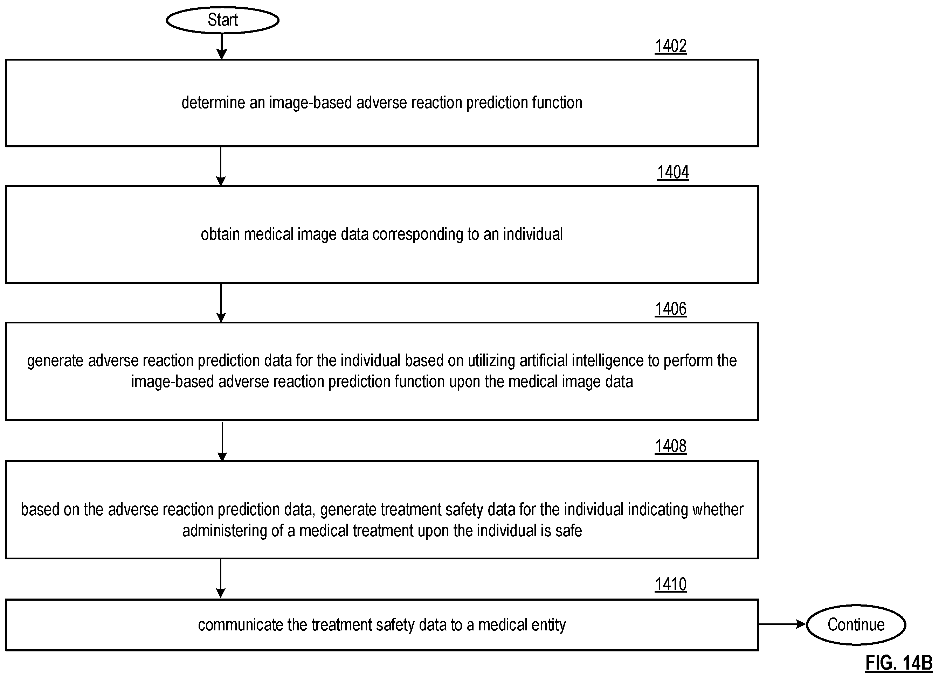

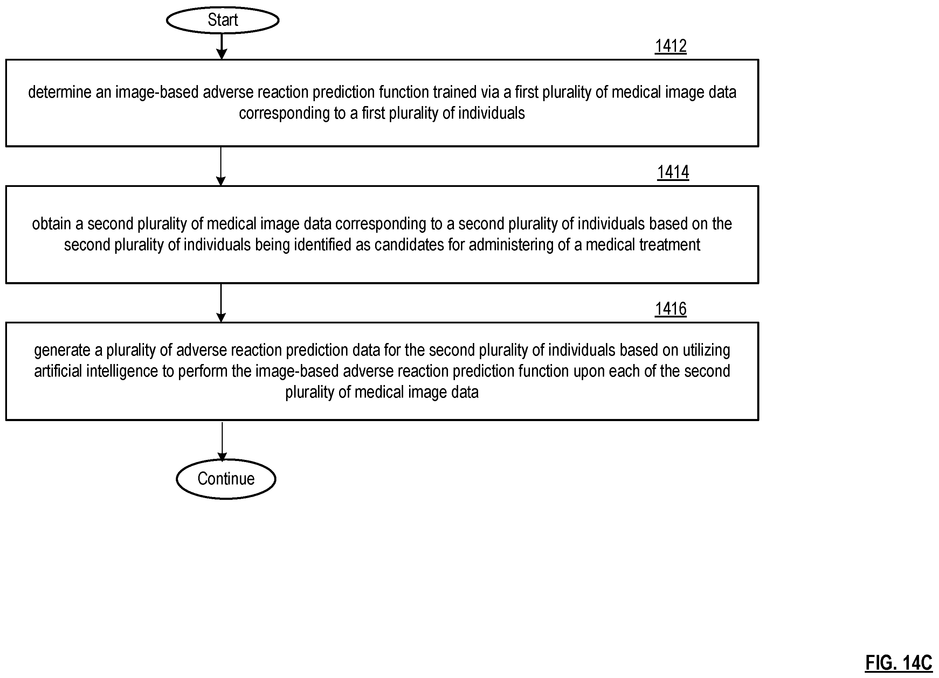

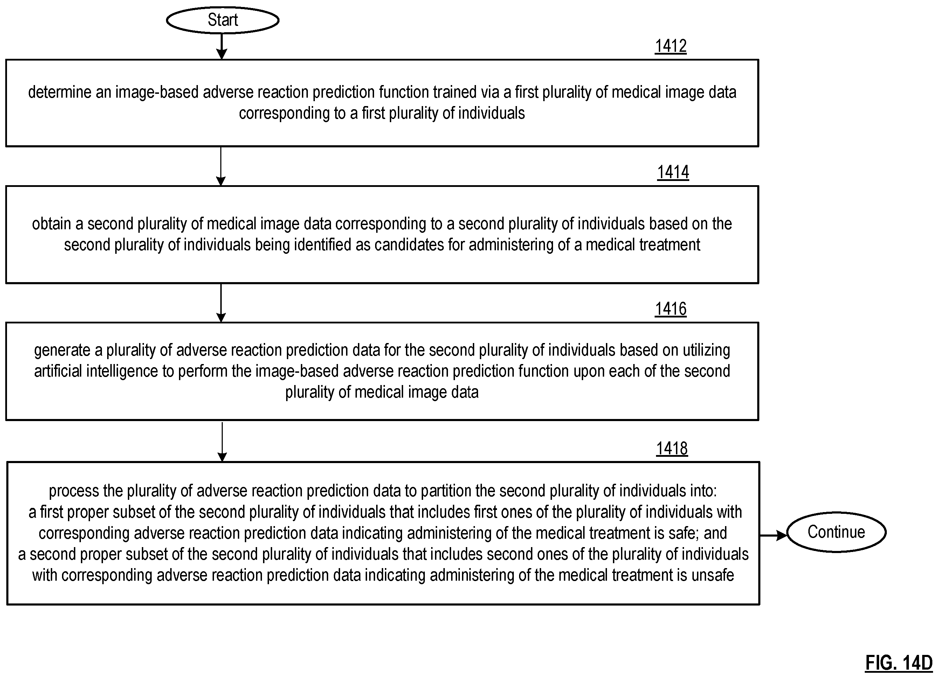

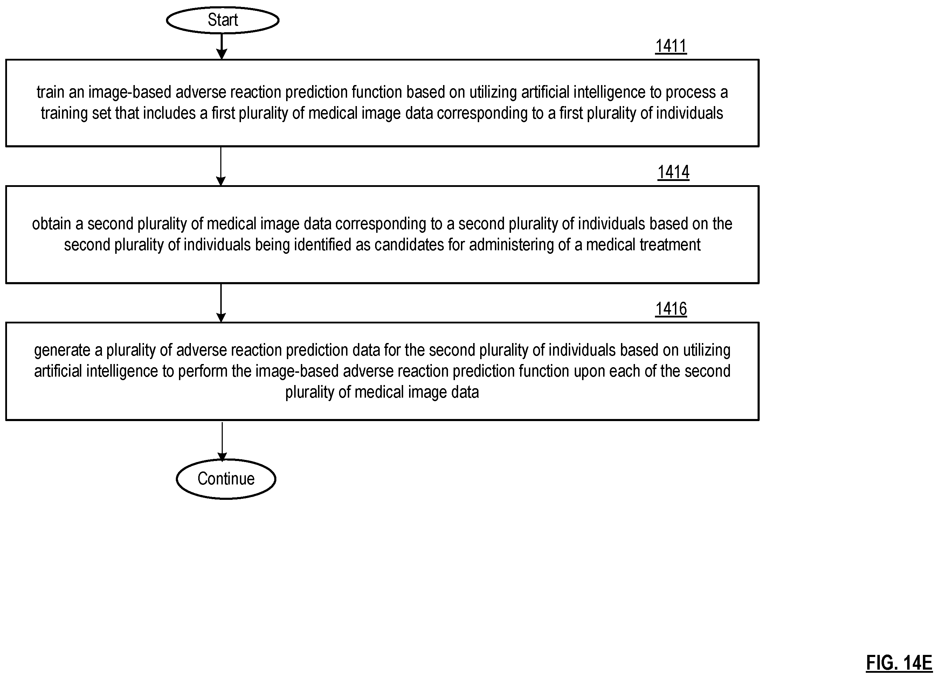

Figures 14A - 14E illustrate methods for execution by at least one processor. For example, some or all of Figure 14A, 14B, 14C, 14D, and/or 14E can be executed via medical data processing system 500 and/or can be executed based on communicating with a medical data processing system 500. For example, some or all of the steps of Figure 14A, 14B, 14C, 14D, and/or 14E can be performed in conjunction with implementing some or all features and/or functionality of the medical data processing system of one or more of Figures 11A - 13D. Alternatively or in addition, some or all of the steps of Figure 14A, 14B, 14C, 14D, and/or 14E can be performed in conjunction with implementing some or all features and/or functionality of any embodiment of the adverse reaction model training system 1106; the adverse reaction prediction system 1107; the treatment safety assessment system 1108, the medical condition detection model training system 1116, the medical condition detection system 1117, the treatment candidacy identification system 1118, and/or the adverse reaction-based trial participant exclusion system 1302 described herein. Alternatively or in addition, some or all of the steps of Figure 14A, 14B, 14C, 14D, and/or 14E can be performed in conjunction with implementing some or all features and/or functionality of the trial arm assignment module 730 and/or the randomization algorithm 800 of one or more of Figures 6A - 6K, and/or any embodiment of trial arm assignment module 730 and/or the randomization algorithm 800 described herein. Alternatively or in addition, some or all of the steps of Figure 14A, 14B, 14C, 14D, and/or 14E can be performed in conjunction with implementing some or all features and/or functionality of the medical outcome prognostication model training system 506, the medical outcome prognostication system 507, and/or the prognosis-based trial arm assignment system 508 of one or more of Figures 4A - 4J, and/or any embodiment of medical outcome prognostication model training system 506, the medical outcome prognostication system 507, and/or the prognosis-based trial arm assignment system 508 described herein Alternatively or in addition, some or all of the steps of Figure 14A, 14B, 14C, 14D, and/or 14E can be performed in conjunction by and/or via communication with one or more data sources 575, one or more requesting entities 576, one or more medical product testing processing systems 597 of one or more medical product testing entities 577, one or more medical product manufacturing processing systems 597 of one or more medical product manufacturing entities 577, one or more AI platforms 588, and/or one or more trial participation assignment entities 587. Alternatively or in addition, some or all of the steps of Figure 14A, 14B, 14C, 14D, and/or 14E can be performed in conjunction with the system 100 of Figure 1B, for example, in conjunction with implementing the imaging-based prognostication (IPRO) framework 200A and/or at least one corresponding model 112, for example, in conjunction with some or all features and/or functionality presented in Figure 8A and/or the example embodiments of Figures 8B - 9R. Alternatively or in addition, some or all of the steps of Figure 14A, 14B, 14C, 14D, and/or 14E can be performed by, and/or based on communications between, any one or more computing devices 700, any one or more corresponding server systems, and/or any processing system that includes at least one processor and/or at least one memory.

Some or all steps of the method of Figure 14A, 14B, 14C, 14D, and/or 14E can performed in conjunction with performance and/or can be implemented based on performing one or more steps of any other method described herein, such as any method of Figures 8B - 8G, and/or any method of Figures 10A - 10G. Some or all steps of Figures 14A - 14E can be performed in conjunction with each other, where performing any steps of any of the Figures 14A - 14E can be performed in conjunction with performing steps one or more other ones of the Figures 14A - 14E.

As illustrated in Figure 14A, step 1402 includes determine an image-based adverse reaction prediction function (e.g. adverse reaction prediction function 1171). Step 1404 includes obtaining medical image data corresponding to an individual (e.g. medical data 562 of a medical image type capturing at least one anatomical feature of the individual). Step 1406 includes generating adverse reaction prediction data (e.g. adverse reaction prediction data 1130) for the individual based on utilizing artificial intelligence to perform the image-based adverse reaction prediction function upon the medical image data. For example, the adverse reaction prediction system 1107 is implemented to perform some or all of steps 1402, 1404, and/or 1406.

Alternatively to or in addition to any of the foregoing, as illustrated in Figure 14B, step 1408 and/or 1410 can optionally be performed after performing some or all of steps 1402, 1404, and/or 1406. Step 1408 includes generating, based on the adverse reaction prediction data, treatment safety data (e.g. treatment safety data 1135) for the individual indicating whether administering of a medical treatment upon the individual is safe. Step 1410 includes communicating the treatment safety data to a medical entity (e.g. the medical entity corresponds to one or more data sources 575, one or more requesting entities 576, one or more medical product testing processing systems 597 of one or more medical product testing entities 577, one or more medical product manufacturing processing systems 597 of one or more medical product manufacturing entities 577, one or more AI platforms 588, and/or one or more trial participation assignment entities 587). Alternatively to or in addition to any of the foregoing, communicating the treatment safety data includes at least one of: transmitting or otherwise sending the treatment safety data (e.g. to the medical entity); displaying the trial arm assignment data (e.g. via a display device of a computing device associated with the medical entity, for example, for observation by a physician, pharmacist, scientist conducting a clinical trial, medical professional, person responsible for determining whether patients can be included in a clinical study, or other person); and/or storing the trial arm assignment data (e.g. in data storage system 560 and/or other storage resources).

As illustrated in Figure 14C, step 1412 includes determining an image-based adverse reaction prediction function trained via a first plurality of medical image data corresponding to a first plurality of individuals. Step 1414 includes obtaining a second plurality of medical image data corresponding to a second plurality of individuals based on the second plurality of individuals being identified as candidates for administering of a medical treatment. Step 1416 includes generate a plurality of adverse reaction prediction data for the second plurality of individuals based on utilizing artificial intelligence to perform the image-based adverse reaction prediction function upon each of the second plurality of medical image data.

Alternatively to or in addition to any of the foregoing, performance of step 1412 can implement performance of step 1402. Alternatively to or in addition to any of the foregoing, performance of steps 1414 and/or 1416 can include performing steps 1404 and/or 1406 multiple times, for each of the second plurality of individuals. Alternatively to or in addition to any of the foregoing, performing steps 1414 and/or 1416 can include first obtaining all of the second plurality of image data, and then generating the plurality of adverse reaction prediction data once all of the second plurality of image data is obtained. Alternatively to or in addition to any of the foregoing, performing steps 1414 and/or 1416 can include, in a first time frame, obtaining a first one of the second plurality of image data and generating first corresponding adverse reaction data for the first one of the second plurality of image data, and in a second time frame, obtaining a second one of the second plurality of image data and generating second corresponding adverse reaction data for the second one of the second plurality of image data, where some or all of the second plurality of image data and plurality of adverse reaction prediction data is obtained and generated, respectively, over time, for example, as corresponding individuals are identified as candidates for administering of a medical treatment over time.

Alternatively to or in addition to any of the foregoing, as illustrated in Figure 14D, step 1418 can be performed after performing some or all of steps 1412, 1414, and/or 1416. Step 1418 includes processing the plurality of adverse reaction prediction data to partition the second plurality of individuals into a first proper subset of the second plurality of individuals that includes first ones of the plurality of individuals with corresponding adverse reaction prediction data indicating administering of the medical treatment is safe; and a second proper subset of the second plurality of individuals that includes second ones of the plurality of individuals with corresponding adverse reaction prediction data indicating administering of the medical treatment is unsafe. Alternatively to or in addition to any of the foregoing, step 1418 can be performed by a same or different entity that performed some or all of steps 1412, 1414, and/or 1416. Alternatively to or in addition to any of the foregoing, the method can include communicating (e.g. sending, displaying, and/or storing) the plurality of adverse reaction prediction data to a medical entity, where the medical entity then processes the plurality of adverse reaction prediction data to partition the second plurality of individuals into a first proper subset of the second plurality of individuals that includes first ones of the plurality of individuals with corresponding adverse reaction prediction data indicating administering of the medical treatment is safe; and a second proper subset of the second plurality of individuals that includes second ones of the plurality of individuals with corresponding adverse reaction prediction data indicating administering of the medical treatment is unsafe. Alternatively to or in addition to any of the foregoing, partitioning the second plurality of individuals occurs all at once, once all of the plurality of adverse reaction prediction data has been generated. Alternatively to or in addition to any of the foregoing, partitioning the second plurality of individuals occurs over time, for example, in response to the plurality of adverse reaction prediction data being generated over time. Alternatively to or in addition to any of the foregoing, performing steps 1414 and/or 1416 and/or 1418 can include, in a first time frame, obtaining a first one of the second plurality of image data for a first individual of the second plurality of individuals, generating first corresponding adverse reaction data for the first one of the second plurality of image data, and/or assigning the first individual of the second plurality of individuals to either the first proper subset or the second proper subset based on whether administering the first corresponding adverse reaction data to the first individual is safe or unsafe; and in a second time frame, obtaining a second one of the second plurality of image data for a second one of the second plurality of individuals, generating second corresponding adverse reaction data for the second one of the second plurality of image data, and/or assigning the second individual of the second plurality of individuals to either the first proper subset or the second proper subset based on whether administering the second corresponding adverse reaction data to the first individual is safe or unsafe; where some or all of the plurality of individuals are assigned to either the first proper subset or the second proper subset over time, for example, as corresponding individuals are identified as candidates for administering of a medical treatment over time.

Alternatively to or in addition to any of the foregoing, as illustrated in Figure 14E, step 1411 can be performed prior to performing some or all of steps 1412, 1414, and/or 1416. Step 1411 includes training an image-based adverse reaction prediction function based on utilizing artificial intelligence to process a training set that includes a first plurality of medical image data corresponding to a first plurality of individuals.

In some or all embodiments, image-based adverse reaction prediction function of step 1402 can be implemented via some or all features and/or functionality of any embodiment of adverse reaction prediction function 1171 described herein. In some or all embodiments, image-based adverse reaction prediction function of step 1402 can be implemented via some or all features and/or functionality of any embodiment of model 112 described herein. Alternatively to or in addition to any of the foregoing, step 1402 is performed via implementing some or all features and/or functionality of adverse reaction prediction model training system 1106 to train image-based adverse reaction prediction function, for example, based on tuning parameters of corresponding model parameter data 661. Alternatively to or in addition to any of the foregoing, the image-based adverse reaction prediction function of step 1402 can be determined via any other means (e.g. based on being accessed in memory, being received via the network, being generated/trained by another entity, based on being automatically trained and/or generated, for example, as step 1411 of the method of Figure 14E, based on having being previously trained via a separate entity, based on being configured based on user input, and/or based on otherwise being determined).

Alternatively to or in addition to any of the foregoing, the image-based adverse reaction prediction function determined in step 1402 was trained based on utilizing artificial intelligence. Alternatively to or in addition to any of the foregoing, the method further includes training the image-based adverse reaction prediction function, for example, based on utilizing artificial intelligence to process a training set. Alternatively to or in addition to any of the foregoing, the training set includes a first plurality of medical image data. Alternatively to or in addition to any of the foregoing, training set includes a corresponding plurality of adverse reaction data, for example, labeling adverse reaction categories 1152 and/or corresponding measurements of adverse reactions exhibited by corresponding ones of the first plurality of individuals with corresponding ones of the first plurality of medical image data. Alternatively to or in addition to any of the foregoing, training set includes a corresponding plurality of medical treatment history data, for example, labeling medical treatment classifications 1151 and/or corresponding dosage/timeframe/ of medical treatment classifications 1151 administered to corresponding ones of the first plurality of individuals with corresponding ones of the first plurality of medical image data.

Alternatively to or in addition to any of the foregoing, some or all of the first plurality of medical image data can be implemented via any embodiment of medical data 562 described herein. Alternatively to or in addition to any of the foregoing, some or all of the first plurality of medical image data can include a plurality of values 601. Alternatively to or in addition to any of the foregoing, some or all of the corresponding plurality of adverse reaction data can be implemented via any embodiment of outcome data 638 and/or medical findings data 616 described herein. Alternatively to or in addition to any of the foregoing, some or all of the type of adverse reaction data can be implemented via any embodiment of medical outcome type 637 described herein.

Alternatively to or in addition to any of the foregoing, the first plurality of medical image data and/or the corresponding plurality of adverse reaction data can be included in the training set based on being received from a data source 575. Alternatively to or in addition to any of the foregoing, the first plurality of medical image data and/or the corresponding plurality of medical outcome data can be included in the training set based on being stored in, accessed via, and/or received from a data storage system, such as medical data storage 561.

Alternatively to or in addition to any of the foregoing, the method further includes requesting the first plurality of medical image data of the training set and/or further includes requesting the corresponding plurality of adverse reaction data (e.g. via generating a corresponding at least one request and/or via sending to the at least one request to a data storage system or a data source), where the first plurality of medical image data and/or the corresponding plurality of adverse reaction data of the training set is obtained in response to the at least one request. Alternatively to or in addition to any of the foregoing, requesting the plurality of medical image data is based on configuring at least one request for medical image data meeting a set of parameters, where the first plurality of medical image data meets the set of parameters based on the request. Alternatively to or in addition to any of the foregoing, the set of parameters indicates the at least one medical image data type/modality, for example, where the first plurality of medical image data is of the at least one medical image data type/modality based on the at least one request for the first plurality of medica image data and/or the corresponding plurality of adverse reaction data indicating the at least one medical image data type. Alternatively to or in addition to any of the foregoing, the set of parameters indicates one or more adverse reaction categories 1152, where the corresponding plurality of adverse reaction data corresponds to the one or more adverse reaction categories 1152 based on the at least one request for the first plurality of medical data and/or the corresponding plurality of medical outcome data indicating the one or more adverse reaction categories 1152. Alternatively to or in addition to any of the foregoing, the set of parameters indicates one or more medical treatment classifications 1151, where some or all of the corresponding first plurality of individuals to which the first plurality of medical image data corresponds underwent medical treatment corresponding to the one or more medical treatment classifications 1151, for example, as indicated in their patient data 564, based on the at least one request for the first plurality of medical image data and/or the corresponding plurality of adverse reaction data indicating the one or more medical treatment classifications 1151.

Alternatively to or in addition to any of the foregoing, the medical image data of step 1404 and/or some or all of the second plurality of medical image data of step 1414 can be obtained based on being received from a data source 575. Alternatively to or in addition to any of the foregoing, the medical image data of step 1404 and/or some or all of the second plurality of medical image data of step 1414 can be obtained based on being stored in, accessed via, and/or received from a data storage system, such as medical data storage 561.

Alternatively to or in addition to any of the foregoing, the medical image data of step 1404 and/or some or all of the second plurality of medical image data of step 1414 is distinct from medical image data utilized to train the adverse reaction prediction function (e.g. distinct from the first plurality of medical data utilized to train the image-based adverse reaction prediction function). Alternatively to or in addition to any of the foregoing, the medical image data of step 1404 and/or some or all of the second plurality of medical image data of step 1414 corresponds to pre-trial medical data for a corresponding individual based on being a prospective participant of a clinical trial, for example, testing the medical treatment and/or involving administering of the medical treatment.

Alternatively to or in addition to any of the foregoing, the method further includes requesting the medical image data of step 1404 and/or some or all of the second plurality of medical image data of step 1414 (e.g. via generating a corresponding at least one request and/or via sending to the at least one request to a data storage system or a data source), where the medical image data of step 1404 and/or some or all of the second plurality of medical image data of step 1414 is obtained in response to the request. Alternatively to or in addition to any of the foregoing, requesting the plurality of medical image data is based on configuring at least one request for medical data meeting a set of parameters, where the medical image data of step 1404 and/or some or all of the second plurality of medical image data of step 1414 meets the set of parameters based on the at least one request. Alternatively to or in addition to any of the foregoing, the set of parameters indicates the at least one medical image data type/modality, for example, where the medical image data of step 1404 and/or some or all of the second plurality of medical image data of step 1414 is of the at least one medical image data type/modality based on the at least one request for the medical image data of step 1404 and/or some or all of the second plurality of medical image data of step 1414 indicating the at least one medical image data type/modality. Alternatively to or in addition to any of the foregoing, the set of parameters indicates the individual or the second plurality of individuals, (e.g. based on patient identifiers of the plurality of prospective clinical trial participants), for example, where the medical image data of step 1404 and/or some or all of the second plurality of medical image data of step 1414 corresponds to the individual or the second plurality of individuals, respectively, based on the at least one request for the medical image data of step 1404 and/or some or all of the second plurality of medical image data of step 1414 indicating the individual or the second plurality of individuals, respectively. Alternatively to or in addition to any of the foregoing, the set of parameters indicates a corresponding clinical trial (e.g. based on a clinical trial identifier of the clinical trial), for example, where the plurality of medical image data of step 1404 and/or some or all of the second plurality of medical image data of step 1414 corresponds to the individual or the second plurality of individuals, respectively, based on being a prospective participants of the clinical trial and based on the at least one request for the medical image data of step 1404 and/or some or all of the second plurality of medical image data of step 1414 indicating the clinical trial.

Step 1416 includes generating a plurality of adverse reaction prediction data, for example, based on the second plurality of medical data being obtained in step 1404. Alternatively to or in addition to any of the foregoing, step 1416 is performed via implementing some or all features and/or functionality of adverse reacting prediction system 1107 to perform the adverse reaction prediction function.

Alternatively to or in addition to any of the foregoing, step 1418 of partitioning the second plurality of individuals into the first proper subset and the second proper subset is based on automatically identifying which individuals belong to which subset based on automatically processing the plurality of adverse reaction prediction data, for example, by generating corresponding treatment safety data for each adverse reaction prediction data indicating whether the medical treatment is safe to administer to a corresponding one of the second plurality of individuals (e.g. as a binary value). Alternatively to or in addition to any of the foregoing, partitioning the second plurality of individuals into the first proper subset and the second proper subset is based on providing the adverse reaction prediction data and/or corresponding treatment safety data for use in a selection process, where this selection process is optionally performed: via an automated process; via at least one human; or via a combination of automated processes and human-conducted processes, for example, intervention with at least one automated process.

Alternatively to or in addition to any of the foregoing, the first proper subset and the second proper subset are mutually exclusive. Alternatively to or in addition to any of the foregoing, the first proper subset and the second proper subset are collectively exhaustive with respect to the second plurality of individuals.

In various examples, some or all of the plurality of adverse reaction data is generated at the same time via collectively processing corresponding medical image data, where corresponding ones of the second plurality of individuals are partitioned at a same time accordingly. In various examples, some or all of the plurality of adverse reaction data is generated at the different times via collectively processing corresponding medical image data at different times (e.g. as corresponding individuals are identified as prospective candidates at different times), where corresponding ones of the second plurality of individuals are partitioned at a same time accordingly (e.g. a first individual is assigned to either the first proper subset or the second proper subset at a first time based on their corresponding treatment safety data, while a second individual is assigned to either the first proper subset or the second proper subset at a second time after the first time (e.g. on a different date) based on their corresponding treatment safety data.

In various examples, each of the plurality of adverse reaction prediction data includes an adverse reaction score. In various examples, partitioning the second plurality of individuals into the first proper subset and the second proper subset is based on comparing the adverse reaction score of each of the plurality of adverse reaction prediction data to a predefined score threshold. In various examples, the predefined score threshold is configured to distinguish between safe and unsafe administering of the medical treatment.

In various examples, the adverse reaction score is based on a predicted probability that a corresponding individual of the second plurality of individuals will have an adverse reaction to the medical treatment based on the image-based adverse reaction prediction function being trained to predict probability of the adverse reaction as a function of medical image data. In various examples, the predefined score threshold corresponds to a threshold probability value. In various examples, the first proper subset of individuals includes first ones of the second plurality of individuals with corresponding adverse reaction scores indicating a corresponding probability of encountering the adverse reaction that falls below the threshold probability value. In various examples, the second proper subset of individuals includes second ones of the second plurality of individuals with corresponding adverse reaction scores indicating a corresponding probability of encountering the adverse reaction that exceeds the threshold probability value. In various examples, an adverse rection score equal to the threshold probability value is configured to be assigned to the first proper subset of individuals. In various examples, an adverse rection score equal to the threshold probability value is configured to be assigned to the second proper subset of individuals.

In various examples, the adverse reaction score is based on a predicted probability that a corresponding individual of the second plurality of individuals will not have an adverse reaction to the medical treatment based on the image-based adverse reaction prediction function being trained to predict probability of the adverse reaction as a function of medical image data. In various examples, the predefined score threshold corresponds to a threshold probability value. In various examples, the first proper subset of individuals includes first ones of the second plurality of individuals with corresponding adverse reaction scores indicating a corresponding probability of not encountering the adverse reaction that exceeds the threshold probability value. In various examples, the second proper subset of individuals includes second ones of the second plurality of individuals with corresponding adverse reaction scores indicating a corresponding probability of not encountering the adverse reaction that falls below the threshold probability value. In various examples, an adverse rection score equal to the threshold probability value is configured to be assigned to the first proper subset of individuals. In various examples, an adverse rection score equal to the threshold probability value is configured to be assigned to the second proper subset of individuals.

In various examples, the training set further includes at least one of: adverse reaction data for at least some first ones of the first plurality of individuals indicating side effects observed for the at least some first ones of the first plurality of individuals; or medical treatment history for at least some second ones of the of the first plurality of individuals indicating medical treatments administered to at least some second ones of the of the first plurality of individuals.

In various examples, the medical treatment is administered to only the second ones of the second plurality of individuals included in the second proper subset of individuals. In various examples, medical treatment is not administered to the first ones of the second plurality of individuals included in the first proper subset of individuals.

In various examples, the medical treatment is a pharmaceutical compound. In various examples, the medical treatment is an immunotherapy treatment. In various examples, the second plurality of individuals are identified for prospective treatment via the immunotherapy treatment based on being diagnosed with at least one type of cancer. In various examples, the first proper subset of individuals includes first ones of the second plurality of individuals with corresponding adverse reaction prediction data indicating administering of immunotherapy treatment is safe. In various examples, the second proper subset of individuals includes second ones of the second plurality of individuals with corresponding adverse reaction prediction data indicating administering of immunotherapy treatment is unsafe.

In various examples, the image-based adverse reaction prediction function is trained and performed in conjunction with implementing a medical modeling platform (e.g. medical imaging platform 505) that includes at least one model trained via training data that includes the first plurality of medical image data. In various examples, the image-based adverse reaction prediction function is performed based on applying the at least one model trained via the training data.

In various examples, the medical treatment belongs to one medical treatment classification of a plurality of medical treatment classifications. In various examples, the at least one model is trained to enable generation of adverse reaction prediction data corresponding to different ones of the plurality of medical treatment classifications. In various examples, the method further includes obtaining a third plurality of medical image data corresponding to a third plurality of individuals based on the third plurality of individuals being identified as candidates for administering of a second medical treatment belonging to a second medical treatment classification of the plurality of medical treatment classifications. In various examples, the method further includes generating a second plurality of adverse reaction prediction data for the third plurality of individuals based on utilizing artificial intelligence to apply the at least one model to each of the third plurality of medical image data to generate corresponding adverse reaction prediction data of the second plurality of adverse reaction prediction data. In various examples, the second plurality of adverse reaction prediction data is processed to partition the third plurality of individuals into a third proper subset of the third plurality of individuals and a fourth proper subset of the third plurality of individuals. In various examples, the third proper subset of individuals includes first ones of the third plurality of individuals with corresponding adverse reaction prediction data indicating administering of the second medical treatment is safe. In various examples, the fourth proper subset of individuals includes second ones of the third plurality of individuals with corresponding adverse reaction prediction data indicating administering of the second medical treatment is unsafe.

In various examples, the at least one model is trained to enable generation of adverse reaction prediction data corresponding to different ones of a plurality of adverse reaction categories. In various examples, each of the plurality of adverse reaction prediction data indicates probability of a corresponding one of the second plurality of individuals having an adverse reaction corresponding to a first adverse reaction category of the plurality of adverse reaction categories. In various examples, the method further includes generating a second plurality of adverse reaction prediction data based on utilizing artificial intelligence to apply the at least one model to generate corresponding adverse reaction prediction data of the second plurality of adverse reaction prediction data. In various examples, each of the second plurality of adverse reaction prediction data indicates probability having an adverse reaction corresponding to a second adverse reaction category of the plurality of adverse reaction categories.

In various examples, the plurality of adverse reaction categories include at least one of: a plurality of symptom-based adverse reaction categories; a plurality of severity-based adverse reaction categories; a plurality of immunologic adverse reaction categories; a plurality of nonimmunologic adverse reaction categories; and/or a plurality of temporal-based reaction categories.

In various examples, the image-based adverse reaction prediction function is trained to output a corresponding plurality of adverse reaction prediction data for each medical image data input. In various examples, the plurality of adverse reaction prediction data is generated for the second plurality of individuals based on utilizing artificial intelligence to apply the at least one model to each of the second plurality of medical image data to generate corresponding adverse reaction prediction data of the plurality of adverse reaction prediction data. In various examples, the second plurality of adverse reaction prediction data is also generated for the second plurality of individuals based on utilizing artificial intelligence to apply the at least one model to each of the second plurality of medical image data to generate corresponding adverse reaction prediction data of the second plurality of adverse reaction prediction data. In various examples, each of the second plurality of adverse reaction prediction data corresponds to one of the second plurality of individuals. In various examples, partitioning the second plurality of individuals into the first proper subset and the second proper subset is based on further processing the second plurality of adverse reaction prediction data.

In various examples, the method further includes obtaining a third plurality of medical image data corresponding to a third plurality of individuals based on the third plurality of individuals being identified as candidates for administering of a second medical treatment. In various examples, the second plurality of adverse reaction prediction data is generated for the third plurality of individuals based on utilizing artificial intelligence to apply the at least one model to each of the third plurality of medical image data to generate corresponding adverse reaction prediction data of the second plurality of adverse reaction prediction data.

In various examples, the at least one model is trained to process input data corresponding to different medical imaging modalities of a plurality of different medical imaging modalities based on being trained via a set of training data that includes at least the first plurality of medical image data and an additional plurality of medical image data. In various examples, the first plurality of medical image data includes first corresponding medical images having a first medical imaging modality of the plurality of different medical imaging modalities. In various examples, the additional plurality of medical image data includes additional corresponding medical images having a second medical image modality of the plurality of medical imaging modalities.

In various examples, each of the second plurality of medical image data includes a corresponding medical image, captured for a corresponding one of the second plurality of individuals, having the first medical imaging modality.

In various examples, each of a first subset of the second plurality of medical image data includes a corresponding medical image, captured for a corresponding one of the second plurality of individuals, having the first medical imaging modality. In various examples, a first corresponding subset of adverse reaction prediction data of the plurality of adverse reaction prediction data is generated for a first corresponding subset of patients based on applying the at least one model to process corresponding medical images having the first medical imaging modality. In various examples, each of a second subset of the second plurality of medical image data includes a corresponding medical image, captured for a corresponding one of the second plurality of individuals, having the second medical imaging modality. In various examples, a second corresponding subset of adverse reaction prediction data of the plurality of adverse reaction prediction data is generated for a second corresponding subset of patients based on applying the at least one model to process corresponding medical images having the second medical imaging modality.

In various examples, at least one of the second plurality of medical image data includes multiple medical images that includes one corresponding medical image having the first medical imaging modality, captured for a corresponding one of the second plurality of individuals on a first date, and another corresponding medical image having the first medical imaging modality, captured for the corresponding one of the second plurality of individuals on a second date after the first date. In various examples, at least one corresponding adverse reaction prediction data of the plurality of adverse reaction prediction data is generated for at least one corresponding patient based on applying the at least one model to the multiple medical images.

In various examples, at least one of the second plurality of medical image data includes multiple medical images that includes one corresponding medical image, captured for a corresponding one of the second plurality of individuals, having the first medical imaging modality, and another corresponding medical image, captured for the corresponding one of the second plurality of individuals on a second date after the first date, having the second medical imaging modality. In various examples, at least one corresponding adverse reaction prediction data of the plurality of adverse reaction prediction data is generated for at least one corresponding patient based on applying the at least one model to the multiple medical images.

In various examples, the at least one model is trained to process input data that includes additional, non-imaging-based data based on being trained via a set of training data that includes at least the first plurality of medical image data and a plurality of additional, non-imaging-based data. In various examples, some or all of the second plurality of individuals has corresponding input data that includes a corresponding one of the second plurality of medical image data and corresponding non-imaging-based data. In various examples, the corresponding non-imaging-based data includes non-imaging-based device-captured medical data, demographic data; patient history data, medical report text data; and/or risk factor data. In various examples, adverse reaction prediction data is generated for the each of the second plurality of individuals based on applying the at least one model to both the corresponding one of the second plurality of medical image data and the corresponding non-imaging-based data of the corresponding input data.

In various examples, the medical treatment is configured to treat a medical condition. In various examples, the at least one model is further trained to detect the medical condition based processing medical image data. In various examples, the method further includes obtaining a third plurality of medical image data for the third plurality of individuals. In various examples, the method further includes generating a plurality of medical condition detection data for the third plurality of individuals based on utilizing artificial intelligence to apply the at least one model to each of the third plurality of medical image data to generate corresponding medical condition detection data of the plurality of medical condition detection data. In various examples, the plurality of medical condition detection data is processed to identify a third proper subset of the third plurality of individuals and a fourth proper subset of the third plurality of individuals. In various examples, the third proper subset includes first ones of the third plurality of individuals having medical condition detection data indicating the medical condition is detected. In various examples, the fourth proper subset includes second ones of the third plurality of individuals having medical condition detection data indicating the medical condition is undetected. In various examples, at least one of the second plurality of individuals is automatically identified as a candidate for the medical treatment based on being included in the fourth proper subset of the third plurality of individuals. In various examples, at least one corresponding medical image data of the second plurality of medical image data is processed to generate both corresponding medical condition detection data and corresponding adverse reaction prediction data for the at least one of the second plurality of individuals.

In various examples, the second plurality of individuals are candidates for administering of the medical treatment based on being prospective clinical trial participants of a clinical trial conducted to test the medical treatment. In various examples, each of the second plurality of medical image data corresponds to pre-trial medical data for a corresponding one of the second plurality of individuals. In various examples, the second proper subset of individuals are excluded from participation in the clinical trial based on having corresponding adverse reaction prediction data indicating administering of the medical treatment is unsafe. In various examples, the first proper subset of individuals are not excluded from participation in the clinical trial based on having corresponding adverse reaction prediction data indicating administering of the medical treatment is safe. In various examples, each of the first proper subset of individuals are assigned to a corresponding trial arm of a set of trial arms of the clinical trial based on not being excluded from participation in the clinical trial. In various examples, each of the first proper subset of individuals are assigned to a corresponding trial arm of a set of trial arms of the clinical trial via performance of some or all steps of one or more of Figures 10A - 10G.

In various examples, the operational instructions, when executed by the at least one processor, further cause the medical data processing system to generate a plurality of treatment safety data for the second plurality of individuals based on processing the plurality of adverse reaction prediction data. In various examples, each of the plurality of treatment safety data indicates whether administering of the medical treatment is safe or unsafe for a corresponding one of the second plurality of individuals based on processing a corresponding one of the plurality of adverse reaction prediction data for the corresponding one of the second plurality of individuals. In various examples, partitioning the second plurality of individuals into the first proper subset and the second proper subset is based on processing the plurality of treatment safety data.

In various examples, the method further includes communicating each of the plurality of adverse reaction prediction data and/or each of the plurality of treatment safety data to a medical entity associated with administering the medical treatment. In various examples, communicating each of the plurality of adverse reaction prediction data and/or each of the plurality of treatment safety data to the medical entity includes transmitting the plurality of adverse reaction prediction data and/or the plurality of treatment safety data to at least one computing device associated with the medical entity, storing the adverse reaction prediction data and/o the plurality of treatment safety data in storage resources associated with the medical entity, displaying the plurality of adverse reaction prediction data and/or the plurality of treatment safety data via at least one display device associated with the medical entity, and/or dictating the plurality of adverse reaction prediction data and/or the plurality of treatment safety data via at least one microphone associated with the medical entity.

In various examples, performing the image-based adverse reaction prediction function upon each of the second plurality of medical image data includes generating corresponding risk characteristic detection data. In various examples, the corresponding risk characteristic detection data is processed to determine whether risk characteristics mapped to adverse reactions of the medical treatment are detected in the each of the second plurality of medical image data. In various examples, the corresponding adverse reaction prediction data is generated based on the corresponding risk characteristic detection data. In various examples, the first proper subset of individuals includes first ones of the second plurality of individuals with corresponding adverse reaction prediction data indicating administering of the medical treatment is safe based on having corresponding risk characteristic detection data indicating no detection of risk characteristics mapped to adverse reactions of the medical treatment. In various examples, the second proper subset of individuals includes second ones of the second plurality of individuals with corresponding adverse reaction prediction data indicating administering of the medical treatment is unsafe based on having corresponding risk characteristic detection data indicating detection of at least one risk characteristics mapped to at least one adverse reaction of the medical treatment.

In various embodiments, any one of more of the various examples described herein are implemented in conjunction with performing some or all steps of Figure 14A, Figure 14B, Figure 14C, Figure 14D, and/or Figure 14E. In various embodiments, any set of the various examples described herein, can implemented in tandem, for example, in conjunction with performing some or all steps of Figure 14A, Figure 14B, Figure 14C, Figure 14D, and/or Figure 14E, for example, optionally in conjunction with performing one or more steps of any one or more of Figures 10A - 10G.

In various embodiments, at least one memory device, memory section, and/or memory resource (e.g., a non-transitory computer readable storage medium) can store operational instructions that, when executed by one or more processing modules of one or more computing devices of a database system, cause the one or more computing devices to perform any or all of the method steps of Figure 14A, Figure 14B, Figure 14C, Figure 14D, and/or Figure 14E. described above, for example, in conjunction with further implementing any one or more of the various examples described above.

In various embodiments, a medical data processing system includes at least one processor and at least one memory that stores operational instructions. In various embodiments, the operational instructions, when executed by the at least one processor, cause the database system to perform some or all steps of Figure 14A, Figure 14B, Figure 14C, Figure 14D, and/or Figure 14E., for example, in conjunction with further implementing any one or more of the various examples described above.

In various embodiments, the operational instructions, when executed by the at least one processor, cause the medical data processing system to: train an image-based adverse reaction prediction function based on utilizing artificial intelligence to process a training set that includes a first plurality of medical image data corresponding to a first plurality of individuals; obtain a new medical image data corresponding to a new individual based on the new individual identified as candidates for administering of a medical treatment; and/or generate adverse reaction prediction data for the new individual based on utilizing artificial intelligence to perform the image-based adverse reaction prediction function upon the new medical image data to generate corresponding adverse reaction prediction data for the new individual. Alternatively or in addition to any of the foregoing, the adverse reaction prediction data is processed to determine whether administering of the medical treatment to the new individual is safe.

Alternatively or in addition to any of the foregoing, the operational instructions, when executed by the at least one processor, cause the medical data processing system to: train an image-based adverse reaction prediction function based on utilizing artificial intelligence to process a training set that includes a first plurality of medical image data corresponding to a first plurality of individuals; obtain a second plurality of medical image data corresponding to a second plurality of individuals based on the second plurality of individuals being identified as candidates for administering of a medical treatment; and/or generate a plurality of adverse reaction prediction data for the second plurality of individuals based on utilizing artificial intelligence to perform the image-based adverse reaction prediction function upon each of the second plurality of medical image data to generate corresponding adverse reaction prediction data of the plurality of adverse reaction prediction data. Alternatively or in addition to any of the foregoing, the plurality of adverse reaction prediction data is processed to partition the second plurality of individuals into a first proper subset of the second plurality of individuals and a second proper subset of the second plurality of individuals. Alternatively or in addition to any of the foregoing, the first proper subset of individuals includes first ones of the second plurality of individuals with corresponding adverse reaction prediction data indicating administering of the medical treatment is safe. Alternatively or in addition to any of the foregoing, the second proper subset of individuals includes second ones of the second plurality of individuals with corresponding adverse reaction prediction data indicating administering of the medical treatment is unsafe.

Alternatively to or in addition to any of the foregoing, the first proper subset and the second proper subset are mutually exclusive. Alternatively to or in addition to any of the foregoing, the first proper subset and the second proper subset are collectively exhaustive with respect to the second plurality of individuals.

Alternatively or in addition to any of the foregoing, some or all of the plurality of adverse reaction data is generated at the same time via collectively processing corresponding medical image data, where corresponding ones of the second plurality of individuals are partitioned at a same time accordingly. Alternatively or in addition to any of the foregoing, some or all of the plurality of adverse reaction data is generated at the different times via collectively processing corresponding medical image data at different times (e.g. as corresponding individuals are identified as prospective candidates at different times), where corresponding ones of the second plurality of individuals are partitioned at a same time accordingly (e.g. a first individual is assigned to either the first proper subset or the second proper subset at a first time based on their corresponding treatment safety data, while a second individual is assigned to either the first proper subset or the second proper subset at a second time after the first time (e.g. on a different date) based on their corresponding treatment safety data.

Alternatively or in addition to any of the foregoing, each of the plurality of adverse reaction prediction data includes an adverse reaction score. Alternatively or in addition to any of the foregoing, partitioning the second plurality of individuals into the first proper subset and the second proper subset is based on comparing the adverse reaction score of each of the plurality of adverse reaction prediction data to a predefined score threshold. Alternatively or in addition to any of the foregoing, the predefined score threshold is configured to distinguish between safe and unsafe administering of the medical treatment.

Alternatively or in addition to any of the foregoing, the adverse reaction score is based on a predicted probability that a corresponding individual of the second plurality of individuals will have an adverse reaction to the medical treatment based on the image-based adverse reaction prediction function being trained to predict probability of the adverse reaction as a function of medical image data. Alternatively or in addition to any of the foregoing, the predefined score threshold corresponds to a threshold probability value. Alternatively or in addition to any of the foregoing, the first proper subset of individuals includes first ones of the second plurality of individuals with corresponding adverse reaction scores indicating a corresponding probability of encountering the adverse reaction that falls below the threshold probability value. Alternatively or in addition to any of the foregoing, the second proper subset of individuals includes second ones of the second plurality of individuals with corresponding adverse reaction scores indicating a corresponding probability of encountering the adverse reaction that exceeds the threshold probability value. Alternatively or in addition to any of the foregoing, an adverse rection score equal to the threshold probability value is configured to be assigned to the first proper subset of individuals. Alternatively or in addition to any of the foregoing, an adverse rection score equal to the threshold probability value is configured to be assigned to the second proper subset of individuals.

Alternatively or in addition to any of the foregoing, the adverse reaction score is based on a predicted probability that a corresponding individual of the second plurality of individuals will not have an adverse reaction to the medical treatment based on the image-based adverse reaction prediction function being trained to predict probability of the adverse reaction as a function of medical image data. Alternatively or in addition to any of the foregoing, the predefined score threshold corresponds to a threshold probability value. Alternatively or in addition to any of the foregoing, the first proper subset of individuals includes first ones of the second plurality of individuals with corresponding adverse reaction scores indicating a corresponding probability of not encountering the adverse reaction that exceeds the threshold probability value. Alternatively or in addition to any of the foregoing, the second proper subset of individuals includes second ones of the second plurality of individuals with corresponding adverse reaction scores indicating a corresponding probability of not encountering the adverse reaction that falls below the threshold probability value. Alternatively or in addition to any of the foregoing, an adverse rection score equal to the threshold probability value is configured to be assigned to the first proper subset of individuals. Alternatively or in addition to any of the foregoing, an adverse rection score equal to the threshold probability value is configured to be assigned to the second proper subset of individuals.

Alternatively or in addition to any of the foregoing, the training set further includes at least one of: adverse reaction data for at least some first ones of the first plurality of individuals indicating side effects observed for the at least some first ones of the first plurality of individuals; or medical treatment history for at least some second ones of the of the first plurality of individuals indicating medical treatments administered to at least some second ones of the of the first plurality of individuals.

Alternatively or in addition to any of the foregoing, the medical treatment is administered to only the second ones of the second plurality of individuals included in the second proper subset of individuals. Alternatively or in addition to any of the foregoing, medical treatment is not administered to the first ones of the second plurality of individuals included in the first proper subset of individuals.

Alternatively or in addition to any of the foregoing, the medical treatment is a pharmaceutical compound. Alternatively or in addition to any of the foregoing, the medical treatment is an immunotherapy treatment. Alternatively or in addition to any of the foregoing, the second plurality of individuals are identified for prospective treatment via the immunotherapy treatment based on being diagnosed with at least one type of cancer. Alternatively or in addition to any of the foregoing, the first proper subset of individuals includes first ones of the second plurality of individuals with corresponding adverse reaction prediction data indicating administering of immunotherapy treatment is safe. Alternatively or in addition to any of the foregoing, the second proper subset of individuals includes second ones of the second plurality of individuals with corresponding adverse reaction prediction data indicating administering of immunotherapy treatment is unsafe.

Alternatively or in addition to any of the foregoing, the image-based adverse reaction prediction function is trained and performed in conjunction with implementing a medical modeling platform (e.g. medical imaging platform 505) that includes at least one model trained via training data that includes the first plurality of medical image data. Alternatively or in addition to any of the foregoing, the image-based adverse reaction prediction function is performed based on applying the at least one model trained via the training data.

Alternatively or in addition to any of the foregoing, the medical treatment belongs to one medical treatment classification of a plurality of medical treatment classifications. Alternatively or in addition to any of the foregoing, the at least one model is trained to enable generation of adverse reaction prediction data corresponding to different ones of the plurality of medical treatment classifications. Alternatively or in addition to any of the foregoing, the operational instructions, when executed by the at least one processor, further cause the medical data processing system to obtain a third plurality of medical image data corresponding to a third plurality of individuals based on the third plurality of individuals being identified as candidates for administering of a second medical treatment belonging to a second medical treatment classification of the plurality of medical treatment classifications. Alternatively or in addition to any of the foregoing, the operational instructions, when executed by the at least one processor, further cause the medical data processing system to generate a second plurality of adverse reaction prediction data for the third plurality of individuals based on utilizing artificial intelligence to apply the at least one model to each of the third plurality of medical image data to generate corresponding adverse reaction prediction data of the second plurality of adverse reaction prediction data. Alternatively or in addition to any of the foregoing, the second plurality of adverse reaction prediction data is processed to partition the third plurality of individuals into a third proper subset of the third plurality of individuals and a fourth proper subset of the third plurality of individuals. Alternatively or in addition to any of the foregoing, the third proper subset of individuals includes first ones of the third plurality of individuals with corresponding adverse reaction prediction data indicating administering of the second medical treatment is safe. Alternatively or in addition to any of the foregoing, the fourth proper subset of individuals includes second ones of the third plurality of individuals with corresponding adverse reaction prediction data indicating administering of the second medical treatment is unsafe.

Alternatively or in addition to any of the foregoing, the at least one model is trained to enable generation of adverse reaction prediction data corresponding to different ones of a plurality of adverse reaction categories. Alternatively or in addition to any of the foregoing, each of the plurality of adverse reaction prediction data indicates probability of a corresponding one of the second plurality of individuals having an adverse reaction corresponding to a first adverse reaction category of the plurality of adverse reaction categories. Alternatively or in addition to any of the foregoing, the operational instructions, when executed by the at least one processor, further cause the medical data processing system to generate a second plurality of adverse reaction prediction data based on utilizing artificial intelligence to apply the at least one model to generate corresponding adverse reaction prediction data of the second plurality of adverse reaction prediction data. Alternatively or in addition to any of the foregoing, each of the second plurality of adverse reaction prediction data indicates probability having an adverse reaction corresponding to a second adverse reaction category of the plurality of adverse reaction categories.

Alternatively or in addition to any of the foregoing, the plurality of adverse reaction categories include at least one of: a plurality of symptom-based adverse reaction categories; a plurality of severity-based adverse reaction categories; a plurality of immunologic adverse reaction categories; a plurality of nonimmunologic adverse reaction categories; and/or a plurality of temporal-based reaction categories.

Alternatively or in addition to any of the foregoing, the image-based adverse reaction prediction function is trained to output a corresponding plurality of adverse reaction prediction data for each medical image data input. Alternatively or in addition to any of the foregoing, the plurality of adverse reaction prediction data is generated for the second plurality of individuals based on utilizing artificial intelligence to apply the at least one model to each of the second plurality of medical image data to generate corresponding adverse reaction prediction data of the plurality of adverse reaction prediction data. Alternatively or in addition to any of the foregoing, the second plurality of adverse reaction prediction data is also generated for the second plurality of individuals based on utilizing artificial intelligence to apply the at least one model to each of the second plurality of medical image data to generate corresponding adverse reaction prediction data of the second plurality of adverse reaction prediction data. Alternatively or in addition to any of the foregoing, each of the second plurality of adverse reaction prediction data corresponds to one of the second plurality of individuals. Alternatively or in addition to any of the foregoing, partitioning the second plurality of individuals into the first proper subset and the second proper subset is based on further processing the second plurality of adverse reaction prediction data.

Alternatively or in addition to any of the foregoing, the operational instructions, when executed by the at least one processor, further cause the medical data processing system to obtain a third plurality of medical image data corresponding to a third plurality of individuals based on the third plurality of individuals being identified as candidates for administering of a second medical treatment. Alternatively or in addition to any of the foregoing, the second plurality of adverse reaction prediction data is generated for the third plurality of individuals based on utilizing artificial intelligence to apply the at least one model to each of the third plurality of medical image data to generate corresponding adverse reaction prediction data of the second plurality of adverse reaction prediction data.

Alternatively or in addition to any of the foregoing, the at least one model is trained to process input data corresponding to different medical imaging modalities of a plurality of different medical imaging modalities based on being trained via a set of training data that includes at least the first plurality of medical image data and an additional plurality of medical image data. Alternatively or in addition to any of the foregoing, the first plurality of medical image data includes first corresponding medical images having a first medical imaging modality of the plurality of different medical imaging modalities. Alternatively or in addition to any of the foregoing, the additional plurality of medical image data includes additional corresponding medical images having a second medical image modality of the plurality of medical imaging modalities.

Alternatively or in addition to any of the foregoing, each of the second plurality of medical image data includes a corresponding medical image, captured for a corresponding one of the second plurality of individuals, having the first medical imaging modality.

Alternatively or in addition to any of the foregoing, each of a first subset of the second plurality of medical image data includes a corresponding medical image, captured for a corresponding one of the second plurality of individuals, having the first medical imaging modality. Alternatively or in addition to any of the foregoing, a first corresponding subset of adverse reaction prediction data of the plurality of adverse reaction prediction data is generated for a first corresponding subset of patients based on applying the at least one model to process corresponding medical images having the first medical imaging modality. Alternatively or in addition to any of the foregoing, each of a second subset of the second plurality of medical image data includes a corresponding medical image, captured for a corresponding one of the second plurality of individuals, having the second medical imaging modality. Alternatively or in addition to any of the foregoing, a second corresponding subset of adverse reaction prediction data of the plurality of adverse reaction prediction data is generated for a second corresponding subset of patients based on applying the at least one model to process corresponding medical images having the second medical imaging modality.

Alternatively or in addition to any of the foregoing, at least one of the second plurality of medical image data includes multiple medical images that includes one corresponding medical image having the first medical imaging modality, captured for a corresponding one of the second plurality of individuals on a first date, and another corresponding medical image having the first medical imaging modality, captured for the corresponding one of the second plurality of individuals on a second date after the first date. Alternatively or in addition to any of the foregoing, at least one corresponding adverse reaction prediction data of the plurality of adverse reaction prediction data is generated for at least one corresponding patient based on applying the at least one model to the multiple medical images.

Alternatively or in addition to any of the foregoing, at least one of the second plurality of medical image data includes multiple medical images that includes one corresponding medical image, captured for a corresponding one of the second plurality of individuals, having the first medical imaging modality, and another corresponding medical image, captured for the corresponding one of the second plurality of individuals on a second date after the first date, having the second medical imaging modality. Alternatively or in addition to any of the foregoing, at least one corresponding adverse reaction prediction data of the plurality of adverse reaction prediction data is generated for at least one corresponding patient based on applying the at least one model to the multiple medical images.

Alternatively or in addition to any of the foregoing, the at least one model is trained to process input data that includes additional, non-imaging-based data based on being trained via a set of training data that includes at least the first plurality of medical image data and a plurality of additional, non-imaging-based data. Alternatively or in addition to any of the foregoing, some or all of the second plurality of individuals has corresponding input data that includes a corresponding one of the second plurality of medical image data and corresponding non-imaging-based data. Alternatively or in addition to any of the foregoing, the corresponding non-imaging-based data includes non-imaging-based device-captured medical data, demographic data; patient history data, medical report text data; and/or risk factor data. Alternatively or in addition to any of the foregoing, adverse reaction prediction data is generated for the each of the second plurality of individuals based on applying the at least one model to both the corresponding one of the second plurality of medical image data and the corresponding non-imaging-based data of the corresponding input data.

Alternatively or in addition to any of the foregoing, the medical treatment is configured to treat a medical condition. Alternatively or in addition to any of the foregoing, the at least one model is further trained to detect the medical condition based processing medical image data. Alternatively or in addition to any of the foregoing, the operational instructions, when executed by the at least one processor, further cause the medical data processing system to obtain a third plurality of medical image data for the third plurality of individuals. Alternatively or in addition to any of the foregoing, the operational instructions, when executed by the at least one processor, further cause the medical data processing system to generate a plurality of medical condition detection data for the third plurality of individuals based on utilizing artificial intelligence to apply the at least one model to each of the third plurality of medical image data to generate corresponding medical condition detection data of the plurality of medical condition detection data. Alternatively or in addition to any of the foregoing, the plurality of medical condition detection data is processed to identify a third proper subset of the third plurality of individuals and a fourth proper subset of the third plurality of individuals. Alternatively or in addition to any of the foregoing, the third proper subset includes first ones of the third plurality of individuals having medical condition detection data indicating the medical condition is detected. Alternatively or in addition to any of the foregoing, the fourth proper subset includes second ones of the third plurality of individuals having medical condition detection data indicating the medical condition is undetected. Alternatively or in addition to any of the foregoing, at least one of the second plurality of individuals is automatically identified as a candidate for the medical treatment based on being included in the fourth proper subset of the third plurality of individuals. Alternatively or in addition to any of the foregoing, at least one corresponding medical image data of the second plurality of medical image data is processed to generate both corresponding medical condition detection data and corresponding adverse reaction prediction data for the at least one of the second plurality of individuals.

Alternatively or in addition to any of the foregoing, the second plurality of individuals are candidates for administering of the medical treatment based on being prospective clinical trial participants of a clinical trial conducted to test the medical treatment. Alternatively or in addition to any of the foregoing, each of the second plurality of medical image data corresponds to pre-trial medical data for a corresponding one of the second plurality of individuals. Alternatively or in addition to any of the foregoing, the second proper subset of individuals are excluded from participation in the clinical trial based on having corresponding adverse reaction prediction data indicating administering of the medical treatment is unsafe. Alternatively or in addition to any of the foregoing, the first proper subset of individuals are not excluded from participation in the clinical trial based on having corresponding adverse reaction prediction data indicating administering of the medical treatment is safe. Alternatively or in addition to any of the foregoing, each of the first proper subset of individuals are assigned to a corresponding trial arm of a set of trial arms of the clinical trial based on not being excluded from participation in the clinical trial. Alternatively or in addition to any of the foregoing, each of the first proper subset of individuals are assigned to a corresponding trial arm of a set of trial arms of the clinical trial via performance of some or all steps of one or more of Figures 10A - 10G.

Alternatively or in addition to any of the foregoing, the operational instructions, when executed by the at least one processor, further cause the medical data processing system to generate a plurality of treatment safety data for the second plurality of individuals based on processing the plurality of adverse reaction prediction data. Alternatively or in addition to any of the foregoing, each of the plurality of treatment safety data indicates whether administering of the medical treatment is safe or unsafe for a corresponding one of the second plurality of individuals based on processing a corresponding one of the plurality of adverse reaction prediction data for the corresponding one of the second plurality of individuals. Alternatively or in addition to any of the foregoing, partitioning the second plurality of individuals into the first proper subset and the second proper subset is based on processing the plurality of treatment safety data.

Alternatively or in addition to any of the foregoing, the operational instructions, when executed by the at least one processor, further cause the medical data processing system to communicate each of the plurality of adverse reaction prediction data and/or each of the plurality of treatment safety data to a medical entity associated with administering the medical treatment. Alternatively or in addition to any of the foregoing, communicating each of the plurality of adverse reaction prediction data and/or each of the plurality of treatment safety data to the medical entity includes transmitting the plurality of adverse reaction prediction data and/or the plurality of treatment safety data to at least one computing device associated with the medical entity, storing the adverse reaction prediction data and/o the plurality of treatment safety data in storage resources associated with the medical entity, displaying the plurality of adverse reaction prediction data and/or the plurality of treatment safety data via at least one display device associated with the medical entity, and/or dictating the plurality of adverse reaction prediction data and/or the plurality of treatment safety data via at least one microphone associated with the medical entity.

Alternatively or in addition to any of the foregoing, performing the image-based adverse reaction prediction function upon each of the second plurality of medical image data includes generating corresponding risk characteristic detection data. Alternatively or in addition to any of the foregoing, the corresponding risk characteristic detection data is processed to determine whether risk characteristics mapped to adverse reactions of the medical treatment are detected in the each of the second plurality of medical image data. Alternatively or in addition to any of the foregoing, the corresponding adverse reaction prediction data is generated based on the corresponding risk characteristic detection data. Alternatively or in addition to any of the foregoing, the first proper subset of individuals includes first ones of the second plurality of individuals with corresponding adverse reaction prediction data indicating administering of the medical treatment is safe based on having corresponding risk characteristic detection data indicating no detection of risk characteristics mapped to adverse reactions of the medical treatment. Alternatively or in addition to any of the foregoing, the second proper subset of individuals includes second ones of the second plurality of individuals with corresponding adverse reaction prediction data indicating administering of the medical treatment is unsafe based on having corresponding risk characteristic detection data indicating detection of at least one risk characteristics mapped to at least one adverse reaction of the medical treatment.

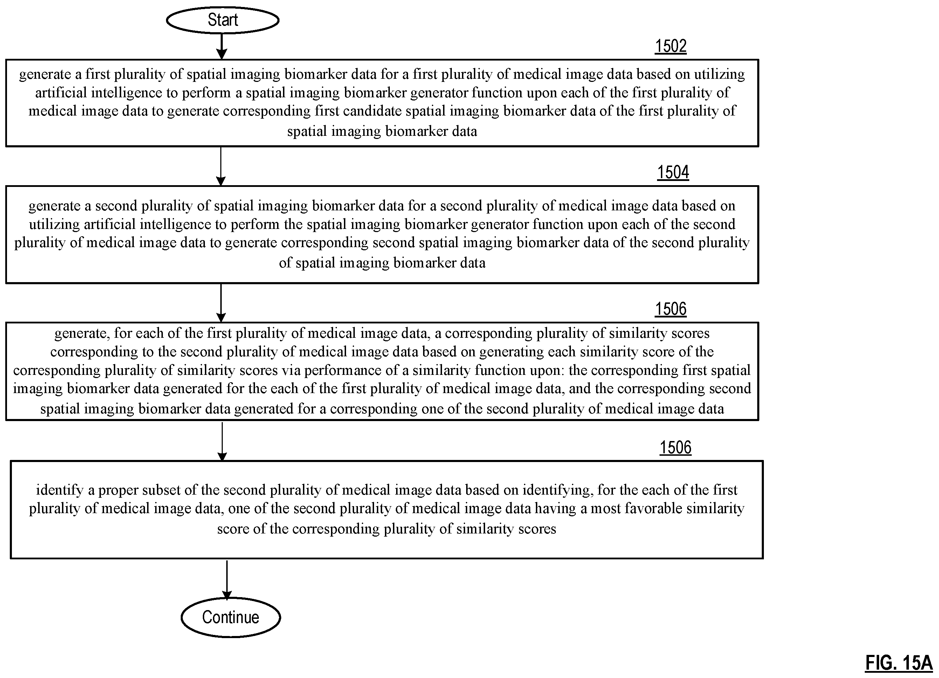

Figures 15A illustrates a method for execution by at least one processor. For example, some or all of Figure 15A can be executed via medical data processing system 500 and/or can be executed based on communicating with a medical data processing system 500. For example, some or all of the steps of Figure 15A can be performed in conjunction with implementing some or all features and/or functionality of the medical data processing system of any of the Figures described herein. Alternatively or in addition, some or all of the steps of Figure 15A can be performed in conjunction with the system 100 of Figure 1B, for example, in conjunction with implementing the imaging-based prognostication (IPRO) framework 200A and/or at least one corresponding model 112, for example, in conjunction with some or all features and/or functionality presented in Figure 8A and/or the example embodiments of Figures 8B - 9R. Alternatively or in addition, some or all of the steps of Figure 15A can be performed by, and/or based on communications between, any one or more computing devices 700, any one or more corresponding server systems, and/or any processing system that includes at least one processor and/or at least one memory.

Step 1502 includes generating a first plurality of spatial imaging biomarker data for a first plurality of medical image data based on utilizing artificial intelligence to perform a spatial imaging biomarker generator function upon each of the first plurality of medical image data to generate corresponding first spatial imaging biomarker data (e.g. corresponding clinical trial candidate spatial imaging biomarker data) of the first plurality of spatial imaging biomarker data.

Step 1504 includes generating a second plurality of spatial imaging biomarker data for a second plurality of medical image data based on utilizing artificial intelligence to perform the spatial imaging biomarker generator function upon each of the second plurality of medical image data to generate corresponding second spatial imaging biomarker data (e.g. corresponding real-world patient spatial imaging biomarker data) of the second plurality of spatial imaging biomarker data.

Step 1506 includes generating, for each of the first plurality of medical image data, a corresponding plurality of similarity scores corresponding to the second plurality of medical image data based on generating each similarity score of the corresponding plurality of similarity scores via performance of a similarity function upon: the corresponding clinical trial candidate spatial imaging biomarker data generated for the each of the first plurality of medical image data, and the corresponding real-world patient spatial imaging biomarker data generated for a corresponding one of the second plurality of medical image data.

Step 1508 includes identifying a proper subset of the second plurality of medical image data based on identifying, for the each of the first plurality of medical image data, one of the second plurality of medical image data having a most favorable similarity score of the corresponding plurality of similarity scores.

Alternatively or in addition to any of the foregoing, the method can further include obtaining the first plurality of medical image data.

Alternatively or in addition to any of the foregoing, the first plurality of medical image data can correspond to a first plurality of individuals (e.g. based on the first plurality of individuals being identified as candidates for participation in a clinical trial).

Alternatively or in addition to any of the foregoing, the method can further include obtaining the second plurality of medical image data.

Alternatively or in addition to any of the foregoing, the second plurality of medical image data can correspond to a second plurality of individuals based on the second plurality of individuals corresponding to real-world patients distinct from the first plurality of individuals.

Alternatively or in addition to any of the foregoing, the method can further include training the spatial imaging biomarker generator function based on utilizing artificial intelligence to process a training set that includes a third plurality of medical image data corresponding to a third plurality of individuals distinct from the first plurality of individuals and the second plurality of individuals.

Alternatively or in addition to any of the foregoing, a full plurality of spatial imaging biomarker data includes the first plurality of spatial imaging biomarker data and the second plurality of spatial imaging biomarker data. Alternatively or in addition to any of the foregoing, each spatial imaging biomarker data of the full plurality of spatial imaging biomarker data includes a plurality of spatial imaging biomarkers generated via performing the spatial imaging biomarker generator function.

Alternatively or in addition to any of the foregoing, the plurality of spatial imaging biomarkers includes: at least one subcutaneous fat-based biomarker; at least one skeletal muscle-based biomarker; at least one lesion-based biomarker; at least one pleural effusion-based biomarker; and/or at least one visceral fat-based biomarker.

Alternatively or in addition to any of the foregoing, performing the spatial imaging biomarker generator function upon each medical image data of a full plurality of medical image data that includes the first plurality of medical image data the second plurality of medical image data includes: segmenting the each medical image data into a plurality of anatomical type-based segments. Alternatively or in addition to any of the foregoing, the plurality of anatomical type-based segments includes: at least one subcutaneous fat segment; at least one skeletal muscle segment; at least one lesion segment; at least one pleural effusion segment; and/or at least one visceral fat segment.

Alternatively or in addition to any of the foregoing, performing the spatial imaging biomarker generator function upon each medical image data of a full plurality of medical image data that includes the first plurality of medical image data the second plurality of medical image data includes: processing each of the plurality of anatomical type-based segments to generate a corresponding spatial imaging biomarker of the plurality of spatial imaging biomarkers. Alternatively or in addition to any of the foregoing: the at least one subcutaneous fat-based biomarker is generated via processing the at least one subcutaneous fat segment; the at least one skeletal muscle-based biomarker is generated via processing the at least one skeletal muscle segment; the at least one lesion-based biomarker is generated via processing the at least one lesion segment; the at least one pleural effusion-based biomarker is generated via processing the at least one pleural effusion segment; and/or the at least one visceral fat-based biomarker is generated via processing the at least one visceral fat segment.

Alternatively or in addition to any of the foregoing, the plurality of spatial imaging biomarkers includes over 5,000 different spatial imaging biomarkers.

Alternatively or in addition to any of the foregoing, the similarity function is performed upon: a first vector having a first ordered set of values for the corresponding clinical trial candidate spatial imaging biomarker data, and a second vector having a second ordered set of values for the corresponding clinical trial candidate spatial imaging biomarker data. Alternatively or in addition to any of the foregoing, the first vector and the second vector have a same number of values in an ordering corresponding to a predetermined ordering of the plurality of spatial imaging biomarkers.

Alternatively or in addition to any of the foregoing, performing the similarity function includes performing a cosine similarity function upon the first vector and the second vector.

Alternatively or in addition to any of the foregoing, the first plurality of medical image data includes a first plurality of 3D computed tomography (CT) scans, and/or the second plurality of medical image data includes a second plurality of CT scans.

Alternatively or in addition to any of the foregoing, an external control arm of the clinical trial is implemented to include the proper subset of the second plurality of medical image data. Alternatively or in addition to any of the foregoing, a treatment arm of the clinical trial is implemented to include the first plurality of individuals.

Alternatively or in addition to any of the foregoing, the method further includes generating the external control arm of the clinical trial to include the proper subset of the second plurality of medical image data.

Alternatively or in addition to any of the foregoing, a medical data processing system comprises: at least one processor; and/or at least one memory storing operational instructions that, when executed by the at least one processor, cause the medical data processing system to perform some or all steps of Figure 15A, in the alternative or addition to any other steps and/or portion of any other method, process, function, and/or algorithm described herein.

Alternatively or in addition to any of the foregoing, the operational instructions, when executed by the at least one processor, cause the medical data processing system to: obtain a first plurality of medical image data corresponding to a first plurality of individuals based on the first plurality of individuals being identified as candidates for participation in a clinical trial; generate a first plurality of spatial imaging biomarker data for the first plurality of medical image data based on utilizing artificial intelligence to perform the spatial imaging biomarker generator function upon each of the first plurality of medical image data to generate corresponding clinical trial candidate spatial imaging biomarker data of the first plurality of spatial imaging biomarker data; obtain a second plurality of medical image data corresponding to a second plurality of individuals based on the second plurality of individuals corresponding to real-world patients distinct from the first plurality of individuals; generate a second plurality of spatial imaging biomarker data for the second plurality of medical image data based on utilizing artificial intelligence to perform a spatial imaging biomarker generator function upon each of the second plurality of medical image data to generate corresponding real-world patient spatial imaging biomarker data of the second plurality of spatial imaging biomarker data; generate, for each of the first plurality of medical image data, a corresponding plurality of similarity scores corresponding to the second plurality of medical image data based on generating each similarity score of the corresponding plurality of similarity scores via performance of a similarity function upon: the corresponding clinical trial candidate spatial imaging biomarker data generated for the each of the first plurality of medical image data, and the corresponding real-world patient spatial imaging biomarker data generated for a corresponding one of the second plurality of medical image data; and/or identify a proper subset of the second plurality of medical image data based on identifying, for the each of the first plurality of medical image data, one of the second plurality of medical image data having a most favorable similarity score of the corresponding plurality of similarity scores.

Alternatively or in addition to any of the foregoing, the operational instructions, when executed by the at least one processor, further cause the medical data processing system to: train the spatial imaging biomarker generator function based on utilizing artificial intelligence to process a training set that includes a third plurality of medical image data corresponding to a third plurality of individuals distinct from the first plurality of individuals and the second plurality of individuals.

Alternatively or in addition to any of the foregoing, a full plurality of spatial imaging biomarker data includes the first plurality of spatial imaging biomarker data and the second plurality of spatial imaging biomarker data. Alternatively or in addition to any of the foregoing, each spatial imaging biomarker data of the full plurality of spatial imaging biomarker data includes a plurality of spatial imaging biomarkers generated via performing the spatial imaging biomarker generator function.

Alternatively or in addition to any of the foregoing, the plurality of spatial imaging biomarkers includes: at least one subcutaneous fat-based biomarker; at least one skeletal muscle-based biomarker; at least one lesion-based biomarker; at least one pleural effusion-based biomarker; and at least one visceral fat-based biomarker.

Alternatively or in addition to any of the foregoing, performing the spatial imaging biomarker generator function upon each medical image data of a full plurality of medical image data that includes the first plurality of medical image data the second plurality of medical image data includes segmenting the each medical image data into a plurality of anatomical type-based segments. Alternatively or in addition to any of the foregoing, the plurality of anatomical type-based segments includes: at least one subcutaneous fat segment; at least one skeletal muscle segment; at least one lesion segment; at least one pleural effusion segment; and/or at least one visceral fat segment.

Alternatively or in addition to any of the foregoing, performing the spatial imaging biomarker generator function upon each medical image data of a full plurality of medical image data that includes the first plurality of medical image data the second plurality of medical image data includes processing each of the plurality of anatomical type-based segments to generate a corresponding spatial imaging biomarker of the plurality of spatial imaging biomarkers. Alternatively or in addition to any of the foregoing: the at least one subcutaneous fat-based biomarker is generated via processing the at least one subcutaneous fat segment; the at least one skeletal muscle-based biomarker is generated via processing the at least one skeletal muscle segment; the at least one lesion-based biomarker is generated via processing the at least one lesion segment; the at least one pleural effusion-based biomarker is generated via processing the at least one pleural effusion segment; and/or the at least one visceral fat-based biomarker is generated via processing the at least one visceral fat segment.

Alternatively or in addition to any of the foregoing, the plurality of spatial imaging biomarkers includes over 5,000 different spatial imaging biomarkers.

Alternatively or in addition to any of the foregoing, the similarity function is performed upon: a first vector having a first ordered set of values for the corresponding clinical trial candidate spatial imaging biomarker data, and a second vector having a second ordered set of values for the corresponding clinical trial candidate spatial imaging biomarker data. Alternatively or in addition to any of the foregoing, the first vector and the second vector have a same number of values in an ordering corresponding to a predetermined ordering of the plurality of spatial imaging biomarkers.

Alternatively or in addition to any of the foregoing, performing the similarity function includes performing a cosine similarity function upon the first vector and the second vector.

Alternatively or in addition to any of the foregoing, the first plurality of medical image data includes a first plurality of 3D computed tomography (CT) scans and/or the second plurality of medical image data includes a second plurality of CT scans.

Alternatively or in addition to any of the foregoing, an external control arm of the clinical trial is implemented to include the proper subset of the second plurality of medical image data. Alternatively or in addition to any of the foregoing, a treatment arm of the clinical trial is implemented to include the first plurality of individuals.

Alternatively or in addition to any of the foregoing, the operational instructions, when executed by the at least one processor, further cause the medical data processing system to generate the external control arm of the clinical trial to include the proper subset of the second plurality of medical image data.

It is noted that terminologies as may be used herein such as bit stream, stream, signal sequence, etc. (or their equivalents) have been used interchangeably to describe digital information whose content corresponds to any of a number of desired types (e.g., data, video, speech, text, graphics, audio, etc. any of which may generally be referred to as 'data').

As may be used herein, the terms "substantially" and "approximately" provides an industry-accepted tolerance for its corresponding term and/or relativity between items. For some industries, an industry-accepted tolerance is less than one percent and, for other industries, the industry-accepted tolerance is 10 percent or more. Other examples of industry-accepted tolerance range from less than one percent to fifty percent. Industry-accepted tolerances correspond to, but are not limited to, component values, integrated circuit process variations, temperature variations, rise and fall times, thermal noise, dimensions, signaling errors, dropped packets, temperatures, pressures, material compositions, and/or performance metrics. Within an industry, tolerance variances of accepted tolerances may be more or less than a percentage level (e.g., dimension tolerance of less than +/- 1%). Some relativity between items may range from a difference of less than a percentage level to a few percent. Other relativity between items may range from a difference of a few percent to magnitude of differences.

As may also be used herein, the term(s) "configured to", "operably coupled to", "coupled to", and/or "coupling" includes direct coupling between items and/or indirect coupling between items via an intervening item (e.g., an item includes, but is not limited to, a component, an element, a circuit, and/or a module) where, for an example of indirect coupling, the intervening item does not modify the information of a signal but may adjust its current level, voltage level, and/or power level. As may further be used herein, inferred coupling (i.e., where one element is coupled to another element by inference) includes direct and indirect coupling between two items in the same manner as "coupled to".

As may even further be used herein, the term "configured to", "operable to", "coupled to", or "operably coupled to" indicates that an item includes one or more of power connections, input(s), output(s), etc., to perform, when activated, one or more its corresponding functions and may further include inferred coupling to one or more other items. As may still further be used herein, the term "associated with", includes direct and/or indirect coupling of separate items and/or one item being embedded within another item.

As may be used herein, the term "compares favorably", indicates that a comparison between two or more items, signals, etc., indicates an advantageous relationship that would be evident to one skilled in the art in light of the present disclosure, and based, for example, on the nature of the signals/items that are being compared. As may be used herein, the term "compares unfavorably", indicates that a comparison between two or more items, signals, etc., fails to provide such an advantageous relationship and/or that provides a disadvantageous relationship. Such an item/signal can correspond to one or more numeric values, one or more measurements, one or more counts and/or proportions, one or more types of data, and/or other information with attributes that can be compared to a threshold, to each other and/or to attributes of other information to determine whether a favorable or unfavorable comparison exists. Examples of such an advantageous relationship can include: one item/signal being greater than (or greater than or equal to) a threshold value, one item/signal being less than (or less than or equal to) a threshold value, one item/signal being greater than (or greater than or equal to) another item/signal, one item/signal being less than (or less than or equal to) another item/signal, one item/signal matching another item/signal, one item/signal substantially matching another item/signal within a predefined or industry accepted tolerance such as 1%, 5%, 10% or some other margin, etc. Furthermore, one skilled in the art will recognize that such a comparison between two items/signals can be performed in different ways. For example, when the advantageous relationship is that signal 1 has a greater magnitude than signal 2, a favorable comparison may be achieved when the magnitude of signal 1 is greater than that of signal 2 or when the magnitude of signal 2 is less than that of signal 1. Similarly, one skilled in the art will recognize that the comparison of the inverse or opposite of items/signals and/or other forms of mathematical or logical equivalence can likewise be used in an equivalent fashion. For example, the comparison to determine if a signal X > 5 is equivalent to determining if -X < -5, and the comparison to determine if signal A matches signal B can likewise be performed by determining -A matches -B or not(A) matches not(B). As may be discussed herein, the determination that a particular relationship is present (either favorable or unfavorable) can be utilized to automatically trigger a particular action. Unless expressly stated to the contrary, the absence of that particular condition may be assumed to imply that the particular action will not automatically be triggered. In other examples, the determination that a particular relationship is present (either favorable or unfavorable) can be utilized as a basis or consideration to determine whether to perform one or more actions. Note that such a basis or consideration can be considered alone or in combination with one or more other bases or considerations to determine whether to perform the one or more actions. In one example where multiple bases or considerations are used to determine whether to perform one or more actions, the respective bases or considerations are given equal weight in such determination. In another example where multiple bases or considerations are used to determine whether to perform one or more actions, the respective bases or considerations are given unequal weight in such determination.

As may be used herein, one or more claims may include, in a specific form of this generic form, the phrase "at least one of a, b, and c" or of this generic form "at least one of a, b, or c", with more or less elements than "a", "b", and "c". In either phrasing, the phrases are to be interpreted identically. In particular, "at least one of a, b, and c" is equivalent to "at least one of a, b, or c" and shall mean a, b, and/or c. As an example, it means: "a" only, "b" only, "c" only, "a" and "b", "a" and "c", "b" and "c", and/or "a", "b", and "c".

As may also be used herein, the terms "processing module", "processing circuit", "processor", "processing circuitry", and/or "processing unit" may be a single processing device or a plurality of processing devices. Such a processing device may be a microprocessor, micro-controller, digital signal processor, microcomputer, central processing unit, field programmable gate array, programmable logic device, state machine, logic circuitry, analog circuitry, digital circuitry, and/or any device that manipulates signals (analog and/or digital) based on hard coding of the circuitry and/or operational instructions. The processing module, module, processing circuit, processing circuitry, and/or processing unit may be, or further include, memory and/or an integrated memory element, which may be a single memory device, a plurality of memory devices, and/or embedded circuitry of another processing module, module, processing circuit, processing circuitry, and/or processing unit. Such a memory device may be a read-only memory, random access memory, volatile memory, non-volatile memory, static memory, dynamic memory, flash memory, cache memory, and/or any device that stores digital information. Note that if the processing module, module, processing circuit, processing circuitry, and/or processing unit includes more than one processing device, the processing devices may be centrally located (e.g., directly coupled together via a wired and/or wireless bus structure) or may be distributedly located (e.g., cloud computing via indirect coupling via a local area network and/or a wide area network). Further note that if the processing module, module, processing circuit, processing circuitry and/or processing unit implements one or more of its functions via a state machine, analog circuitry, digital circuitry, and/or logic circuitry, the memory and/or memory element storing the corresponding operational instructions may be embedded within, or external to, the circuitry comprising the state machine, analog circuitry, digital circuitry, and/or logic circuitry. Still further note that, the memory element may store, and the processing module, module, processing circuit, processing circuitry and/or processing unit executes, hard coded and/or operational instructions corresponding to at least some of the steps and/or functions illustrated in one or more of the Figures. Such a memory device or memory element can be included in an article of manufacture.

One or more embodiments have been described above with the aid of method steps illustrating the performance of specified functions and relationships thereof. The boundaries and sequence of these functional building blocks and method steps have been arbitrarily defined herein for convenience of description. Alternate boundaries and sequences can be defined so long as the specified functions and relationships are appropriately performed. Any such alternate boundaries or sequences are thus within the scope and spirit of the claims. Similarly, flow diagram blocks may also have been arbitrarily defined herein to illustrate certain significant functionality.

To the extent used, the flow diagram block boundaries and sequence could have been defined otherwise and still perform the certain significant functionality. Such alternate definitions of both functional building blocks and flow diagram blocks and sequences are thus within the scope and spirit of the claims. One of average skill in the art will also recognize that the functional building blocks, and other illustrative blocks, modules and components herein, can be implemented as illustrated or by discrete components, application specific integrated circuits, processors executing appropriate software and the like or any combination thereof.

In addition, a flow diagram may include a "start" and/or "continue" indication. The "start" and "continue" indications reflect that the steps presented can optionally be incorporated in or otherwise used in conjunction with one or more other routines. In addition, a flow diagram may include an "end" and/or "continue" indication. The "end" and/or "continue" indications reflect that the steps presented can end as described and shown or optionally be incorporated in or otherwise used in conjunction with one or more other routines. In this context, "start" indicates the beginning of the first step presented and may be preceded by other activities not specifically shown. Further, the "continue" indication reflects that the steps presented may be performed multiple times and/or may be succeeded by other activities not specifically shown. Further, while a flow diagram indicates a particular ordering of steps, other orderings are likewise possible provided that the principles of causality are maintained.

The one or more embodiments are used herein to illustrate one or more aspects, one or more features, one or more concepts, and/or one or more examples. A physical embodiment of an apparatus, an article of manufacture, a machine, and/or of a process may include one or more of the aspects, features, concepts, examples, etc. described with reference to one or more of the embodiments discussed herein. Further, from figure to figure, the embodiments may incorporate the same or similarly named functions, steps, modules, etc. that may use the same or different reference numbers and, as such, the functions, steps, modules, etc. may be the same or similar functions, steps, modules, etc. or different ones.

Unless specifically stated to the contra, signals to, from, and/or between elements in a figure of any of the figures presented herein may be analog or digital, continuous time or discrete time, and single-ended or differential. For instance, if a signal path is shown as a single-ended path, it also represents a differential signal path. Similarly, if a signal path is shown as a differential path, it also represents a single-ended signal path. While one or more particular architectures are described herein, other architectures can likewise be implemented that use one or more data buses not expressly shown, direct connectivity between elements, and/or indirect coupling between other elements as recognized by one of average skill in the art.

The term "module" is used in the description of one or more of the embodiments. A module implements one or more functions via a device such as a processor or other processing device or other hardware that may include or operate in association with a memory that stores operational instructions. A module may operate independently and/or in conjunction with software and/or firmware. As also used herein, a module may contain one or more sub-modules, each of which may be one or more modules.

As may further be used herein, a computer readable memory includes one or more memory elements. A memory element may be a separate memory device, multiple memory devices, or a set of memory locations within a memory device. Such a memory device may be a read-only memory, random access memory, volatile memory, non-volatile memory, static memory, dynamic memory, flash memory, cache memory, a quantum register or other quantum memory and/or any other device that stores data in a non-transitory manner. Furthermore, the memory device may be in a form of a solid-state memory, a hard drive memory or other disk storage, cloud memory, thumb drive, server memory, computing device memory, and/or other non-transitory medium for storing data. The storage of data includes temporary storage (i.e., data is lost when power is removed from the memory element) and/or persistent storage (i.e., data is retained when power is removed from the memory element). As used herein, a transitory medium shall mean one or more of: (a) a wired or wireless medium for the transportation of data as a signal from one computing device to another computing device for temporary storage or persistent storage; (b) a wired or wireless medium for the transportation of data as a signal within a computing device from one element of the computing device to another element of the computing device for temporary storage or persistent storage; (c) a wired or wireless medium for the transportation of data as a signal from one computing device to another computing device for processing the data by the other computing device; and (d) a wired or wireless medium for the transportation of data as a signal within a computing device from one element of the computing device to another element of the computing device for processing the data by the other element of the computing device. As may be used herein, a non-transitory computer readable memory is substantially equivalent to a computer readable memory. A non-transitory computer readable memory can also be referred to as a non-transitory computer readable storage medium.

One or more functions associated with the methods and/or processes described herein can be implemented via a processing module that operates via the non-human "artificial" intelligence (AI) of a machine. Examples of such AI include machines that operate via anomaly detection techniques, decision trees, association rules, expert systems and other knowledge-based systems, computer vision models, artificial neural networks, convolutional neural networks, support vector machines (SVMs), Bayesian networks, genetic algorithms, feature learning, sparse dictionary learning, preference learning, deep learning and other machine learning techniques that are trained using training data via unsupervised, semi-supervised, supervised and/or reinforcement learning, generative AI models, and/or other AI. The human mind is not equipped to perform such AI techniques, not only due to the complexity of these techniques, but also due to the fact that artificial intelligence, by its very definition - requires "artificial" intelligence - i.e. machine/non-human intelligence.

One or more functions associated with the methods and/or processes described herein can alternatively or additionally be implemented communication with a separate processing module that operates via the non-human "artificial" intelligence (AI) of a machine, for example, based on communication with this separate processing module to: configure training of a corresponding model via artificial intelligence by this separate processing module in conjunction with implementing one or more embodiments; receive model data for a corresponding model trained via artificial intelligence by this separate processing module for use in conjunction with implementing one or more embodiments; send model output processing via a corresponding model trained via artificial intelligence by this separate processing module in conjunction with implementing one or more embodiments; receive model output for a corresponding model trained and/or executed via artificial intelligence by this separate processing module for use in conjunction with implementing one or more embodiments; and/or otherwise communicating with the separate processing module to supply input and/or receive output in conjunction with a corresponding AI model being trained and/or applied via the separate processing module.

One or more functions associated with the methods and/or processes described herein can be implemented as a large-scale system that is operable to receive, transmit and/or process data on a large-scale. As used herein, a large-scale refers to a large number of data, such as one or more kilobytes, megabytes, gigabytes, terabytes or more of data that are received, transmitted and/or processed. Such receiving, transmitting and/or processing of data cannot practically be performed by the human mind on a large-scale within a reasonable period of time, such as within a second, a millisecond, microsecond, a real-time basis or other high speed required by the machines that generate the data, receive the data, convey the data, store the data and/or use the data.

One or more functions associated with the methods and/or processes described herein can require data to be manipulated in different ways within overlapping time spans. The human mind is not equipped to perform such different data manipulations independently, contemporaneously, in parallel, and/or on a coordinated basis within a reasonable period of time, such as within a second, a millisecond, microsecond, a real-time basis or other high speed required by the machines that generate the data, receive the data, convey the data, store the data and/or use the data.

One or more functions associated with the methods and/or processes described herein can be implemented in a system that is operable to electronically receive digital data via a wired or wireless communication network and/or to electronically transmit digital data via a wired or wireless communication network. Such receiving and transmitting cannot practically be performed by the human mind because the human mind is not equipped to electronically transmit or receive digital data, let alone to transmit and receive digital data via a wired or wireless communication network.

One or more functions associated with the methods and/or processes described herein can be implemented in a system that is operable to electronically store digital data in a memory device. Such storage cannot practically be performed by the human mind because the human mind is not equipped to electronically store digital data.

One or more functions associated with the methods and/or processes described herein may operate to cause an action by a processing module directly in response to a triggering event -- without any intervening human interaction between the triggering event and the action. Any such actions may be identified as being performed "automatically", "automatically based on" and/or "automatically in response to" such a triggering event. Furthermore, any such actions identified in such a fashion specifically preclude the operation of human activity with respect to these actions - even if the triggering event itself may be causally connected to a human activity of some kind.

While particular combinations of various functions and features of the one or more embodiments have been expressly described herein, other combinations of these features and functions are likewise possible. The present disclosure is not limited by the particular examples disclosed herein and expressly incorporates these other combinations.

Some or all features and/or functionality of the various embodiments described herein can be implemented via implementing any features and/or functionality presented in the Appendix section that follows, for example, via implementing medical data processing system (e.g. in conjunction with implementing some or all of the method of Figure 15A) to: generate and/or process external control arms; utilize an AI model (e.g. implemented via imaging-based prognostication framework 200A, one or more functions 571, and/or one or more AI platforms 588) to analyze each of a set of CT scans (e.g. collected from a completed lung cancer trial or other completed clinical trial, each optionally implemented as medical data 562 and/or having corresponding medical data metadata 610, for example, each for a given patient having corresponding patient data 564); identifying a real-world patient for each of the set of CT scans (e.g. in a corresponding other CT scan in a second set of other CT scans separate from those of the set of CT scans) with the most similar imaging features (and/or otherwise having sufficiently similar imaging features with the given scan, for example, based on the pair of scans having a computed similarity score comparing favorably to a similarly threshold), for example, via applying cosine similarity or other similarity measure; and/or generating an external control arm (e.g. a computational control arm) that includes a set of real-world patients, each identified for a corresponding one of the set of CT scans (e.g. based on being most similar to the corresponding one of the set of CT scans); and/or any other features and/or functionality presented in the Appendix section that follows. For example, some or all features and or functionality presented in the Appendix section (e.g. features and/or functionality relating to example embodiments of applying IPRO) can be applied to implement any embodiments and/or examples described herein, such as any embodiments of the via imaging-based prognostication framework 200A, one or more functions 571, one or more AI platforms 588, medical data processing system 500, some or all steps of the method of Figure 15A, and/or any other systems, methods, functions, processes, and/or algorithms described herein.

## Claims

1. A method comprising:
obtaining a first plurality of medical image data corresponding to a first plurality of individuals based on the first plurality of individuals being identified as candidates for participation in a clinical trial;
generating a first plurality of spatial imaging biomarker data for the first plurality of medical image data based on utilizing artificial intelligence to perform a spatial imaging biomarker generator function upon each of the first plurality of medical image data to generate corresponding clinical trial candidate spatial imaging biomarker data of the first plurality of spatial imaging biomarker data;
obtaining a second plurality of medical image data corresponding to a second plurality of individuals based on the second plurality of individuals corresponding to real-world patients distinct from the first plurality of individuals;
generating a second plurality of spatial imaging biomarker data for the second plurality of medical image data based on utilizing artificial intelligence to perform the spatial imaging biomarker generator function upon each of the second plurality of medical image data to generate corresponding real-world patient spatial imaging biomarker data of the second plurality of spatial imaging biomarker data;
generating, for each of the first plurality of medical image data, a corresponding plurality of similarity scores corresponding to the second plurality of medical image data based on generating each similarity score of the corresponding plurality of similarity scores via performance of a similarity function upon: the corresponding clinical trial candidate spatial imaging biomarker data generated for the each of the first plurality of medical image data, and the corresponding real-world patient spatial imaging biomarker data generated for a corresponding one of the second plurality of medical image data; and
identifying a proper subset of the second plurality of medical image data based on identifying, for the each of the first plurality of medical image data, one of the second plurality of medical image data having a most favorable similarity score of the corresponding plurality of similarity scores.

2. The method of claim 1, further comprising:
training the spatial imaging biomarker generator function based on utilizing artificial intelligence to process a training set that includes a third plurality of medical image data corresponding to a third plurality of individuals distinct from the first plurality of individuals and the second plurality of individuals.

3. The method of claim 1, wherein a full plurality of spatial imaging biomarker data includes the first plurality of spatial imaging biomarker data and the second plurality of spatial imaging biomarker data, and wherein each spatial imaging biomarker data of the full plurality of spatial imaging biomarker data includes a plurality of spatial imaging biomarkers generated via performing the spatial imaging biomarker generator function.

4. The method of claim 3, wherein the plurality of spatial imaging biomarkers includes:
At least one subcutaneous fat-based biomarker;
At least one skeletal muscle-based biomarker;
At least one lesion-based biomarker;
At least one pleural effusion-based biomarker; and
At least one visceral fat-based biomarker.

5. The method of claim 4, wherein performing the spatial imaging biomarker generator function upon each medical image data of a full plurality of medical image data that includes the first plurality of medical image data the second plurality of medical image data includes:
segmenting the each medical image data into a plurality of anatomical type-based segments, wherein the plurality of anatomical type-based segments includes: at least one subcutaneous fat segment; at least one skeletal muscle segment; at least one lesion segment; at least one pleural effusion segment; and at least one visceral fat segment;
processing each of the plurality of anatomical type-based segments to generate a corresponding spatial imaging biomarker of the plurality of spatial imaging biomarkers, wherein the at least one subcutaneous fat-based biomarker is generated via processing the at least one subcutaneous fat segment; wherein the at least one skeletal muscle-based biomarker is generated via processing the at least one skeletal muscle segment; wherein the at least one lesion-based biomarker is generated via processing the at least one lesion segment; wherein the at least one pleural effusion-based biomarker is generated via processing the at least one pleural effusion segment; and wherein the at least one visceral fat-based biomarker is generated via processing the at least one visceral fat segment.

6. The method of claim 3, wherein the plurality of spatial imaging biomarkers includes over 5,000 different spatial imaging biomarkers.

7. The method of claim 3, wherein the similarity function is performed upon: a first vector having a first ordered set of values for the corresponding clinical trial candidate spatial imaging biomarker data, and a second vector having a second ordered set of values for the corresponding clinical trial candidate spatial imaging biomarker data, wherein the first vector and the second vector have a same number of values in an ordering corresponding to a predetermined ordering of the plurality of spatial imaging biomarkers.

8. The method of claim 7, wherein performing the similarity function includes performing a cosine similarity function upon the first vector and the second vector.

9. The method of claim 1, wherein the first plurality of medical image data includes a first plurality of 3D computed tomography (CT) scans, and wherein the second plurality of medical image data includes a second plurality of CT scans.

10. The method of claim 1, wherein an external control arm of the clinical trial is implemented to include the proper subset of the second plurality of medical image data, and wherein a treatment arm of the clinical trial is implemented to include the first plurality of individuals.

11. A medical data processing system comprises:
at least one processor; and
at least one memory storing operational instructions that, when executed by the at least one processor, cause the medical data processing system to:
obtain a first plurality of medical image data corresponding to a first plurality of individuals based on the first plurality of individuals being identified as candidates for participation in a clinical trial;
generate a first plurality of spatial imaging biomarker data for the first plurality of medical image data based on utilizing artificial intelligence to perform the spatial imaging biomarker generator function upon each of the first plurality of medical image data to generate corresponding clinical trial candidate spatial imaging biomarker data of the first plurality of spatial imaging biomarker data;
obtain a second plurality of medical image data corresponding to a second plurality of individuals based on the second plurality of individuals corresponding to real-world patients distinct from the first plurality of individuals;
generate a second plurality of spatial imaging biomarker data for the second plurality of medical image data based on utilizing artificial intelligence to perform a spatial imaging biomarker generator function upon each of the second plurality of medical image data to generate corresponding real-world patient spatial imaging biomarker data of the second plurality of spatial imaging biomarker data;
generate, for each of the first plurality of medical image data, a corresponding plurality of similarity scores corresponding to the second plurality of medical image data based on generating each similarity score of the corresponding plurality of similarity scores via performance of a similarity function upon: the corresponding clinical trial candidate spatial imaging biomarker data generated for the each of the first plurality of medical image data, and the corresponding real-world patient spatial imaging biomarker data generated for a corresponding one of the second plurality of medical image data; and
identify a proper subset of the second plurality of medical image data based on identifying, for the each of the first plurality of medical image data, one of the second plurality of medical image data having a most favorable similarity score of the corresponding plurality of similarity scores.

12. The medical data processing system of claim 11, further comprising:
training the spatial imaging biomarker generator function based on utilizing artificial intelligence to process a training set that includes a third plurality of medical image data corresponding to a third plurality of individuals distinct from the first plurality of individuals and the second plurality of individuals.

13. The medical data processing system of claim 11, wherein a full plurality of spatial imaging biomarker data includes the first plurality of spatial imaging biomarker data and the second plurality of spatial imaging biomarker data, and wherein each spatial imaging biomarker data of the full plurality of spatial imaging biomarker data includes a plurality of spatial imaging biomarkers generated via performing the spatial imaging biomarker generator function.

14. The medical data processing system of claim 13, wherein the plurality of spatial imaging biomarkers includes:
At least one subcutaneous fat-based biomarker;
At least one skeletal muscle-based biomarker;
At least one lesion-based biomarker;
At least one pleural effusion-based biomarker; and
At least one visceral fat-based biomarker.

15. The medical data processing system of claim 14, wherein performing the spatial imaging biomarker generator function upon each medical image data of a full plurality of medical image data that includes the first plurality of medical image data the second plurality of medical image data includes:
segmenting the each medical image data into a plurality of anatomical type-based segments, wherein the plurality of anatomical type-based segments includes: at least one subcutaneous fat segment; at least one skeletal muscle segment; at least one lesion segment; at least one pleural effusion segment; and at least one visceral fat segment;
processing each of the plurality of anatomical type-based segments to generate a corresponding spatial imaging biomarker of the plurality of spatial imaging biomarkers, wherein the at least one subcutaneous fat-based biomarker is generated via processing the at least one subcutaneous fat segment; wherein the at least one skeletal muscle-based biomarker is generated via processing the at least one skeletal muscle segment; wherein the at least one lesion-based biomarker is generated via processing the at least one lesion segment; wherein the at least one pleural effusion-based biomarker is generated via processing the at least one pleural effusion segment; and wherein the at least one visceral fat-based biomarker is generated via processing the at least one visceral fat segment.

16. The medical data processing system of claim 13, wherein the plurality of spatial imaging biomarkers includes over 5,000 different spatial imaging biomarkers.

17. The medical data processing system of claim 11, wherein the similarity function is performed upon: a first vector having a first ordered set of values for the corresponding clinical trial candidate spatial imaging biomarker data, and a second vector having a second ordered set of values for the corresponding clinical trial candidate spatial imaging biomarker data, wherein the first vector and the second vector have a same number of values in an ordering corresponding to a predetermined ordering of the plurality of spatial imaging biomarkers.

18. The medical data processing system of claim 17, wherein performing the similarity function includes performing a cosine similarity function upon the first vector and the second vector.

19. The medical data processing system of claim 11, wherein an external control arm of the clinical trial is implemented to include the proper subset of the second plurality of medical image data, and wherein a treatment arm of the clinical trial is implemented to include the first plurality of individuals.

20. A method comprising:
generating a first plurality of spatial imaging biomarker data for a first plurality of medical image data based on utilizing artificial intelligence to perform a spatial imaging biomarker generator function upon each of the first plurality of medical image data to generate corresponding first candidate spatial imaging biomarker data of the first plurality of spatial imaging biomarker data;
generating a second plurality of spatial imaging biomarker data for a second plurality of medical image data based on utilizing artificial intelligence to perform the spatial imaging biomarker generator function upon each of the second plurality of medical image data to generate corresponding second spatial imaging biomarker data of the second plurality of spatial imaging biomarker data;
generating, for each of the first plurality of medical image data, a corresponding plurality of similarity scores corresponding to the second plurality of medical image data based on generating each similarity score of the corresponding plurality of similarity scores via performance of a similarity function upon: the corresponding first spatial imaging biomarker data generated for the each of the first plurality of medical image data, and the corresponding second spatial imaging biomarker data generated for a corresponding one of the second plurality of medical image data; and
identifying a proper subset of the second plurality of medical image data based on identifying, for the each of the first plurality of medical image data, one of the second plurality of medical image data having a most favorable similarity score of the corresponding plurality of similarity scores.
